# EUROPÄISCHE PATENTSCHRIFT

(11) **EP 1 373 533 B1**
(45) Veröffentlichungstag und Bekanntmachung des Hinweises auf die Patenterteilung: **05.11.2008**
(21) Anmeldenummer: 02727367.1
(22) Anmeldetag: 07.03.2002
(51) Int. Cl.: C12N 15/82, C12N 15/54, C12N 9/10, A01H 5/00

(54) **ERHÖHUNG DES VITAMIN-E-GEHALTS IN ORGANISMEN DURCH ERHÖHUNG DER TYROSINAMINOTRANSFERASE-AKTIVITÄT**
INCREASE IN THE VITAMIN E CONTENT IN ORGANISMS DUE TO AN INCREASE IN THE TYROSINE AMINOTRANSFERASE ACTIVITY
AUGMENTATION DE LA TENEUR EN VITAMINE E DANS DES ORGANISMES PAR ACCROISSEMENT DE L'ACTIVITE DE LA TYROSINE AMINOTRANSFERASE

(30) Priorität: 09.03.2001 DE 10111676
(43) Veröffentlichungstag der Anmeldung: 02.01.2004
(73) Patentinhaber: Sungene GmbH & Co. KGaA, 06466 Gatersleben (DE)
(72) Erfinder: BADUR, Ralf, 67117 Limburgerhof (DE); GEIGER, Michael, 06484 Quedlinburg (DE); SALCHERT, Klaus-Dieter, 06507 Gernrode (DE); TROPF, Susanne, 06484 Quedlinburg (DE); LEMKE, Rainer, 06484 Quedlinburg (DE)
(74) Vertreter: Bieberbach, Andreas
(86) Internationale Anmeldenummer: PCT/EP2002/002492
(87) Internationale Veröffentlichungsnummer: WO 2002/072848

(56) Entgegenhaltungen:
- WO-A-00/08169
- WO-A-00/61771
- DIETRICH J B ET AL: "EXPRESSION OF MAMMALIAN TYROSINE AMINOTRANSFERASE IN SACCHAROMYCES-CEREVISIAE AND ESCHERICHIA-COLI PURIFICATION TO HOMOGENEITY AND CHARACTERIZATION OF THE ENZYME OVERPRODUCED IN THE BACTERIA" EUROPEAN JOURNAL OF BIOCHEMISTRY, Bd. 201, Nr. 2, 1991, Seiten 399-408, XP008016021 ISSN: 0014-2956
- RETTENMEIER R ET AL: "ISOLATION AND CHARACTERIZATION OF THE HUMAN TYROSINE AMINOTRANSFERASE GENE" NUCLEIC ACIDS RESEARCH, Bd. 18, Nr. 13, 1990, Seiten 3853-3862, XP001148886 ISSN: 0305-1048
- MONTEMARTINI MARISA ET AL: "A recombinant tyrosine aminotransferase from Trypanosoma cruzi has both tyrosine aminotransferase and alanine aminotransferase activities." FEMS MICROBIOLOGY LETTERS, Bd. 133, Nr. 1-2, 1995, Seiten 17-20, XP002237972 ISSN: 0378-1097
- SHINTANI D ET AL: "ELEVATING THE VITAMIN E CONTENT OF PLANTS THROUGH METABOLIC ENGINEERING" SCIENCE, AMERICAN ASSOCIATION FOR THE ADVANCEMENT OF SCIENCE,, US, Bd. 282, Nr. 282, 1998, Seiten 2098-2100, XP000887122 ISSN: 0036-8075 in der Anmeldung erwähnt
- FIEDLER E ET AL: "THE FORMATION OF HOMOGENTISATE IN THE BIOSYNTHESIS OF TOCOPHEROL AND PLASOQUINONE IN SPINACH CHLOROPLASTS" PLANTA, SPRINGER VERLAG, DE, Bd. 155, Nr. 6, 1982, Seiten 511-515, XP001058088 ISSN: 0032-0935
- SCHULTZ G: "BIOSYNTHESIS OF ALPHA-TOCOPHEROL IN CHLOROPLASTS OF HIGHER PLANTS" FETT WISSENSCHAFT TECHNOLOGIE- FAT SCIENCE TECHNOLOGY, CONRADIN INDUSTRIEVERLAG. LEINFELDEN ECHTERDINGEN, DE, Bd. 92, Nr. 2, 1990, Seiten 86-91, XP001058085 ISSN: 0931-5985
- ANNA LOPOUKHINA: "Characterization of coronatine-regulated genes from Arabidopsis thaliana" 1999, Ruhr-Universität Bochum

## Beschreibung

Die vorliegende Erfindung betrifft ein Verfahren zur Herstellung von Vitamin E durch Kultivierung von Pflanzen die gegenüber dem Wildtyp eine erhöhte Tyrosinaminotransferase- Aktivität aufweisen, sowie die genetisch veränderten Organismen, insbesondere Pflanzen selbst.

Die in der Natur vorkommenden acht Verbindungen mit Vitamin E-Aktivität sind Derivate des 6-Chromanols (Ullmann's Encyclopedia of Industrial Chemistry, Vol. A 27 (1996), VCH Verlagsgesellschaft, Chapter 4., 478-488, Vitamin E). Die Gruppe der Tocopherole (1a-d) weist eine gesättigte Seitenkette auf, die Gruppe der Tocotrienole (2a-d) eine ungesättigte Seitenkette: 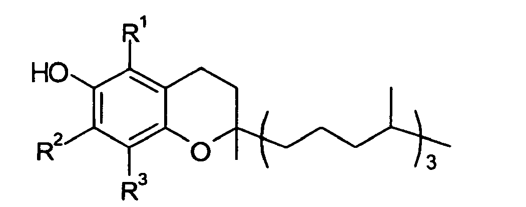 1a, α-Tocopherol: R¹ = R² = R³ = CH₃
1b, β-Tocopherol: R¹ = R³ = CH₃, R² = H
1c, γ-Tocopherol: R¹ = H, R² = R³ = CH₃
1d, δ-Tocopherol: R¹ = R² = H, R³ = CH₃ 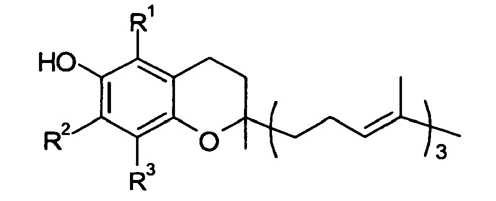 2a, α-Tocotrienol: R¹ = R² = R³ = CH₃
2b, β-Tocotrienol: R¹ = R³ = CH₃, R² = H
2c, γ-Tocotrienol: R¹ = H, R² = R³ = CH₃
2d, δ-Tocotrienol: R¹ = R² = H, R³ = CH₃

In der vorliegenden Erfindung werden unter Vitamin E alle vorstehend erwähnten Tocopherole und Tocotrienole mit Vitamin-E-Aktivität verstanden.

Diese Verbindungen mit Vitamin-E-Aktivität sind wichtige natürliche fett-löslicheAntioxidantien. Ein Mangel an Vitamin E führt bei Menschen und Tierenzu pathophysiologischen Situationen.Vitamin E-Verbindungen haben daher einen hohen wirtschaftlichen Wert als Zusatzstoffe im Food- und Feed-Bereich, in pharmazeutischen Formulierungen und in kosmetischen Anwendungen.

Ein wirtschaftliches Verfahren zur Herstellung von Vitamin E-Verbindungen sowie Nahrungs- und Futtermittel mit erhöhtem Vitamin E-Gehalt sind daher von großer Bedeutung.

Besonders wirtschaftliche Verfahren sind biotechnologische Verfahren unter Ausnutzung natürlicher oder durch genentische Veränderung optimierter Vitamin-E-produzierender Organismen.

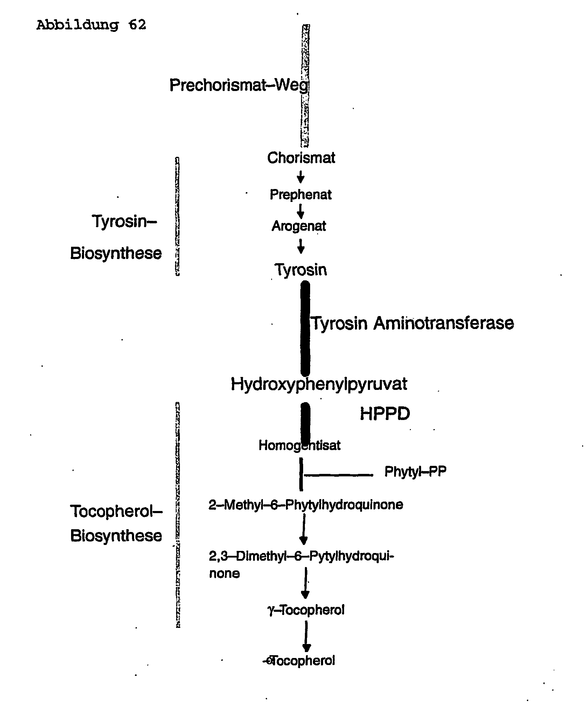

Abbildung 62 zeigt ein Biosyntheseschema von α-Tocopherol in höheren Pflanzen.

In höheren Pflanzen wird Tyrosin ausgehend von Chorismat über Prephenat und Arogenat gebildet. Die aromatische Aminosäure Tyrosin wird durch das Enzym Tyrosinaminotransferase in Hydroxyphenyl-Pyruvat umgewandelt, welches durch Dioxygenierung in Homogentisinsäure überführt wird.

Die Homogentisinsäure wird anschließend an Phytylpyrophosphat (PPP) bzw. Geranylgeranylpyrophosphat gebunden, um die Vorläufer von α-Tocopherol und α-Tocotrienol, das 2-Methyl-6-phytylhydrochinol bzw. das 2-Methyl-6-geranylgeranylhydrochinol zu bilden. Durch Methylierungsschritte mit S-Adenosylmethionin als Methyl-Gruppen-Donor entsteht zunächst 2,3-Dimethyl-6-phytylquinol, dann durch Zyklisierung γ-Tocopherol und durch nochmalige Methylierung α-Tocopherol.

Es sind Versuche bekannt, in transgenen Organismen durch Überexpression einzelner Biosynthesegene eine Erhöhung des Metabolitflusses zur Steigerung des Tocopherol- bzw. Tocotrienolgehaltes zu erreichen.

WO 97/27285 beschreibt eine Modifikation des Tocopherol-Gehaltes durch verstärkte Expression bzw. durch Herunterregulation des Enzyms p-Hydroxyphenylpyruvatdioxygenase (HPPD).

WO 99/04622 bzw. D. DellaPenna et al., Science 1998, 282, 2098-2100 beschreiben Gensequenzen codierend für eine γ-Tocopherolmethyltransferase aus Synechocystis PCC6803 und Arabidopsis thaliana und deren Einbau in transgene Pflanzen, die einen modifizierten Vitamin E-Gehalt aufweisen.

WO 99/23231 zeigt, daß die Expression einer Geranylgeranyl-Reductase in transgenen Pflanzen eine gesteigerte Tocopherolbiosynthese zur Folge hat.

WO 00/08169 beschreibt Gensequenzen codierend eine 1-Deoxy-D-Xylose-5-Phosphat-Synthase und eine Geranyl-Geranyl-Pyrophosphat Oxidoreduktase und deren Einbau in transgene Pflanzen, die einen modifizierten Vitamin E-Gehalt aufweisen.

WO 00/68393 und WO 00/63391 beschreiben Gensequenzen codierend eine Phytyl/Prenyl-Transferase und deren Einbau in transgene Pflanzen, die einen modifizierten Vitamin E-Gehalt aufweisen.

In WO 00/61771 wird postuliert, daß die Kombination eines Gens aus dem Sterol-Stoffwechsel in Kombination mit einem Gen aus dem Tocopherolstoffwechsel zu einer Erhöhung des Tocopherolgehalts in transgenen Pflanzen führen kann.

In einer von A. Lopoukhina verfassten Doktorarbeit (*Characterization of coronatine regulated genes from Arabidopsis thaliana*, Doktorarbeit an der Ruhr-Universität Bochum, Lehrstuhl für Pflanzenphysiologie, 1999) und in einem Posterbeitrag von H. Holländer-Czytko et al. auf der Botanikertagung 2000 in Jena vom 17-22.9.2000 werden durch das Phytotoxin Coronatin induzierbare Gene aus Arabidopsis thaliana offenbart. Bei einem dieser Gene weist die abgeleitete Aminosäuresequenz eine Homologie von ca. 35 % mit bekannten Tyrosinaminotransferasen auf. Durch heterologe Expression des putativen Tyrosinaminotransferase-Gens in E.coli konnte eine geringe Enzymaktivität einer Tyrosinaminotransferase nachgewiesen werden. Es wird offenbart, daß die Behandlung von Pflanzen mit Coronatin und die Verwundung von Pflanzen zu einer Akkumulation der putativen Tyrosinaminotransferase-spezifischen mRNA, der putativen Tyrosinaminotransferase und der meßbaren Enzymaktivität führt. Ferner wird auf Seite 72 f. der Doktorarbeit offenbart, daß es bekannt sei, daß die Verwundung von Pflanzen zu einer Bildung von reaktiven Sauerstoffspezies führt, die durch antioxidative Verbindungen wie Tocopherol, Carotinoide oder Rosmarinsäure abgefangen werden.

Alle diese Methoden, bis auf den zuletzt erwähnten Stand der Technik, liefern zwar genetisch veränderte Organismen, insbesondere Pflanzen, die in der Regel einen modifizierten Gehalt an Vitamin E aufweisen, weisen jedoch den Nachteil auf, daß die Höhe des Gehalts an Vitamin E in den im Stand der Technik bekannten genetisch veränderten Organismen noch nicht zufriedenstellend ist.

Der Erfindung lag daher die Aufgabe zugrunde ein weiteres Verfahren zur Herstellung von Vitamin E durch Kultivierung von Organismen zur Verfügung zu stellen, bzw. weitere transgene Organismen, die Vitamin E herstellen, zur Verfügung zu stellen, die optimierte Eigenschaften, wie beispielsweise einen höheren Gehalt an Vitamin E aufweisen und den geschilderten Nachteil des Standes der Technik nicht aufweisen.

Demgemäß wurde ein Verfahren zur Herstellung von Vitamin E gefunden, indem man Pflanzen kultiviert, die gegenüber dem Wildtyp eine erhöhte Tyrosinaminotransferase-Aktivität aufweisen.

Unter Tyrosinaminotransferase-Aktivität wird die Enzymaktivität einer Tyrosinaminotransferase verstanden.

Unter einer Tyrosinaminotransferase wird ein Protein verstanden, das die enzymatische Aktivität aufweist, Tyrosin in 4-Hydroxyphenylpyruvat umzuwandeln.

Dementsprechend wird unter Tyrosinaminotransferase-Aktivität die in einer bestimmten Zeit durch das Protein Tyrosinaminotransferase umgesetzte Menge Tyrosin bzw. gebildete Menge 4-Hydroxyphenylpyruvat verstanden.

Bei einer erhöhten Tyrosinaminotransferase-Aktivität gegenüber dem Wildtyp wird somit im Vergleich zum Wildtyp in einer bestimmten Zeit durch das Protein Tyrosinaminotransferase die umgesetzte Menge Tyrosin bzw. die gebildete Menge 4-Hydroxyphenylpyruvat erhöht.

Vorzugsweise beträgt diese Erhöhung der Tyrosinaminotransferase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der Tyrosinaminotransferase-Aktivität des Wildtyps.

Unter einem Wildtyp wird der entsprechende nicht genetisch veränderte Ausgangsorganismus verstanden. Vorzugsweise und insbesondere in Fällen in denen der Organismus oder der Wildtyp nicht eindeutig zuordenbar ist, wird unter Wildtyp für die Erhöhung der Tyrosinaminotransferase-Aktivität, die Erhöhung der nachstehend beschriebenen Hydroxyphenylpyruvat-Dioxygenase-Aktivität, die Erhöhung der nachstehend beschriebenen Homogentisat-Phytyltransferase-Aktivität, die Erhöhung der nachstehend beschriebenen Geranyl-Geranyl-Pyrophosphat-Oxidoreduktase-Aktivität, die Erhöhung der nachstehend beschriebenen 2-Methyl-6-Phytylhydrochinon-Methyltransferase-Aktivität, die Erhöhung der nachstehend beschriebenen Tocopherolcyclase-Aktivität, die Erhöhung der nachstehend beschriebenen γ-Tocopherol-Methyltransferase-Aktivität, die Reduzierung der nachstehend beschriebenen Homogentisat-Dioxygenase-Aktivität, die Reduzierung der nachstehend beschriebenen Maleylacetoacetat-Isomerase-Aktivität und die Reduzierung der nachstehend beschriebenen Fumarylacetoacetat-Hydrolase-Aktivität, sowie für die Erhöhung des Gehalts an Vitamin E ein Referenzorganismus verstanden. Dieser Referenzorganismus ist vorzugsweise *Brassica napus cv Westar.*

Die Erhöhung der Tyrosinaminotransferase-Aktivität kann durch verschiedene Wege erfolgen, beispielsweise durch Ausschalten von hemmenden Regulationsmechanismen auf Translations- und Proteinebene oder durch Erhöhung der Genexpression einer Nukleinsäure codierend eine Tyrosinaminotransferase gegenüber dem Wildtyp, beispielsweise durch Induzierung des Tyrosinaminotransferase-Gens durch Phytotoxine wie beispielsweise Coronatin oder durch Einbringen von Nukleinsäuren codierend eine Tyrosinaminotransferase.

Unter Erhöhung der Genexpression einer Nukleinsäure codierend eine Tyrosinaminotransferase wird erfindungsgemäß auch die Manipulation der Expression der Pflanzen eigenen endogenen Tyrosinaminotransferasen verstanden. Dies kann beispielsweise durch Veränderung der Promotor DNA-Sequenz für Tyrosinaminotransferasen kodierende Gene erreicht werden. Eine solche Veränderung, die eine veränderte oder vorzugsweise erhöhte Expressionsrate mindestens eines endogenen Tyrosinaminotransferase Gens zur Folge hat, kann durch Deletion oder Insertion von DNA Sequenzen erfolgen.

Es ist, wie vorstehend beschrieben, möglich, die Expression mindestens einer endogenen Tyrosinamintransferase durch die Applikation exogener Stimuli zu verändern. Dies kann durch besondere physiologische Bedingungen, also durch die Applikation von Fremdsubstanzen erfolgen.

Des weiteren kann eine veränderte bzw. erhöhte Expression mindestens eines endogenen Tyrosinaminotransferase Gens dadurch erzielt werden, dass ein im nicht transformierten Organismus nicht vorkommendes Regulatorprotein mit dem Promotor dieser Gene in Wechselwirkung tritt.

Solch ein Regulator kann ein chimäres Protein darstellen, welches aus einer DNA-Bindedomäne und einer Transkriptionsaktivator-Domäne besteht, wie beispielsweise in WO 96/06166 beschrieben.

In einer bevorzugten Ausführungsform erfolgt die Erhöhung der Tyrosinaminotransferase-Aktivität gegenüber dem Wildtyp durch eine Erhöhung der Genexpression einer Nukleinsäure codierend eine Tyrosinaminotransferase.

In einer weiter bevorzugten Ausführungsform erfolgt die Erhöhung der Genexpression einer Nukleinsäure codierend eine Tyrosinaminotransferase durch Einbringen von Nukleinsäuren codierend eine Tyrosinaminotransferase in die Pflanze.

Dazu kann prinzipiell jedes Tyrosinaminotransferase-Gen, also jede Nukleinsäuren die eine Tyrosinaminotransferase codiert verwendet werden. Bei genomischen Tyrosinaminotransferase-Nukleinsäure-Sequenzen aus eukaryontischen Quellen, die Introns enthalten, sind für den Fall daß der Wirtsorganismus nicht in der Lage ist oder nicht in die Lage versetzt werden kann, die entsprechenden Tyrosinaminotransferase zu exprimieren, bevorzugt bereits prozessierte Nukleinsäuresequenzen, wie die entsprechenden cDNAs zu verwenden.

Alle in der Beschreibung erwähnten Nukleinsäuren können beispielsweise eine RNA-, DNA- oder cDNA-Sequenz sein.

Beispiele für Nukleinsäuren codierend eine Tyrosinaminotransferase bzw. Beispiele für Tyrosinaminontransferasen sind
die sechs putativen Tyrosinaminotransferasen TAT I bis TAT VI aus *Arabidopsis thaliana* TATI: CAA23026 (Nukleinsäure: SEQ. ID. NO. 5, Protein: SEQ. ID. NO. 6), TAT II: CAA23025, TAT III: AAD23027 (Nukleinsäure: SEQ. ID. NO. 7, Protein: SEQ. ID. NO. 8) , TAT IV: CAA16881, TAT V: AAD21706 (Nukleinsäure: SEQ. ID. NO. 9, Protein: SEQ. ID. NO. 10), TAT VI: (Nukleinsäure: SEQ. ID. NO. 11, Protein: SEQ. ID. NO. 12)
die Tyrosinaminotransferase aus *Rattus norvegicus* (Nukleinsäure: SEQ. ID. NO. 1, Protein: SEQ. ID. NO. 2),
eine Variante der Tyrosinaminotransferase aus Rattus *norvegicus* (Nukleinsäure: SEQ. ID. NO. 3, Protein: SEQ. ID. NO. 4),
die humane Tyrosinaminotransferase (Accesion No. XP_008081),
die Tyrosinaminotransferase aus *Tryposoma rangeli* (Accesion No. AF165323_1),
die Tyrosinaminotransferase aus *Tryposoma cruzi* (Accesion No. AI 622965) oder
die Tyrosinaminotransferase aus *Rhizobium meliloti* (Accesion No. L05065).

Bevorzugt verwendet man Nukleinsäuren, die Proteine kodieren, enthaltend die Aminosäuresequenz SEQ. ID. NO. 2 oder eine von dieser Sequenz durch Substitution, Insertion oder Deletion von Aminosäuren abgeleitete Sequenz, die eine Identität von mindestens 20 %, vorzugsweise mindestens 33 %, bevorzugter mindestens 35 %,bevorzugter mindestens 50%, noch bevorzugter mindestens 70 %, am bevorzugtesten mindestens 90 % auf Aminosäureebene mit der Sequenz SEQ. ID. NO. 2, und die die enzymatische Eigenschaft einer Tyrosinaminotransferase aufweisen.

Die Sequenz SEQ. ID. NO. 2 stellt die Aminosäuresequenz der Tyrosinaminotransferase aus *Rattus norvegicus* dar.

Unter dem Begriff "Substitution" ist in der Beschreibung der Austausch einer oder mehrerer Aminosäuren durch eine oder mehrere Aminosäuren zu verstehen. Bevorzugt werden sog. konservative Austausche durchgeführt, bei denen die ersetzte Aminosäure eine ähnliche Eigenschaft hat wie die ursprüngliche Aminosäure, beispielsweise Austausch von Glu durch Asp, Gln durch Asn, Val durch Ile, Leu durch Ile, Ser durch Thr.

Deletion ist das Ersetzen einer Aminosäure durch eine direkte Bindung. Bevorzugte Positionen für Deletionen sind die Termini des Polypeptides und die Verknüpfungen zwischen den einzelnen Proteindomänen.

Insertionen sind Einfügungen von Aminosäuren in die Polypeptidkette, wobei formal eine direkte Bindung durch ein oder mehrere Aminosäuren ersetzt wird.

Unter Identität zwischen zwei Proteinen wird die Identität der Aminosäuren über die jeweils gesamte Proteinlänge verstanden, insbesondere die Identität die durch Vergleich mit Hilfe der Lasergene Software der Firma DNASTAR, inc.Madison, Wisconsin (USA) unter Anwendung der Clustal Methode (Higgins DG, Sharp PM. Fast and sensitive multiple sequence alignments on a microcomputer. Comput Appl. Biosci. 1989 Apr;5(2) : 151-1) unter Einstellung folgender Parameter berechnet wird:

Multiple alignment parameter:

| | |
|---|---|
| Gap penalty | 10 |
| Gap length penalty | 10 |
| Pairwise alignment parameter: | |
| K-tuple | 1 |
| Gap penalty | 3 |
| Window | 5 |
| Diagonals saved | 5 |

Unter einem Protein, das eine Identität von mindestens 20 % auf Aminosäureebene mit der Sequenz SEQ. ID. NO. 2 aufweist, wird dementsprechend ein Protein verstanden, das bei einem Vergleich seiner Sequenz mit der Sequenz SEQ. ID. NO. 2, insbesondere nach obigen Programmalgorithmus mit obigem Parametersatz eine Identität von mindestens 20 % aufweist.

Die bekannten Tyrosinaminotransferasen weisen mit der SEQ. ID. NO. 2 (Tyrosinaminotransferasen aus *Rattus norvegicus*) nach obigem Programmalgorithmus mit obigem Parametersatz folgende Identität [%] der Aminosäuresequenzen auf:

| | |
|---|---|
| CAA23026 (TAT I) | 26,8 % |
| CAA23025 (TAT II) | 22,3 % |
| AAD23027 (TAT III) | 28,3 % |
| CAA16881 (TAT IV) | 29,8 % |
| AAD21706 (TAT V) | 30,0 % |
| TAT VI K19.P17.14 | 33.3 % |
| AF165323_1 (*Tryposoma rangeli)* | *33,3 %* |
| XP_008081 (human) | 91,6 % |

In einer weiter bevorzugten Ausführungsform werden Nukleinsäuren in Pflanzen eingebracht, die Proteine kodieren, enthaltend die Aminosäuresequenz der Tyrosinaminotransferase aus Rattus *norvegicus* SEQ. ID. NO. 2 oder die Aminosäuresequenz der humanen Tyrosinaminotransferase (Accesion No. XP_008081).

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der organismusspezifischen codon usage häufig verwendet werden. Die codon usage läßt sich anhand von Computerauswertungen anderer, bekannter Gene der betreffenden Organismen leicht ermitteln.

Soll das Protein beispielsweise in einer Pflanze exprimiert werden, so ist es häufig vorteilhaft, die codon usage der Pflanze bei der Rückübersetzung zu verwenden.

In einer besonders bevorzugten Ausführungsform bringt man eine Nukleinsäure, enthaltend die Sequenz SEQ. ID. NO. 1 in die Pflanze ein.

Die Sequenz SEQ. ID. NO. 1 stellt die cDNA der Tyrosinaminotransferase aus *Rattus norvegicus* (Accesion No. NM_012668) dar.

In einer bevorzugten Ausführungsform werden Pflanzen kultiviert, die gegenüber dem Wildtyp zusätzlich eine erhöhte Aktivität mindestens einer der Aktivitäten, ausgewählt aus der Gruppe Hydroxyphenylpyruvat-Dioxygenase-Aktivität, Homogentisat-Phytyltransferase-Aktivität, Geranyl-Geranyl-Pyrophosphat-Oxidoreduktase-Aktivität, 2-Methyl-6-Phytylhydrochinon-Methyltransferase-Aktivität, Tocopherolcyclase-Aktivität und γ-Tocopherol-Methyltransferase-Aktivität aufweisen.

Unter Hydroxyphenylpyruvat-Dioxygenase-Aktivität wird die Enzymaktivität einer Hydroxyphenylpyruvat-Dioxygenase verstanden.

Unter einer Hydroxyphenylpyruvat-Dioxygenase wird ein Protein verstanden, das die enzymatische Aktivität aufweist, Hydroxyphenylpyruvat in Homogentisat umzuwandeln.

Dementsprechend wird unter Hydroxyphenylpyruvat-Dioxygenase-Aktivität die in einer bestimmten Zeit durch das Protein Hydroxyphenylpyruvat-Dioxygenase umgesetzte Menge Hydroxyphenylpyruvat bzw. gebildete Menge Homogentisat verstanden.

Bei einer erhöhten Hydroxyphenylpyruvat-Dioxygenase-Aktivität gegenüber dem Wildtyp wird somit im Vergleich zum Wildtyp in einer bestimmten Zeit durch das Protein Hydroxyphenylpyruvat-Dioxygenase die umgesetzte Menge Hydroxyphenylpyruvat bzw. die gebildete Menge Homogentisat erhöht.

Vorzugsweise beträgt diese Erhöhung der Hydroxyphenylpyruvat-Dioxygenase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der Hydroxyphenylpyruvat-Dioxygenase-Aktivität des Wildtyps.

Unter Homogentisat-Phytyltransferase-Aktivität wird die Enzymaktivität einer Homogentisat-Phytyltransferase verstanden. Unter einer Homogentisat-Phytyltransferase wird ein Protein verstanden, das die enzymatische Aktivität aufweist, Homogentisat und Phytylpyrophosphat in 2-Methyl-6-Phytylhydrochinol umzuwandeln.

Dementsprechend wird unter Homogentisat-Phytyltransferase-Aktivität die in einer bestimmten Zeit durch das Protein Homogentisat-Phytyltransferase umgesetzte Menge Homogentisat oder Phytylpyrophosphat bzw. gebildete Menge 2-Methyl-6-Phytylhydrochinol verstanden.

Bei einer erhöhten Homogentisat-Phytyltransferase-Aktivität gegenüber dem Wildtyp wird somit im Vergleich zum Wildtyp in einer bestimmten Zeit durch das Protein Homogentisat-Phytyltransferase die umgesetzte Menge Homogentisat oder Phytylpyrophosphat bzw. die gebildete Menge 2-Methyl-6-Phytylhydrochinol erhöht.

Vorzugsweise beträgt diese Erhöhung der Homogentisat-Phytyltransferase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der Homogentisat-Phytyltransferase-Aktivität des Wildtyps.

Unter Geranyl-Geranyl-Pyrophosphat-Oxidoreduktase-Aktivität wird die Enzymaktivität einer Geranyl-Geranyl-Pyrophosphat-Oxidoreduktase verstanden.

Unter einer Geranyl-Geranyl-Pyrophosphat-Oxidoreduktase wird ein Protein verstanden, das die enzymatische Aktivität aufweist, Geranyl-Gerany-Pyrophosphat in Phytylpyrophosphat umzuwandeln.

Dementsprechend wird unter Geranyl-Geranyl-Pyrophosphat-Oxidoreduktase-Aktivität die in einer bestimmten Zeit durch das Protein Geranyl-Geranyl-Pyrophosphat-Oxidoreduktase umgesetzte Menge Geranyl-Gerany-Pyrophosphat bzw. gebildete Menge Phytylpyrophosphat verstanden.

Bei einer erhöhten Geranyl-Geranyl-Pyrophosphat-Oxidoreduktase-Aktivität gegenüber dem Wildtyp wird somit im Vergleich zum Wildtyp in einer bestimmten Zeit durch das Protein Geranyl-Geranyl-Pyrophosphat-Oxidoreduktase die umgesetzte Menge Geranyl-Gerany-Pyrophosphat bzw. die gebildete Menge Phytylpyrophosphat erhöht.

Vorzugsweise beträgt diese Erhöhung der Geranyl-Geranyl-Pyrophosphat-Oxidoreduktase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der Geranyl-Geranyl-Pyrophosphat-Oxidoreduktase-Aktivität des Wildtyps.

Unter 2-Methyl-6-Phytylhydrochinon-Methyltransferase-Aktivität wird die Enzymaktivität einer 2-Methyl-6-Phytylhydrochinon-Methyltransferase verstanden.

Unter einer 2-Methyl-6-Phytylhydrochinon-Methyltransferase wird ein Protein verstanden, das die enzymatische Aktivität aufweist, 2-Methyl-6-Phytylhydrochinol in 2,3-Dimethyl-6-Phytylhydrochinol umzuwandeln.

Dementsprechend wird unter 2-Methyl-6-Phytylhydrochinon-Methyltransferase-Aktivität die in einer bestimmten Zeit durch das Protein 2-Methyl-6-Phytylhydrochinon-Methyltransferase umgesetzte Menge 2-Methyl-6-Phytylhydrochinol bzw. gebildete Menge 2,3-Dimethyl-6-Phytylhydrochinol verstanden.

Bei einer erhöhten 2-Methyl-6-Phytylhydrochinon-Methyltransferase-Aktivität gegenüber dem Wildtyp wird somit im Vergleich zum Wildtyp in einer bestimmten Zeit durch das Protein 2-Methyl-6-Phytylhydrochinon-Methyltransferase die umgesetzte Menge 2-Methyl-6-Phytylhydrochinol bzw. die gebildete Menge 2,3-Dimethyl-6-Phytylhydrochinol erhöht.

Vorzugsweise beträgt diese Erhöhung der 2-Methyl-6-Phytylhydrochinon-Methyltransferase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der 2-Methyl-6-Phytylhydrochinon-Methyltransferase-Aktivität des Wildtyps.

Unter Tocopherolcyclase-Aktivität wird die Enzymaktivität einer Tocopherolcyclase verstanden.

Unter einer Tocopherolcyclase wird ein Protein verstanden, das die enzymatische Aktivität aufweist, 2,3-Dimethyl-6-Phytylhydrochinol in γ-Tocopherol umzuwandeln.

Dementsprechend wird unter Tocopherolcyclase-Aktivität die in einer bestimmten Zeit durch das Protein Tocopherolcyclase umgesetzte Menge 2,3-Dimethyl-6-Phytylhydrochinol bzw. gebildete Menge γ-Tocopherol verstanden.

Bei einer erhöhten Tocopherolcyclase-Aktivität gegenüber dem Wildtyp wird somit im Vergleich zum Wildtyp in einer bestimmten Zeit durch das Protein Tocopherolcyclase die umgesetzte Menge 2,3-Dimethyl-6-Phytylhydrochinol bzw. die gebildete Menge γ-Tocopherol erhöht.

Vorzugsweise beträgt diese Erhöhung der Tocopherolcyclase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der Tocopherolcyclase-Aktivität des Wildtyps.

Unter γ-Tocopherol-Methyltransferase-Aktivität wird die Enzymaktivität einer γ-Tocopherol-Methyltransferase verstanden.

Unter einer γ-Tocopherol-Methyl transferase wird ein Protein verstanden, das die enzymatische Aktivität aufweist, γ-Tocopherol in α-Tocopherol umzuwandeln.

Dementsprechend wird unter γ-Tocopherol-Methyltransferase-Aktivität die in einer bestimmten Zeit durch das Protein γ-Tocopherol-Methyltransferase umgesetzte Menge γ-Tocopherol bzw. gebildete Menge α-Tocopherol verstanden.

Bei einer erhöhten γ-Tocopherol-Methyltransferase-Aktivität gegenüber dem Wildtyp wird somit im Vergleich zum Wildtyp in einer bestimmten Zeit durch das Protein γ-Tocopherol-Methyltransferase die umgesetzte Menge γ-Tocopherol bzw. die gebildete Menge α-Tocopherol erhöht.

Vorzugsweise beträgt diese Erhöhung der γ-Tocopherol-Methyltransferase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der γ-Tocopherol-Methyltransferase-Aktivität des Wildtyps.

Die Erhöhung mindestens einer der Aktivitäten, ausgewählt aus der Gruppe Hydroxyphenylpyruvat-Dioxygenase-Aktivität, Homogentisat-Phytyltransferase-Aktivität, Geranyl-Geranyl-Pyrophosphat-Oxidoreduktase-Aktivität, 2-Methyl-6-Phytylhydrochinon-Methyltransferase-Aktivität, Tocopherolcyclase-Aktivität und γ-Tocopherol-Methyltransferase-Aktivität kann unabhängig voneinander durch verschiedene Wege erfolgen, beispielsweise durch Ausschalten von hemmenden Regulationsmechanismen auf Expressions- und Protein-ebene oder durch Erhöhung der Genexpression der entsprechenden Nukleinsäuren, also der Erhöhung der Genexpression mindestens einer Nukleinsäure ausgewählt aus der Gruppe, Nukleinsäuren kodierend eine Hydroxyphenylpyruvat-Dioxygenase, Nukleinsäuren kodierend eine Homogentisat-Phytyltransferase, Nukleinsäuren kodierend eine Geranyl-Geranyl-Pyrophosphat-Oxidoreduktase, Nukleinsäuren kodierend eine 2-Methyl-6-Phytylhydrochinon-Methyltransferase, Nukleinsäuren kodierend eine Tocopherolcyclase und Nukleinsäuren kodierend eine γ-Tocopherol-Methyltransferase gegenüber dem Wildtyp.

Die Erhöhung der Genexpression der entsprechenden Nukleinsäure gegenüber dem Wildtyp kann ebenfalls durch verschiedene Wege erfolgen, beispielsweise durch Induzierung der entsprechenden Gene durch Aktivatoren, also durch Induzierung des Hydroxyphenylpyruvat-Dioxygenase-Gens, Homogentisat-Phytyltransferase-Gens, Geranyl-Geranyl-Pyrophosphat-Oxidoreduktase-Gens, 2-Methyl-6-Phytylhydrochinon-Methyltransferase-Gens, Tocopherolcyclase-Gens oder γ-Tocopherol-Methyltransferase-Gens durch Aktivatoren oder durch Einbringen von einer oder mehrerer Genkopien der entsprechenden Nukleinsäuren, also durch Einbringen mindestens einer der Nukleinsäuren, ausgewählt aus der Gruppe, Nukleinsäuren kodierend eine Hydroxyphenylpyruvat-Dioxygenase, Nukleinsäuren kodierend eine Homogentisat-Phytyltransferase, Nukleinsäuren kodierend eine Geranyl-Geranyl-Pyrophosphat-Oxidoreduktase, Nukleinsäuren kodierend eine 2-Methyl-6-Phytylhydrochinon-Methyltransferase, Nukleinsäuren kodierend eine Tocopherolcyclase und Nukleinsäuren kodierend eine γ-Tocopherol-Methyltransferase in die Pflanze.

Unter Erhöhung der Genexpression einer Nukleinsäure codierend eine Hydroxyphenylpyruvat-Dioxygenase, Homogentisat-Phytyltransferase, Geranyl-Geranyl-Pyrophosphat-Oxidoreduktase, 2-Methyl-6-Phytylhydrochinon-Methyltransferase, Tocopherolcyclase oder γ-Tocopherol-Methyltransferase wird erfindungsgemäß auch die Manipulation der Expression der Pflanzen eigenen, endogenen Hydroxyphenylpyruvat- Dioxygenasen, Homogentisat-Phytyltransferasen, Geranyl-Geranyl- Pyrophosphat- Oxidoreduktasen, 2-Methyl-6-Phytylhydrochinon- Methyltransferasen, Tocopherolcyclasen oder γ-Tocopherol-Methyltransferasen verstanden.

Dies kann beispielsweise durch Veränderung der Promotor DNA-Sequenz für Hydroxyphenylpyruvat-Dioxygenase, Homogentisat-Phytyltransferase, Geranyl-Geranyl-Pyrophosphat-Oxidoreduktase, 2-Methyl-6-Phytylhydrochinon-Methyltransferase, Tocopherolcyclase oder γ-Tocopherol-Methyltransferase kodierende Gene erreicht werden. Eine solche Veränderung, die eine erhöhte Expressionsrate des entsprechenden Gens zur Folge hat, kann beispielsweise durch Deletion oder Insertion von DNA Sequenzen erfolgen.

Es ist, wie vorstehend beschrieben, möglich, die Expression der endogenen Hydroxyphenylpyruvat-Dioxygenase, Homogentisat-Phytyltransferase, Geranyl-Geranyl-Pyrophosphat-Oxidoreduktase, 2-Methyl-6-Phytylhydrochinon-Methyltransferase, Tocopherolcyclase oder γ-Tocopherol-Methyltransferase durch die Applikation exogener Stimuli zu verändern. Dies kann durch besondere physiologische Bedingungen, also durch die Applikation von Fremdsubstanzen erfolgen.

Desweiteren kann eine veränderte bzw. erhöhte Expression endogener Hydroxyphenylpyruvat-Dioxygenase-, Homogentisat-Phytyltransferase-, Geranyl-Geranyl-Pyrophosphat-Oxidoreduktase-, 2-Methyl-6-Phytylhydrochinon-Methyltransferase-, Tocopherolcyclase- oder γ-Tocopherol-Methyltransferase-Gene dadurch erzielt werden, dass ein in der nicht transformierten Pflanze nicht vorkommendes Regulator-Protein mit dem Promotor dieser Gene in Wechselwirkung tritt.

Solch ein Regulator kann ein chimäres Protein darstellen, welches aus einer DNA-Bindedomäne und einer Transkriptionsaktivator-Domäne besteht, wie beispielsweise in WO 96/06166 beschrieben.

In einer bevorzugten Ausführungsform erfolgt die Erhöhung der Genexpression einer Nukleinsäure codierend eine Hydroxyphenylpyruvat-Dioxygenase, im folgenden auch HPPD genannt, durch Einbringen von mindestens einer Nukleinsäuren codierend eine HPPD in die Pflanze.

Dazu kann prinzipiell jedes HPPD-Gen, also jede Nukleinsäure, die eine HPPD codiert, verwendet werden.

Bei genomischen HPPD-Nukleinsäure-Sequenzen aus eukaryontischen Quellen, dieIntronsenthalten, sind für den Fall daß der Wirtsorganismus nicht in der Lage ist oder nicht in die Lage versetzt werden kann, die entsprechende HPPD zu exprimieren, bevorzugt bereits prozessierte Nukleinsäuresequenzen, wie die entsprechenden cDNAs zu verwenden.

Beispiele für HPPD-Gene sind Nukleinsäuren, codierend eine HPPD aus *Arabidopsis thaliana* (Nukleinsäure: Seq. ID. No. 13, Protein: Seq. ID. No. 14) oder eine HPPD aus Gerste (WO 99/04021).

In den erfindungsgemäßen transgenen Pflanzen liegt also in dieser bevorzugten Ausführungsform gegenüber dem Wildtyp mindestens ein weiteres HPPD-Gen vor. In dieser bevorzugten Ausführungsform weist die Pflanze dementsprechend mindestens eine exogene Nukleinsäure, codierend eine HPPD oder mindestens zwei endogene Nukleinsäuren, codierend eine HPPD auf.

Bevorzugt verwendet man in vorstehend beschriebener bevorzugter Ausführungsform Nukleinsäuren, die Proteine kodieren, enthaltend die Aminosäuresequenz SEQ. ID. NO. 14 oder eine von dieser Sequenz durch Substitution, Insertion oder Deletion von Aminosäuren abgeleitete Sequenz, die eine Identität von mindestens 30 %, vorzugsweise mindestens 50%, bevorzugter mindestens 70%, noch bevorzugter mindestens 90%, am bevorzugtesten mindestens 95% auf Aminosäureebene mit der Sequenz SEQ. ID. NO. 14, und die die enzymatische Eigenschaft einer HPPD aufweisen.

Die Sequenz SEQ. ID. NO. 14 stellt die Aminosäuresequenz der HPPD aus *Arabisopsis thaliana* dar.

Unter einem Protein, das eine Identität von mindestens 30 % auf Aminosäureebene mit der Sequenz SEQ. ID. NO. 14 aufweist, wird dementsprechend ein Protein verstanden, das bei einem Vergleich seiner Sequenz mit der Sequenz SEQ. ID. NO. 14, insbesondere nach obigen Programmalgorithmus mit obigem Parametersatz eine Identität von mindestens 30 % aufweist.

Weitere Beispiele für HPPD und HPPD-Gene lassen sich beispielsweise aus verschiedenen Organismen, deren genomische Sequenz bekannt ist, durch Homologievergleiche der Aminosäuresequenzen oder der entsprechenden rückübersetzten Nukleinsäuresequenzen aus Datenbanken mit der SeQ ID. NO. 14 leicht auffinden.

Die HPPD aus Gerste weist beispielsweise mit der HPPD aus *Arabisopsis thaliana* (Seq. ID. No. 14) eine Identität von 57,5% auf.

Weitere Beispiele für HPPD und HPPD-Gene lassen sich weiterhin beispielsweise ausgehend von der Sequenz SEQ. ID. No. 13 aus verschiedenen Organismen deren genomische Sequenz nicht bekannt ist, durch Hybridisierungs- und PCR-Techniken in an sich bekannter Weise leicht auffinden.

In einer weiter besonders bevorzugten Ausführungsform werden zur Erhöhung der Hydroxyphenylpyruvat-Dioxygenase-Aktivität Nukleinsäuren in Pflanzen eingebracht, die Proteine kodieren, enthaltend die Aminosäuresequenz der HPPD aus *Arabidopsois thaliana* (SEQ. ID. NO. 14).

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der organismusspezifischen codon usage häufig verwendet werden. Die codon usage läßt sich anhand von Computerauswertungen anderer, bekannter Gene der betreffenden Organismen leicht ermitteln.

Soll das Protein beispielsweise in Pflanzen exprimiert werden, so ist es häufig vorteilhaft, die codon usage der Pflanze bei der Rückübersetzung zu verwenden.

In einer besonders bevorzugten Ausführungsform bringt man eine Nukleinsäure, enthaltend die Sequenz SEQ. ID. NO. 13 in die Pflanze ein.

Die Sequenz SEQ. ID. NO. 13 stellt die genomische DNA aus A. thaliana dar, die die HPPD der Sequenz SEQ ID NO. 14 codiert.

Alle vorstehend erwähnten HPPD-Gene sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al . (1989), Molecular cloning: A laboratory manual, Cold Spring Harbor Laboratory Press, beschrieben.

In einer bevorzugten Ausführungsform erfolgt die Erhöhung der Genexpression einer Nukleinsäure codierend eine Homogentisat-Phytyltransferase, im folgenden auch HPT genannt, durch Einbringen von mindestens einer Nukleinsäuren codierend eine HPT in die Pflanze.

Dazu kann prinzipiell jedes HPT-Gen, also jede Nukleinsäure, die eine HPT codiert, verwendet werden.

Bei genomischen HPT-Nukleinsäure-Sequenzen aus eukaryontischen Quellen, die Introns enthalten, sind fürdenFall daß der Wirtsorganismus nicht in der Lage ist oder nicht in die Lage versetzt werden kann, die entsprechende HPT zu exprimieren, bevorzugt bereits prozessierte Nukleinsäuresequenzen, wie die entsprechenden cDNAs zu verwenden.

Beispiele für HPT-Gene sind Nukleinsäuren, codierend eine HPT aus *Arabidopsis thaliana* (Nukleinsäure: Seq. ID. No. 15, Protein: Seq. ID. No. 16) oder Nukleinsäuren, codierend eine HPT aus *Glycine max, Heliantus annus, Nicotiana tabacum, Physcomitrella patens, Brassica napus, Oryza sativa, Hordeum vulgaris* oder *Synechocystis sp. PCC6803.*

In den erfindungsgemäßen transgenen Pflanzen liegt also in dieser bevorzugten Ausführungsform gegenüber dem Wildtyp mindestens ein weiteres HPT-Gen vor. In dieser bevorzugten Ausführungsform weist die Pflanze dementsprechend mindestens eine exogene Nukleinsäure, codierend eine HPT oder mindestens zwei endogene Nukleinsäuren, codierend eine HPT auf.

Bevorzugt verwendet man in vorstehend beschriebener bevorzugter Ausführungsform Nukleinsäuren, die Proteine kodieren, enthaltend die Aminosäuresequenz SEQ. ID. NO. 16 oder eine von dieser Sequenz durch Substitution, Insertion oder Deletion von Aminosäuren abgeleitete Sequenz, die eine Identität von mindestens 30 %, vorzugsweise mindestens 50%, bevorzugter mindestens 70%, noch bevorzugter mindestens 90%, am bevorzugtesten mindestens 95% auf Aminosäureebene mit der Sequenz SEQ. ID. NO. 16, und die die enzymatische Eigenschaft einer HPT aufweisen.

Die Sequenz SEQ. ID. NO. 16 stellt die Aminosäuresequenz der HPT aus *Arabisopsis thaliana* dar.

Unter einem Protein, das eine Identität von mindestens 30 % auf Aminosäureebene mit der Sequenz SEQ. ID. NO. 16 aufweist, wird dementsprechend ein Protein verstanden, das bei einem Vergleich seiner Sequenz mit der Sequenz SEQ. ID. NO. 16, insbesondere nach obigen Programmalgorithmus mit obigem Parametersatz eine Identität von mindestens 30 % aufweist.

Weitere Beispiele für HPT und HPT-Gene lassen sich beispielsweise aus verschiedenen Organismen, deren genomische Sequenz bekannt ist, durch Homologievergleiche der Aminosäuresequenzen oder der entsprechenden rückübersetzten Nukleinsäuresequenzen aus Datenbanken mit der SeQ ID. NO. 16 leicht auffinden.

Die HPT aus *Synechocystis sp. PCC6803* weist beispielsweise mit der HPT aus *Arabisopsis thaliana* (Seq. ID. No. 16) eine Identität von 40, 9 % auf.

Weitere Beispiele für HPT und HPT-Gene lassen sich weiterhin beispielsweise ausgehend von der Sequenz SEQ. ID. No. 15 aus verschiedenen Organismen deren genomische Sequenz nicht bekannt ist, durch Hybridisierungs- und PCR-Techniken in an sich bekannter Weise leicht auffinden.

In einer weiter besonders bevorzugten Ausführungsform werden zur Erhöhung der Homogentisat-Phytyltransferase-Aktivität Nukleinsäuren in Pflanzen eingebracht, die Proteine kodieren, enthaltend die Aminosäuresequenz der HPT aus *Arabidopsois thaliana* (SEQ. ID. NO. 16).

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der organismusspezifischen codon usage häufig verwendet werden. Die codon usage läßt sich anhand von Computerauswertungen anderer, bekannter Gene der betreffenden Organismen leicht ermitteln.

Soll das Protein beispielsweise in Pflanzen exprimiert werden, so ist es häufig vorteilhaft, die codon usage der Pflanze bei der Rückübersetzung zu verwenden.

In einer besonders bevorzugten Ausführungsform bringt man eine Nukleinsäure, enthaltend die Sequenz SEQ. ID. NO. 15 in die Pflanze ein.

Die Sequenz SEQ. ID. NO. 15 stellt die genomische DNA aus A. thaliana dar, die die HPT der Sequenz SEQ ID NO. 16 codiert.

Alle vorstehend erwähnten HPT-Gene sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

In einer bevorzugten Ausführungsform erfolgt die Erhöhung der Genexpression einer Nukleinsäure codierend eine Geranyl-Geranyl-Pyrophosphat-Oxidoreduktase, im folgenden auch GGPPOR genannt, durch Einbringen von mindestens einer Nukleinsäuren codierend eine GGPPOR in die Pflanze.

Dazu kann prinzipiell jedes GGPPOR-Gen, also jede Nukleinsäure, die eine GGPPOR codiert, verwendet werden.

Bei genomischen GGPPOR-Nukleinsäure-Sequenzen aus eukaryontischen Quellen, die Introns enthalten, sind für den Fall daß der Wirtsorganismus nicht in der Lage ist oder nicht in die Lage versetzt werden kann, die entsprechende GGPPOR zu exprimieren, bevorzugt bereits prozessierte Nukleinsäuresequenzen, wie die entsprechenden cDNAs zu verwenden.

Beispiele für GGPPOR-Gene sind Nukleinsäuren, codierend eine GGPPOR aus *Nicotania tabacum* (Nukleinsäure: Seq. ID. No. 17, Protein: Seq. ID. No. 18) oder Nukleinsäuren, codierend eine GGPPOR aus *Arabidopsis thaliana, Glycine max, Heliantus annus*, *Physcomitrella patens, Brassica napus*, *Oryza sativa, Hordeum vulgaris* oder *Synechocystis sp. PCC6803.*

In den erfindungsgemäßen transgenen Pflanzen liegt also in dieser bevorzugten Ausführungsform gegenüber dem Wildtyp mindestens ein weiteres GGPPOR-Gen vor. In dieser bevorzugten Ausführungsform weist die Pflanze dementsprechend mindestens eine exogene Nukleinsäure, codierend eine GGPPOR oder mindestens zwei endogene Nukleinsäuren, codierend eine GGPPOR auf.

Bevorzugt verwendet man in vorstehend beschriebener bevorzugter Ausführungsform Nukleinsäuren, die Proteine kodieren, enthaltend die Aminosäuresequenz SEQ. ID. NO. 18 oder eine von dieser Sequenz durch Substitution, Insertion oder Deletion von Aminosäuren abgeleitete Sequenz, die eine Identität von mindestens 30 %, vorzugsweise mindestens 50%, bevorzugter mindestens 70%, noch bevorzugter mindestens 90%, am bevorzugtesten mindestens 95% auf Aminosäureebene mit der Sequenz SEQ. ID. NO. 18, und die die enzymatische Eigenschaft einer GGPPOR aufweisen.

Die Sequenz SEQ. ID. NO. 18 stellt die Aminosäuresequenz der GGPPOR aus *Nicotania tabacum* dar.

Unter einem Protein, das eine Identität von mindestens 30 % auf Aminosäureebene mit der Sequenz SEQ. ID. NO. 18 aufweist, wird dementsprechend ein Protein verstanden, das bei einem Vergleich seiner Sequenz mit der Sequenz SEQ. ID. NO. 18, insbesondere nach obigen Programmalgorithmus mit obigem Parametersatz eine Identität von mindestens 30 % aufweist.

Weitere Beispiele für GGPPOR und GGPPOR-Gene lassen sich beispielsweise aus verschiedenen Organismen, deren genomische Sequenz bekannt ist, durch Homologievergleiche der Aminosäuresequenzen oder der entsprechenden rückübersetzten Nukleinsäuresequenzen aus Datenbanken mit der SeQ ID. NO. 18 leicht auffinden.

Die GGPPOR aus *Arabidopsis thaliana* weist beispielsweise mit der GGPPOR aus *Nicotania tabacum* (Seq. ID. No. 18) eine Identität von 80 % auf.

Weitere Beispiele für GGPPOR und GGPPOR-Gene lassen sich weiterhin beispielsweise ausgehend von der Sequenz SEQ. ID. No. 17 aus verschiedenen Organismen deren genomische Sequenz nicht bekannt ist, durch Hybridisierungs- und PCR-Techniken in an sich bekannter Weise leicht auffinden.

In einer weiter besonders bevorzugten Ausführungsform werden zur Erhöhung der Geranyl-Geranyl-Pyrophosphat-Oxidoreduktase-Aktivität Nukleinsäuren in Pflanzen eingebracht, die Proteine kodieren, enthaltend die Aminosäuresequenz der GGPPOR aus *Nicotania tabacum* (SEQ. ID. NO. 18).

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der organismusspezifischen codon usage häufig verwendet werden. Die codon usage läßt sich anhand von Computerauswertungen anderer, bekannter Gene der betreffenden Organismen leicht ermitteln.

Soll das Protein beispielsweise in Pflanzen exprimiert werden, so ist es häufig vorteilhaft, die codon usage der Pflanze bei der Rückübersetzung zu verwenden.

In einer besonders bevorzugten Ausführungsform bringt man eine Nukleinsäure, enthaltend die Sequenz SEQ. ID. NO. 17 in die Planze ein.

Die Sequenz SEQ. ID. NO. 17 stellt die genomische DNA aus Nicotiana tabacum dar, die die GGPPOR der Sequenz SEQ ID NO. 18 codiert.

Alle vorstehend erwähnten GGPPOR-Gene sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

In einer bevorzugten Ausführungsform erfolgt die Erhöhung der Genexpression einer Nukleinsäure codierend eine 2-Methyl-6-Phytylhydrochinon-Methyltransferase, im folgenden auch MT1 genannt, durch Einbringen von mindestens einer Nukleinsäuren codierend eine MT1 in die Pflanze.

Dazu kann prinzipiell jedes MT1-Gen, also jede Nukleinsäure, die eine MT1 codiert, verwendet werden.

Bei genomischen MT1-Nukleinsäure-Sequenzen aus eukaryontischen Quellen, die Introns enthalten, sind für den Fall daß der Wirtsorganismus nicht in der Lage ist oder nicht in die Lage versetzt werden kann, die entsprechende MT1 zu exprimieren, bevorzugt bereits prozessierte Nukleinsäuresequenzen, wie die entsprechenden cDNAs zu verwenden.

Beispiele für MT1-Gene sind Nukleinsäuren, codierend eine MT1 aus *Synechocystis sp. PCC6803* (Nukleinsäure: Seq. ID. No. 19, Protein: Seq. ID. No. 20).

In den erfindungsgemäßen transgenen Pflanzen liegt also in dieser bevorzugten Ausführungsform gegenüber dem Wildtyp mindestens ein weiteres MT1-Gen vor. In dieser bevorzugten Ausführungsform weist die Pflanze dementsprechend mindestens eine exogene Nukleinsäure, codierend eine MT1 oder mindestens zwei endogene Nukleinsäuren, codierend eine MT1 auf.

Bevorzugt verwendet man in vorstehend beschriebener bevorzugter Ausführungsform Nukleinsäuren, die Proteine kodieren, enthaltend die Aminosäuresequenz SEQ. ID. NO. 20 oder eine von dieser Sequenz durch Substitution, Insertion oder Deletion von Aminosäuren abgeleitete Sequenz, die eine Identität von mindestens 30 %, vorzugsweise mindestens 50%, bevorzugter mindestens 70%, noch bevorzugter mindestens 90%, am bevorzugtesten mindestens 95% auf Aminosäureebene mit der Sequenz SEQ. ID. NO. 20, und die die enzymatische Eigenschaft einer MT1 aufweisen.

Die Sequenz SEQ. ID. NO. 20 stellt die Aminosäuresequenz der MT1 aus *Synechocystis sp. PCC6803* dar.

Unter einem Protein, das eine Identität von mindestens 30 % auf Aminosäureebene mit der Sequenz SEQ. ID. NO. 20 aufweist, wird dementsprechend ein Protein verstanden, das bei einem vergleich seiner Sequenz mit der Sequenz SEQ. ID. NO. 20, insbesondere nach obigen Programmalgorithmus mit obigem Parametersatz eine Identität von mindestens 30 % aufweist.

Weitere Beispiele für MT1 und MT1-Gene lassen sich beispielsweise aus verschiedenen Organismen, deren genomische Sequenz bekannt ist, durch Homologievergleiche der Aminosäuresequenzen oder der entsprechenden rückübersetzten Nukleinsäuresequenzen aus Datenbanken mit der SeQ ID. NO. 20 leicht auffinden.

Weitere Beispiele für MT1 und MT1-Gene lassen sich weiterhin beispielsweise ausgehend von der Sequenz SEQ. ID. No. 19 aus verschiedenen Organismen deren genomische Sequenz nicht bekannt ist, durch Hybridisierungs- und PCR-Techniken in an sich bekannter Weise leicht auffinden.

In einer weiter besonders bevorzugten Ausführungsform werden zur Erhöhung der 2-Methyl-6-Phytylhydrochinon-Methyltransferase-Aktivität Nukleinsäuren in Pflanzen eingebracht, die Proteine kodieren, enthaltend die Aminosäuresequenz der MT1 aus *Synechocystis sp. PCC6803* (SEQ. ID. NO. 20).

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der organismusspezifischen codon usage häufig verwendet werden. Die codon usage läßt sich anhand von Computerauswertungen anderer, bekannter Gene der betreffenden Organismen leicht ermitteln.

Soll das Protein beispielsweise in Pflanzen exprimiert werden, so ist es häufig vorteilhaft, die codon usage der Pflanze bei der Rückübersetzung zu verwenden.

In einer besonders bevorzugten Ausführungsform bringt man eine Nukleinsäure, enthaltend die Sequenz SEQ. ID. NO. 19 in die Pflanze ein.

Die Sequenz SEQ. ID. NO. 19 stellt die genomische DNA aus *Synechocystis sp. PCC6803* dar, die die MT1 der Sequenz SEQ ID NO. 20 codiert.

Alle vorstehend erwähnten MT1-Gene sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

In einer bevorzugten Ausführungsform erfolgt die Erhöhung der Genexpression einer Nukleinsäure codierend eine Tocopherolcyclase, im folgenden auch CYC genannt, durch Einbringen von mindestens einer Nukleinsäuren codierend eine CYC in die Pflanze.

Dazu kann prinzipiell jedes CYC-Gen, also jede Nukleinsäure, die eine CYC codiert, verwendet werden.

Bei genomischen CYC-Nukleinsäure-Sequenzen aus eukaryontischen Quellen, dieIntronsenthalten, sind für den Fall daß der Wirtsorganismus nicht in der Lage ist oder nicht in die Lage versetzt werden kann, die entsprechende CYC zu exprimieren, bevorzugt bereits prozessierte Nukleinsäuresequenzen, wie die entsprechenden cDNAs zu verwenden.

Beispiele für CYC-Gene sind Nukleinsäuren, codierend eine CYC aus *Synechocystis sp. PCC6803* (Nukleinsäure: Seq. ID. No. 21, Protein: Seq. ID. No. 22) oder Nukleinsäuren, codierend eine CYC aus *Glycine max, Heliantus annus, Nicotiana tabacum*, *Physcomitrella patens, Brassica napus, Oryza sativa, Arabidopsis thaliana* oder *Hordeum vulgaris.*

In den erfindungsgemäßen transgenen Pflanzen liegt also in dieser bevorzugten Ausführungsform gegenüber dem Wildtyp mindestens ein weiteres CYC-Gen vor. In dieser bevorzugten Ausführungsform weist die Pflanze dementsprechend mindestens eine exogene Nukleinsäure, codierend eine CYC oder mindestens zwei endogene Nukleinsäuren, codierend eine CYC auf.

Bevorzugt verwendet man in vorstehend beschriebener bevorzugter Ausführungsform Nukleinsäuren, die Proteine kodieren, enthaltend die Aminosäuresequenz SEQ. ID. NO. 22 oder eine von dieser Sequenz durch Substitution, Insertion oder Deletion von Aminosäuren abgeleitete Sequenz, die eine Identität von mindestens 30 %, vorzugsweise mindestens 50%, bevorzugter mindestens 70%, noch bevorzugter mindestens 90%, am bevorzugtesten mindestens 95% auf Aminosäureebene mit der Sequenz SEQ. ID. NO. 22, und die die enzymatische Eigenschaft einer CYC aufweisen.

Die Sequenz SEQ. ID. NO. 22 stellt die Aminosäuresequenz der CYC aus *Synechocystis sp. PCC6803* dar.

Unter einem Protein, das eine Identität von mindestens 30 % auf Aminosäureebene mit der Sequenz SEQ. ID. NO. 22 aufweist, wird dementsprechend ein Protein verstanden, das bei einem Vergleich seiner Sequenz mit der Sequenz SEQ. ID. NO. 22, insbesondere nach obigen Programmalgorithmus mit obigem Parametersatz eine Identität von mindestens 30 % aufweist.

Weitere Beispiele für CYC und CYC-Gene lassen sich beispielsweise aus verschiedenen Organismen, deren genomische Sequenz bekannt ist, durch Homologievergleiche der Aminosäuresequenzen oder der entsprechenden rückübersetzten Nukleinsäuresequenzen aus Datenbanken mit der SeQ ID. NO. 22 leicht auffinden.

Die CYC aus Arabidopsis thaliana weist beispielsweise mit der CYC aus *Synechocystis sp. PCC6803* (Seq. ID. No. 22) eine Identität von 29,1 % auf.

Weitere Beispiele für CYC und CYC-Gene lassen sich weiterhin beispielsweise ausgehend von der Sequenz SEQ. ID. No. 21 aus verschiedenen Organismen deren genomische Sequenz nicht bekannt ist, durch Hybridisierungs- und PCR-Techniken in an sich bekannter Weise leicht auffinden.

In einer weiter besonders bevorzugten Ausführungsform werden zur Erhöhung der Tocopherolcyclase-Aktivität Nukleinsäuren in Pflanzen eingebracht, die Proteine kodieren, enthaltend die Aminosäuresequenz der CYC aus *Synechocystis sp. PCC6803* (SEQ. ID. NO. 22).

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der organismusspezifischen codon usage häufig verwendet werden. Die codon usage läßt sich anhand von Computerauswertungen anderer, bekannter Gene der betreffenden Organismen leicht ermitteln.

Soll das Protein beispielsweise in Pflanzen exprimiert werden, so ist es häufig vorteilhaft, die codon usage der Pflanze bei der Rückübersetzung zu verwenden.

In einer besonders bevorzugten Ausführungsform bringt man eine Nukleinsäure, enthaltend die Sequenz SEQ. ID. NO. 21 in die Pflanze ein.

Die Sequenz SEQ. ID. NO. 21 stellt die genomische DNA aus *Synechocystis sp. PCC6803* dar, die die CYC der Sequenz SEQ ID NO. 22 codiert.

Alle vorstehend erwähnten CYC-Gene sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

In einer bevorzugten Ausführungsform erfolgt die Erhöhung der Genexpression einer Nukleinsäure codierend eine γ-Tocopherol-Methyltransferase, im folgenden auch γ-TMT genannt, durch Einbringen von mindestens einer Nukleinsäuren codierend eine γ-TMT in die Pflanze.

Dazu kann prinzipiell jedes γ-TMT-Gen, also jede Nukleinsäure, die eine γ-TMT codiert, verwendet werden.

Bei genomischen γ-TMT-Nukleinsäure-Sequenzen aus eukaryontischen Quellen, die Introns enthalten, sind für den Fall daß der Wirtsorganismus nicht in der Lage ist oder nicht in die Lage versetzt werden kann, die entsprechende γ-TMT zu exprimieren, bevorzugt bereits prozessierte Nukleinsäuresequenzen, wie die entsprechenden cDNAs zu verwenden.

Beispiele für γ-TMT-Gene sind Nukleinsäuren, codierend eine γ-TMT aus *Arabidopsis thaliana* (Nukleinsäure: Seq. ID. No. 23, Protein: Seq. ID. No. 24) oder Nukleinsäuren, codierend eine γ-TMT aus *Glycine max, Heliantus annus, Nicotiana tabacum, Physcomitrella patens*, *Brassica napus, Oryza sativa*, *Hordeum vulgaris* oder *Synechocystis sp. PCC6803.*

In den erfindungsgemäßen transgenen Pflanzen liegt also in dieser bevorzugten Ausführungsform gegenüber dem Wildtyp mindestens ein weiteres γ-TMT-Gen vor. In dieser bevorzugten Ausführungsform weist die Pflanze dementsprechend mindestens eine exogene Nukleinsäure, codierend eine γ-TMT oder mindestens zwei endogene Nukleinsäuren, codierend eine γ-TMT auf.

Bevorzugt verwendet man in vorstehend beschriebener bevorzugter Ausführungsform Nukleinsäuren, die Proteine kodieren, enthaltend die Aminosäuresequenz SEQ. ID. NO. 24 oder eine von dieser Sequenz durch Substitution, Insertion oder Deletion von Aminosäuren abgeleitete Sequenz, die eine Identität von mindestens 30 %, vorzugsweise mindestens 50%, bevorzugter mindestens 70%, noch bevorzugter mindestens 90%, am bevorzugtesten mindestens 95% auf Aminosäureebene mit der Sequenz SEQ. ID. NO. 24, und die die enzymatische Eigenschaft einer γ-TMT aufweisen.

Die Sequenz SEQ. ID. NO. 24 stellt die Aminosäuresequenz der γ-TMT aus *Arabisopsis thaliana* dar.

Unter einem Protein, das eine Identität von mindestens 30 % auf Aminosäureebene mit der Sequenz SEQ. ID. NO. 24 aufweist, wird dementsprechend ein Protein verstanden, das bei einem Vergleich seiner Sequenz mit der Sequenz SEQ. ID. NO. 24, insbesondere nach obigen Programmalgorithmus mit obigem Parametersatz eine Identität von mindestens 30 % aufweist.

Weitere Beispiele für γ-TMT und γ-TMT-Gene lassen sich beispielsweise aus verschiedenen Organismen, deren genomische Sequenz bekannt ist, durch Homologievergleiche der Aminosäuresequenzen oder der entsprechenden rückübersetzten Nukleinsäuresequenzen aus Datenbanken mit der SeQ ID. NO. 24 leicht auffinden.

Die γ-TMT aus Synechocystis sp. PCC6803 weist beispielsweise mit der γ-TMT aus *Arabisopsis thaliana* (Seq. ID. No. 24) eine Identität von 26,7 % auf.

Weitere Beispiele für γ-TMT und γ-TMT-Gene lassen sich weiterhin beispielsweise ausgehend von der Sequenz SEQ. ID. No. 23 aus verschiedenen Organismen deren genomische Sequenz nicht bekannt ist, durch Hybridisierungs- und PCR-Techniken in an sich bekannter Weise leicht auffinden.

In einer weiter besonders bevorzugten Ausführungsform werden zur Erhöhung der γ-Tocopherol-Methyltransferase-Aktivität Nukleinsäuren in Pflanzen eingebracht, die Proteine kodieren, enthaltend die Aminosäuresequenz der γ-TMT aus *Arabidopsois thaliana* (SEQ. ID. NO. 24).

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der organismusspezifischen codon usage häufig verwendet werden. Die codon usage läßt sich anhand von Computerauswertungen anderer, bekannter Gene der betreffenden Organismen leicht ermitteln.

Soll das Protein beispielsweise in Pflanzen exprimiert werden, so ist es häufig vorteilhaft, die codon usage der Pflanze bei der Rückübersetzung zu verwenden.

In einer besonders bevorzugten Ausführungsform bringt man eine Nukleinsäure, enthaltend die Sequenz SEQ. ID. NO. 23 in die Pflanze ein.

Die Sequenz SEQ. ID. NO. 23 stellt die genomische DNA aus A. thaliana dar, die die γ-TMT der Sequenz SEQ ID NO. 24 codiert.

Alle vorstehend erwähnten γ-TMT-Gene sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

In einer weiter bevorzugten Ausführungsform des Verfahrens weisen die Pflanzen gegenüber dem Wildtyp zusätzlich eine reduzierte Aktivität mindestens einer der Aktivitäten, ausgewählt aus der Gruppe Homogentisat-Dioxygenase-Aktivität, Maleylacetoacetat-Isomerase-Aktivität und Fumarylacetoacetat-Hydrolase-Aktivität auf.

Unter einer reduzierten Aktivität wird sowohl die reduzierte als auch das komplette Ausschalten der Aktivität verstanden. Eine Reduzierung einer Aktivität umfasst demnach auch eine mengenmässige Verringerung des entsprechenden Proteins in der Pflanze bis hin zu einem vollständigen Fehlen des entsprechenden Proteins, beispielsweise zu testen durch eine fehlende Nachweisbarkeit der entsprechenden Enzymaktivität oder eine fehlende immunologische Nachweisbarkeit der entsprechenden Proteine.

Unter Homogentisat-Dioxygenase-Aktivität wird die Enzymaktivität einer Homogentisat-Dioxygenase verstanden.

Unter einer Homogentisat-Dioxygenase wird ein Protein verstanden, das die enzymatische Aktivität aufweist, Homogentisat in Maleylacetoacetat umzuwandeln.

Dementsprechend wird unter Homogentisat-Dioxygenase-Aktivität die in einer bestimmten Zeit durch das Protein Homogentisat-Dioxygenase umgesetzte Menge Homogentisat bzw. gebildete Menge Maleylacetoacetat verstanden.

Bei einer reduzierten Homogentisat-Dioxygenase-Aktivität gegenüber dem Wildtyp wird somit im Vergleich zum Wildtyp in einer bestimmten Zeit durch das Protein Homogentisat-Dioxygenase die umgesetzte Menge Homogentisat bzw. die gebildete Menge Maleylacetoacetat reduziert.

Vorzugsweise beträgt diese Reduzierung der Homogentisat-Dioxygenase-Aktivität mindestens 5 %, weiter bevorzugt mindestens 20 %, weiter bevorzugt mindestens 50 %, weiter bevorzugt 100 %. Besonders bevorzugt ist die Homogentisat-Dioxygenase-Aktivität komplett ausgeschaltet.

Unter Maleylacetoacetat-Isomerase-Aktivität wird die Enzymaktivität einer Maleylacetoacetat-Isomerase verstanden.

Unter einer Maleylacetoacetat-Isomerase wird ein Protein verstanden, das die enzymatische Aktivität aufweist, Maleylacetoacetat in Fumarylacetoacetat umzuwandeln.

Dementsprechend wird unter Maleylacetoacetat-Isomerase-Aktivität die in einer bestimmten Zeit durch das Protein Maleylacetoacetat-Isomerase umgesetzte Menge Maleylacetoacetat bzw. gebildete Menge Fumarylacetoacetat verstanden.

Bei einer reduzierten Maleylacetoacetat-Isomerase-Aktivität gegenüber dem Wildtyp wird somit im Vergleich zum Wildtyp in einer bestimmten Zeit durch das Protein Maleylacetoacetat-Isomerase die umgesetzte Menge Maleylacetoacetat bzw. die gebildete Menge Fumarylacetoacetat reduziert.

Vorzugsweise beträgt diese Reduzierung der Maleylacetoacetat-Isomerase-Aktivität mindestens 5 %, weiter bevorzugt mindestens 20 %, weiter bevorzugt mindestens 50 %, weiter bevorzugt 100 %. Besonders bevorzugt ist die Maleylacetoacetat-Isomerase-Aktivität komplett ausgeschaltet.

Unter Fumarylacetoacetat-Hydrolase-Aktivität wird die Enzymaktivität einer Fumarylacetoacetat-Hydrolase verstanden.

Unter einer Fumarylacetoacetat-Hydrolase wird ein Protein verstanden, das die enzymatische Aktivität auf weist, Fumarylacetoacetat in Fumarat umzuwandeln.

Dementsprechend wird unter Fumarylacetoacetat-Hydrolase-Aktivität die in einer bestimmten Zeit durch das Protein Fumarylacetoacetat-Hydrolase umgesetzte Menge Fumarylacetoacetat bzw. gebildete Menge Fumarat verstanden.

Bei einer reduzierten Fumarylacetoacetat-Hydrolase-Aktivität gegenüber dem Wildtyp wird somit im Vergleich zum Wildtyp in einer bestimmten Zeit durch das Protein Fumarylacetoacetat-Hydrolase die umgesetzte Menge Fumarylacetoacetat bzw. die gebildete Menge Fumarat reduziert.

Vorzugsweise beträgt diese Reduzierung der Fumarylacetoacetat-Hydrolase-Aktivität mindestens 5 %, weiter bevorzugt mindestens 20 %, weiter bevorzugt mindestens 50 %, weiter bevorzugt 100 %. Besonders bevorzugt ist die Fumarylacetoacetat-Hydrolase-Aktivität komplett ausgeschaltet.

Die Homogentisat-Dioxygenase wird im folgenden auch als HGD bezeichnet, die Maleylacetoacetat-Isomerase wird im folgenden auch als MAAI bezeichnet und die Fumarylacetoacetat-Hydrolase wird wird im folgenden auch als FAAH bezeichnet.

Es zahlreiche Möglichkeiten, um die HGD-, MAAI - und/oder FAAH-Aktivität in gewünschter Weise zu reduzieren.

Eine mögliche Methode umfasst die Verwendung mindestens einer Nukleinsäuresequenz, im folgenden auch anti-HGD, anti-MAAI bzw. anti-FAAH genannt, welche zu einer antisense-Nukleinsäuresequenz transkribierbar ist, die zur Inhibition der HGD-, MAAI- und/oder FAAH-Aktivität befähigt ist, beispielsweise indem sie die Expression von endogener HGD, MAAI und/oder FAAH inhibiert.

Diese anti-HGD, anti-MAAI oder anti-FAAH-Nukleinsäuresequenzen können gemäss einer bevorzugten Ausführungsform die in antisense-Orientierung insertierte kodierende Nukleinsäuresequenz der HGD MAAI und/oder FAAH oder funktional äquivalente Fragment der jeweiligen Sequenzen enthalten.

Vorteilhaft kann die antisense-Strategie mit einem Ribozym-Verfahren gekoppelt werden. Ribozyme sind katalytisch aktive RNA Sequenzen, die gekoppelt an die antisense Sequenzen, die Zielsequenzen katalytisch spalten (Tanner NK. FEMS Microbiol Rev. 1999; 23 (3):257-75). Dies kann die Effizienz einer anti-sense Strategie erhöhen.

Weitere Methoden zur Reduzierung der HGD-, MAAI- und/oder FAAH-Expression, insbesondere in Pflanzen als Organismen umfassen die zu Kosuppression führende Überexpression homologer HGD-, MAAI- und/oder FAAH-Nukleinsäuresequenzen (Jorgensen et al. , Plant Mol. Biol. 1996, 31 (5):957-973) oder die Induktion des spezifischen RNA-Abbaus durch die Pflanze mit Hilfe eines viralen Expressionssystems (Amplikon) (Angell, SM et al., Plant J. 1999, 20 (3) : 357-362). Diese Methoden werden auch als "post-transcriptional gene silencing" (PTGS) bezeichnet.

Weitere Methoden sind die Einführung von Nonsense-Mutationen in das Endogen mittels Einführung von RNA/DNA-Oligonukleotiden in die Pflanze (Zhu et al., Nat. Biotechnol. 2000, 18(5):555-558) oder die Generierung von Knockout-Mutanten mit Hilfe von z.B. T-DNA-Mutagenese (Koncz et al., Plant Mol. Biol. 1992, 20(5) :963-976) oder homologer Rekombination (Hohn, B.undPuchta, H, Proc. Natl. Acad. Sci. USA. 1999, 96:8321-8323.). Ferner ist eine Genüberexpression oder -repression auch mit spezifischen DNA-bindenden Faktoren z.B. mit den oben erwähnten Faktoren vom Typus der Zinkfingertranskriptionsfaktoren. Ferner können Faktoren in eine Zelle eingebracht werden, die das Zielprotein selber inhibieren. Die Protein-bindenden Faktoren können z.B. Aptamere sein (Famulok M, und Mayer G. Curr Top Microbiol Immunol. 1999; 243:123-36).

Eine weitere Methode zur Reduzierung mindestens einer der vorstehend beschriebenen Aktivitäten ist die Verwendung von RNA die einem Bereich mit Doppel-Strang-Struktur aufweist und in diesem Bereich eine Nukleinsäuresequenz aufweist, die mit einem Teil der zu reduzierenden Zielsequenz identisch ist. Eine ausführliche Beschreibung dieser Methode, die auch RNAi-Technologie genannt wird, ist in WO 99/32619 offenbart.

In einer bevorzugten Ausführungsform erfolgt die zusätzlichen Reduzierung mindestens einer der Aktivitäten, ausgewählt aus der Gruppe HGD-, MAAI- und FAAH-Aktivität durch Reduzierung der Genexpression mindestens einer Nukleinsäure, ausgewählt aus der Gruppe Nukleinsäuren kodierend eine Homogentisat-Dioxygenase, Nukleinsäuren kodierend eine Maleylacetoacetat-Isomerase und Nukleinsäuren kodierend eine Fumarylacetoacetat-Hydrolase gegenüber dem Wildtyp.

Eine Reduzierung der Genexpression mindestens einer Nukleinsäure, ausgewählt aus der Gruppe Nukleinsäuren kodierend eine Homogentisat-Dioxygenase, Nukleinsäuren kodierend eine Maleylacetoacetat-Isomerase und Nukleinsäuren kodierend eine Fumarylacetoacetat-Hydrolase gegenüber dem Wildtyp kann, wie vorstehend beschrieben, bevorzugt durch Verwendung folgender Methoden erreicht werden:
a) Einführung von antisense-Nukleinsäuresequenzen;
b) Einführung von antisense-Nukleinsäuresequenzen kombiniert mit einem Ribozym-Verfahren
c) Einführung von für homologe HGD-, MAAI und/oder FAAH-kodierende und zu Kosuppression führende Nukleinsäuresequenzen
d) Einführung von HGD-, MAAI und/oder FAAH-Abbau bewirkende virale Nukleinsäuresequenzen und Expressionskonstrukte;
e) Einführung von Nonsense-Mutanten von endogenen HGD-, MAAI und/oder FAAH kodierenden Nukleinsäuresequenzen;
f) Einführung von Knockout-Mutanten;
g) Einführung von zu homologer Rekombination geeigneten Nukleinsäuresequenzen;
h) Einführung von RNA, die einen Bereich mit Doppel-Strang-Struktur aufweist und in diesem Bereich eine Nukleinsäuresequenz aufweist, die mit einem Teil der Pflanzen eigenen Ziel-Nukleinsäuresequenz identisch ist.

Auch eine kombinierte Anwendung der vorstehend beschriebenen Methoden ist denkbar.

In einer besonders bevorzugten Ausführungsform des Verfahrens weisen die Pflanzen eine reduzierte Homogentisat-Dioxygenase- Aktivität auf.

Dies wird besonders bevorzugt dadurch erreicht, daß man in die Pflanze eine RNA einbringt, die einen Bereich mit Doppel-Strang-Struktur aufweist und in diesem Bereich eine Nukleinsäuresequenz aufweist, die mit einem Teil der Pflanzen eigenen Nukleinsäure, codierend eine Homogentisat-Dioxygenase identisch ist. Eine ausführliche Beschreibung dieser Methode, die auch RNAi-Technologie genannt wird, ist in WO 99/32619 offenbart.

Je nach verwendeter Pflanze ist demnach ein unterschiedliches Teilfragment der Pflanzen eigenen Nukleinsäure, codierend eine Homogentisat-Dioxygenase, zu verwenden.

SEQ. ID. No. 25 stellt beispielsweise ein Teilfragment der HGD-codierenden Nukleinsäure aus *Brassica napus* dar, welches, in ein entsprechendes RNAi-Konstrukt integriert, die HGD-Aktivität in *Brassica napus* reduziert.

In weiteren bevorzugten Ausführungsformen des erfindungsgemäßen Verfahrens erfolgt die Herstellung von Vitamin E durch Kultivierung von Pflanzen, die gegenüber dem Wildtyp
eine erhöhte Tyrosinaminotransferase-Aktivität aufweisen,
eine erhöhte Tyrosinaminotransferase-Aktivität und eine erhöhte Hydroxyphenylpyruvat-Dioxygenase-Aktivität aufweisen,
eine erhöhte Tyrosinaminotransferase-Aktivität und eine erhöhte Homogentisat-Phytyltransferase-Aktivität aufweisen,
eine erhöhte Tyrosinaminotransferase-Aktivität und eine erhöhte Geranyl-Geranyl-Pyrophosphat-Oxidoreduktase-Aktivität aufweisen,
eine erhöhte Tyrosinaminotransferase-Aktivität und eine erhöhte 2-Methyl-6-Phytylhydrochinon-Methyltransferase-Aktivität aufweisen,
eine erhöhte Tyrosinaminotransferase-Aktivität und eine erhöhte Tocopherolcyclase-Aktivität aufweisen,
eine erhöhte Tyrosinaminotransferase-Aktivität und eine erhöhte γ-Tocopherol-Methyltransferase-Aktivität aufweisen,
eine erhöhte Tyrosinaminotransferase-Aktivität und eine reduzierte Homogentisat-Dioxygenase-Aktivität aufweisen,
eine erhöhte Tyrosinaminotransferase-Aktivität und eine reduzierte Maleylacetoacetat-Isomerase-Aktivität aufweisen,
eine erhöhte Tyrosinaminotransferase-Aktivität und eine reduzierte Fumarylacetoacetat-Hydrolase-Aktivität aufweisen,
eine erhöhte Tyrosinaminotransferase-Aktivität, eine erhöhte Hydroxyphenylpyruvat-Dioxygenase-Aktivität und eine reduzierte Homogentisat-Dioxygenase-Aktivität aufweisen,
eine erhöhte Tyrosinaminotransferase-Aktivität und eine erhöhte Homogentisat-Phytyltransferase-Aktivität und eine reduzierte Homogentisat-Dioxygenase-Aktivität aufweisen,
eine erhöhte Tyrosinaminotransferase-Aktivität und eine erhöhte Geranyl-Geranyl-Pyrophosphat-Oxidoreduktase-Aktivität und eine reduzierte Homogentisat-Dioxygenase-Aktivität aufweisen,
eine erhöhte Tyrosinaminotransferase-Aktivität und eine erhöhte 2-Methyl-6-Phytylhydrochinon-Methyltransferase-Aktivität und eine reduzierte Homogentisat-Dioxygenase-Aktivität aufweisen,
eine erhöhte Tyrosinaminotransferase-Aktivität und eine erhöhte Tocopherolcyclase-Aktivität und eine reduzierte Homogentisat-Dioxygenase-Aktivität aufweisen,
eine erhöhte Tyrosinaminotransferase-Aktivität und eine erhöhte γ-Tocopherol-Methyltransferase-Aktivität und eine reduzierte Homogentisat-Dioxygenase-Aktivität aufweisen,
eine erhöhte Tyrosinaminotransferase-Aktivität und eine erhöhte Hydroxyphenylpyruvat-Dioxygenase-Aktivität, und eine Homogentisat-Phytyltransferase-Aktivität, und eine reduzierte Homogentisat-Dioxygenase-Aktivität aufweisen,
eine erhöhte Tyrosinaminotransferase-Aktivität und eine erhöhte Hydroxyphenylpyruvat-Dioxygenase-Aktivität, und eine Geranyl-Geranyl-Pyrophosphat-Oxidoreduktase-Aktivität, und eine reduzierte Homogentisat-Dioxygenase-Aktivität aufweisen,
eine erhöhte Tyrosinaminotransferase-Aktivität und eine erhöhte Hydroxyphenylpyruvat-Dioxygenase-Aktivität, und eine 2-Methyl-6-Phytylhydrochinon-Methyltransferase-Aktivität, und eine reduzierte Homogentisat-Dioxygenase-Aktivität aufweisen,
eine erhöhte Tyrosinaminotransferase-Aktivität und eine erhöhte Hydroxyphenylpyruvat-Dioxygenase-Aktivität, und eine Tocopherolcyclase-Aktivität, und eine reduzierte Homogentisat-Dioxygenase-Aktivität aufweisen,
eine erhöhte Tyrosinaminotransferase-Aktivität und eine erhöhte Hydroxyphenylpyruvat-Dioxygenase-Aktivität, und eine γ-Tocopherol-Methyltransferase, und eine reduzierte Homogentisat-Dioxygenase-Aktivität aufweisen,
eine erhöhte Tyrosinaminotransferase-Aktivität und eine erhöhte Hydroxyphenylpyruvat-Dioxygenase-Aktivität, und eine Homogentisat-Phytyltransferase-Aktivität, und eine Geranyl-Geranyl-Pyrophosphat-Oxidoreduktase-Aktivität, und eine reduzierte Homogentisat-Dioxygenase-Aktivität aufweisen,
eine erhöhte Tyrosinaminotransferase-Aktivität und eine erhöhte Hydroxyphenylpyruvat-Dioxygenase-Aktivität, und eine Homogentisat-Phytyltransferase-Aktivität, und eine 2-Methyl-6-Phytylhydrochinon-Methyltransferase-Aktivität, und eine reduzierte Homogentisat-Dioxygenase-Aktivität aufweisen,
eine erhöhte Tyrosinaminotransferase-Aktivität und eine erhöhte Hydroxyphenylpyruvat-Dioxygenase-Aktivität, und eine Homogentisat-Phytyltransferase-Aktivität, und ein Tocopherolcyclase-Aktivität, und eine reduzierte Homogentisat-Dioxygenase-Aktivität aufweisen,
eine erhöhte Tyrosinaminotransferase-Aktivität und eine erhöhte Hydroxyphenylpyruvat-Dioxygenase-Aktivität, und eine Homogentisat-Phytyltransferase-Aktivität und eine γ-Tocopherol-Methyltransferase, und eine reduzierte Homogentisat-Dioxygenase-Aktivität aufweisen,
eine erhöhte Tyrosinaminotransferase-Aktivität und eine erhöhte Hydroxyphenylpyruvat-Dioxygenase-Aktivität, und eine Homogentisat-Phytyltransferase-Aktivität, und eine Geranyl-Geranyl-Pyrophosphat-Oxidoreduktase-Aktivität, und eine 2-Methyl-6-Phytylhydrochinon-Methyltransferase-Aktivität, und eine reduzierte Homogentisat-Dioxygenase-Aktivität aufweisen,
eine erhöhte Tyrosinaminotransferase-Aktivität und eine erhöhte Hydroxyphenylpyruvat-Dioxygenase-Aktivität, und eine Homogentisat-Phytyltransferase-Aktivität, und eine Geranyl-Geranyl-Pyrophosphat-Oxidoreduktase-Aktivität, Tocopherolcyclase-Aktivität, und eine reduzierte Homogentisat-Dioxygenase-Aktivität aufweisen,
eine erhöhte Tyrosinaminotransferase-Aktivität und eine erhöhte Hydroxyphenylpyruvat-Dioxygenase-Aktivität, Homogentisat-Phytyltransferase-Aktivität, und eine Geranyl-Geranyl-Pyrophosphat-Oxidoreduktase-Aktivität, und eine und eine γ-Tocopherol-Methyltransferase, und eine reduzierte Homogentisat-Dioxygenase-Aktivität aufweisen,
eine erhöhte Tyrosinaminotransferase-Aktivität und eine erhöhte Hydroxyphenylpyruvat-Dioxygenase-Aktivität, und eine Homogentisat-Phytyltransferase-Aktivität, und eine Geranyl-Geranyl-Pyrophosphat-Oxidoreduktase-Aktivität, und eine 2-Methyl-6-Phytylhydrochinon-Methyltransferase-Aktivität, und eine Tocopherolcyclase-Aktivität, und eine reduzierte Homogentisat-Dioxygenase-Aktivität aufweisen,
eine erhöhte Tyrosinaminotransferase-Aktivität und eine erhöhte Hydroxyphenylpyruvat-Dioxygenase-Aktivität, und eine Homogentisat-Phytyltransferase-Aktivität, und eine Geranyl-Geranyl-Pyrophosphat- Oxidoreduktase-Aktivität, und eine 2-Methyl-6-Phytylhydrochinon- Methyltransferase-Aktivität, und eine γ-Tocopherol-Methyl- transferase, und eine reduzierte Homogentisat-Dioxygenase-Aktivität aufweisen,
eine erhöhte Tyrosinaminotransferase-Aktivität und eine erhöhte Hydroxyphenylpyruvat-Dioxygenase-Aktivität, und eine Homogentisat-Phytyltransferase-Aktivität, und eine Geranyl-Geranyl- Pyrophosphat-Oxidoreduktase-Aktivität, und eine 2-Methyl-6- Phytylhydrochinon-Methyltransferase-Aktivität, und eine Tocopherolcyclase-Aktivität, und eine γ-Tocopherol-Methyl- transferase, und eine reduzierte Homogentisat-Dioxygenase-Aktivität aufweisen,

Unter Organismen werden prokaryontische Organismen oder eukaryontische Organismen, wie beispielsweise Bakterien, Hefen, Algen, Moose, Pilze oder Pflanzen, verstanden, die in der Lage sind, als Wildtyp oder durch genetische Veränderung Vitamin E herzustellen. Organismen sind photosynthetisch aktive Organismen, wie beispielsweise Cyanobakterien, Moose, Algen oder Pflanzen, die bereits als Wildtyp in der Lage sind, Vitamin E herzustellen.

Besonders bevorzugte erfindungsgemäße Organismen sind Pflanzen.

Bevorzugte Pflanzen sind Tagetes, Sonnenblume, Arabidopsis, Tabak, Roter Pfeffer, Soja, Tomate, Aubergine, Paprika, Möhre, Karotte, Kartoffel, Mais, Salate und Kohlarten, Getreide, Alfalfa, Hafer, Gerste, Roggen, Weizen, Triticale, Hirse, Reis, Luzerne, Flachs, Baumwolle, Hanf, Brassicacaen wie beispielsweise Raps oder Canola, Zuckerrübe, Zuckerrohr, Nuß- und Weinspezies oder Holzgewächse wie beispielsweise Espe oder Eibe.

Besonders bevorzugt sind *Arabidopsis thaliana, Tagetes* erecta, *Brassica napus, Nicotiana tabacum,* Sonnenblume, Canola, Kartoffel oder Soja.

Im erfindungsgemäßen Verfahren zur Herstellung von Vitamin E wird vorzugsweise dem Kultivierungsschritt der genetisch veränderten Pflanzen, im folgenden auch transgene Pflanzen bezeichnet, ein Ernten der Pflanzen und ein Isolieren von Vitamin E aus den Pflanzen angeschlossen.

Das Ernten der Pflanzen erfolgt in an sich bekannter Weise der jeweiligen Pflanze entsprechend. Pflanzenzellen, die durch Fermentation in flüssigen Nährmedien kultiviert werden, können beispielsweise durch Zentrifugieren, Dekantieren oder Filtrieren abgetrennt werden. Pflanzen werden in an sich bekannter Weise auf Nährböden gezogen und entsprechend geerntet.

Die Isolierung von Vitamin E aus der geernteten Biomasse erfolgt in an sich bekannter Weise, beispielsweise durch Extraktion und gegebenenfalls weiterer chemische oder physikalischer Reinigungsprozesse, wie beispielsweise Fällungsmethoden, Kristallographie, thermische Trennverfahren, wie Rektifizierverfahren oder physikalische Trennverfahren, wie beispielsweise Chromatographie.

Die Isolierung von Vitamin E aus Öl-haltigen Pflanzen erfolgt beispielsweise bevorzugt durch chemische Umwandlung und Destillation aus Pflanzenölen oder aus den bei der Desodorierung pflanzlicher Öle anfallenden Wasserdampfdestillate (Dämpferkondensate).

Weitere Isolierverfahren von Vitamin E aus Dämpferkondensaten sind beispielsweise in DE 31 26 110 A1, EP 171 009 A2, GB 2 145 079, EP 333 472 A2 und WO 94/05650 beschrieben.

Die Herstellung der transgenen Pflanzen erfolgt vorzugsweise durch Transformation der Pflanzen, mit einem Nukleinsäurekonstrukt, das die vorstehend beschriebenen Nukleinsäuren codierend eine Tyrosinaminotransferase enthält, die mit einem oder mehreren Regulationssignalen funktionell verknüpft sind, die die Transkription und Translation in Pflanzen gewährleisten.

Vorzugsweise enhalten die erfindungsgemäßen Nukleinsäurekonstrukte zusätzlich eine, zwei oder drei Nukleinsäuren, ausgewählt aus der Gruppe Nukleinsäuren kodierend eine Hydroxyphenylpyruvat-Dioxygenase, Nukleinsäuren kodierend eine Homogentisat-Phytyltransferase, Nukleinsäuren kodierend eine Geranyl-Geranyl-Pyrophosphat-Oxidoreduktase, Nukleinsäuren kodierend eine 2-Methyl-6-Phytylhydrochinon-Methyltransferase, Nukleinsäuren kodierend eine Tocopherolcyclase und Nukleinsäuren kodierend eine γ-Tocopherol-Methyltransferase, die mit einem oder mehreren Regulationssignalen funktionell verknüpft sind, die die Transkription und Translation in Pflanzen gewährleisten.

In einer weiteren bevorzugten Ausführungsform enthalten die vorstehend beschriebenen Nukleinsäurekonstrukte zusätzlich funktionell verknüpft eine RNA, die einen Bereich mit Doppel-Strang-Struktur aufweist und in diesem Bereich eine Nukleinsäuresequenz aufweist, die mit einem Teil einer Nukleinsäure, codierend eine Homogentisat-Dioxygenase identisch ist.

Es ist, insbesondere in Pflanzen, technisch nur schwer zu realisieren, mehr als vier Aktivitäten mit einem Nukleinsäurekonstrukt zu erhöhen oder zu erniederigen. Daher werden bevorzugt Kombinationen von Nukleinsäurekonstrukten verwendet um die Aktivitäten, insbesondere ummehr als 4 Aktivitäten in der Pflanze zu erhöhen oder zu erniedrigen.

Es ist jedoch auch möglich, genetisch veränderte Pflanzen zu kreuzen, die bereits veränderte Aktivitäten enthalten. Beispielsweise ist es durch Kreuzen von genetisch veränderten Pflanzen, die jeweils zwei veränderte Aktivitäten enthalten, möglich, Pflanzen mit vier veränderten Aktivitäten herzustellen. Gleiches kann auch erreicht werden, indem man eine Kombination von zwei Nukleinsäurekonstrukten die jeweils 2 Aktivitäten verändern in die Pflanze einführt.

In einer bevorzugten Ausführungsform werden die bevorzugten genetisch veränderten Pflanzen durch Einbringen von Kombinationen von Nukleinsäurekonstrukten hergestellt.

Dementsprechend betrifft die Erfindung insbesondere eine Kombination aus Nukleinsäurekonstrukten, wobei die Kombination ein Nukleinsäurekonstrukt, enthaltend die vorstehend beschriebenen Nukleinsäuren codierend eine Tyrosinaminotransferase, funktionell verknüpft mit einem oder mehreren Regulationssignalen, die die Transkription und Translation in Pflanzen gewährleisten, und
a) mindestens ein weiteres Nukleinsäurekonstrukt, ausgewählt aus der Gruppe A bis F
   A Nukleinsäurekonstrukt, enthaltend Nukleinsäuren codierend eine Hydroxyphenylpyruvat-Dioxygenase, die mit einem oder mehreren Regulationssignalen funktionell verknüpft sind, die die Transkription und Translation in Pflanzen gewährleisten,
   B Nukleinsäurekonstrukt, enthaltend Nukleinsäuren codierend eine Homogentisat-Phytyltransferase, die mit einem oder mehreren Regulationssignalen funktionell verknüpft sind, die die Transkription und Translation in Pflanzen gewährleisten und
   C Nukleinsäurekonstrukt, enthaltend Nukleinsäuren codierend eine Geranyl-Geranyl-Pyrophosphat-Oxidoreduktase, die mit einem oder mehreren Regulationssignalen funktionell verknüpft sind, die die Transkription und Translation in Pflanzen gewährleisten,
   D Nukleinsäurekonstrukt, enthaltend Nukleinsäuren codierend eine 2-Methyl-6-Phytylhydrochinon-Methyltransferase, die mit einem oder mehreren Regulationssignalen funktionell verknüpft sind, die die Transkription und Translation in Pflanzen gewährleisten,
   E Nukleinsäurekonstrukt, enthaltend Nukleinsäuren codierend eine Tocopherolcyclase, die mit einem oder mehreren Regulationssignalen funktionell verknüpft sind, die die Transkription und Translation in Pflanzen gewährleisten und
   F Nukleinsäurekonstrukt, enthaltend Nukleinsäuren codierend eine γ-Tocopherol-Methyltransferase, die mit einem oder mehreren Regulationssignalen funktionell verknüpft sind, die die Transkription und Translation in Pflanzen gewährleisten,
   oder
b) mindestens ein weiteres Nukleinsäurekonstrukt, enthaltend zwei, drei oder vier Nukleinsäurekonstrukte, ausgewählt aus der Gruppe der Nukleinsäurekonstrukte A bis F,
umfasst.

Diese Nukleinsäurekonstrukte, in denen die kodierenden Nukleinsäuresequenzen mit einem oder mehreren Regulationssignalen funktionell verknüpft sind, die die Transkription und Translation in Pflanzen gewährleisten, werden im folgenden auch Expressionskassetten genannt.

Dementsprechend betrifft die Erfindung ferner Nukleinsäurekonstrukte, insbesondere als Expressionskassette fungierende Nukleinsäurekonstrukte, enthaltend eine Nukleinsäure codierend eine Tyrosinaminotransferase, die mit einem oder mehreren Regulationssignalen funktionell verknüpft ist, die die Transkription und Translation in Pflanzen gewährleisten.

Vorzugsweise enthalten die Regulationssignale einen oder mehrere Promotoren, die die Transkription und Translation in Pflanzen gewährleisten.

Die Expressionskassetten beinhalten Regulationssignale, also regulative Nukleinsäuresequenzen, welche die Expression der kodierenden Sequenz in der Wirtszelle steuern. Gemäß einer bevorzugten Ausführungsform umfaßt eine Expressionskassette stromaufwärts, d.h. am 5'-Ende der kodierenden Sequenz, einen Promotor und stromabwärts, d.h. am 3'-Ende, ein Polyadenylierungssignal und gegebenenfalls weitere regulatorische Elemente, welche mit der dazwischenliegenden kodierenden Sequenz für mindestens eines der vorstehend beschriebenen Gene operativ verknüpft sind. Unter einer operativen Verknüpfung versteht man die sequenzielle Anordnung von Promotor, kodierender Sequenz, Terminator und ggf. weiterer regulativer Elemente derart, daß jedes der regulativen Elemente seine Funktion bei der Expression der kodierenden Sequenz bestimmungsgemäß erfüllen kann.

Bei der Verwendung von Pflanzen als Organismus enthalten die erfindungsgemäßen Nukleinsäurekonstrukte und Expressionskassetten vorzugsweise eine Nukleinsäure kodierend ein plastidäres Transitpeptid, das die Lokalisation in Plastiden gewährleistet.

Im folgenden werden beispielhaft die bevorzugten Nukleinsäurekonstrukte, Expressionskassetten und Vektoren für Pflanzen und Verfahren zur Herstellung von transgenen Pflanzen, sowie die transgenen Pflanzen selbst beschrieben.

Die zur operativen Verknüpfung bevorzugten aber nicht darauf beschränkten Sequenzen sind Targeting-Sequenzen zur Gewährleistung der subzellulären Lokalisation im Apoplasten, in der Vakuole, in Plastiden, im Mitochondrium, im Endoplasmatischen Retikulum (ER), im Zellkern, in Ölkörperchen oder anderen Kompartimenten und Translationsverstärker wie die 5'-Führungssequenz aus dem Tabak-Mosaik-Virus (Gallie et al., Nucl. Acids Res. 15 (1987), 8693 -8711).

Als Promotoren der Expressionskassette ist grundsätzlich jeder Promotor geeignet, der die Expression von Fremdgenen in Pflanzen steuern kann. Vorzugsweise verwendet man insbesondere einen pflanzlichen Promotor oder einen Promotor, der einem Pflanzenvirus entstammt. Insbesondere bevorzugt ist der CaMV 35S-Promotor aus dem Blumenkohl-Mosaik-Virus (Franck et al., Cell 21 (1980), 285-294). Dieser Promotor enthält bekanntlich unterschiedliche Erkennungssequenzen für transkriptionale Effektoren, die in ihrer Gesamtheit zu einer permanenten und konstitutiven Expression des eingeführten Gens führen (Benfey et al., EMBO J. 8 (1989), 2195-2202).

Die Expressionskassette kann auch einen chemisch induzierbaren Promotor enthalten, durch den die Expression des Ziel-Gens in der Pflanze zu einem bestimmten Zeitpunkt gesteuert werden kann. Derartige Promotoren wie z.B. der PRP1-Promotor (Ward et al., Plant. Mol. Biol. 22 (1993), 361-366), ein durch Salicylsäure induzierbarer Promotor (WO 95/19443), ein durch Benzenesulfonamid-induzierbarer (EP-A 388186), ein durch Tetrazyklin-induzierbarer (Gatz et al., (1992) Plant J. 2, 397-404), ein durch Abscisinsäure-induzierbarer (EP-A335528) bzw.ein durch Ethanol-oder Cyclohexanon-induzierbarer (WO 93/21334) Promotor können beispielsweise verwendet werden.

Weiterhin sind insbesondere solche Promotoren bevorzugt, die die Expression in Geweben oder Pflanzenteilen sicherstellen, in denen beispielsweise die Biosynthese von Vitamin E bzw. dessen Vorstufen stattfindet. Insbesondere zu nennen sind Promotoren, die eine blattspezifische Expression gewährleisten. Zu nennen sind der Promotor der cytosolischen FBPase aus Kartoffel oder der ST-LSI Promotor aus Kartoffel (Stockhaus et al., EMBO J. 8 (1989), 2445-245).

Mit Hilfe eines samenspezifischen Promotors konnte ein Fremdprotein stabil bis zu einem Anteil von 0,67 % des gesamten löslichen Samenproteins in den Samen transgener Tabakpflanzen exprimiert werden (Fiedler und Conrad, Bio/Technology 10 (1995), 1090-1094). Die Expressionskassette kann daher beispielsweise einen samenspezifischen Promotor (bevorzugt den Phaseolin-Promotor (US 5504200), den USP-(Baumlein, H. et al., Mol. Gen. Genet. (1991) 225 (3), 459-467), LEB4-Promotor (Fiedler und Conrad, 1995),
Sucrose-Bindeprotein-Promotor (Zitat), das LEB4-Signalpeptid, das zu exprimierende Gen und ein ER-Retentionssignal enthalten.

Der Biosyntheseort von Vitamin E ist in Pflanzen unter anderem das Blattgewebe, so daß eine blattspezifische Expression der erfindungsgemäßen Nukleinsäuren kodierend eine Tyrosinaminotransferase sinnvoll ist. Dies ist jedoch nicht einschränkend, da die Expression auch in allen übrigen Teilen der Pflanze - besonders in fetthaltigen Samen - gewebespezifisch erfolgen kann.

Eine weitere bevorzugte Ausführungsform betrifft deshalb eine samenspezifische Expression der vorstehend beschriebenen Nukleinsäuren.

Darüberhinaus ist eine konstitutive Expression von exogenen Ziel-Genen von Vorteil. Andererseits kann aber auch eine induzierbare Expression wünschenswert erscheinen.

Die Wirksamkeit der Expression der transgen exprimierten Ziel-Gene kann beispielsweise *in vitro* durch Sproßmeristemvermehrung ermittelt werden. Zudem kann eine in Art und Höhe veränderte Expression des Ziel-Gens und deren Auswirkung auf die Vitamin E-Biosyntheseleistung an Testpflanzen in Gewächshausversuchen getestet werden.

Die Herstellung einer Expressionskassette erfolgt vorzugsweise durch Fusion eines geeigneten Promotors mit einer vorstehend beschriebenen Ziel-Nukleinsäure und vorzugsweise einer zwischen Promotor und Ziel-Nukleinsäure-Sequenz inserierten Nukleinsäure, die für ein chloroplastenspezifisches Transitpeptid kodiert, sowie einem Polyadenylierungssignal nach gängigen Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley-Interscience (1987) beschrieben sind.

Insbesondere bevorzugt sind insertierte Nukleinsäure-Sequenzen, die ein Targeting in den Plastiden gewährleisten.

Es können auch Expressionskassetten verwendet werden, deren Nukleinsäure-Sequenz für ein Ziel-Protein-Fusionsprotein kodiert, wobei ein Teil des Fusionsproteins ein Transitpeptid ist, das die Translokation des Polypeptides steuert. Bevorzugt sind für die Chloroplasten spezifische Transitpeptide, welche nach Translokation des Ziel-Proteins in die Chloroplasten vom Ziel-Protein-Teil enzymatisch abgespalten werden.

Insbesondere bevorzugt ist das Transitpeptid, das von der plastidären *Nicotiana tabacum* Transketolase oder einem anderen Transitpeptid (z.B. dem Transitpeptid der kleinen Untereinheit der Rubisco oder der Ferredoxin NADP Oxidoreduktase als auch der Isopentenylpyrophosphat Isomerase-2) oder dessen funktionellem Äquivalent abgeleitet ist.

Besonders bevorzugt sind Nukleinsäure-Sequenzen von drei Kassetten des Plastiden-Transitpeptids der plastidären Transketolase aus Tabak in drei Leserastern als KpnI/BamHI Fragmente mit einem ATG-Codon in der NcoI Schnittstelle:

Ein weiteres Beispiel für ein plastidäres Transitpeptid ist das Transitpeptid der plastidären Isopentenyl-pyrophosphat Isomerase-2 (IPP-2) aus Arabidopsis thaliana.

Die erfindungsgemäßen Nukleinsäuren können synthetisch hergestellt oder natürlich gewonnen sein oder eine Mischung aus synthetischen und natürlichen Nukleinsäure-Bestandteilen enthalten, sowie aus verschiedenen heterologen Genabschnitten verschiedener Organismen bestehen.

Bevorzugt sind, wie vorstehend beschrieben, synthetische Nukleotid-Sequenzen mit Kodons, die von Pflanzen bevorzugt werden. Diese von Pflanzen bevorzugten Kodons können aus Kodons mit der höchsten Proteinhäufigkeit bestimmt werden, die in den meisten interessanten Pflanzenspezies exprimiert werden.

Bei der Präparation einer Expressionskassette können verschiedene DNA-Fragmente manipuliert werden, um eine Nukleotid-Sequenz zu erhalten, die zweckmäßigerweise in der korrekten Richtung liest und die mit einem korrekten Leseraster ausgestattet ist. Für die Verbindung der DNA-Fragmente miteinander können an die Fragmente Adaptoren oder Linker angesetzt werden.

Zweckmäßigerweise können die Promotor- und die Terminator-Regionen in Transkriptionsrichtung mit einem Linker oder Polylinker, der eine oder mehrere Restriktionsstellen für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der Promotor kann sowohl nativ bzw. homolog als auch fremdartig bzw. heterolog zur Wirtspflanze sein. Die Expressionskassette beinhaltet vorzugsweise in der 5'-3'-Transkriptionsrichtung den Promotor, eine kodierende Nukleinsäuresequenz oder ein Nukleinsäurekonstrukt und eine Region für die transkriptionale Termination. Verschiedene Terminationsbereiche sind gegeneinander beliebig austauschbar.

Ferner können Manipulationen, die passende Restriktionsschnittstellen bereitstellen oder die überflüssige DNA oder Restriktionsschnittstellen entfernen, eingesetzt werden. Wo Insertionen, Deletionen oder Substitutionen wie z.B. Transitionen und Transversionen in Frage kommen, können *in vitro*-Mutagenese, "primerrepair", Restriktion oder Ligation verwendet werden.

Bei geeigneten Manipulationen, wie z.B. Restriktion, "chewing-back" oder Auffüllen von Überhängen für "bluntends", können komplementäre Enden der Fragmente für die Ligation zur Verfügung gestellt werden.

Bevorzugte Polyadenylierungssignale sind pflanzliche Polyadenylierungssignale, vorzugsweise solche, die im wesentlichen T-DNA-Polyadenylierungssignale aus Agrobacterium tumefaciens, insbesondere des Gens 3 der T-DNA (Octopin Synthase) des Ti-Plasmids pTiACH5 entsprechen (Gielen et al., EMBO J. 3 (1984), 835 ff) oder funktionelle Äquivalente.

Ferner betrifft die Erfindung die Verwendung der vorstehend beschriebenen Nukleinsäuren kodierend ein Tyrosinaminotransferase oder der vorstehend beschriebenen Nukleinsäurekonstrukte oder der Tyrosinaminotransferase zur Herstellung von transgenen Pflanzen.

Vorzugsweise weisen diese transgenen Pflanze gegenüber dem Wildtyp einen erhöhten Gehalt an Vitamin E auf.

Daher betrifft die Erfindung ferner die Verwendung der erfindungsgemäßen Nukleinsäuren oder der erfindungsgemäßen Nukleinsäurekonstrukte zur Erhöhung des Gehalts an Vitamin E in Pflanzen, die als Wildtyp in der Lage sind, Vitamin E zu produzieren.

Es ist bekannt, daß Pflanzen mit einem hohen Vitamin-E-Gehalt eine erhöhte Resistenz gegenüber abiotischem Streß aufweisen. Unter abiotischem Streß wird beispielsweise Kälte, Frost, Trockenheit, Hitze und Salz verstanden.

Daher betrifft die Erfindung weiterhin die Verwendung der erfindungsgemäßen Nukleinsäuren zur Herstellung transgener Pflanzen, die gegenüber dem Wildtyp eine erhöhte Resistenz gegenüber abiotischem Streß aufweisen.

Die vorstehend beschriebenen Proteine und Nukleinsäuren können zur Herstellung von Vitamin E in transgenen Pflanzen verwendet werden.

Die Übertragung von Fremdgenen in das Genom eines Organismus, insbesondere einer Pflanze wird als Transformation bezeichnet.

Dazu können insbesondere bei Pflanzen an sich bekannte Methoden zur Transformation und Regeneration von Pflanzen aus Pflanzengeweben oder Pflanzenzellen zur transienten oder stabilen Transformation genutzt werden.

Geeignete Methoden zur Transformation von Pflanzen sind die Protoplastentransformation durch Polyethylenglykol-induzierte DNA-Aufnahme, das biolistische Verfahren mit der Genkanone - die sogenannte particle bombardment Methode, die Elektroporation, die Inkubation trockener Embryonen in DNA-haltiger Lösung, die Mikroinjektion und der, vorstehend beschriebene, durch Agrobacterium vermittelte Gentransfer. Die genannten Verfahren sind beispielsweise in B. Jenes et al. , Techniques for Gene Transfer, in: Transgenic Plants, Vol. 1, Engineering and Utilization, herausgegeben von S.D. Kung und R. Wu, Academic Press (1993), 128-143 sowie in Potrykus, Annu. Rev. Plant Physiol. Plant Molec. Biol. 42 (1991), 205-225) beschrieben.

Vorzugsweise wird das zu exprimierende Konstrukt in einen Vektor kloniert, der geeignet ist, Agrobacterium tumefaciens zu transformieren, beispielsweise pBin19 (Bevan et al., Nucl. Acids Res. 12 (1984), 8711).

Dementsprechend betrifft die Erfindung weiterhin Vektoren enthaltend die vorstehend beschriebenen Nukleinsäuren, Nukleinsäurekonstrukte oder Expressionskassetten.

Mit einer Expressionskassette transformierte Agrobakterien können in bekannter Weise zur Transformation von Pflanzen verwendet werden, z.B. indem verwundete Blätter oder Blattstücke in einer Agrobakterienlösung gebadet und anschließend in geeigneten Medien kultiviert werden.

Die Expressionskassette kann über die Pflanzen hinaus auch zur Transformation von Bakterien, insbesondere Cyanobakterien, Moosen, Hefen, filamentösen Pilzen und Algen eingesetzt werden.

Zur bevorzugten Herstellung von genetisch veränderten Pflanzen, im folgenden auch transgene Pflanzen bezeichnet, wird die fusionierte Expressionskassette, die eine Tyrosinaminotransferase exprimiert, in einen Vektor, beispielsweise pBin19, kloniert, der geeignet ist, *Agrobacterium tumefaciens* zu transformieren.

Mit einem solchen Vektor transformierte Agrobakterien können dann in bekannter Weise zur Transformation von Pflanzen, insbesondere von Kulturpflanzen verwendet werden, indem beispielsweise verwundete Blätter oder Blattstücke in einer Agrobakterienlösung gebadet und anschließend in geeigneten Medien kultiviert werden.

Die Transformation von Pflanzen durch Agrobakterien ist unter anderem bekannt aus F.F. White, Vectors for Gene Transfer in Higher Plants; in Transgenic Plants, Vol. 1, Engineering and Utilization, herausgegeben von S.D. Kung und R. Wu, Academic Press, 1993, S. 15-38. Aus den transformierten Zellen der verwundeten Blätter bzw. Blattstücke können in bekannter Weise transgene Pflanzen regeneriert werden, die ein in die Expressionskassette integriertes Gen für die Expression einer Nukleinsäure codierend eine Tyrosinaminotransferase enthalten.

Zur Transformation einer Wirtspflanze mit einer für eine Tyrosinaminotransferase kodierenden Nukleinsäure wird eine Expressionskassette als Insertion in einen rekombinanten Vektor eingebaut, dessen Vektor-DNA zusätzliche funktionelle Regulationssignale, beispielsweise Sequenzen für Replikation oder Integration enthält. Geeignete Vektoren sind unter anderem in "Methods in Plant Molecular Biology and Biotechnology" (CRC Press), Kap. 6/7, S. 71-119 (1993) beschrieben.

Beispielhaft kann die pflanzliche Expressionskassette in ein Derivat des Transformationsvektors pBin-19 mit 35s Promotor (Bevan, M., Nucleic Acids Research 12: 8711-8721 (1984)) eingebaut werden.

Unter Verwendung der oben zitierten Rekombinations- und Klonierungstechniken können die Expressionskassetten in geeignete Vektoren kloniert werden, die ihre Vermehrung, beispielsweise in E. coli, ermöglichen. Geeignete Klonierungsvektoren sind u.a. pBR332, pUC-Serien, M13mp-Serien und pACYC184. Besonders geeignet sind binäre Vektoren, die sowohl in *E*. *coli* als auch in Agrobakterien replizieren können.

Die Erfindung betrifft daher ferner die Verwendung der vorstehend beschriebenen Nukleinsäuren, der vorstehend beschriebenen Nukleinsäurekonstrukte, insbesondere der Expressionskassetten zur Herstellung von genetisch veränderten Pflanzen oder zur Transformation von Pflanzen, -zellen, -geweben oder Pflanzenteilen.

Vorzugsweise ist Ziel der Verwendung die Erhöhung des Gehaltes der Pflanze oder Pflanzenteile an Vitamin E.

Dabei kann je nach Wahl des Promotors die Expression spezifisch in den Blättern, in den Samen, Blütenblättern oder anderen Teilen der Pflanze erfolgen.

Dementsprechend betrifft die Erfindung ferner ein Verfahren zur Herstellung von genetisch veränderten Pflanzen indem man eine vorstehend beschriebene Nukleinsäure oder ein vorstehend beschriebenes Nukleinsäurekonstrukt oder eine vorstehend beschriebene Kombination von Nukleinsäurekonstrukten in das Genom der Ausgangspflanze einführt.

Die Erfindung betrifft ferner die vorstehend beschriebenen genetisch veränderten Pflanzen selbst.

Wie vorstehend erwähnt, weisen die genetisch veränderten Pflanzen einen erhöhten Gehalt Vitamin E auf.

Die Erhöhung der Tyrosinamintransferase-Aktivität in der Pflanze führt zu einem weiteren Effekt. Es wird nicht nur der Gesamt-Vitamin E-Gehalt erhöht sondern es erfolgt zusätzlich eine selektive Erhöhung der Tocotrienole im Vergleich zu den Tocopherolen.

Als Organismen und zur Herstellung von Organismen mit einem erhöhten Gehalt an Vitamin E im Vergleich zum Wildtyp werden besonders bevorzugt Pflanzenals Ausgangsorganismen und dementsprechend auch als genetisch veränderte Organismen verwendet.

Solche transgenen Pflanzen, deren Vermehrungsgut, sowie deren Pflanzenzellen, -gewebe oder -teile sind ein weiterer Gegenstand der vorliegenden Erfindung.

Bevorzugte Pflanzen sind, wie vorstehend ausgeführt Tagetes, Sonnenblume, Arabidopsis, Tabak, Roter Pfeffer, Soja, Tomate, Aubergine, Paprika, Möhre, Karotte, Kartoffel, Mais, Salate und Kohlarten, Getreide, Alfalfa, Hafer, Gerste, Roggen, Weizen, Triticale, Hirse, Reis, Luzerne, Flachs, Baumwolle, Hanf, Brassicacaen wie beispielsweise Raps oder Canola, Zuckerrübe, Zuckerrohr, Nuß- und Weinspezies oder Holzgewächse wie beispielsweise Espe oder Eibe.

Besonders bevorzugt sind *Arabidopsis thaliana, Tagetes erecta, Brassica napus, Nicotiana tabacum,* Sonnenblume, Canola, Kartoffel oder Soja.

Die genetisch veränderten Pflanzen können, können wie vorstehend beschrieben zur Herstellung von Vitamin E verwendet werden.

Von Menschen und Tieren verzehrbare erfindungsgemäße, genetisch veränderte Pflanzen mit erhöhtem Gehalt an Vitamin-E können auch beispielsweise direkt oder nach an sich bekannter Prozessierung als Nahrungsmittel oder Futtermittel oder als Futter- und Nahrungsergänzungsmittel verwendet werden.

Die erfindungsgemäß, genetisch veränderten Pflanzen können ferner zur Herstellung von Vitamin E-haltigen Extrakten verwendet werden.

Erhöhung des Gehaltes an Vitamin E bedeutet im Rahmen der vorliegenden Erfindung vorzugsweise die künstlich erworbene Fähigkeit einer erhöhten Biosyntheseleistung dieser Verbindungen in der Pflanze gegenüber der nicht gentechnisch modifizierten Pflanze, vorzugsweise für die Dauer mindestens einer Pflanzengeneration.

Unter einem erhöhten Gehalt an Vitamin E wird in der Regel ein erhöhter Gehalt an Gesamt-Tocopherol verstanden. Unter einem erhöhten Gehalt an Vitamin E wird aber auch insbesondere ein veränderter Gehalt der vorstehend beschriebenen 8 Verbindungen mit Tocopherolaktivität verstanden.

Beispielsweise führt das Einbringen eines Tyrosinaminotransferase-Gens in Pflanzen überraschenderweise zu einem besonders erhöhten Anstieg des Gehalts an Tocotrienolen.

Die Erfindung wird durch die nun folgenden Beispiele erläutert, ist aber nicht auf diese beschränkt:

Allgemeine Experimentelle Bedingungen:
Sequenzanalyse rekombinanter DNA

Die Sequenzierung rekombinanter DNA-Moleküle erfolgte mit einem Laserfluoreszenz-DNA-Sequenzierer der Firma Licor (Vertrieb durch MWG Biotech, Ebersbach) nach der Methode von Sanger (Sanger et al., Proc. Natl. Acad. Sci. USA 74 (1977), 5463-5467).

### Beispiel 1

Klonierung des Tyrosinaminotransferase-Gens kodierend die Tyrosinaminotransferase aus *Rattus norvegicus.*

Die Präparation der RNA aus Rattenleber erfolgte in an sich bekannter Weise wie von S. Kar und BJ. Carr in Biochem. Biophys. Res. Commun. 1995, 212(1), 21-6 (*Differential display and cloning of messenger RNAs from the late phase of rat liver regeneration),* beschrieben.

Die cDNA Synthese wurde unter Verwendung des SuperScript II cDNA Synthese Kit (Gibco BRL) gemäß den Herstellerangaben durchgeführt.

Die Nukleinsäure kodierend eine Tyrosinaminotransferase wurde mittels *polymerase chain reaction* (PCR) aus *Rattus norvegicus* unter Verwendung eines sense spezifischen Primers (Tyrosinaminotransferase 5' SEQ.-ID Nr. 3) und eines antisense spezifischen Primers (Tyrosin-Aminotransferase 3' SEQ.-ID Nr. 4) amplifiziert.

### Die PCR Bedingungen waren die folgenden:

Die PCR erfolgte in einem 50µl Reaktionsansatz in dem enthalten war:
- 2 µl einer *Rattus norvegicus* cDNA (hergestellt wie oben beschrieben)
- 0,2 mM dATP, dTTP, dGTP, dCTP
- 1,5 mM Mg(OAc)₂
- 5 µg Rinderserum-Albumin
- 40 pmol Tyrosin-Aminotransferase 5'Primer
- 40 pmol Tyrosin-Aminotransferase 3'Primer
- 15 µl 3,3× rTth DNA Polymerase XLPuffer (PE Applied Biosystems)
- 5 U rTth DNA Polymerase XL (PE Applied Biosystems)

Die PCR wurde unter folgenden Zyklus Bedingungen durchgeführt:
Schritt 1: 5 Minuten 94°C (Denaturierung)
Schritt 2: 3 Sekunden 94°C
Schritt 3:1 Minute 55°C (Annealing)
Schritt 4: 2 Minuten 72°C (Elongation)
   30 Wiederholungen der Schritte 2-4
Schritt 5: 10 Minuten 72°C (Post-Elongation)
Schritt 6: 4°C (Warteschleife)
Das Amplikon wurde unter Verwendung von Standardmethoden in den PCR Klonierungsvektor pGEM-Te (Promega) kloniert. Die Identität des erzeugten Amplikons im Vektor pGEMTe/RnTATAse1 wurde durch Sequenzierung unter Verwendung des M13F (-40) Primers und des M13R Primers bestätigt (SEQ. ID. No. 1 und SEQ. ID. NO. 3).

### Beispiel 2

Klonierung des Tyrosin-Aminotransferase-Gens 1 kodierend für die Tyrosin-Aminotransferase 1 aus *Arabidospsis thaliana.*

Die DNA kodierend für das Tyrosin-Aminotransferase-Gen 1 wurde mittels polymerase chain reaction (PCR) aus *Arabidopsis thaliana* unter Verwendung eines sense spezifischen Primers (At1Tyrosin-Aminotransferase 5' SEQ. ID. No. 28) und eines antisense spezifischen Primers (At1Tyrosin-Aminotransferase 3' SEQ. ID. No. 29) amplifiziert.
Die PCR Bedingungen waren die folgenden:
Die PCR erfolgte in einem 50µl Reaktionsansatz in dem enthalten war:
- 2µl einer *Arabidopsis thaliana* cDNA
- 0,2 mM dATP, dTTP, dGTP, dCTP
- 1,5 mM Mg(OAc)₂
- 5µg Rinderserum-Albumin
- 40pmol At1Tyrosin-Aminotransferase 5'Primer
- 40pmol At1Tyrosin-Aminotransferase 3'Primer
- 15µl 3,3x rTth DNA Polymerase XLPuffer (PE Applied Biosystems)
- 5U rTth DNA Polymerase XL (PE Applied Biosystems)

Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 55°C (Annealing)
Schritt 4: 2 Minuten 72°C (Elongation)
   30 Wiederholungen der Schritte 2-4
Schritt 5: 10 Minuten 72°C (Post-Elongation)

Das Amplikon wurde unter Verwendung von Standardmethoden in den PCR Klonierungsvektor pGEM-Te (Promega) kloniert. Die Identität des erzeugten Amplikons im Vektor pGEMTe/AtTATAse1 wurde durch vollständige Sequenzierung unter Verwendung des M13F (-40) Primers und des M13R bestätigt (Seq. ID. No. 5).

### Beispiel 3

Klonierung des Tyrosin-Aminotransferase-Gens 3 kodierend für die Tyrosin-Aminotransferase 3 aus *Arabidopsis thaliana.*

Die DNA kodierend für das Tyrosin-Aminotransferase-Gen 3 wurde mittels polymerase chain reaction (PCR) aus *Arabidopsis thaliana* unter Verwendung eines sense spezifischen Primers (At3Tyrosin-Aminotransferase 5': SEQ. ID. No. 30) und eines antisense spezifischen Primers (At3Tyrosin-Aminotransferase 3': SEQ. ID. No. 31) amplifiziert.
Die PCR Bedingungen waren die folgenden:
Die PCR erfolgte in einem 50µl Reaktionsansatz in dem enthalten war:
- 2µl einer *Arabidopsis thaliana* cDNA
- 0,2 mM dATP, dTTP, dGTP, dCTP
- 1,5 mM Mg(OAc)₂
- 5µg Rinderserum-Albumin
- 40pmol At3Tyrosin-Aminotransferase 5'Primer
- 40pmol Ar3Tyrosin-Aminotransferase 3'Primer
- 15µl 3,3x rTth DNA Polymerase XLPuffer (PE Applied Biosystems)
- 5U rTth DNA Polymerase XL (PE Applied Biosystems)

Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 56°C (Annealing)
Schritt 4: 2 Minuten 72°C (Elongation)
   30 Wiederholungen der Schritte 2-4
Schritt 5: 10 Minuten 72°C (Post-Elongation)

Das Amplikon wurde unter Verwendung von Standardmethoden in den PCR Klonierungsvektor pGEM-Te (Promega) kloniert. Die Identität des erzeugten Amplikons im Vektor pGEMTe/AtTATAse3 wurde durch vollständige Sequenzierung unter Verwendung des M13F (-40) und des M13R Primers bestätigt (Seq. ID. Nr. 7).

### Beispiel 4

Klonierung des Tyrosin-Aminotransferase-Gens 5 kodierend für die Tyrosin-Aminotransferase 5 aus *Arabidopsis thaliana.*

Die DNA kodierend für das Tyrosin-Aminotransferase-Gen 5 wurde mittels polymerase chain reaction (PCR) aus *Arabidopsis thaliana* unter Verwendung eines sense spezifischen Primers (At5Tyrosin-Aminotransferase 5': SEQ. ID. No. 32) und eines antisense spezifischen Primers (At5Tyrosin-Aminotransferase 3': SEQ. ID. No. 33) amplifiziert.

Die PCR Bedingungen waren die folgenden:
Die PCR erfolgte in einem 50µl Reaktionsansatz in dem enthalten war:
- 2µl einer *Arabidopsis thaliana*. cDNA
- 0,2 mM dATP, dTTP, dGTP, dCTP
- 1,5 mM Mg(OAc)₂
- 5µg Rinderserum-Albumin
- 40pmol At5Tyrosin-Aminotransferase 5'Primer
- 40pmol At5Tyrosin-Aminotransferase 3'Primer
- 15µl 3,3x rTth DNA Polymerase XLPuffer (PE Applied Biosystems)
- 5U rTth DNA Polymerase XL (PE Applied Biosystems)

Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 56°C (Annealing)
Schritt 4: 2 Minuten 72°C (Elongation)
   30 Wiederholungen der Schritte 2-4
Schritt 5: 10 Minuten 72°C (Post-Elongation)

Das Amplikon wurde unter Verwendung von Standardmethoden in den PCR Klonierungsvektor pGEM-Te (Promega) kloniert. Die Identität des erzeugten Amplikons im Vektor pGEMTe/AtTATAse5 wurde durch vollständige Sequenzierung unter Verwendung des M13F (-40) und des M13R Primers bestätigt (Seq.. ID. No. 9).

### Beispiel 5

Klonierung des Tyrosin-Aminotransferase-Gens 6 kodierend für die Tyrosin-Aminotransferase 6 aus *Arabidopsis thaliana*.

Die DNA kodierend für das Tyrosin-Aminotransferase-Gen 6 wird mittels polymerase chain reaction (PCR) aus *Arabidopsis thaliana* unter Verwendung eines sense spezifischen Primers (At6Tyrosin-Aminotransferase 5': SEQ. ID. No. 34) und eines antisense spezifischen Primers (At6Tyrosin-Aminotransferase 3': SEQ. ID. No. 35) amplifiziert.
Die PCR Bedingungen waren die folgenden:
Die PCR erfolgte in einem 50µl Reaktionsansatz in dem enthalten war:
- 2µl einer *Arabidopsis thaliana* cDNA
- 0,2 mM dATP, dTTP, dGTP, dCTP
- 1,5 mM Mg(OAc)₂
- 5µg Rinderserum-Albumin
- 40pmol At6Tyrosin-Aminotransferase 5'Primer
- 40pmol Ar6Tyrosin-Aminotransferase 3'Primer
- 15µl 3,3x rTth DNA Polymerase XLPuffer (PE Applied Biosystems)
- 5U rTth DNA Polymerase XL (PE Applied Biosystems)

Die PCR wird unter folgenden Zyklus Bedingungen durchgeführt:
Schritt 1: 5 Minuten 94°C (Denaturierung)
Schritt 2: 3 Sekunden 94°C
Schritt 3: 1 Minute 56°C (Annealing)
Schritt 4: 2 Minuten 72°C (Elongation)
   30 Wiederholungen der Schritte 2-4
Schritt 5: 10 Minuten 72°C (Post-Elongation)

Das Amplikon wird unter Verwendung von Standardmethoden in den PCR Klonierungsvektor pGEM-Te (Promega) kloniert. Die Identität des erzeugten Amplikons im Vektor pGEMTe/AtTATAse6 wird durch vollständige Sequenzierung unter Verwendung des M13F (-40) und des M13R Primers bestätigt (Seq. ID. Nr. 11).

### Beispiel 6

Klonierung des Geranylgeranylpyrophosphate-Oxidoreduktase-Gens kodierend für die Geranylgeranylpyrophosphate-Oxidoreduktase aus *Nicotiana tabacum*

Die DNA kodierend für Geranylgeranylpyrophosphate-Oxidoreduktase -Gens wurde mittels polymerase chain reaction (PCR) aus *Nicotiana* tabacum unter Verwendung eines sense spezifischen Primers (Geranylgeranylpyrophosphate-Oxidoreduktase 5': SEQ. ID. NO. 36) und eines antisense spezifischen Primers (Geranylgeranylpyrophosphate-Oxidoreduktase 3': SEQ. ID. No. 37) amplifiziert.

Die PCR Bedingungen waren die folgenden:
Die PCR erfolgte in einem 50µl Reaktionsansatz in dem enthalten war:
- 2µl einer *Nicotiana tabacum* cDNA
- 0,2 mM dATP, dTTP, dGTP, dCTP
- 1,5 mM Mg(OAc)₂
- 5µg Rinderserum-Albumin
- 40pmol Geranylgeranylpyrophosphate-Oxidoreduktase 5'Primer
- 40pmol Geranylgeranylpyrophosphate-Oxidoreduktase 3'Primer
- 15µl 3,3x rTth- DNA Polymerase Puffer (PE Applied Biosystems)
- 5U rTth DNA Polymerase (PE Applied Biosystems)
Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 56°C (Annealing)
Schritt 4: 2 Minuten 72°C (Elongation)
   30 Wiederholungen der Schritte 2-4
Schritt 5: 10 Minuten 72°C (Post-Elongation)

Das Amplikon wurde unter Verwendung von Standardmethoden in den PCR Klonierungsvektor pGEMTe (Promega) kloniert. Die Identität des erzeugten Amplikons im Vektor pGEMTe/NtGGPPOR wurde durch vollständige Sequenzierung unter Verwendung des M13F (-40) und des M13R Primers bestätigt(Seq. ID. No. 17).

### Beispiel 7

Klonierung des Hydroxyphenylpyruvat-Dioxygenase-Gens kodierend für die Hydroxyphenylpyruvat-Dioxygenase aus *Arabidopsis thaliana*.

Die DNA kodierend für das Hydroxyphenylpyruvat-Dioxygenase-Gens wurde mittels polymerase chain reaction (PCR) aus *Arabidopsis thaliana* unter Verwendung eines sense spezifischen Primers (AtHydroxyphenylpyruvat-Dioxygenase 5': SEQ. ID. No. 38) und eines antisense spezifischen Primers (AtHydroxyphenylpyruvat-Dioxygenase 3': SEQ. ID. Nr. 39) amplifiziert.

### Die PCR Bedingungen waren die folgenden:

Die PCR erfolgte in einem 50µl Reaktionsansatz in dem enthalten war:
- 2µl einer *Arabidopsis thaliana* cDNA
- 0,2 mM dATP, dTTP, dGTP, dCTP
- 1,5 mM Mg(OAc)₂
- 5µg Rinderserum-Albumin
- 40pmol AtHydroxyphenylpyruvat-Dioxygenase 5'Primer
- 40pmol AtHydroxyphenylpyruvat-Dioxygenase 3'Primer
- 15µl 3,3x rTth DNA Polymerase XLPuffer (PE Applied Biosystems)
- 5U rTth DNA Polymerase XL (PE Applied Biosystems)

Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 58°C (Annealing)
Schritt 4: 2 Minuten 72°C (Elongation)
   30 Wiederholungen der Schritte 2-4
Schritt 5: 10 Minuten 72°C (Post-Elohgation)
Das Amplikon wurde unter Verwendung von Standardmethoden in den PCR Klonierungsvektor pGEM-Te (Promega) kloniert. Die Identität des erzeugten Amplikons im Vektor pGEMTe/AtHPPD wurde durch vollständige Sequenzierung unter Verwendung des M13F (-40) und des M13R Primers bestätigt (Seq. ID. No. 13).

### Beispiel 8

Klonierung des Homogentisat-Prenyltransferase-Gens kodierend für die Homogentisat-Prenyltransferase aus *Arabidopsis thaliana*.

Die DNA kodierend für das Homogentisinsäure-Prenyltransferase-Gens wurde mittels polymerase chain reaction (PCR) aus *Arabidopsis thaliana* unter Verwendung eines sense spezifischen Primers (AtHomogentisat-Prenyltransferase 5': SEQ. ID. Nr. 40) und eines antisense spezifischen Primers (AtHomogentisat-Prenyltransferase 3': SEQ. ID. No. 41) amplifiziert.

Die PCR Bedingungen waren die folgenden:
Die PCR erfolgte in einem 50µl Reaktionsansatz in dem enthalten war:
- 2µl einer *Arabidopsis thaliana* cDNA
- 0,2 mM dATP, dTTP, dGTP, dCTP
- 1,5 mM Mg(OAc)₂
- 5µg Rinderserum-Albumin
- 40pmol AtHomogentisinsäure-Prenyltransferase 5'Primer
- 40pmol AtHomogentisinsäure-Prenyltransferase 3'Primer
- 15µl 3,3x rTth DNA Polymerase XLPuffer (PE Applied Biosystems)
- 5U rTth DNA Polymerase XL (PE Applied Biosystems)

Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 58°C (Annealing)
Schritt 4: 2 Minuten 72°C (Elongation)
   30 Wiederholungen der Schritte 2-4
Schritt 5: 10 Minuten 72°C (Post-Elongation)

Das Amplikon wurde unter Verwendung von Standardmethoden in den PCR Klonierungsvektor pGEM-Te (Promega) kloniert. Die Identität des erzeugten Amplikons im Vektor pGEMTe/AtHPT wurde durch vollständige Sequenzierung unter Verwendung des M13F (-40) und des M13R Primers bestätigt (Seq. ID. No. 15).

### Beispiel 9

Klonierung des 2-Methyl-6-Phytylhydrochinon-Methyltransferase-Gens kodierend für die 2-Methyl-6-Phytylhydrochinon-Methyltransferase aus *Synechocystis* sp. PCC6803.

Die DNA kodierend für das 2-Methyl-6-Phytylhydrochinon-Methyltransferase-Gens wurde mittels polymerase chain reaction (PCR) aus *Synechocystis* sp. PCC6803 unter Verwendung eines sense spezifischen Primers (2-Methyl-6-Phytylhydrochinon-Methyltransferase 5': SEQ. ID. No. 42) und eines antisense spezifischen Primers (2-Methyl-6-Phytylhydrochinon-Methyltransferase 3': SEQ. ID. No. 43) amplifiziert.

### Die PCR Bedingungen waren die folgenden:

Die PCR erfolgte in einem 50µl Reaktionsansatz in dem enthalten war:
- 2µl einer *Synechocystis* sp. PCC6803 DNA
- 0,2 mM dATP, dTTP, dGTP, dCTP
- 1,5 mM Mg(OAc)₂
- 5µg Rinderserum-Albumin
- 40pmol 2-Methyl-6-Phytylhydrochinon-Methyltransferase 5'Primer
- 40pmol 2-Methyl-6-Phytylhydrochinon-Methyltransferase 3'Primer
- 15µl 3,3x rTth DNA Polymerase XLPuffer (PE Applied Biosystems)
- 5U rTth DNA Polymerase XL (PE Applied Biosystems)

Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 58°C (Annealing)
Schritt 4: 2 Minuten 72°C (Elongation)
   30 Wiederholungen der Schritte 2-4
Schritt 5: 10 Minuten 72°C (Post-Elongation)

Das Amplikon wurde unter Verwendung von Standardmethoden in den PCR Klonierungsvektor pGEM-Te (Promega) kloniert. Die Identität des erzeugten Amplikons im Vektor pGEMTe/SynMT1 wurde durch vollständige Sequenzierung unter Verwendung des M13F (-40) und des M13R Primers bestätigt (Seq ID. No. 19).

### Beispiel 10

Klonierung des Tocopherolcyclase-Gens (auch 2,3-Dimethyl-5-Phytylplastochinol-Zyklase-Gen genannt) kodierend für die Tocopherolcyclase (auch 2,3-Dimethyl-5-Phytylplastochinol-Zyklase genannt) aus *Synechocystis* sp. PCC6803.

Die DNA kodierend für 2,3-Dimethyl-5-Phytylplastochinol-Zyklase-Gens wurde mittels polymerase chain reaction (PCR) aus *Synechocystis* sp. PCC6803 unter Verwendung eines sense spezifischen Primers (2,3-Dimethyl-5-Phytylplastochinol-Zyklase 5': SEQ. ID. No. 44) und eines antisense spezifischen Primers (2,3-Dimethyl-5-Phytylplastochinol-Zyklase 3': SEQ.-ID Nr. 45) amplifiziert.

Die PCR Bedingungen waren die folgenden:
Die PCR erfolgte in einem 50µl Reaktionsansatz in dem enthalten war:
- 2µl einer *Synechocystis* sp. PCC6803 DNA
- 0,2 mM dATP, dTTP, dGTP, dCTP
- 1,5 mM Mg(OAc)₂
- 5µg Rinderserum-Albumin
- 40pmol 2,3-Dimethyl-5-Phytylplastochinol-Zyklase 5'Primer
- 40pmol 2,3-Dimethyl-5-Phytylplastochinol-Zyklase 3'Primer
- 15µl 10 x Pfu1-Turbo DNA Polymerase Puffer (Stratagene)
- 5U Pfu1-Turbo DNA Polymerase (Stratagene)

Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 60°C (Annealing)
Schritt 4: 1,5 Minuten 72°C (Elongation)
   30 Wiederholungen der Schritte 2-4
Schritt 5: 10 Minuten 72°C (Post-Elongation)

Das Amplikon wurde unter Verwendung von Standardmethoden in den PCR Klonierungsvektor pCRTopo4blunt (Invitrogen) kloniert. Die Identität des erzeugten Amplikons imd Vektor pCR4topoblunt/SynCyc wurde durch vollständige Sequenzierung unter Verwendung des M13F (-20) und des M13R Primers bestätigt (Seq. ID. No. 21).

### Beispiel 11

Klonierung des γ-Tocopherol-Methyltransferase-Gens kodierend für die γ-Tocopherol-Methyltransferase aus *Arabidopsis thaliana.*

Die DNA kodierend für das γ-Tocopherol-Methyltransferase-Gens wurde mittels polymerase chain reaction (PCR) aus *Arabidopsis thaliana* unter Verwendung eines sense spezifischen Primers (Atγ-Tocopherol-Methyltransferase 5': SEQ. ID. No. 46) und eines antisense spezifischen Primers (Atγ-Tocopherol-Methyltransferase 3': SEQ. ID. No. 47) amplifiziert.

Die PCR Bedingungen waren die folgenden:
Die PCR erfolgte in einem 50µl Reaktionsansatz in dem enthalten war:
- 2µl einer *Arabidopsis thaliana* cDNA
- 0,2 mM dATP, dTTP, dGTP, dCTP
- 1,5 mM Mg(OAc)₂
- 5µg Rinderserum-Albumin
- 40pmol Atγ-Tocopherol-Methyltransferase 5'Primer
- 40pmol Atγ-Tocopherol-Methyltransferase 3'Primer
- 15µl 3,3x rTth DNA Polymerase XLPuffer (PE Applied Biosystems)
- 5U rTth DNA Polymerase XL (PE Applied Biosystems)

Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 58°C (Annealing)
Schritt 4: 2 Minuten 72°C (Elongation)
   30 Wiederholungen der Schritte 2-4
Schritt 5: 10 Minuten 72°C (Post-Elongation)

Das Amplikon wurde unter Verwendung von Standardmethoden in den PCR Klonierungsvektor pGEM-Te (Promega) kloniert. Die Identität des erzeugten Amplikons im Vektor pGEMTe/AtγTMT wurde durch vollständige Sequenzierung unter Verwendung des M13F (-40) und des M13R Primers bestätigt (Seq. ID. Nr. 23).

### Beispiel 12

Klonierung eines Teilfragmentes des Homogentisinsäure-Dioxygenase-Gens kodierend für die Homogentisinsäure-Dioxygenase aus *Brassica napus.*

Die DNA kodierend für ein Teilfragmentes das Homogentisinsäure-Dioxygenase-Gens wurde mittels polymerase chain reaction (PCR) aus *Brassica napus* unter Verwendung eines sense spezifischen Primers (Homogentisinsäure-Dioxygenase 5': SEQ. ID. No. 48) und eines antisense spezifischen Primers (Homogentisinsäure-Dioxygenase 3': SEQ. ID. No. 49) amplifiziert.

Die PCR Bedingungen waren die folgenden:
Die PCR erfolgte in einem 50µl Reaktionsansatz in dem enthalten war:
- 2µl einer *Brassica napus* cDNA
- 0,2 mM dATP, dTTP, dGTP, dCTP
- 1,5 mM Mg(OAc)₂
- 5µg Rinderserum-Albumin
- 40pmol Homogentisinsäure-Dioxygenase 5'Primer
- 40pmol Homogentisinsäure-Dioxygenase 3'Primer
- 15µl 3,3x rTth- DNA Polymerase Puffer (PE Applied Biosystems)
- 5U rTth DNA Polymerase (PE Applied Biosystems)

Die PCR wurde unter folgenden Zyklus-Bedingungen durchgeführt:
Schritt 1: 5 Minuten 94°C (Denaturierung)
Schritt 2: 3 Sekunden 94°C
Schritt 3: 1 Minute 56°C (Annealing)
Schritt 4: 2 Minuten 72°C (Elongation)
   30 Wiederholungen der Schritte 2-4
Schritt 5: 10 Minuten 72°C (Post-Elongation)

Das Amplikon wurde unter Verwendung von Standardmethoden in den PCR Klonierungsvektor pGEMTe (Promega) kloniert. Die Identität des erzeugten Amplikons im Vektor pGEMTe/*BnHGD wurde durch vollständige Sequenzierung unter Verwendung des M13F (-40) und des M13R Primers bestätigt (Seq. ID. No. 25).

### Beispiel 13

Erzeugung des DNA Konstruktes zur samenspezifischen Unterdrückung der Expression des Homogentisat-Dioxygenase-Gens aus *Brassica napus*.

Zur Herstellung von chimären DNA Konstrukten zur Erzeugung transgener *Brassica napus* Pflanzen, die eine reduzierte Expression des Homogentisat-Dioxygenase Gens aus *Brassica napus* unter Kontrolle eines samenspezifischen Promotors exprimieren, wurde der Vektor pSUN2 (WO 02/00900) verwendet. Dieser Vektor wurde so verändert, dass er den samenspezifichen Promotor des Vicilin Gens aus *Vicia faba* (Weschke W., Bassüner R., van Hai N., Czihal A.., Bäumlein H., Wobus U. The structure of a Vicia faba Vicilin Gene. Biochem.Physiol.Pflanzen 183,233-242 (1988)), und das 2 Intron (IV2) des ST-LS1 Gens aus *Solanum tuberosum*. (Vancanneyt G., Schmidt R., O'Connor-Sanchez A., Willmitzer L., Rocha-Sosa M. Construction of an intron-containing marker gene: Splicing of the intron in transgenic plants and its use in monitoring early events in Agrobacterium-mediated plant transformation. MGG (1990)), und das Terminationssignal-2 des Octopin-Synthase Gens aus *Agrobakterium tumifaciens* (Gielen et al. 1984) enthält.

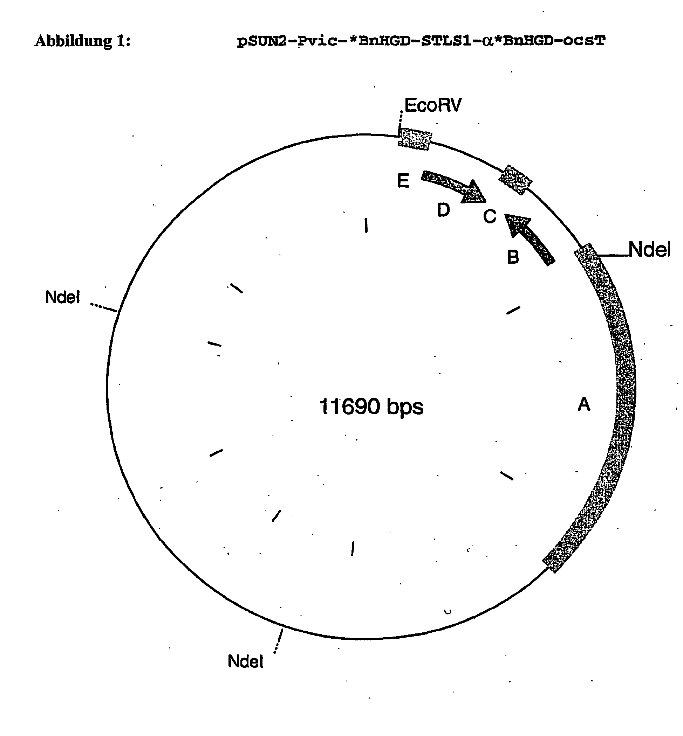

Das DNA Fragment kodierend für das Teilfragment des Homogentisinsäure-Dioxygenase-Gen aus *Brassica napus* wurde als SacI/ScaI Fragment aus dem Plasmid pGEMTe/*BnHGD in den mit Sma1 geöffneten pSUN2-Pvic-STLS1-ocsT kloniert, nachdem die überstehenden Enden des Fragmentes mit der T4 Polymerase in glatte Enden überführt wurden. Das resultierende Plasmid pSUN2-Pvic-*BnHGD-STLS1-ocsT wurde mit ScaI verdaut. In diesen lineariserten Vektor wurde erneut das Fragment des Homogentisinsäure-Dioxygenase-Gen aus *Brassica napus* als geglättetes SacI/ScaI Fragment aus dem Plasmid pGEMTe/*BnHGD kloniert. Dabei wurde darauf geachtet, dass die beiden BnHGD-Fragmente in gegenläufiger Orientierung auf beiden Seiten des STLS1 Introns vorhanden sind. Dieses Plasmid (pSUN2-Pvic-*BnHGD-STLS1-α*BnHGD-ocsT (Abbildung 1) wird zur Erzeugung transgener *Brassica napus* bzw. *A.thaliana* Pflanzen verwendet.

Fragment A (2559 Bp) in Abbildung 1 beinhaltet den Promotor des Vicilin Gens aus Vicia faba, Fragment B (580 Bp) kodiert für einen Fragment des Homogentisinsäure Dioxygenase Gen aus *Brassica napus*. Fragment C (190Bp) kodiert für das 2 Intron (IV2) des ST-LS1 Gens aus *Solanum tuberosum.* Fragment D ist identisch mit Fragment B jedoch im Vektor gegenläufig zu B orientiert. Fragment E (208Bp) kodiert für das Terminationssignal-2 des Octopin-Gens.

### Beispiel 14

Erzeugung von DNA Konstrukten zur Expression der Tyrosin-Aminotransferase aus *Rattus norvegicus* unter Kontrolle eines samenspezifischen Promotors.

Zur Herstellung von chimären DNA Konstrukten zur Erzeugung transgener *A.thaliana, Nicotiana tabacum bzw. Brassica napus* Pflanzen, die die Tyrosin-Aminotransferase aus *Rattus norvegicus* unter Kontrolle eines samenspezifischen Promotors exprimieren, wurde der Vektor pSUN2 (Patent WO 02/00900) verwendet. Dieser Vektor wurde so verändert, dass er den samenspezifichen Promotor des *Vicia faba* Unknown-Seed-Protein-Gens (USPP) (Bäumlein H., Boerjan W., Nagy I., Bassüner R., van Montagu M., Inzé D., Wobus U. A novel seed protein geen from Vicia faba is developmentally regulated in transgenic tobacco and Arabidopsis plants. MGG 225:459-467 (1991) ), die Sequenz kodierend für das Chloroplasten-Transitpeptid des *Vicia faba* Ribulose-Bisphosphat-Carboxylase *(rbcS) Gens* (Guerineau F., Woolston S., Brooks L., Mullineaux P. An expression cassette for targeting foreign proteins into chloroplasts. Nucleic Acids Res 16(23): 11380. (1988)) und das Terminationssignal des Nopalin-Synthase Gens aus *A.tumefaciens* (Depicker A, Stachel S, Dhaese P, Zambryski P, Goodman HM.Nopaline synthase: transcript mapping and DNA sequence.J Mol Appl Genet. 1982;1(6):561-73.) enthält.

Das DNA Fragment kodierend für das Tyrosin-Aminotransferäse-Gen aus *Rattus norvegicus* wurde als EcoR5 Fragment aus dem Plasmid pGEMTe/RnTATase in den pSUN2-USPP-rbcS-nosT kloniert, nachdem dieser mit dem Restriktionsenzym Sma1 verdaut wurde. Dadurch wurde eine Translationsfusion mit dem Transitpeptid der Ribulose-Bisphosphat-Carboxylase *(rbcs)* erzeugt und somit ein Import der Tyrosin-Aminotransferase aus *Rattus norvegicus* in die Plastiden gewährleistet.

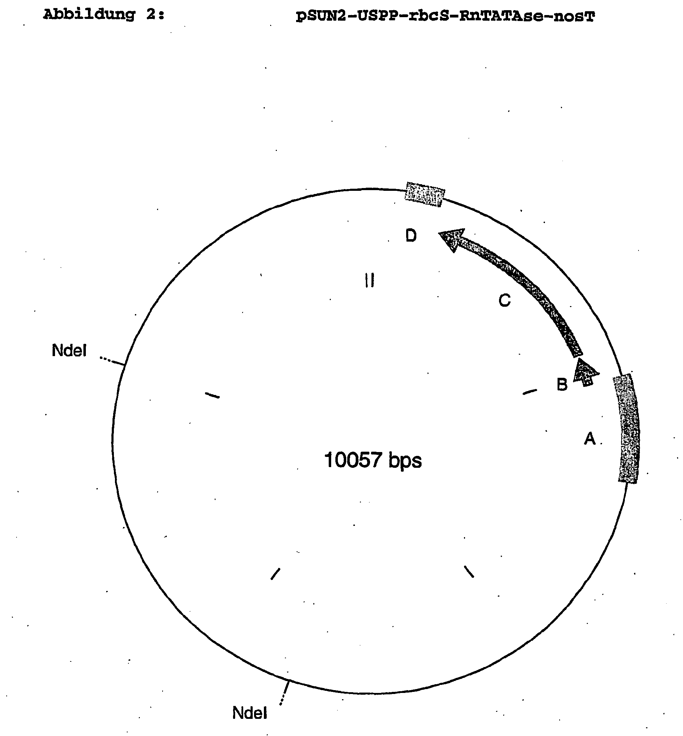

Dieses Plasmid (pSUN2USPP-rbcS-RnTATase-nosT, Abbildung 2) wird zur Erzeugung transgener *Brassica napus* bzw. *A.thaliana* Pflanzen verwendet.

Fragment A (678Bp) in Abbildung 2 beinhaltet den Promotor des Unknow-Seed-Protein-Gens (USPP) aus Vicia faba, Fragment B (235Bp) kodiert für das Transitpeptid der Ribulose-Bisphosphat-Carboxylase *(rbcS) aus Vicia faba*. Fragment C (1365 Bp) kodiert für das Tyrosin-Aminotransferase-Gen aus *Rattus norvegicus*. Fragment D (272Bp) kodiert für das Terminationssignal des Nopalin-Synthase Gens aus *A.tumefaciens*.

### Beispiel 15

Erzeugung von DNA Konstrukten zur Expression der Tyrosin-Aminotransferase 1 aus *Arabidopsis thaliana* unter Kontrolle eines samenspezifischen Promotors.

Zur Herstellung von chimären DNA Konstrukten zur Erzeugung transgener *Brassica napus* Pflanzen, die die Tyrosin-Aminotransferase aus *Arabidopsis thaliana* unter Kontrolle eines samenspezifischen Promotors exprimieren, wurde der Vektor pSUN2 (WO 02/00900) verwendet.

Dieser Vektor wurde so verändert, dass er den samenspezifichen Promotor des Vicia faba "Unknown-Seed-Protein" (USPP) (Bäumlein et al., 1991) und das Terminationssignal des Nopalin-Synthase Gens aus Agrobakterium tumifaciens (GIELEN, J., de BEUCKELEER, M., SEURINCK, J., DEBROECK, H., de GREVE, H., LEMMERS, M., van MONTAGU, M., SCHELL, J. The complete nucleotide sequence of the TL-DNA of the Agrobacterium tumefaciens plasmid pTiAch5. EMBO J. 3: 835-846. (1984)) enthält.

Das DNA Fragment kodierend für das Tyrosin-Aminotransferase-Gen 1 aus *Arabidopsis thaliana* wurde aus dem Plasmid pGEMTe/AtTATase1 als Sal1 Fragment isoliert, und nachdem die Sal1 Ende mit dem Klenow Enzym aufgefüllt wurde, in den pSUN2-USPP-nosT kloniert, nachdem dieser mit dem Restriktionsenzym Sma1 parcial verdaut wurde (Grösse des linearisierten Vektors 8250Bp).

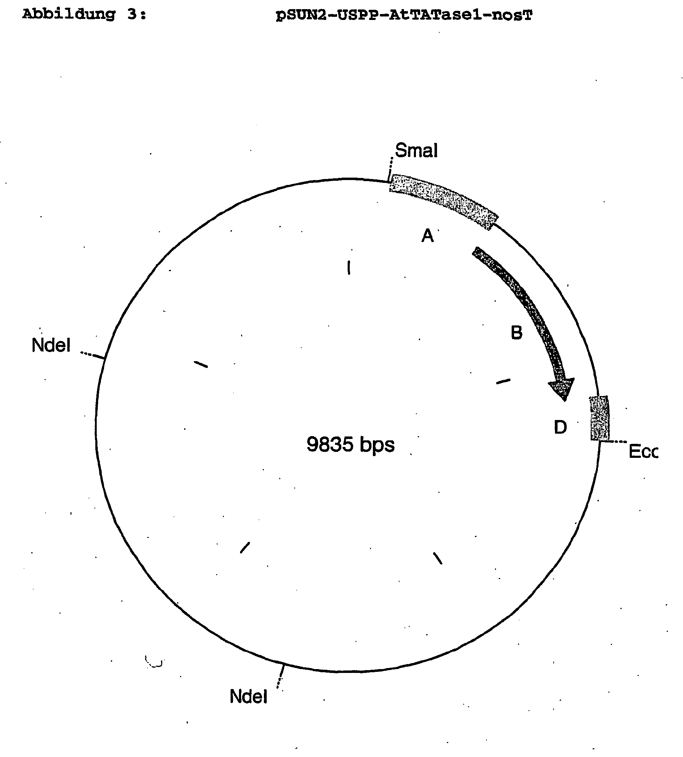

Dieses Plasmid (pSUN2-USPP-AtTATase1-nosT, Abbildung3) wird zur Erzeugung transgener *Brassica napus* Pflanzen verwendet.
Fragment A (678Bp) in Abbildung 3 beinhaltet den Promotor des Unknow-Seed-Protein-Gens (USPP)aus Vicia faba, Fragment B (1269 Bp) kodiert für das Tyrosin-Aminotransferase-Gen 1 aus *Arabidopsis thaliana* und Fragment C (272Bp) kodiert für das Terminationssigna1 des Nopalin-Synthase Gens.

### Beispiel 16

Erzeugung von DNA Konstrukten zur Expression der Tyrosin-Aminotransferase 3 aus *Arabidopsis thaliana* unter Kontrolle eines samenspezifischen Promotors.

Zur Herstellung von chimären DNA Konstrukten zur Erzeugung transgener *Brassica napus* Pflanzen, die die Tyrosin-Aminotransferase 3 aus *Arabidopsis thaliana* unter Kontrolle eines samenspezifischen Promotors exprimieren, wurde der Vektor pSUN2 (WO 02/00900) verwendet.

Dieser Vektor wurde so verändert, dass er den samenspezifichen Promotor des Vicia faba "Unknown-Seed-Protein" (USPP) (Bäumlein et al., 1991) und das Terminationssignal des Nopalin-Synthase Gens aus Agrobakterium (Gielen et al. 1984) enthält.

Das DNA Fragment kodierend für das Tyrosin-Aminotransferase-Gen 3 aus *Arabidopsis thaliana* wurde aus dem Plasmid pGEMTe/AtTATase3 als Sal1 Fragment isoliert, und nachdem das Sal1 Ende mit dem Klenow Enzym aufgefüllt wurde, in den pSUN2-USPP-nosT kloniert, nachdem dieser mit dem Restriktionsenzym Sma1 parcial verdaut wurde(Grösse des linearisierten Vektors 8250Bp).

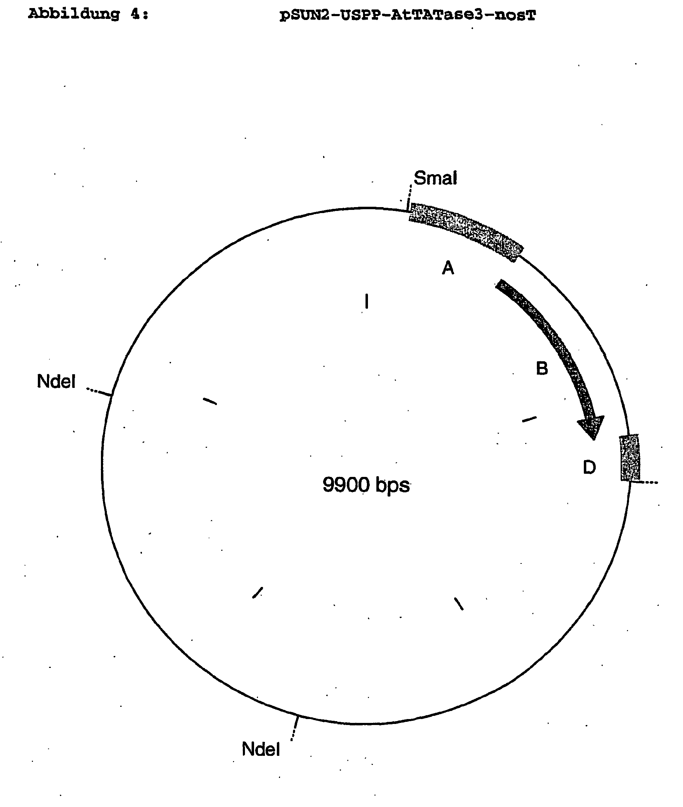

Dieses Plasmid (pSUN2USPP-AtTATase3-nosT, Abbildung 4) wird zur Erzeugung transgener *Brassica napus* Pflanzen verwendet.

Fragment A (678 Bp) in Abbildung 4 beinhaltet den Promotor des "Unknow-Seed-Protein-Gens" (USPP) aus Vicia faba, Fragment B (1334 Bp) kodiert für das Tyrosin-Aminotransferase-Gen 3 aus *Arabidopsis thaliana* und Fragment C (272Bp) kodiert für das Terminationssignal des Nopalin-Synthase Gens.

### Beispiel 17

Erzeugung von DNA Konstrukten zur Expression der Tyrosin-Aminotransferase 5 aus *Arabidopsis thaliana* unter Kontrolle eines samenspezifischen Promotors.

Zur Herstellung von chimären DNA Konstrukten zur Erzeugung transgener *Brassica napus* Pflanzen, die die Tyrosin-Aminotransferase 5 aus *Arabidopsis thaliana* unter Kontrolle eines samenspezifischen Promotors exprimieren, wurde der Vektor pSUN2 (WO 02/00900) verwendet.

Dieser Vektor wurde so verändert, dass er den samenspezifichen Promotor des Vicia faba "Unknown-Seed-Protein" (USPP) (Bäumlein et al., 1991) und das Terminationssignal des Nopalin-Synthase Gens aus Agrobakterium (Gielen et al. 1984) enthält.

Das DNA Fragment kodierend für das Tyrosin-Aminotransferase-Gen 5 aus *Arabidopsis thaliana* wurde aus dem Plasmid pGEMTe/AtTATase5 als BamH1 Fragment isoliert, und nachdem das BamH1 Ende mit dem Klenow Enzym aufgefüllt wurde, in den pSUN2-USPP-nosT kloniert, nachdem dieser mit dem Restriktionsenzym Sma1 parcial verdaut wurde (Grösse des linearisierten Vektors 8250Bp).

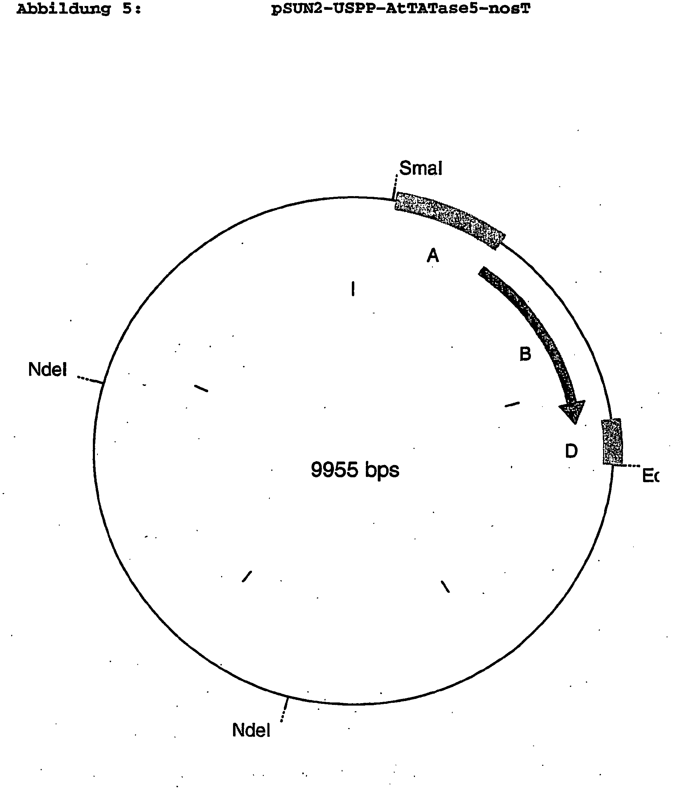

Dieses Plasmid (pSUN2-USPP-AtTATase5-nosT, Abbildung5) wird zur Erzeugung transgener *Brassica napus* Pflanzen verwendet.

Fragment A (678 Bp) in Abbildung 5 beinhaltet den Promotor des "Unknow-Seed-Protein-Gens" aus Vicia faba, Fragment B (1389 Bp) kodiert für das Tyrosin-Aminotransferase-Gen 5 aus *Arabidopsis thaliana* und Fragment C (272Bp) kodiert für das Terminationssignal des Nopalin-Synthase Gens.

### Beispiel 18

Erzeugung von DNA Konstrukten zur Expression der Tyrosin-Aminotransferase 6 aus *Arabidopsis thaliana* unter Kontrolle eines samenspezifischen Promotors.

Zur Herstellung von chimären DNA Konstrukten zur Erzeugung transgener *Brassica napus* Pflanzen, die die Tyrosin-Aminotransferase 6 aus *Arabidopsis thaliana* unter Kontrolle eines samenspezifischen Promotors exprimieren, wird der Vektor pSUN2 (WO 02/00900) verwendet.

Dieser Vektor wird so verändert, dass er den samenspezifichen Promotor des Vicia faba "Unknown-Seed-Protein-Gens" (USPP) (Bäumlein et al., 1991) und das Terminationssignal des Nopalin-Synthase Gens aus Agrobakterium (Gielen et al. 1984) enthält. Das DNA Fragment kodierend für das Tyrosin-Aminotransferase-Gen 6 aus *Arabidopsis thaliana* wird aus dem Plasmid pGEMTe/AtTATase5 als Sal1 Fragment isoliert, die Sal1 Ende werden mit dem Klenow Enzym aufgefüllt, in den pSUN2-USPP-nosT kloniert, der mit dem Restriktionsenzym Sma1 parcial wird (Grösse des linearisierten Vektors 8250Bp).

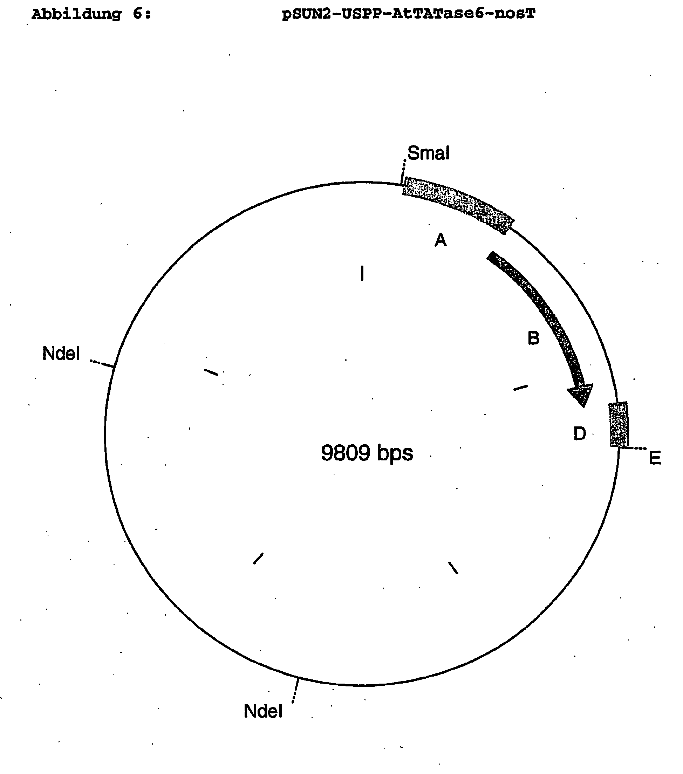

Dieses Plasmid (pSUN2-USPP-AtTATase6-nosT, Abbildung6) wird zur Erzeugung transgener *Brassica napus* Pflanzen verwendet.

Fragment A (678 Bp) in Abbildung 6 beinhaltet den Promotor des "Unknow-Seed-Protein-Gens" aus Vicia faba, Fragment B (1243 Bp) kodiert für das Tyrosin-Aminotransferase-Gen 6 aus *Arabidopsis thaliana* und Fragment C (272Bp) kodiert für das Terminationssignal des Nopalin-Synthase-Gens.

### Beispiel 19

Erzeugung von DNA Konstrukten zur Expression der Geranylgeranylpyrophosphate-Oxidoreduktase aus *Nicotiana tabacum* unter Kontrolle eines samenspezifischen Promotors.

Zur Herstellung von chimären DNA Konstrukten zur Erzeugung transgener *Brassica napus* Pflanzen, die die Geranylgeranylpyrophosphate-Oxidoreduktase aus *Nicotianum tabacum* unter Kontrolle eines samenspezifischen Promotors exprimieren, wurde der Vektor pSUN2 (WO 02/00900) verwendet.

Zuerst wurde der Vektor puc19 (New England Biolabs) so verändert, das er den samenspezifichen Promotor des Legumin B4 Gens (Kafatos et al., 1986), und das Terminationssignal der Nopalin-Synthase aus *A.tumefaciens* (Depicker et al., 1982) enthält. Der resultierende Vektor heißt puc19-LeB4-nosT.

Das DNA Fragment kodierend für das Geranylgeranylpyrophosphate-Oxidoreduktase-Gen aus *Nicotiana tabacum* wurde als KpnI/Sal1 Fragment in puc19LeB4nosT kloniert, nachdem dieser mit den Restriktionsenzymen KpnI/Sal1 verdaut wurde.

Aus dem Vektor puc19-LeB4-NtGGPPOR-nosT, wurde die DNA bestehend aus LeB4-Promotor, Geranylgeranylpyrophosphate-Oxidoreduktase-Gen (Nucleotid 1 bis 1323 von Seq. ID 7) als Sma1/Hind3 Fragment isoliert und in den Vektor pSUN2 kloniert, nachdem dieser mit dem Restriktionsenzym Sma1/Hind3 verdaut wurde. Der daraus resultierende Vektor heißt pSUN2-LeB4-NtGGPPOR(nuc.1-1323). Aus dem Vektor puc19-LeB4-NtGGPPOR-nosT, wurde die DNA bestehend aus Geranylgeranylpyrophosphate-Oxidoreduktase-Gen (Nucleotid 1319 bis 1509 von Seq. ID. No 17), nos-Terminationssequenz als Hind3 Fragement isoliert und in den Vektor pSUN2-LeB4-NtGGPPOR(nuc.1-1323) eingefügt, nachdem dieser ebenfalls mit Hind3 geschnitten wurde.

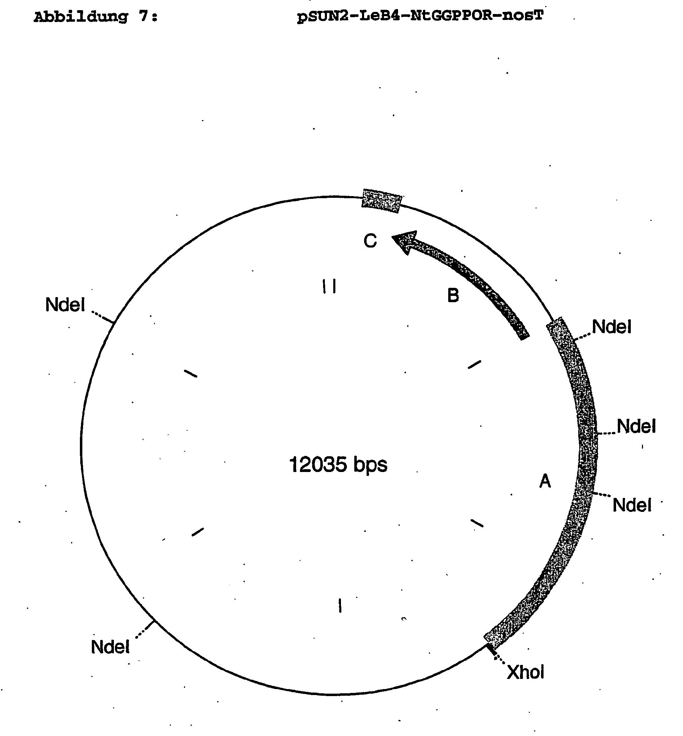

Dieses Plasmid (pSUN2-LeB4-NtGGPPOR-nosT, Abbildung7) wird zur Erzeugung transgener *Brassica napus* Pflanzen verwendet. Fragment A (2764 Bp) in Abbildung 7 beinhaltet den Promotor des LeguminB4-Gens aus Vicia faba, Fragment B (1509Bp) kodiert für das Geranylgeranylpyrophosphate-Oxidoreduktase-Gen aus *Nicotiana* tabacum und Fragment C kodiert (272Bp) für das Terminationssignal des Nopalin-Synthase-Gens.

### Beispiel 20

Erzeugung von DNA Konstrukten zur Expression der Hydroxyphenylpyruvate-dioxygenase aus *Arabidopsis thaliana* unter Kontrolle eines samenspezifischen Promotors.

Zur Herstellung von chimären DNA Konstrukten zur Erzeugung transgener *Brassica napus* Pflanzen, die die Hydroxyphenylpyruvate-dioxygenase aus *Arabidopsis thaliana* unter Kontrolle eines samenspezifischen Promotors exprimieren, wird der Vektor pSUN2 (WO 02/00900) verwendet.

Dieser Vektor wird so verändert, dass er den samenspezifichen Promotor des *Vicia faba* "Unknown-Seed-Protein-Gens" (USPP)(Bäumlein et al., 1988) und das Terminationssignal-1 des Octopin-Synthase-Gens aus *A.tumefaciens* (Depicker et al., 1982) enthält.

Das DNA Fragment kodierend für das Hydroxyphenylpyruvate-dioxygenase aus *Arabidopsis thaliana* wird aus dem Plasmid pGEMTe/AtHPPD als BamH1/Sal1 Fragment isoliert und nachdem das BamH1 Ende und Sal1 Ende mit dem Klenow Enzym aufgefüllt werden, in den mit dem Restriktionsenzym Sma1 parcial verdauten Vektor pSUN2-USPP-ocsT kloniert (Grösse des linearisierten Vektors 8691Bp).

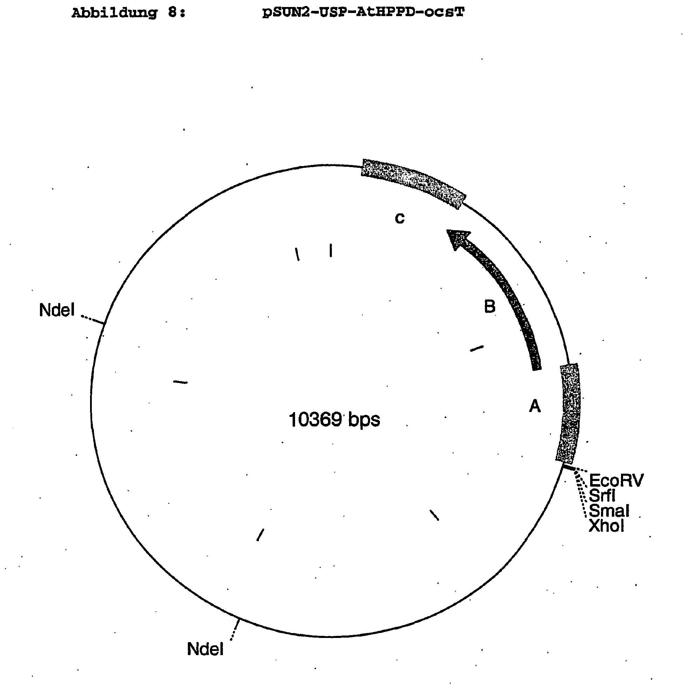

Dieses Plasmid (pSUN2-USPP-AtHPPD-ocsT, Abbildung 8) wird zur Erzeugung transgener *Brassica napus* Pflanzen verwendet.

Fragment A (678 Bp) in Abbildung 8 beinhaltet den Promotor des "Unknow-Seed-Protein-Gens" aus Vicia faba, Fragment B (1338 Bp) kodiert für das Hydroxyphenylpyruvate-Dioxygenase-Gen aus Arabi*dopsis thaliana* und Fragment C (713Bp) kodiert für das Terminationssignal-1 des Octopin-Synthase Gens.

### Beispiel 21

Erzeugung von DNA Konstrukten zur Expression der Homogentisinsäure-Phytyltransferase aus *Arabidopsis thaliana* unter Kontrolle eines samenspezifischen Promotors.

Zur Herstellung von chimären DNA Konstrukten zur Erzeugung transgener *Brassica napus* Pflanzen, die die Homogentisinsäure-Phytyltransferase aus *Arabidopsis thaliana* unter Kontrolle eines samenspezifischen Promotors exprimieren, wird der Vektor pSUN2 (Wo 02/00900) verwendet.

Dieser Vektor wird so verändert, dass er den samenspezifichen Promotor des Vicia faba "Unknown-Seed-Protein-Gens" (USPP) (Bäumlein et al., 1991) und das Terminationssignal-1 des Nopalin-Synthase-Gens aus *A.tumefaciens* (Depicker et al.,1982) enthält.

Das DNA Fragment kodierend für das Homogentisinsäure Phytyltransferase aus *Arabidopsis thaliana* wird aus dem Plasmid pGEMTe/AtHPT als BamH1 Fragment isoliert, die BamH1 Ende werden mit dem Klenow Enzym aufgefüllt und in den Sma1 parcial verdauten pSUN2-USPP-ocsT kloniert (Grösse des linearisierten Vektors 88691Bp).

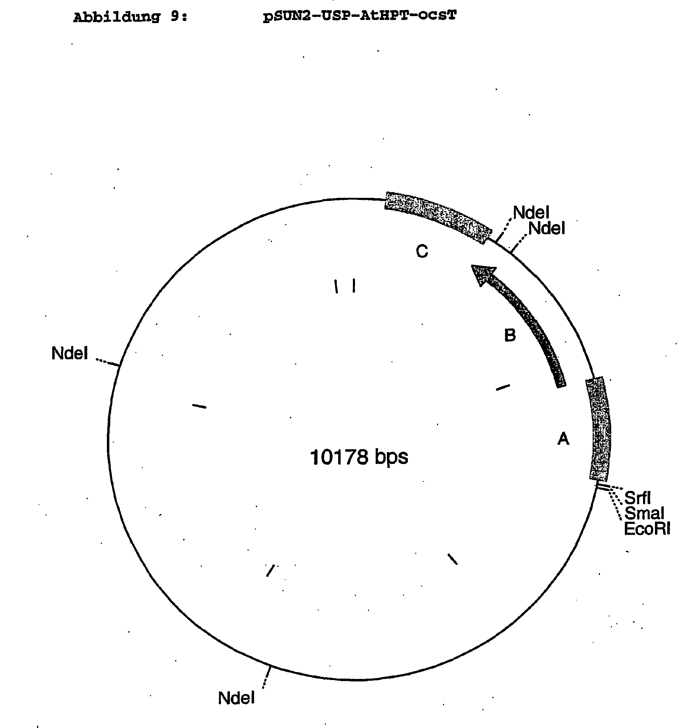

Dieses Plasmid (pSUN2-USPP-AtHPT-ocsT, Abbildung 9) wird zur Erzeugung transgener *Brassica napus* Pflanzen verwendet.

Fragment A ( 678 Bp) in Abbildung 9 beinhaltet den Promotor des "Unknow-Seed-Protein-Gens" aus Vicia faba, Fragment B (1182 Bp) kodiert für das Homogentisinsäure-Phytyltransferase-Gen aus Ara*bidopsis thaliana* und Fragment C (713Bp) kodiert für das Terminationssignal-1 des Octopin-Synthase Gens.

### Beispiel 22

Erzeugung von DNA Konstrukten zur Expression der 2-Methyl-6-Phytylhydrochinon-Methyltransferase aus *Synechocystis* sp. PCC6803 unter. Kontrolle eines samenspezifischen Promotors.

Zur Herstellung von chimären DNA Konstrukten zur Erzeugung transgener *Brassica napus* Pflanzen, die die 2-Methyl-6-Phytylhydrochinol Methyltransferas aus *Synechocystis* sp. PCC6803 unter Kontrolle eines samenspezifischen Promotors exprimieren, wird der Vektor pSUN2 (WO 02/00900) verwendet.

Dieser Vektor wird so verändert, dass er den samenspezifichen Promotor des LeguminB4-Gens (Kafatos et al., 1986), die Sequenz kodierend für das Transitpeptid der *A.thaliana* plastiden-spezifischen Isopentenyl-pyrophosphat Isomerase-2 (IPP-2) und das Terminationssignal des Nopalin-Synthase-Gens aus *A.tumefaciens* (Depicker et al., 1982)) enthält.

Das DNA Fragment kodierend für das 2-Methyl-6-Phytylhydrochinon-Methyltransferase aus *Synechocystis* sp. PCC6803 wird aus dem Plasmid pGEMTe/SynMT1 als BamH1 Fragment isoliert, die BamH1 Ende werden mit dem Klenow Enzym aufgefüllt und in den Sal1 verdauten pSUN2-Leb4P-IPP-nosT kloniert, dessen Sal1 Enden ebenfalls mit dem Klenow Enzym aufgefüllt werden. Dadurch wird eine Translationsfusion mit dem Transitpetid der IPP-2 erzeugt und somit ein Import der 2-Methyl-6-Phytylhydrochinon-Methyltransferase in den Chloroplasten gewährleistet.

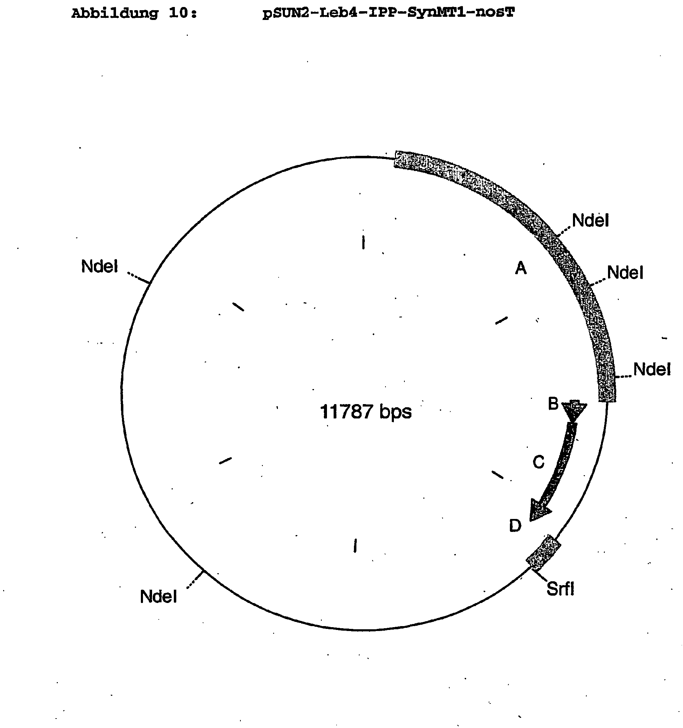

Dieses Plasmid (pSUN2LeB4-IPP-SynMT1-nosT, Abbildung 10) wird zur Erzeugung transgener *Brassica napus* Pflanzen verwendet.

Fragment A (2764 Bp) in Abbildung 10 beinhaltet den Promotor des LeguminB4-Gens aus Vicia faba, Fragment B (235Bp) kodiert für das Transitpeptid der *A.thaliana* Isopentenyl-pyrophosphat-Isomerase-2. Fragment C (957 Bp) kodiert für das 2-Methyl-6-Phytylhydrochinol Methyltransferase Gen aus *Synechocystis* sp. PCC6803 und Fragment D (272Bp) kodiert für das Terminationssignal des Nopalin-Synthase-Gens.

### Beispiel 23

Erzeugung von DNA Konstrukten zur Expression der 2,3-Dimethyl-5-Phytylplastochinol-Zyklase aus *Synechocystis* sp. PCC6803 unter Kontrolle eines samenspezifischen Promotors.

Zur Herstellung von chimären DNA Konstrukten zur Erzeugung transgener *Brassica napus* Pflanzen, die die 2,3-Dimethyl-5-Phytylplastochinol-Zyklase aus *Synechocystis* sp. PCC6803 unter Kontrolle eines samenspezifischen Promotors exprimieren, wird der Vektor pSUN2 (Wo 02/00900) verwendet.

Dieser Vektor wird so verändert, dass er den samenspezifichen Promotor des Legumin-B4-Gens (Kafatos et al., 1986), die Sequenz kodierend für das Transitpeptid der *A.thaliana* plastiden-spezifischen Isopentenyl-pyrophosphat Isomerase-2 (IPP-2) und das Terminationssignal des Nopalin-Synthase Gens aus *A.tumefaciens* (Depicker et al., 1982) enthält.

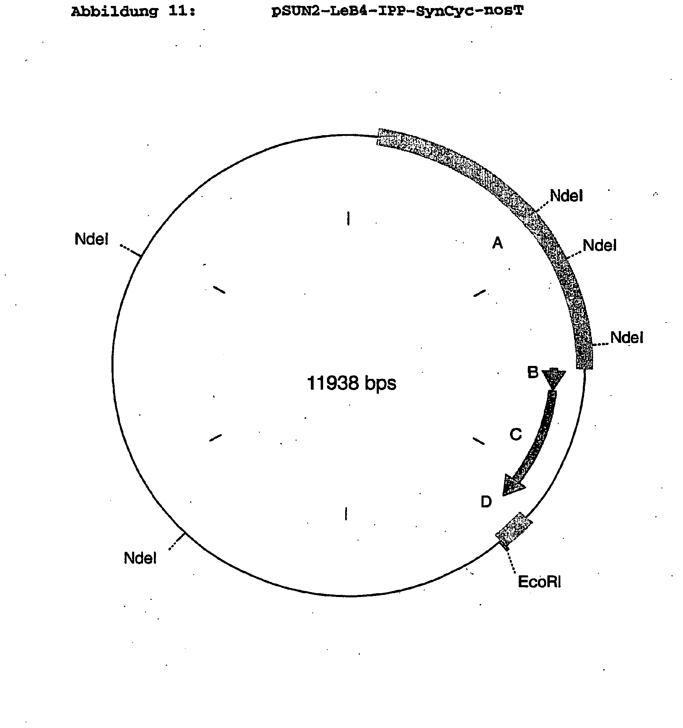

Das DNA Fragment kodierend für das 2,3-Dimethyl-5-Phytylplastochinol-Zyklase aus *Synechocystis* sp. PCC6803 wird aus dem Plasmid pGEMTe/SynCyc als BamH1 Fragment isoliert, die BamH1 Ende werden mit dem Klenow Enzym aufgefüllt und in den Sal1 verdauten pSUN2-Leb4P-IPP-nosT kloniert, dessen Sal1 Enden ebenfalls mit dem Klenow Enzym aufgefüllt werden. Dadurch wird eine Translationsfusion mit dem Transitpetid der IPP-2 erzeugt und somit ein Import der 2,3-Dimethyl-5-Phytylplastochinol-Zyklase in den Chloroplasten gewährleistet. Dieses Plasmid (pSUN2-LeB4P-IPP-SynCyc-nosT, Abbildung 11) wird zur Erzeugung transgener *Brassica napus* Pflanzen verwendet.
Fragment A (2764 Bp) in Abbildung 11 beinhaltet den Promotor des LeguminB4-Gens aus Vicia faba, Fragment B (235Bp) kodiert für das Transitpeptid der *A.thaliana* Isopentenyl-pyrophosphat-Isomerase-2. Fragment C (1100 Bp) kodiert für das 2,3-Dimethyl-5-Phytylplastochinol Zyklase Gen aus *Synechocystis* sp. PCC6803. und Fragment D (272Bp) kodiert für das Terminationssignal des Nopalin-Synthase-Gens.

### Beispiel 24

Erzeugung von DNA Konstrukten zur Expression der γ-Tocopherol-Methyltransferase aus *Arabidopsis thaliana* unter Kontrolle eines samenspezifischen Promotors.

Zur Herstellung von chimären DNA Konstrukten zur Erzeugung transgener *Brassica napus* Pflanzen, die die γ-Tocopherol-Methyltransferase aus *Arabidopsis thaliana* unter Kontrolle eines samenspezifischen Promotors exprimieren, wurde der Vektor pSUN2 (WO 02/00900).verwendet.

Zuerst wurde der Vektor puc19 (New England Biolabs) so verändert, dass er den samenspezifichen Promotor des Sucrose-Binding-Protein-Gens (SBP-P) (DE 19852195 C2) und die 35s-Terminationssequenz des Blumenkohlmosaikvirus (FRANCK, A., GUILLEY, H., JONARD, G., RICHARDS, K., HIRTH, L.. Nucleotide sequence of cauliflower mosaik virus DNA. Cell 21: 285-294. (1980)) enthält. Der resultierende Vektor heißt puc19-SBPP-35ST.

Das DNA Fragment kodierend für das γ-Tocopherol-Methyltransferase-Gen aus *Arabidopsis thaliana* wurde als BamH1/Sal1 Fragment in puc19-SBPP-AtγTMT-35ST kloniert, nachdem dieser mit dem Restriktionsenzym BamH1/Sal1 verdaut wurde.

Mittels PCR wurde die Expressionkassette bestehend aus: SBP-Promotor, γ-Tocopherol-Methyltransferase-Gen aus *Arabidopsis thaliana* und 35sT Terminationssequenz, unter Verwendung eines sense spezifischen Primers (SBPP-XbaI 5': SEQ. ID. No. 50) und eines anti-sense spezifischen Primers (35ST-XbaI 3': SEQ. ID. No. 51), amplifiziert und in den Vektor pCR4topoblunt (Invitrogene) kloniert.

Die PCR Bedingungen waren die folgenden:
Die PCR erfolgte in einem 50µl Reaktionsansatz in dem enthalten war:
- 1µl einer puc19-SBPP-AtγTMT-35ST Plasmid-DNA
- 0,2 mM dATP, dTTP, dGTP, dCTP
- 1,5 mM Mg(OAC)₂
- 5µg Rinderserum-Albumin
- 40pmol SBPP-XbaI 5'Primer
- 40pmol 35ST-XbaI 3'Primer
- 5µl 10x Pfu1 DNA Polymerase Puffer (Stratagene)
- 5U Pfu1 DNA Polymerase (Stratagene)

Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 55°C (Annealing)
Schritt 4: 10 Minuten 68°C (Elongation)
   30 Wiederholungen der Schritte 2-4
Schritt 5: 10 Minuten 72°C (Post-Elongation)

Das DNA Fragment bestehend aus SBP-Promotor, γ-Tocopherol-Methyltransferase-Gen aus *Arabidopsis thaliana* und 35ST Terminationssequenz wurde aus dem Plasmid pCR4TOPOblunt/SBPP-γTMT-35ST als XbaI Fragment isoliert und in den Vektor pSUN2 kloniert, nachdem dieser mit dem Restriktionsenzym XbaI verdaut wurde.

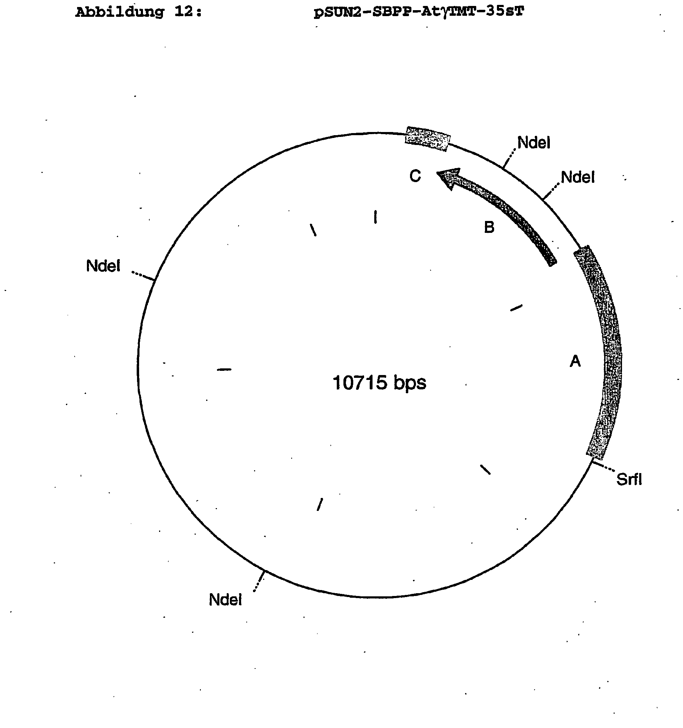

Dieses Plasmid (pSUN2-SBPP-γTMT-35ST, Abbildung 12) wird zur Erzeugung transgener *Brassica napus* Pflanzen verwendet.

Fragment A (1788Bp) in Abbildung 12 beinhaltet den Promotor des SBP-Gens aus Vicia faba, Fragment B (1047Bp) kodiert für das γ-Tocopherol-Methyltransferase-Gen aus *Arabidopsis thaliana* und Fragment C (291Bp) kodiert für den 35s-Terminator des Blumenkohl-mosaikvirus.

### Beispiel 25

Erzeugung von DNA Konstrukten zur Expression des Tyrosin-Aminotransferase Gens aus *Rattus norvegicus* unter Kontrolle eines samenspezifischen Promotors, in Kombination mit der samenspezifischen Unterdrückung der Expression des Homogentisat-Dioxygenase Gens aus *Brassica napus*.

Zur Herstellung von chimären DNA Konstrukten zur Erzeugung transgener *Brassica napus* Pflanzen, die die Tyrosin-Aminotransferase aus *Rattus norvegicus* unter Kontrolle eines samenspezifischen Promotors exprimieren und gleichzeitig die samenspezifische Unterdrückung der Expression des Homogentisat-Dioxygenase-Gens aus *Brassica napus* vermitteln, wurde der Vektor pSUN2-Pvic-BnHGD*-STLS1-αBnHGD*-ocsT und der Vektor pSUN2USPP-rbcS-RnTATase-nosT, verwendet.

Aus dem Vektor pSUN2USPP-rbcS-RnTATase-nosT, wurde mittels PCR die Expressionkassette bestehend aus: USP-Promotor, rbcS Transitpeptid, Tyrosin-Aminotransferase-Gen'aus *Rattus norvegicus* und nos-Terminationssequenz, unter Verwendung eines sense spezifischen Primers (USPP-SRF1 5': SEQ. ID. No. 52) und eines antisense spezifischen Primers (nosT-Srf1 3': SEQ. ID. No. 53), amplifiziert und in den Vektor pCR4topoblunt (Invitrogene) kloniert.

Die PCR Bedingungen waren die folgenden:
Die PCR erfolgte in einem 50µl Reaktionsansatz in dem enthalten war:
- 1µl des pSUN2-USPP-rbcS-RnTATase-nosT Plasmid-DNA
- 0,2 mM dATP, dTTP, dGTP, dCTP
- 1,5 mM Mg(OAc)₂
- 5µg Rinderserum-Albumin
- 40pmol USPP-Srf1 5'Primer
- 40pmol nosT-Srf1 3'Primer
- 5µl 10x Pfu1 Turbo DNA Polymerase Puffer (Stratagene)
- 5U Pfu1 Turbo DNA Polymerase (Stratagene)

Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 55°C (Annealing)
Schritt 4: 8 Minuten 68°C (Elongation)
   30 Wiederholungen der Schritte 2-4
Schritt 5: 10 Minuten 72°C (Post-Elongation)

Das DNA Fragment bestehend aus USP-Promotor, rbcS Transitpeptid, Tyrosin*-Aminotransferase*-Gen aus *Rattus norvegicus* und nos Terminationssequenz wurde aus dem Plasmid pCR4TOPOblunt/USPP-rbcS-RnATase-nosT als Srf1 Fragment isoliert und in den pSUN2-Pvic-*BnHGD-STLS1-α*BnHGD-ocsT kloniert, nachdem dieser mit dem Restriktionsenzym EcoR5 verdaut wurde.

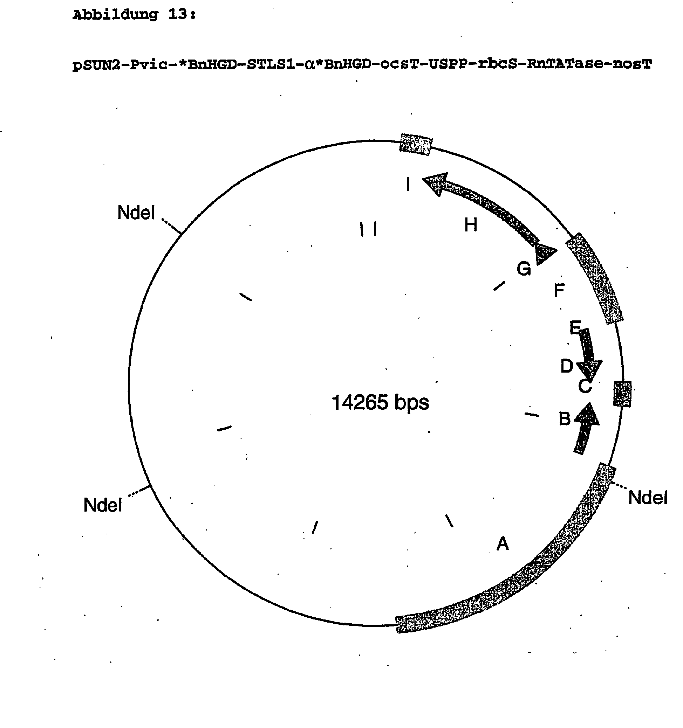

Dieses Plasmid (pSUN2-Pvic-*BnHGD-STLS1-α*BnHGD-ocsT-USPP-rbcS-RnATase-nosT, Abbildung 13) wird zur Erzeugung transgener *Brassica napus* Pflanzen verwendet.

Fragment A (2559 Bp) in Abbildung 13 beinhaltet den Promotor des Vicilin-Gens aus Vicia faba, Fragment B (580 Bp) kodiert für einen Fragment des Homogentisinsäure-Dioxygenase-Gen aus *Brassica napus* und Fragment C (198Bp) kodiert für das 2 Intron (IV2) des ST-LS1 Gens aus Solanum tuberosum. Fragment D ist identisch mit Fragment B jedoch im Vektor gegenläufig zu B orientiert. Fragment E (208Bp) kodiert für das Terminationssignal-2 des Octopin Gens. Fragment F ( 678 Bp) beinhaltet den Promotor des "Unknow-Seed-Protein-Gens" aus Vicia faba, Fragment G (235Bp) kodiert für das Transitpeptid der Ribulose-Bisphosphat-Carboxylase (rbcS) aus *Vicia faba*. Fragment H (1365 Bp) kodiert für das Tyrosin-Aminotransferase-Gen aus Rattus *norvegicus* und Fragment I (272Bp) kodiert für das Terminationssignal des Nopalin-Synthese-Gens aus *Agrobakterium tumifaciens*.

### Beispiel 26

Erzeugung von DNA Konstrukten zur Expression der Tyrosin-aminotransferase-1 aus *Arabidopsis thaliana* unter Kontrolle eines samenspezifischen Promotors und zur samenspezifischen Unterdrückung der Expression des Homogentisinsäure-Dioxygenase-Gens aus *Brassica napus*

Zur Herstellung von chimären DNA Konstrukten zur Erzeugung transgener *Brassica napus* Pflanzen, die die Tyrosin-aminotransferase-1 aus *Arabidopsis thaliana* unter Kontrolle eines samenspezifischen Promotors exprimieren, und die Expression der endogenen Homogentisinsäure-Dioxygenase-Gens aus *Brassica napus* samenspezifisch unterdrücken, wurden die Vektoren pSUN2-USPP-AtTATase1-nosT und der Vektor pSUN2-Pvic-*BnHGD-STLS1-α*BnHGD-ocsT miteinander kombiniert.

Das DNA Fragment kodierend für die Expressionskassette bestehend aus: USP-Promotor, Tyrosin-Aminotransferase-1 Gen aus *Arabidopsis thaliana* und nos-Terminator wurde aus dem Plasmid pSUN2-USPP-At-TATasel-nosT als EcoR1/Sma1 Fragment isoliert, das EcoR1 Ende wurde mit dem Klenow Enzym aufgefüllt und in den mit EcoR5 verdauten pSUNPvic-*BnHGD-STLS1-α*BnHGD-ocsT kloniert.

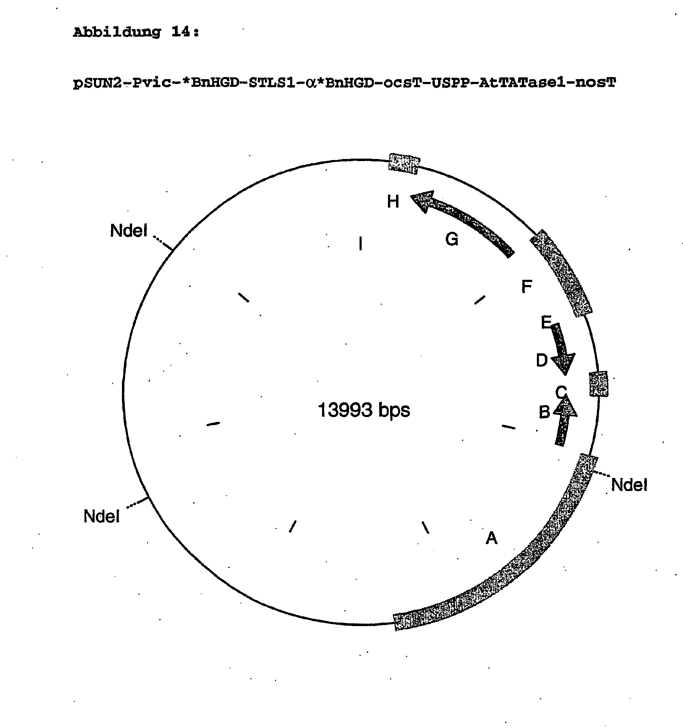

Dieses Plasmid (pSUN2-Pvic-*BnHGD-STLS1-α*BnHGD-ocsT/USPP-AtTA-Tase1-nosT Abbildung 14) wird zur Erzeugung transgener *Brassica napus* Pflanzen verwendet.

Fragment A (2559 Bp) in Abbildung 14 beinhaltet den Promotor des Vicilin-Gens aus Vicia faba, Fragment B (580 Bp) kodiert für einen Fragment des Homogentisinsäure-Dioxygenase-Gen aus *Brassica napus*. Fragment C (190Bp) kodiert für das 2 Intron (IV2) des ST-LS1 Gens aus Solanum tuberosum. Fragment D ist identisch mit Fragment B jedoch im Vektor gegenläufig zu B orientiert. Fragment E (208Bp) kodiert für das Terminationssigna1-2 des Octopin-Gens. Fragment
Fragment F ( 678Bp) beinhaltet den Promotor des Unknow-Seed-Protein-Gens (USPP)aus *Vicia faba*, Fragment G (1269Bp) kodiert für das Tyrosin-Aminotransferase-Gen 1 aus *Arabidopsis thaliana* und Fragment H (272Bp) kodiert für das Terminationssignal des Nopalin-Synthase Gens.

### Beispiel 27

Erzeugung von DNA Konstrukten zur Expression der Tyrosinaminotransferase-3 aus *Arabidopsis thaliana* unter Kontrolle eines samenspezifischen Promotors und zur samenspezifischen Unterdrükkung der Expression des Homogentisinsäure Dioxygenase Gens aus *Brassica napus*

Zur Herstellung von chimären DNA Konstrukten zur Erzeugung transgener *Brassica napus* Pflanzen, die die Tyrosin-Aminotransferase-3 aus *Arabidopsis thaliana* unter Kontrolle eines samenspezifischen Promotors exprimieren, und die Expression der endogenen Homogentisinsäure-Dioxygenase-Gens aus *Brassica napus* samenspezifisch unterdrücken, wurden die Vektoren pSUN2-USPP-AtTATase3-nosT und der Vektor pSUN2-Pvic-*BnHGD-STLS1-α*BnHGD-ocsT miteinander kombiniert.

Das DNA Fragment kodierend für die Expressionskassette bestehend aus: USP-Promotor, Tyrosin-Aminotransferase-3-Gen aus *Arabidopsis thaliana* und nos-Terminator wird aus dem aus dem Plasmid pSUN2-USPP-AtTATase1-nosT als EcoR1/Sma1 Fragment isoliert, das EcoR1 wird mit dem Klenow Enzym aufgefüllt und in den mit EcoR5 verdauten pSUN2-Pvic-*BnHGD-STLS1-α*BnHGD-ocsT kloniert.

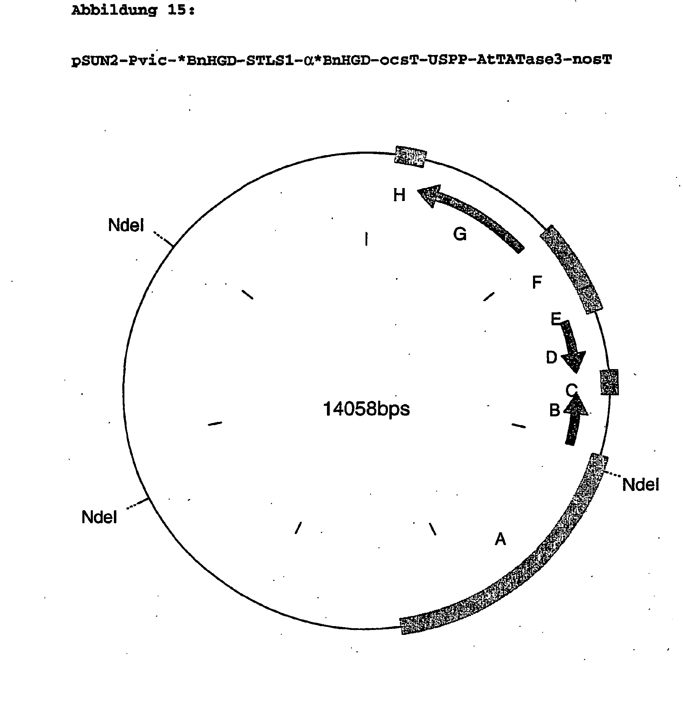

Dieses Plasmid (pSUN2-Pvic-*BnHGD-STLS1-α*BnHGD-ocsT/USPP-AtTA-Tase3-nosT Abbildung 15) wird zur Erzeugung transgener *Brassica napus* Pflanzen verwendet.

Fragment A (2559 Bp) in Abbildung 15 beinhaltet den Promotor des Vicilin-Gens aus Vicia faba, Fragment B (580 Bp) kodiert für einen Fragment des Homogentisinsäure-Dioxygenase-Gen aus *Brassica napus*. Fragment C (190Bp) kodiert für das 2 Intron (IV2) des ST-LS1 Gens aus Solanum tuberosum. Fragment D ist identisch mit Fragment B jedoch im Vektor gegenläufig zu B orientiert. Fragment E (208Bp) kodiert für das Terminationssignal-2 des Octopin Gens. Fragment F (678Bp) beinhaltet den Promotor des "Unknow-Seed-Protein-Gens" (USPP) aus *Vicia faba*, Fragment G (1334Bp) kodiert für das Tyrosin-Aminotransferase-Gen 3 aus *Arabidopsis thaliana* und Fragment H (272Bp) kodiert für das Terminationssignal des Nopalin-Synthase Gens.

### Beispiel 28

Erzeugung von DNA Konstrukten zur Expression der Tyrosin- Aminotransferase-5 aus *Arabidopsis thaliana* unter Kontrolle eines samenspezifischen Promotors und zur samenspezifischen Unterdrückung der Expression des Homogentisinsäure-Dioxygenase-Gens aus *Brassica napus*

Zur Herstellung von chimären DNA Konstrukten zur Erzeugung transgener *Brassica napus* Pflanzen, die die Tyrosin-Aminotransferase-5 aus *Arabidopsis thaliana* unter Kontrolle eines samenspezifischen Promotors exprimieren, und die Expression der endogenen Homogentisinsäure-Dioxygenase-Gens aus *Brassica napus* samenspezifisch unterdrücken, wurden die Vektoren pSUN2-USPP-AtTATase5-nosT und der Vektor pSUN2-Pvic-*BnHGD-STLS1-α*BnHGD-ocsT miteinander kombiniert.

Das DNA Fragment kodierend für die Expressionskassette bestehend aus: USP-Promotor, Tyrosin-Aminotransferase-5-Gen aus *Arabidopsis thaliana* und nos-Terminator wird aus dem aus dem Plasmid pSUN2-USPP-AtTATase5-nosT als Sma1/EcoR1 Fragment isoliert, das EcoR1 Ende mit dem Klenow Enzym aufgefüllt und in den mit EcoR5 verdauten pSUNPvic-*BnHGD-STLS1-α*BnHGD-ocsT kloniert.

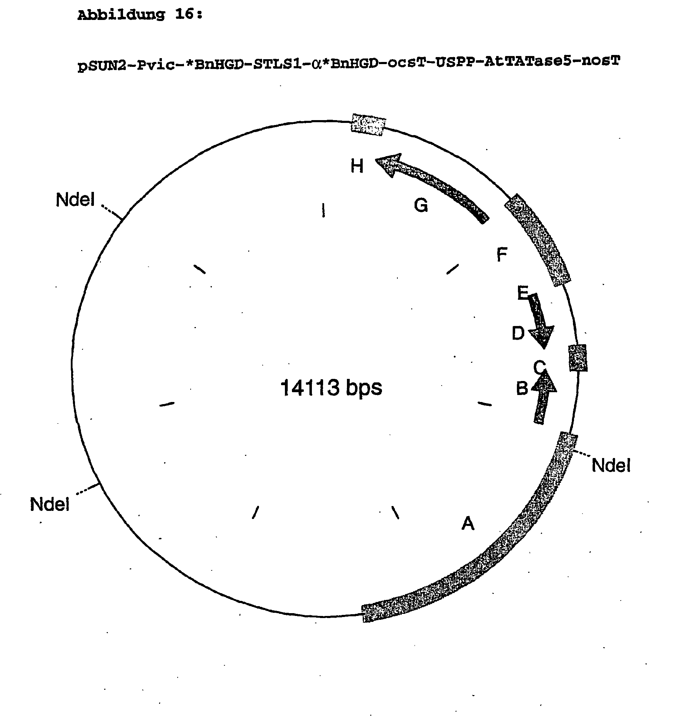

Dieses Plasmid (pSUN2-Pvic-*BnHGD-STLS1-α*BnHGD-ocsT/USPP-AtTA-Tase5-nosT Abbildung 16) wird zur Erzeugung transgener *Brassica napus* Pflanzen verwendet.

Fragment A (2559.Bp) in Abbildung 16 beinhaltet den Promotor des Vicilin-Gens aus Vicia faba, Fragment B (580 Bp) kodiert für einen Fragment des Homogentisinsäure-Dioxygenase-Gen aus *Brassica napus*. Fragment C (190Bp) kodiert für das 2 Intron (IV2) des ST-LS1 Gens aus Solanum *tuberosum*. Fragment D ist identisch mit Fragment B jedoch im Vektor gegenläufig zu B orientiert. Fragment E (208Bp) kodiert für das Terminationssignal-2 des Octopin Gens.

Fragment F (678Bp) beinhaltet den Promotor des "Unknow-Seed-Protein-Gens" aus *Vicia faba*, Fragment G (1389Bp) kodiert für das Tyrosin-Aminotransferase-Gen 5 aus *Arabidopsis thaliana* und Fragment H (272Bp) kodiert für das Terminationssignal des Nopalin-Synthase Gens.

### Beispiel 29

Erzeugung von DNA Konstrukten zur Expression der Tyrosin-Aminotransferase-6 aus *Arabidopsis thaliana* unter Kontrolle eines samenspezifischen Promotors und zur samenspezifischen Unterdrükkung der Expression des Homogentisinsäure-Dioxygenase-Gens aus *Brassica napus*

Zur Herstellung von chimären DNA Konstrukten zur Erzeugung transgener *Brassica napus* Pflanzen, die die Tyrosin-Aminotransferase-6 aus *Arabidopsis thaliana* unter Kontrolle eines samenspezifischen Promotors exprimieren, und die Expression der endogenen Homogentisinsäure-Dioxygenase-Gens aus *Brassica napus* samenspezifisch unterdrücken, wurden die Vektoren pSUN2-USPP-AtTATase6-nosT und der Vektor pSUN2-Pvic-*BnHGD-STLS1-α*BnHGD-ocsT miteinander kombiniert.

Das DNA Fragment kodierend für die Expressionskassette bestehend aus: USP-Promotor, Tyrosin-Aminotransferase-6-Gen aus *Arabidopsis thaliana* und nos-Terminator wird aus dem aus dem Plasmid pSUN2-USPP-AtTATase6-nosT als Sma1/EcoR1 Fragment isoliert, das EcoR1 Ende mit dem Klenow Enzym aufgefüllt und in den mit EcoR5 verdauten pSUNPvic-*BnHGD-STLS1-α*BnHGD-ocsT kloniert.

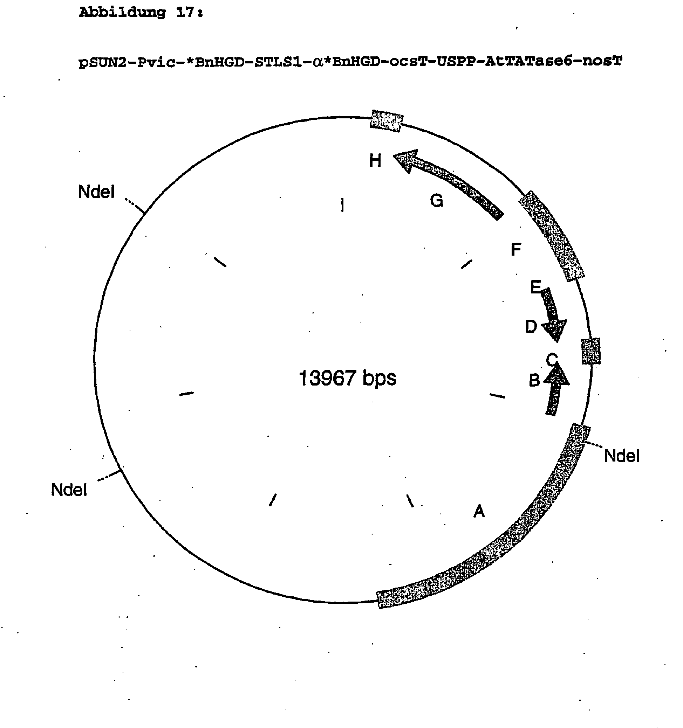

Dieses Plasmid (pSUN2-Pvic-*BnHGD-STLS1-α*BnHGD-ocsT/USPP-AtTA-Tase6-nosT Abbildung 17) wird zur Erzeugung transgener *Brassica napus* Pflanzen verwendet.

Fragment A (2559 Bp) in Abbildung 17 beinhaltet den Promotor des Vicilin-Gens aus Vicia faba, Fragment B (580 Bp) kodiert für einen Fragment des Homogentisinsäure-Dioxygenase-Gen aus *Brassica napus*. Fragment C (190Bp) kodiert für das 2 Intron (IV2) des ST-LS1 Gens aus *Solanum* tuberosum. Fragment D ist identisch mit Fragment B jedoch im Vektor gegenläufig zu B orientiert. Fragment E (208Bp) kodiert für das Terminationssignal-2 des Octopin Gens. Fragment F ( 678Bp) beinhaltet den Promotor des "Unknow-Seed-Protein-Gens" aus *Vicia faba*, Fragment G (1243 Bp) kodiert für das Tyrosin-Aminotransferase-Gen 6 aus *Arabidopsis thaliana* und Fragment H (272Bp) kodiert für das Terminationssignal des Nopalin-Synthase-Gens.

### Beispiel 30

Erzeugung von DNA Konstrukten zur Expression der Tyrosin Aminotransferase aus Rattus *norvegicus* unter Kontrolle eines samenspezifischen Promotors und der Geranylgeranylpyrophosphat-Oxidoreductase aus *Nicotiana tabacum* unter Kontrolle eines samenspezifischen Promotors

Zur Herstellung von chimären DNA Konstrukten zur Erzeugung transgener *Brassica napus* Pflanzen, die die Tyrosin-Aminotransferase aus *Rattus norvegicus* unter Kontrolle eines samenspezifischen Promotors exprimieren, und die Geranylgeranylpyrophosphate-Oxidoreductase aus *Nicotiana tabacum* samenspezifischen expremieren, werden die Vektoren pSUN2-LEB4-NtGGPPOR-nosT und pCR4topoblunt-USPP-rbcS-RnTATase1-nosT miteinander kombiniert.

Das DNA Fragment bestehend aus USP-Promotor, Tyrosin-Aminotransferase aus *Rattus norvegicus* und nos-Terminationssequenz wird aus dem Plasmid pCR4TOPOblunt/USPP-rbcS-RnTATase1-nosT als Srf1 Fragment isoliert und in den mit Xho1 verdauten Vektor pSUN2-LeB4-NtGGPPOR-nosT kloniert, dessen Xho1 Enden zuvor mit dem Klenow Enzym geglättet werden.

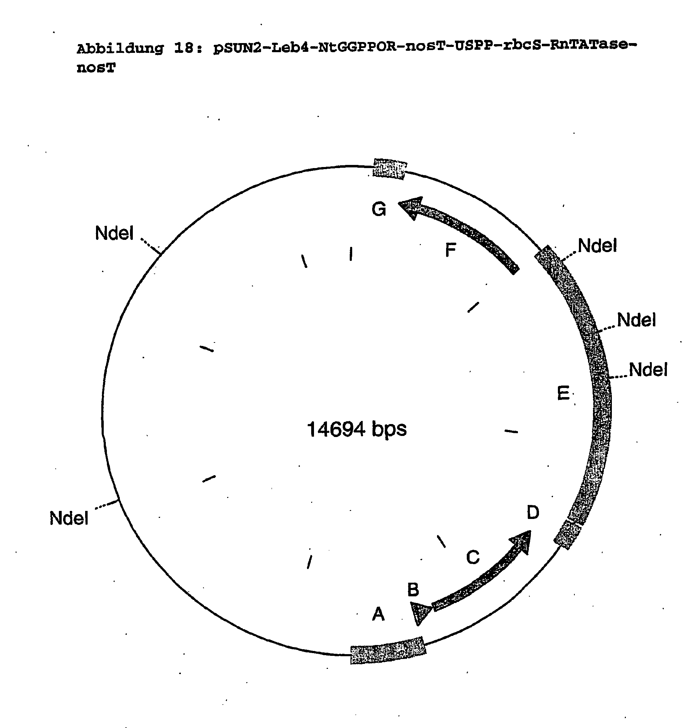

Dieses Plasmid (pSUN2-LeB4-NtGGPPORnosT/USPP-rbcS-RnTATase1-nosT Abbildung 18) wird zur Erzeugung transgener *Brassica napus* Pflanzen verwendet.

Fragment A (678Bp) in Abbildung 18 beinhaltet den Promotor des Unknow-Seed-Protein-Gens (USPP) aus *Vicia faba*, Fragment B (235Bp) kodiert für das Transitpeptid der Ribulose-Bisphosphat-Carboxylase *(rbcS) aus Vicia faba*. Fragment C (1365Bp) kodiert für das Tyrosin-Aminotransferase-Gen aus *Rattus norvegicus.* Fragment D (272Bp) kodiert für das Terminationssigna1 des Nopalin-Synthase Gens aus *A.tumefaciens*.
Fragment E (2764Bp) beinhaltet den Promotor des LeguminB4-Gens aus *Vicia faba*, Fragment F (1509Bp) kodiert für das Geranylgeranylpyrophosphate-Oxidoreduktase-Gen aus *Nicotiana* tabacum,Fragment G (272Bp) kodiert für das Terminationssigna1 des Nopalin-Synthase-Gens.

### Beispiel 31

Erzeugung von DNA Konstrukten zur Expression der Tyrosin Aminotransferase 1 aus *Arabidopsis thaliana* unter Kontrolle eines samenspezifischen Promotors und der Geranylgeranylpyrophosphate-Oxidoreductase aus *Nicotiana tabacum* unter Kontrolle eines samenspezifischen Promotors

Zur Herstellung von chimären DNA Konstrukten zur Erzeugung transgener *Brassica napus* Pflanzen, die die Tyrosin-Aminotransferase-1 aus *Arabidopsis thaliana* unter Kontrolle eines samenspezifischen Promotors exprimieren, und die Geranylgeranylpyrophosphate-Oxidoreductase aus *Nicotiana tabacum* samenspezifischen expremieren, werden die Vektoren pSUN2-LeB4-NtGGPPOR-nosT und pSUN2-USPP-AtTA-Tase1-nosT miteinander kombiniert.

Das DNA Fragment kodierend für die Expressionskassette bestehend aus: USP-Promotor, Tyrosin-Aminotransferase-1-Gen aus *Arabidopsis thaliana* und nos-Terminator wird aus dem Plasmid pSUN2-USPP-AtTA-Tase1-nosT als Sma1/EcoR1 Fragment isoliert, das EcoR1 Ende mit dem Klenow Enzym aufgefüllt und in den mit Xho1 verdauten Vektor pSUN2-LeB4-NtGGPPOR-nosT kloniert, dessen Xho1 Enden zuvor mit dem Klenow Enzym geglättet werden.

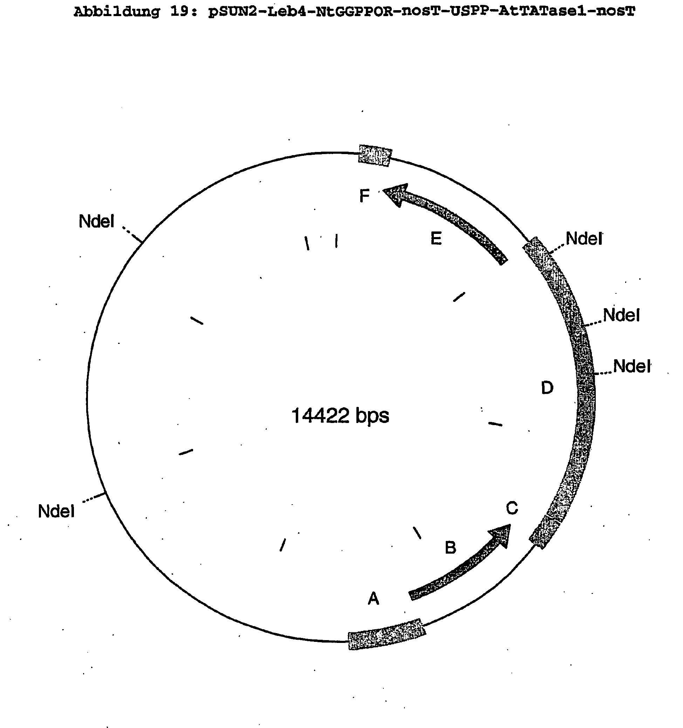

Dieses Plasmid (pSUN2-LeB4-NtGGPPOR-nosT/USPP-AtTATase1-nosT Abbildung 19) wird zur Erzeugung transgener *Brassica napus* Pflanzen verwendet
Fragment A (678Bp) in Abbildung 19 beinhaltet den Promotor des Unknow-Seed-Protein-Gens (USPP) aus *Vicia faba*, Fragment B (1269Bp) kodiert für das Tyrosin-Aminotransferase-Gen 1 aus *Arabidopsis thaliana*und Fragment C (272Bp) kodiert für das Terminationssignal des Nopalin-Synthase Gens. Fragment D (2764Bp) beinhaltet den Promotor des LeguminB4-Gens aus *Vicia faba*, Fragment E (1509Bp) kodiert für das Geranylgeranylpyrophosphate-Oxidoreduktase-Gen aus *Nicotiana tabacum*. Fragment F (272Bp) kodiert für das Terminationssigna1 des Nopalin-Synthase-Gens.

### Beispiel 32

Erzeugung von DNA Konstrukten zur Expression der Tyrosin Aminotransferase 3 aus *Arabidopsis thaliana* unter Kontrolle eines samenspezifischen Promotors und der Geranylgeranylpyrophosphate-Oxidoreductase aus *Nicotiana tabacum* unter Kontrolle eines samenspezifischen Promotors

Zur Herstellung von chimären DNA Konstrukten zur Erzeugung transgener *Brassica napus* Pflanzen, die die Tyrosin-Aminotfansferase-3 aus *Arabidopsis thaliana* unter Kontrolle eines samenspezifischen Promotors exprimieren, und die Geranylgeranylpyrophosphate-Oxidoreductase aus *Nicotiana tabacum* samenspezifischen expremieren, werden die Vektoren pSUN2-LeB4-NtGGPPOR-nosT und pSUN2-USPP-AtTA-Tase3-nosT miteinander kombiniert.

Das DNA Fragment kodierend für die Expressionskassette bestehend aus: USP-Promotor, Tyrosin-Aminotransferase-3-Gen aus *Arabidopsis thaliana* und nos-Terminator wird aus dem Plasmid pSUN2-USPP-AtTA-Tase3-nosT als Sma1/EcoR1 Fragment isoliert, das EcoR1 wird mit dem Klenow Enzym aufgefüllt und in den mit Xho1 verdauten Vektor pSUN2-LeB4-NtGGPPOR-nosT kloniert, dessen Xho1 Enden zuvor mit dem Klenow Enzym geglättet werden.

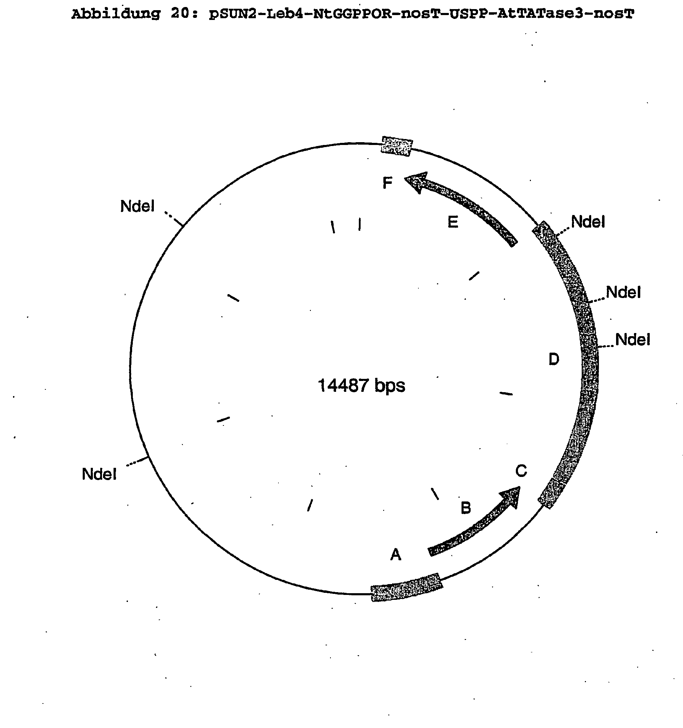

Dieses Plasmid (pSUN2-LeB4-NtGGPPORnosT/USPP-AtTATase3-nosT Abbildung 20) wird zur Erzeugung transgener *Brassica napus* Pflanzen verwendet.

Fragment A (678Bp) in Abbildung 20 beinhaltet den Promotor des "Unknow-Seed-Protein-Gens" (USPP) aus *Vicia faba*, Fragment B (1334Bp) kodiert für das Tyrosin-Aminotransferase-Gen 3 aus *Arabidopsis thaliana*und. Fragment C (272Bp) kodiert für das Terminationssignal des Nopalin-Synthase Gens. Fragment D (2764Bp) beinhaltet den Promotor des LeguminB4-Gens aus *Vicia faba*, Fragment E (1509Bp) kodiert für das Geranylgeranylpyrophosphate-Oxidoreduktase-Gen aus *Nicotiana tabacum*. Fragment F (272Bp) kodiert für das Terminationssignal des Nopalin-Synthase-Gens.

### Beispiel 33

Erzeugung von DNA Konstrukten zur Expression der Tyrosin Aminotransferase 5 aus *Arabidopsis thaliana* unter Kontrolle eines samenspezifischen Promotors und der Geranylgeranylpyrophosphate-Oxidoreductase aus *Nicotiana tabacum* unter Kontrolle eines samenspezifischen Promotors

Zur Herstellung von chimären DNA Konstrukten zur Erzeugung transgener *Brassica napus* Pflanzen, die die Tyrosin-Aminotransferase-5 aus *Arabidopsis thaliana* unter Kontrolle eines samenspezifischen Promotors exprimieren, und die Geranylgeranylpyrophosphate-Oxidoreductase aus *Nicotiana tabacum* samenspezifischen expremieren, werden die Vektoren pSUN2-LeB4-NtGGPPOR-nosT und pSUN2-USPP-AtTA-Tase5-nosT miteinander kombiniert.

Das DNA Fragment kodierend für die Expressionskassette bestehend aus: USP-Promotor, Tyrosin-Aminotransferase-5-Gen aus *Arabidopsis thaliana* und nos-Terminator wird aus dem Plasmid pSUN2-USPP-AtTA-Tase5-nosT als Sma1/EcoR1 Fragment isoliert, das EcoR1 Ende mit dem Klenow Enzym aufgefüllt und in den mit Xho1 verdauten Vektor pSUN2-LeB4-NtGGPPOR-nosT kloniert, dessen Xho1 Enden zuvor mit dem Klenow Enzym geglättet werden.

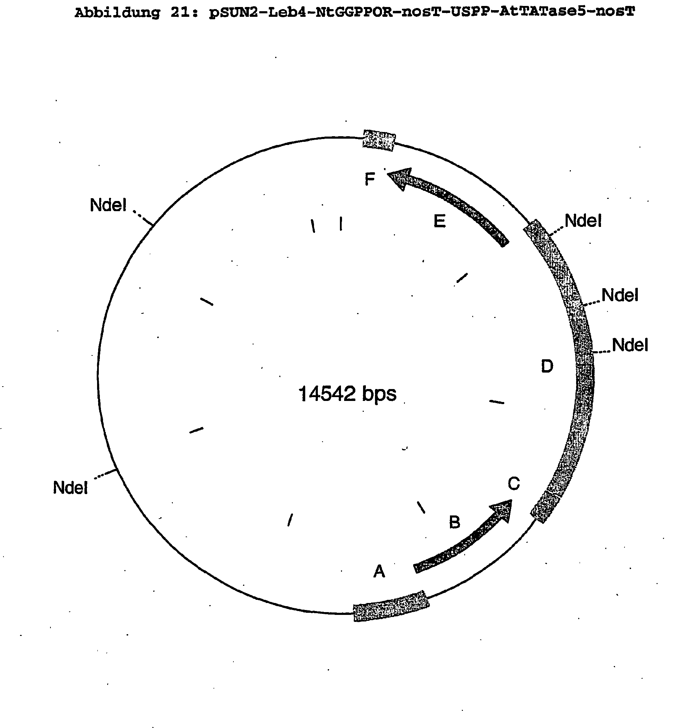

Dieses Plasmid (pSUN2-LeB4-NtGGPPORnosT/USPP-AtTATase5-nosT Abbildung 21) wird zur Erzeugung transgener *Brassica napus* Pflanzen verwendet

Fragment A ( 678Bp) in Abbildung 21 beinhaltet den Promotor des "Unknow-Seed-Protein-Gens" aus *Vicia faba*, Fragment B (1389Bp) kodiert für das Tyrosin-Aminotransferase-Gen 5 aus *Arabidopsis thaliana* und Fragment C (272Bp) kodiert für das Terminationssignal des Nopalin-Synthase Gens. Fragment D (2764Bp) beinhaltet den Promotor des LeguminB4-Gens aus *Vicia faba*, Fragment E (1509Bp) kodiert für das Geranylgeranylpyrophosphate-Oxidoreduktase-Gen aus *Nicotiana tabacum*. Fragment F (272Bp) kodiert für das Terminationssignal des Nopalin-Synthase-Gens

### Beispiel 34

Erzeugung von DNA Konstrukten zur Expression der Tyrosin Aminotransferase 6 aus *Arabidopsis thaliana* unter Kontrolle eines samenspezifischen Promotors und der Geranylgeranylpyrophosphate-Oxidoreductase aus *Nicotiana tabacum* unter Kontrolle eines samenspezifischen Promotors

Zur Herstellung von chimären DNA Konstrukten zur Erzeugung transgener *Brassica napus* Pflanzen, die die Tyrosin-Aminotransferase-6 aus *Arabidopsis thaliana* unter Kontrolle eines samenspezifischen Promotors exprimieren, und die Geranylgeranylpyrophosphate-Oxidoreductase aus *Nicotiana tabacum* samenspezifischen expremieren, werden die Vektoren pSUN2-LeB4-NtGGPPOR-nosT und pSUN2-USPP-AtTA-Tase6-nosT miteinander kombiniert.

Das DNA Fragment kodierend für die Expressionskassette bestehend aus: USP-Promotor, Tyrosin-Aminotransferase-6-Gen aus *Arabidopsis thaliana* und nos-Terminator wird aus dem Plasmid pSUN2-USPP-AtTA-Tase6-nosT als Sma1/EcoR1 Fragment isoliert, das EcoR1 Ende wird mit dem Klenow Enzym aufgefüllt und in den mit Xho1 verdauten Vektor pSUN2-LeB4-NtGGPPOR-nosT kloniert, dessen Xho1 Enden zuvor mit dem Klenow Enzym geglättet werden.

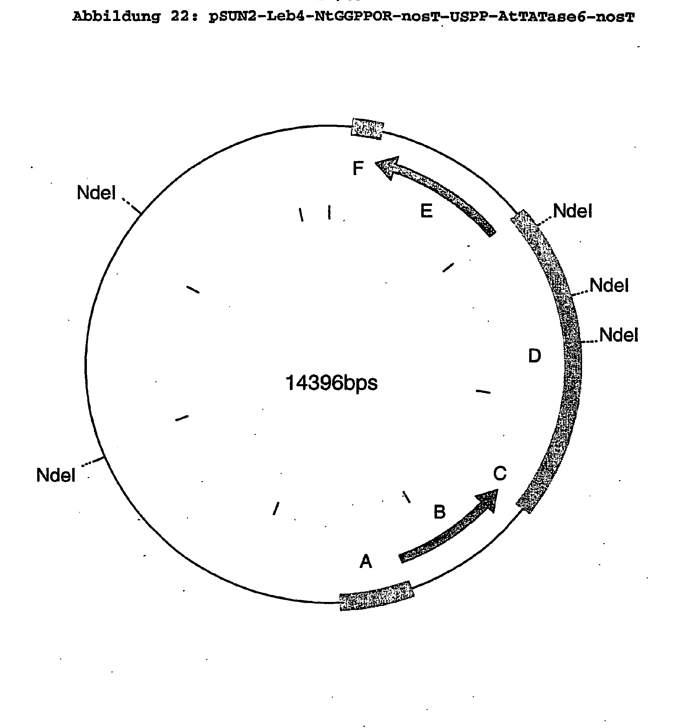

Dieses Plasmid (pSUN2-LeB4-NtGGPPORnosT/USPP-AtTATase6-nosT Abbildung 22) wird zur Erzeugung transgener *Brassica napus* Pflanzen verwendet.

Fragment A ( 678Bp) in Abbildung 22 beinhaltet den Promotor des "Unknow-Seed-Protein-Gens" aus *Vicia faba*, Fragment B (1243 Bp) kodiert für das Tyrosin-Aminotransferase-Gen 6 aus *Arabidopsis thaliana* und Fragment C (272Bp) kodiert für das Terminationssignal des Nopalin-Synthase-Gens. Fragment D (2764Bp) beinhaltet den Promotor des LeguminB4-Gens aus *Vicia faba*, Fragment E (1509Bp) kodiert für das Geranylgeranylpyrophosphate-Oxidoreduktase-Gen aus *Nicotiana tabacum*. Fragment F (272Bp) kodiert für das Terminationssignal des Nopalin-Synthase-Gens

### Beispiel 35

Erzeugung von DNA Konstrukten zur Expression der Tyrosin Aminotransferase aus *Rattus norvegicus* unter Kontrolle eines samenspezifischen Promotors und Hydroxyphenylpyruvat-Dioxygenase aus *Arabidopsis thaliana* unter Kontrolle eines samenspezifischen Promotors

Zur Herstellung von chimären DNA Konstrukten zur Erzeugung transgener *Brassica napus* Pflanzen, die die Tyrosin-Aminotransferase aus *Rattus norvegicus* unter Kontrolle eines samenspezifischen Promotors exprimieren, und die Hydroxyphenylpyruvat-Dioxygenase aus *Arabidopsis thaliana* samenspezifischen expremieren, werden die Vektoren pSUN2-USPP-AtHPPD-ocsT und pCR4topoblunt-USPP-rbcS-RnTATase1-nosT miteinander kombiniert.

Das DNA Fragment bestehend aus USP-Promotor, Tyrosin-Aminotransferase aus *Rattus norvegicus* und nos-Terminationssequenz wird aus dem Plasmid pCR4TOPOblunt/USPP-rbcS-RnTATasel-nosT als Srf1 Fragment isoliert und in den mit Srf1 verdauten Vektor pSUN2-USPP-AtHPPD-ocsT kloniert.

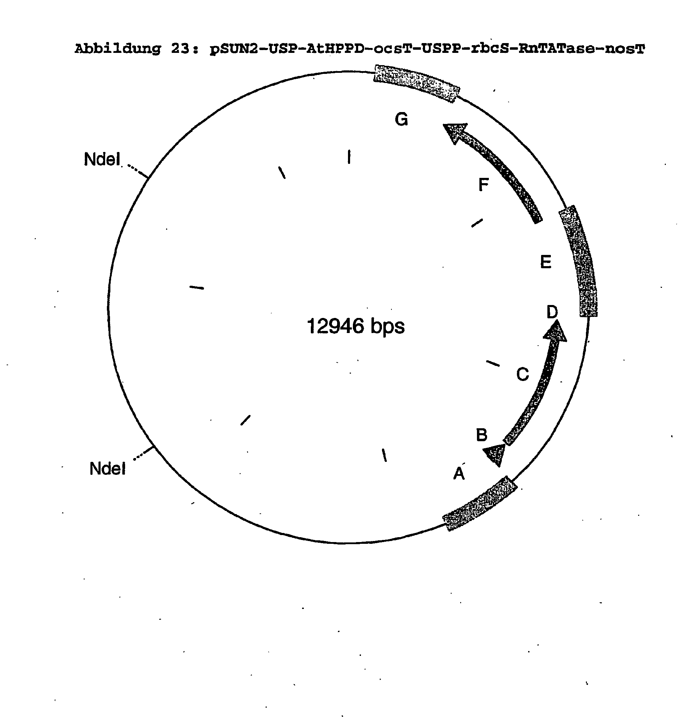

Dieses Plasmid (pSUN2-USPP-AtHPPD-ocsT/USPP-rbcS-RnTATase1-nosT Abbildung 23) wird zur Erzeugung transgener *Brassica napus* Pflanzen verwendet.

Fragment A (678Bp) in Abbildung 23 beinhaltet den Promotor des Unknow-Seed-Protein-Gens (USPP) aus *Vicia faba*, Fragment B (235Bp) kodiert für das Transitpeptid der Ribulose-Bisphosphat-Carboxylase *(rbcS) aus Vicia faba*. Fragment C (1365Bp) kodiert für das Tyrosin-Aminotransferase-Gen aus Rattus *norvegicus*. Fragment D (272Bp) kodiert für das Terminationssignal des Nopalin-Synthase Gens aus *A.tumefaciens*.
Fragment E (678Bp) beinhaltet den Promotor des "Unknow-Seed-Protein-Gens" aus *Vicia faba*, Fragment F (1338Bp) kodiert für das Hydroxyphenylpyruvate-Dioxygenase-Gen aus *Arabidopsis thaliana*. Fragment G (713Bp) kodiert für das Terminationssignal-1 des Octopin-Synthase Gens.

### Beispiel 36

Erzeugung von DNA Konstrukten zur Expression der Tyrosin Aminotransferase 1 aus *Arabidopsis thaliana* unter Kontrolle eines samenspezifischen Promotors und der Hydroxyphenylpyruvat-Dioxygenase aus *Arabidopsis, thaliana* unter Kontrolle eines samenspezifischen Promotors

Zur Herstellung von chimären DNA Konstrukten zur Erzeugung transgener *Brassica napus* Pflanzen, die die Tyrosin-Aminotransferase-1 aus *Arabidopsis thaliana* unter Kontrolle eines samenspezifischen Promotors exprimieren, und Hydroxyphenylpyruvat-Dioxygenase aus *Arabidopsis thaliana* samenspezifischen expremieren, werden die Vektoren pSUN2-USPP-AtHPPD-ocsT und pSUN2-USPP-AtTATase1-nosT miteinander kombiniert.

Das DNA Fragment kodierend für die Expressionskassette bestehend aus: USP-Promotor, Tyrosin-Aminotransferase-1-Gen aus *Arabidopsis thaliana* und nos-Terminator wird aus dem Plasmid pSUN2-USPP-AtTA-Tase1-nosT als Sma1/EcoR1 Fragment isoliert, das EcoR1 Ende mit dem Klenow Enzym aufgefüllt und in den mit Srf1 verdauten Vektor pSUN2-USPP-AtHPPD-ocsT kloniert.

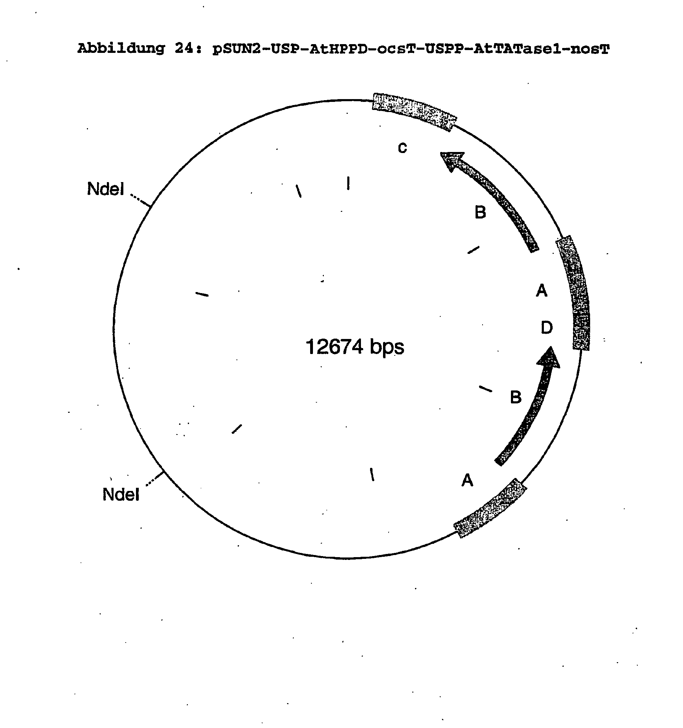

Dieses Plasmid (pSUN2-USPP-AtHPPD-ocsT /USPP-AtTATase1-nosT Abbildung 24) wird zur Erzeugung transgener *Brassica napus* Pflanzen verwendet
Fragment A (678Bp) in Abbildung 24 beinhaltet den Promotor des Unknow-Seed-Protein-Gens (USPP)aus *Vicia faba*, Fragment B (1269Bp) kodiert für das Tyrosin-Aminotransferase-Gen 1 aus *Arabidopsis thaliana* und Fragment C (272Bp) kodiert für das Terminationssignal des Nopalin-Synthase Gens. Fragment D (678Bp) beinhaltet den Promotor des "Unknow-Seed-Protein-Gens" aus *Vicia faba*, Fragment E (1338Bp) kodiert für das Hydroxyphenylpyruvate-Dioxygenase-Gen aus *Arabidopsis thaliana*. Fragment F (713Bp) kodiert für das Terminationssignal-1 des Octopin-Synthase Gens.

### Beispiel 37

Erzeugung von DNA Konstrukten zur Expression der Tyrosin Aminotransferase 3 aus *Arabidopsis thaliana* unter Kontrolle'eines samenspezifischen Promotors und der Hydroxyphenylpyruvat-Dioxygenase aus *Arabidopsis thaliana* unter Kontrolle eines samenspezifischen Promotors

Zur Herstellung von chimären DNA Konstrukten zur Erzeugung transgener *Brassica napus* Pflanzen, die die Tyrosin-Aminotransferase-3 aus *Arabidopsis thaliana* unter Kontrolle eines samenspezifischen Promotors exprimieren, und die Hydroxyphenylpyruvat-Dioxygenase aus *Arabidopsis thaliana* samenspezifischen expremieren, werden die Vektoren pSUN2-USPP-AtHPPD-ocsT und pSUN2-USPP-AtTATase3-nosT miteinander kombiniert.

Das DNA Fragment kodierend für die Expressionskassette bestehend aus: USP-Promotor, Tyrosin-Aminotransferase-3-Gen aus *Arabidopsis thaliana* und nos-Terminator wird aus dem Plasmid pSUN2-USPP-AtTA-Tase3-nosT als Sma1/FcoR1. Fragment isoliert, das EcoR1 Ende wird' mit dem Klenow Enzym aufgefüllt und in den mit Srf1 verdauten Vektor pSUN2-USPP-AtHPPD-ocsT kloniert.

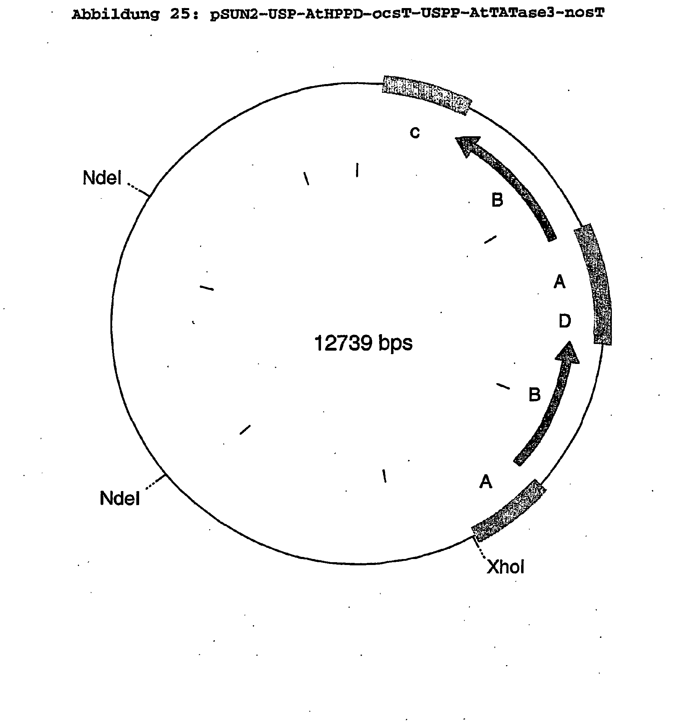

Dieses Plasmid (pSUN2-USPP-AtHPPD-ocsT /USPP-AtTATase3-nosT Abbildung 25) wird zur Erzeugung transgener *Brassica napus* Pflanzen verwendet.

Fragment A (678Bp) IN Abbildung 25 beinhaltet den Promotor des "Unknow-Seed-Protein-Gens" (USPP) aus *Vicia faba*, Fragment B (1334Bp) kodiert für das Tyrosin-Aminotransferase-Gen 3 aus Ara*bidopsis thaliana* und Fragment C (272Bp) kodiert für das Terminationssignal des Nopalin-Synthase Gens. Fragment D (678Bp) beinhaltet den Promotor des "Unknow-Seed-Protein-Gens" aus *Vicia faba*, Fragment E (1338Bp) kodiert für das Hydroxyphenylpyruvate-Dioxygenase-Gen aus *Arabidopsis thaliana*. Fragment F (713Bp) kodiert für das Terminationssignal-1 des Octopin-Synthase Gens.

### Beispiel 38

Erzeugung von DNA Konstrukten zur Expression der Tyrosin Aminotransferase 5 aus *Arabidopsis thaliana* unter Kontrolle eines samenspezifischen Promotors und der Hydroxyphenylpyruvat-Dioxygenase aus *Arabidopsis thaliana* unter Kontrolle eines samenspezifischen Promotors

Zur Herstellung von chimären DNA Konstrukten zur Erzeugung transgener *Brassica napus* Pflanzen, die die Tyrosin-Aminotransferase-5 aus *Arabidopsis thaliana* unter Kontrolle eines samenspezifischen Promotors exprimieren, und die Hydroxyphenylpyruvat-Dioxygenase aus *Arabidopsis thaliana* samenspezifischen expremieren, werden die Vektoren pSUN2-USPP-AtHPPD-ocsT und pSUN2-USPP-AtTATase5-nosT miteinander kombiniert.

Das DNA Fragment kodierend für die Expressionskassette bestehend aus: USP-Promotor, Tyrosin-Aminotransferase-5-Gen aus *Arabidopsis thaliana* und nos-Terminator wird aus dem Plasmid pSUN2-USPP-AtTA-Tase5-nosT als Sma1/EcoR1 Fragment isoliert, das EcoR1 Ende mit dem Klenow Enzym aufgefüllt und in den mit Srf1 verdauten Vektor pSUN2-SBPP-AtγTMT-35sT kloniert.

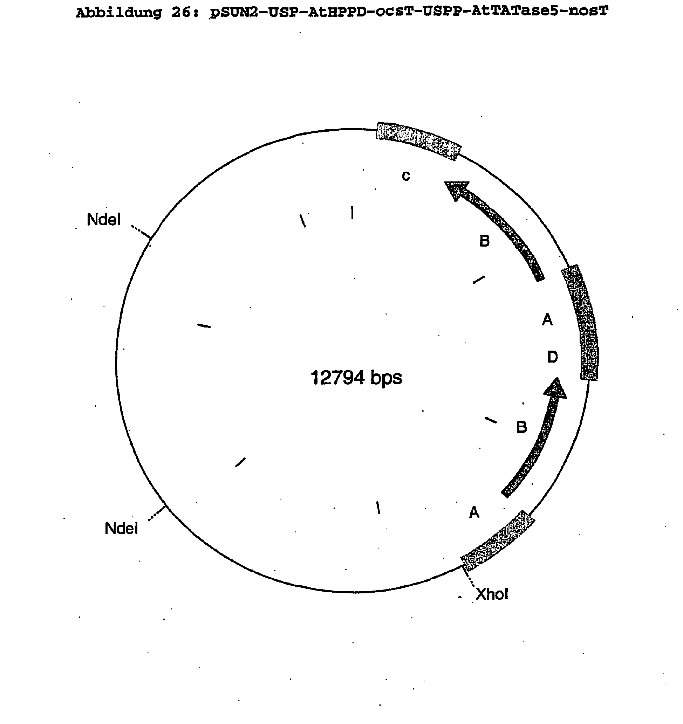

Dieses Plasmid (pSUN2-USPP-AtHPPD-ocsT /USPP-AtTATase5-nosT Abbildung 26) wird zur Erzeugung transgener *Brassica napus* Pflanzen verwendet.

Fragment A (678Bp) in Abbildung 26 beinhaltet den Promotor des "Unknow-Seed-Protein-Gens" aus *Vicia faba*, Fragment B (1389Bp) kodiert für das Tyrosin-Aminotransferase-Gen 5 aus *Arabidopsis thaliana* und Fragment C (272Bp) kodiert für das Terminationssignal des Nopalin-Synthase Gens. Fragment D (678Bp) beinhaltet den Promotor des "Unknow-Seed-Protein-Gens" aus *Vicia faba*, Fragment E (1338Bp) kodiert für das Hydroxyphenylpyruvate-Dioxygenase-Gen aus *Arabidopsis thaliana.* Fragment F (713Bp) kodiert für das Terminationssignal-1 des Octopin-Synthase Gens.

### Beispiel 39

Erzeugung von DNA Konstrukten zur Expression der Tyrosin Aminotransferase 6 aus *Arabidopsis thaliana* unter Kontrolle eines samenspezifischen Promotors und der Hydroxyphenylpyruvat-Dioxygenase aus *Arabidopsis thaliana* unter Kontrolle eines samenspezifischen Promotors

Zur Herstellung von chimären DNA Konstrukten zur Erzeugung transgener *Brassica napus* Pflanzen, die die Tyrosin-Aminotransferase-6 aus *Arabidopsis thaliana* unter Kontrolle eines samenspezifischen Promotors exprimieren, und die Hydroxyphenylpyruvat-Dioxygenase aus *Arabidopsis thaliana* samenspezifischen expremieren, werden die Vektoren pSUN2-USPP-AtHPPD-ocsT und pSUN2-USPP-AtTATase6-nosT miteinander kombiniert.

Das DNA Fragment kodierend für die Expressionskassette bestehend aus: USP-Promotor, Tyrosin-Aminotransferase-6-Gen aus *Arabidopsis thaliana* und nos-Terminator wird aus dem Plasmid pSUN2-USPP-AtTA-Tase6-nosT als Sma1/EcoR1 Fragment isoliert, das EcoR1 Ende wird mit dem Klenow Enzym aufgefüllt und in den mit Srf1 verdauten Vektor pSUN2-USPP-AtHPPD-ocsT kloniert.

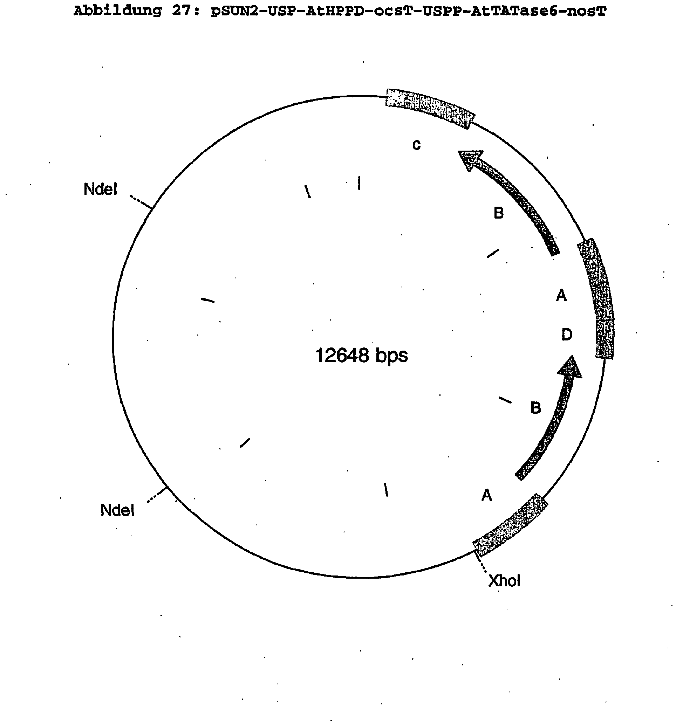

Dieses Plasmid (pSUN2-USPP-AtHPPD-ocsT/USPP-AtTATase6-nosT Abbildung 27) wird zur Erzeugung transgener *Brassica napus* Pflanzen verwendet

Fragment A (678Bp) in Abbildung 27 beinhaltet den Promotor des "Unknow-Seed-Protein-Gens" aus *Vicia faba*, Fragment B (1243 Bp) kodiert für das Tyrosin-Aminotransferase-Gen 6 aus *Arabidopsis thaliana* und Fragment C (272Bp) kodiert für das Terminationssignal des Nopalin-Synthase-Gens. Fragment D (678Bp) beinhaltet den Promotor des "Unknow-Seed-Protein-Gens" aus *Vicia faba*, Fragment E (1338Bp) kodiert für das Hydroxyphenylpyruvate-Dioxygenase-Gen aus *Arabidopsis thaliana*. Fragment F (713Bp) kodiert für das Terminationssignal-1 des Octopin-Synthase Gens.

### Beispiel 40

Erzeugung von DNA Konstrukten zur Expression der Tyrosin Aminotransferase aus *Rattus norvegicus* unter Kontrolle eines samenspezifischen Promotors und Homogentisinsäure-Phytyltransferase aus *Arabidopsis thaliana* unter Kontrolle eines samenspezifischen Promotors

Zur Herstellung von chimären DNA Konstrukten zur Erzeugung transgener *Brassica napus* Pflanzen, die die Tyrosin-Aminotransferase aus *Rattus norvegicus* unter Kontrolle eines samenspezifischen Promotors exprimieren, und die Homogentisinsäure-Phytyltransferase aus *Arabidopsis thaliana* samenspezifischen expremieren, werden die Vektoren pSUN2-USPP-AtHPT-ocsT und pCR4topoblunt-USPP-rbcS-RnTATasel-nosT miteinander kombiniert.

Das DNA Fragment bestehend aus USP-Promotor, Tyrosin-Aminotransferase aus *Rattus norvegicus* und nos-Terminationssequenz wird aus dem Plasmid pCR4TOPOblunt/USPP-rbcS-RnTATase1-nosT als Srf1 Fragment isoliert und in den mit Srf1 verdauten Vektor pSUN2-USPP-AtHPT-ocsT kloniert.

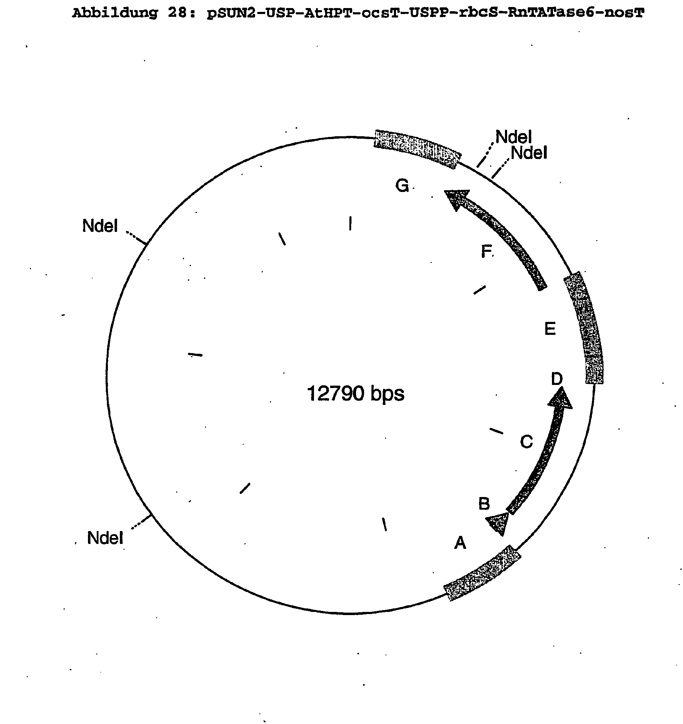

Dieses Plasmid (pSUN2-USPP-AtHPT-ocsT/USPP-rbcS-RnTATase1-nosT (Abbildung 28) wird zur Erzeugung transgener *Brassica napus* Pflanzen verwendet.

Fragment A (678Bp) in Abbildung 28 beinhaltet den Promotor des Unknow-Seed-Protein-Gens (USPP) aus *Vicia faba*, Fragment B (235Bp) kodierend für das Transitpeptid der Ribulose-Bisphosphat-Carboxylase *(rbcS) aus Vicia faba*. Fragment C (1365Bp) kodiert für das Tyrosin-Aminotransferase-Gen aus *Rattus norvegicus*. Fragment D (272Bp) für das Terminationssignal des Nopalin-Synthase Gens aus *A.tumefaciens*.
Fragment E (678Bp) beinhaltet den Promotor des "Unknow-Seed-Protein-Gens" aus *Vicia faba*, Fragment F (1182Bp) kodiert für das Homogentisinsäure-Phytyltransferase-Gen aus *Arabidopsis thaliana*. Fragment G (713Bp) kodiert für das Terminationssignal-1 des Octopin-Synthase Gens.

### Beispiel 41

Erzeugung von DNA Konstrukten zur Expression der Tyrosin Aminotransferase 1 aus *Arabidopsis thaliana* unter Kontrolle eines samenspezifischen Promotors und der Homogentisinsäure-Phytyltransferase aus *Arabidopsis thaliana* unter Kontrolle eines samenspezifischen Promotors

Zur Herstellung von chimären DNA Konstrukten zur Erzeugung transgener *Brassica napus* Pflanzen, die die Tyresin-Aminotransferase-1 aus *Arabidopsis thaliana* unter Kontrolle eines samenspezifischen Promotors exprimieren, und Homogentisinsäure-Phytyltransferase aus *Arabidopsis thaliana* samenspezifischen expremieren, werden die Vektoren pSUN2-USPP-AtHPT-ocsT und pSUN2-USPP-AtTATasel-nosT miteinander kombiniert.

Das DNA Fragment kodierend für die Expressionskassette bestehend aus: USP-Promotor, Tyrosin-Aminotransferase-1-Gen aus *Arabidopsis thaliana* und nos-Terminator wird aus dem Plasmid pSUN2-USPP-AtTA-Tase1-nosT als Sma1/EcoR1 Fragment isoliert, das EcoR1 Ende mit dem Klenow Enzym aufgefüllt und in den mit Srf1 verdauten Vektor pSUN2-USPP-AtHPT-ocsT kloniert.

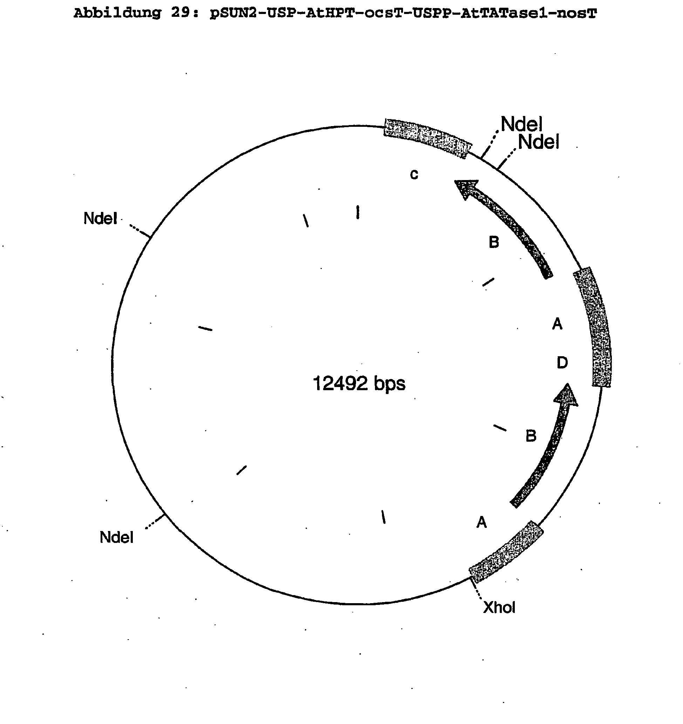

Dieses Plasmid (pSUN2-USPP-AtHPT-ocsT/USPP-AtTATasel-nosT Abbildung 29) wird zur Erzeugung transgener *Brassica napus* Pflanzen verwendet

Fragment A (678Bp) in Abbildung 29 beinhaltet den Promotor des Unknow-Seed-Protein-Gens (USPP)aus *Vicia faba*, Fragment B (1269Bp) kodiert für das Tyrosin-Aminotransferase-Gen 1 aus Ara*bidopsis thaliana* und Fragment C (272Bp) kodiert für das Terminationssignal des Nopalin-Synthase Gens. Fragment D (678Bp) beinhaltet den Promotor des "Unknow-Seed-Protein-Gens" aus *Vicia faba*, Fragment E (1182Bp) kodiert für das Homogentisinsäure-Phytyltransferase-Gen aus *Arabidopsis thaliana*. Fragment F (713Bp) kodiert für das Terminationssignal-1 des Octopin-Synthase Gens.

### Beispiel 42

Erzeugung von DNA Konstrukten zur Expression der Tyrosin Aminotransferase 3 aus *Arabidopsis thaliana* unter Kontrolle eines samenspezifischen Promotors und der Homogentisinsäure-Phytyltransferase aus *Arabidopsis thaliana* unter Kontrolle eines samenspezifischen Promotors

Zur Herstellung von chimären DNA Konstrukten zur Erzeugung transgener *Brassica napus* Pflanzen, die die Tyrosin-Aminotransferase-3 aus *Arabidopsis thaliana* unter Kontrolle eines samenspezifischen Promotors exprimieren, und die Homogentisinsäure-Phytyltransferase aus *Arabidopsis thaliana* samenspezifischen expremieren, werden die Vektoren pSUN2-USPP-AtHPT-ocsT und pSUN2-USPP-AtTA-Tase3-nosT miteinander kombiniert.

Das DNA Fragment kodierend für die Expressionskassette betstehend aus: USP-Promotor, Tyrosin-Aminotransferase-3-Gen aus *Arabidopsis thaliana* und nos-Terminator wird aus dem Plasmid pSUN2-USPP-AtTA-Tase3-nosT als Sma1/EcoR1 Fragment isoliert, das EcoR1 Ende mit dem Klenow Enzym aufgefüllt und in den mit Srf1 verdauten Vektor pSUN2-USPP-AtHPT-ocsT kloniert.

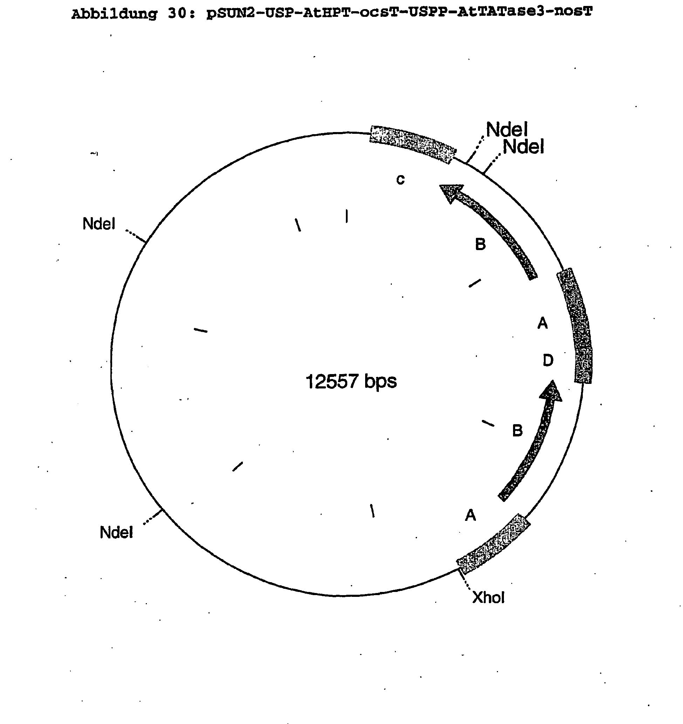

Dieses Plasmid (pSUN2-USPP-AtHPT-ocsT /USPP-AtTATase3-nosT Abbildung 30) wird zur Erzeugung transgener *Brassica napus* Pflanzen verwendet

Fragment A (678Bp) in Abbildung 30 beinhaltet den Promotor des "Unknow-Seed-Protein-Gens" (USPP) aus *Vicia faba*, Fragment B (1334Bp) kodiert für das Tyrosin-Aminotransferase-Gen 3 aus Ara*bidopsis thaliana* und Fragment C (272Bp) kodiert für das Terminationssignal des Nopalin-Synthase Gens. Fragment D (678Bp) beinhaltet den Promotor des "Unknow-Seed-Protein-Gens" aus *Vicia faba*, Fragment E (1182Bp) kodiert für das Homogentisinsäure-Phytyltransferase-Gen aus *Arabidopsis*, *thaliana*. Fragment F (713Bp) kodiert für das Terminationssignal-1 des Octopin-Synthase Gens.

### Beispiel 43

Erzeugung von DNA Konstrukten zur Expression der Tyrosin Aminotransferase 5 aus *Arabidopsis thaliana* unter Kontrolle eines samenspezifischen Promotors und der Homogentisinsäure-Phytyltransferase aus *Arabidopsis thaliana* unter Kontrolle eines samenspezifischen Promotors

Zur Herstellung von chimären DNA Konstrukten zur Erzeugung transgener *Brassica napus* Pflanzen, die die Tyrosin-Aminotransferase-5 aus *Arabidopsis thaliana* unter Kontrolle eines samenspezifischen Promotors exprimieren, und die Homogentisinsäure-Phytyltransferase aus *Arabidopsis thaliana* samenspezifischen expremieren, werden die Vektoren pSUN2-USPP-AtHPT-ocsT und pSUN2-USPP-AtTA-Tase5-nosT miteinander kombiniert.

Das DNA Fragment kodierend für die Expressionskassette bestehend aus: USP-Promotor, Tyrosin-Aminotransferase-5-Gen aus *Arabidopsis thaliana* und nos-Terminator wird aus dem Plasmid pSUN2-USPP-AtTA-Tase5-nosT als Sma1/EcoR1 Fragment isoliert, das EcoR1 Ende mit dem Klenow Enzym aufgefüllt und in den mit Srf1 verdauten Vektor pSUN2- USPP-AtHPT-ocsT kloniert.

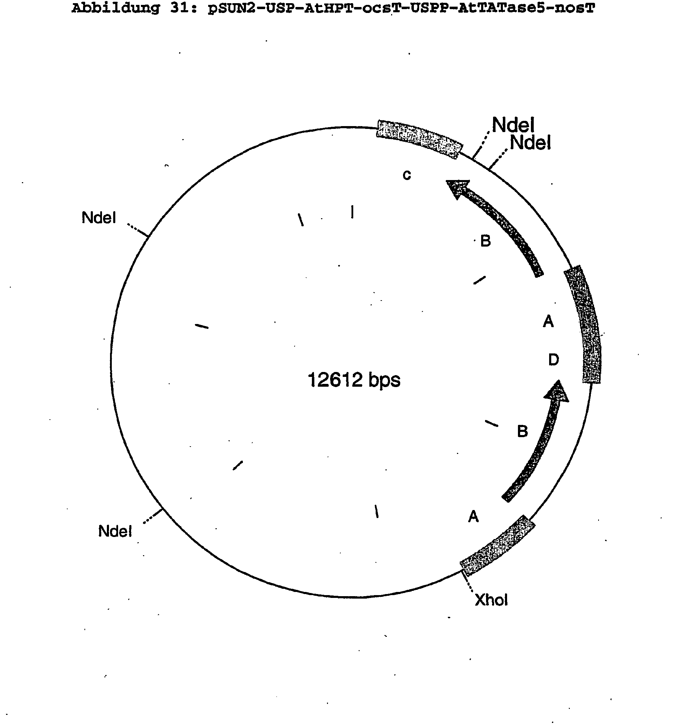

Dieses Plasmid (pSUN2-USPP-AtHPT-ocsT/USPP-AtTATase5-nosT Abbildung 31) wird zur Erzeugung transgener *Brassica napus* Pflanzen verwendet

Fragment A (678Bp) in Abbildung 31 beinhaltet den Promotor des "Unknow-Seed-Protein-Gens" aus *Vicia faba*, Fragment B (1389Bp) kodiert für das Tyrosin-Aminotransferase-Gen 5 aus *Arabidopsis thaliana* und Fragment C (272Bp) kodiert für das Terminationssignal des Nopalin-Synthase Gens. Fragment D (678Bp) beinhaltet den Promotor des "Unknow-Seed-Protein-Gens" aus *Vicia faba*, Fragment E (1182Bp) kodiert für das Homogentisinsäure-Phytyltransferase-Gen aus *Arabidopsis thaliana*. Fragment F (713Bp) kodiert für das Terminationssignal-1 des Octopin-Synthase Gens.

### Beispiel 44

Erzeugung von DNA Konstrukten zur Expression der Tyrosin Aminotransferase 6 aus *Arabidopsis thaliana* unter Kontrolle eines samenspezifischen Promotors und der Homogentisinsäure-Phytyltransferase aus *Arabidopsis thaliana* unter Kontrolle eines samenspezifischen Promotors

Zur Herstellung von chimären DNA Konstrukten zur Erzeugung transgener *Brassica napus* Pflanzen, die die Tyrosin-Aminotransferase-6 aus *Arabidopsis thaliana* unter Kontrolle eines samenspezifischen Promotors exprimieren, und die Homogentisinsäure-Phytyltransferase aus *Arabidopsis thaliana* samenspezifischen expremieren, werden die Vektoren pSUN2-USPP-AtHPT-ocsT und pSUN2-USPP-AtTA-Tase6-nosT miteinander kombiniert.
Das DNA Fragment kodierend für die Expressionskassette bestehend aus: USP-Promotor, Tyrosin-Aminotransferase-6-Gen aus *Arabidopsis thaliana* und nos-Terminator wird aus dem Plasmid pSUN2-USPP-AtTA-Tase6-nosT als Sma1/EcoR1 Fragment isoliert, das EcoR1 Ende wird mit dem Klenow Enzym aufgefüllt und in den mit Srf1 verdauten Vektor pSUN2-USPP-AtHPT-ocsT kloniert.

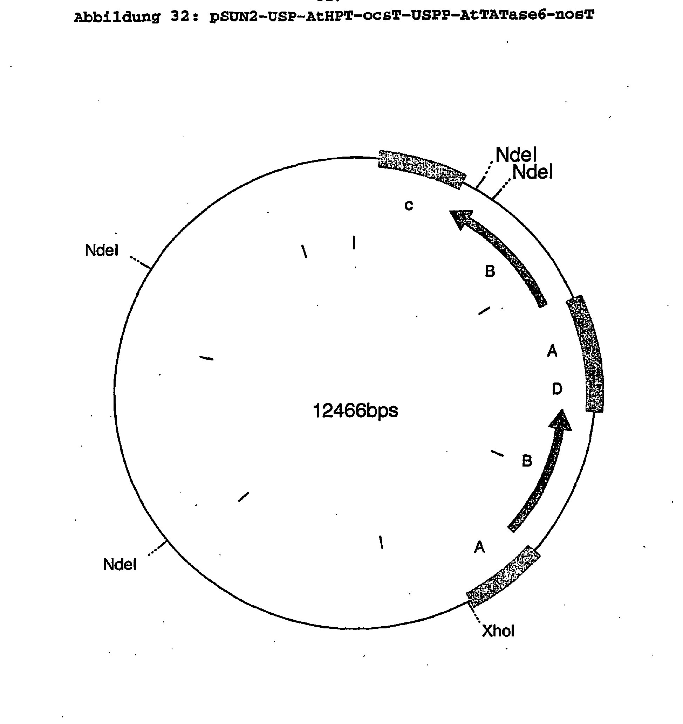

Dieses Plasmid (pSUN2-USPP-AtHPT-ocsT/USPP-AtTATase6-nosT Abbildung 32) wird zur Erzeugung transgener *Brassica napus* Pflanzen verwendet

Fragment A (678Bp) in Abbildung 32 beinhaltet den Promotor des "Unknow-Seed-Protein-Gens" aus *Vicia faba*, Fragment B (1243 Bp) kodiert für das Tyrosin-Aminotransferase-Gen 6 aus *Arabidopsis thaliana* und Fragment C (272Bp) kodiert für das Terminationssignal des Nopalin-Synthase-Gens. Fragment D (678Bp) beinhaltet den Promotor des "Unknow-Seed-Protein-Gens" aus *Vicia faba*, Fragment E (1182Bp) kodiert für das Homogentisinsäure-Phytyltransferase-Gen aus *Arabidopsis thaliana*. Fragment F (713Bp) kodiert für das Terminationssignal-1 des Octopin-Synthase Gens.

### Beispiel 45

Erzeugung von DNA Konstrukten zur Expression der Tyrosin Aminotransferase aus Rattus *norvegicus* unter Kontrolle eines samenspezifischen Promotors und 2-Methyl-6-Phytylhydrochinon-Methyltransferase aus *Synechocystis* sp. PCC6803 unter Kontrolle eines samenspezifischen Promotors Zur Herstellung von chimären DNA Konstrukten zur Erzeugung transgener *Brassica napus* Pflanzen, die die Tyrosin-Aminotransferase aus *Rattus norvegicus* unter Kontrolle eines samenspezifischen Promotors exprimieren, und die 2-Methyl-6-Phytylhydrochinon-Methyltransferase aus *Synechocystis* sp. PCC6803 samenspezifischen expremieren, werden die Vektoren pSUN2-LeB4-IPP-SynMT1-nosT und pCR4topoblunt-USPP-rbcS-RnTA-Tase1-nosT miteinander kombiniert.
Das DNA Fragment bestehend aus USP-Promotor, Tyrosin-Aminotransferase aus *Rattus norvegicus* und nos-Terminationssequenz wird aus dem Plasmid pCR4TOPOblunt/USPP-rbcS-RnTATase1-nosT als Srf1 Fragment isoliert und in den mit Srf1 verdauten Vektor pSUN2-LeB4-IPP-SynMT1-nosT kloniert.

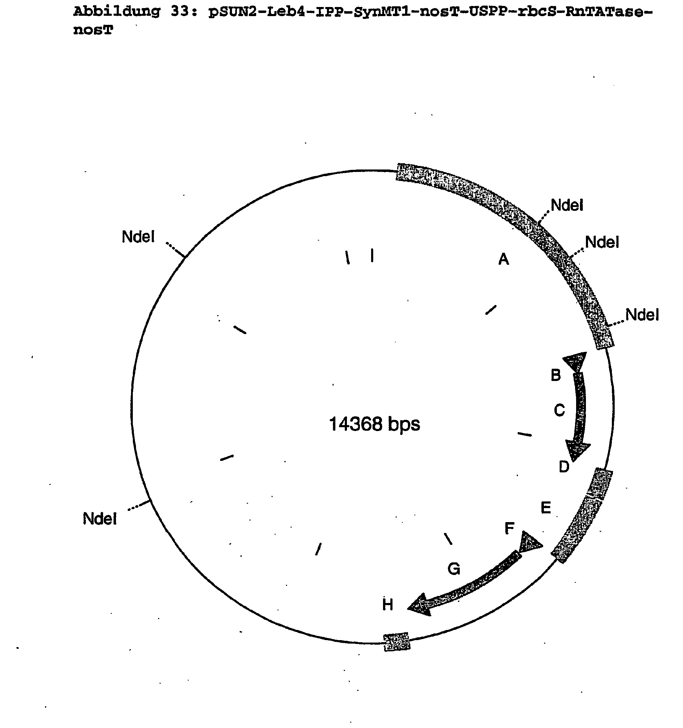

Dieses Plasmid (pSUN2-LeB4-IPP-SynMT1-nosT/USPP-rbcS-RnTA-Tase1-nosT Abbildung 33) wird zur Erzeugung transgener *Brassica napus* Pflanzen verwendet.

Fragment A (2764Bp) in Abbildung 33 beinhaltet den Promotor des LeguminB4-Gens aus *Vicia faba*, Fragment B (235Bp) kodiert für das Transitpeptid der *A.thaliana* Isopentenyl-pyrophosphat-Isomerase-2. Fragment C (957Bp) kodiert für das 2-Methyl-6-Phytylhydrochinol Methyltransferase Gen aus *Synechocystis* sp. PCC6803. Fragment D (272Bp) kodiert für das Terminationssignal des Nopalin-Synthase-Gens. Fragment E (678Bp) beinhaltet den Promotor des Unknow-Seed-Protein-Gens (USPP) aus *Vicia faba*, Fragment F (235Bp) kodiert für das Transitpeptid der Ribulose-Bisphosphat-Carboxylase *(rbcS)* aus *Vicia faba*. Fragment G (1365Bp) kodiert für das Tyrosin-Aminotransferase-Gen aus *Rattus norvegicus.* Fragment H (272Bp) kodiert für das Terminationssignal des Nopalin-Synthase Gens aus *A.tumefaciens*.

### Beispiel 46

Erzeugung von DNA Konstrukten zur Expression der Tyrosin Aminotransferase 1 aus *Arabidopsis thaliana* unter Kontrolle eines samenspezifischen Promotors und der 2-Methyl-6-Phytylhydrochinon-Methyltransferase aus *Synechocystis* sp. PCC6803 unter Kontrolle eines samenspezifischen Promotors

Zur Herstellung von chimären DNA Konstrukten zur Erzeugung transgener *Brassica napus* Pflanzen, die die Tyrosin-Aminotransferase-1 aus *Arabidopsis thaliana* unter Kontrolle eines samenspezifischen Promotors exprimieren, und 2-Methyl-6-Phytylhydrochinon-Methyltransferase aus *Synechocystis* sp. PCC6803 samenspezifischen expremieren, werden die Vektoren pSUN2-LeB4-IPP-SynMT1-nosT und pSUN2-USPP-AtTATase1-nosT miteinander kombiniert.

Das DNA Fragment kodierend für die Expressionskassette bestehend aus: USP-Promotor, Tyrosin-Aminotransferase-1-Gen aus *Arabidopsis thaliana* und nos-Terminator wird aus dem Plasmid pSUN2-USPP-AtTA-Tase1-nosT als Sma1/EcoR1 Fragment isoliert, das EcoR1 Ende mit dem Klenow Enzym aufgefüllt und in den mit Srf1 verdauten Vektor pSUN2-LeB4-IPP-SynMT1-nosT kloniert.

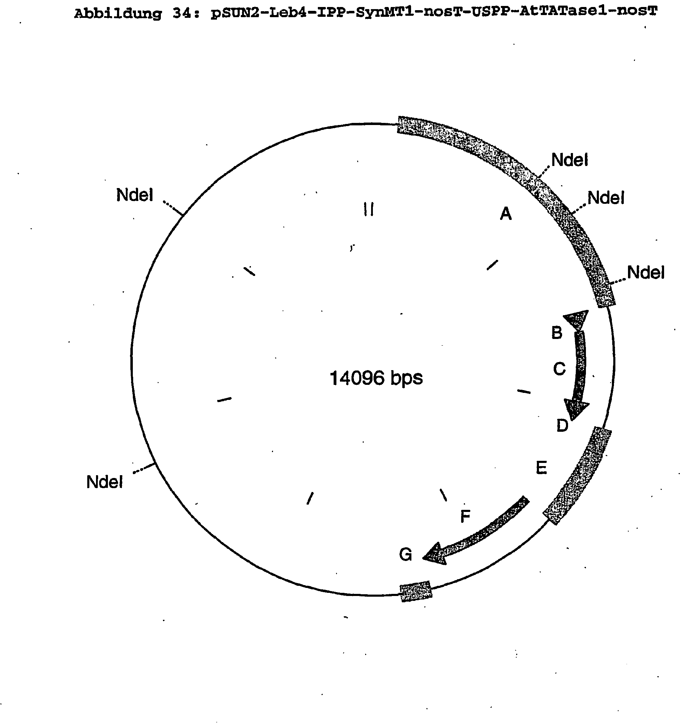

Dieses Plasmid (pSUN2-LeB4-IPP-SynMT1-nosT/USPP-AtTATasel-nosT Abbildung 34) wird zur Erzeugung transgener *Brassica napus* Pflanzen verwendet

Fragment A (2764Bp) in Abbildung 34 beinhaltet den Promotor des LeguminB4-Gens aus *Vicia faba*, Fragment B (235Bp) kodiert für das Transitpeptid der *A.thaliana* Isopentenyl-pyrophosphat-Isomerase-2. Fragment C (957Bp) kodiert für das 2-Methyl-6-Phytylhydrochinol Methyltransferase Gen aus *Synechocystis* sp. PCC6803. Fragment D (272Bp) kodiert für das Terminationssignal des Nopalin-Synthase-Gens. Fragment E ( 678Bp) beinhaltet den Promotor des Unknow-Seed-Protein-Gens (USPP)aus *Vicia faba*, Fragment F (1269Bp) kodiert für das Tyrosin-Aminotransferase-Gen 1 aus *Arabidopsis thaliana*. Fragment G (272Bp) kodiert für das Terminationssignal des Nopalin-Synthase Gens.

### Beispiel 47

Erzeugung von DNA Konstrukten zur Expression der Tyrosin Aminotransferase 3 aus *Arabidopsis thaliana* unter Kontrolle eines samenspezifischen Promotors und der 2-Methyl-6-Phytylhydrochinon-Methyltransferase aus *Synechocystis* sp. PCC6803 unter Kontrolle eines samenspezifischen Promotors

Zur Herstellung von chimären DNA Konstrukten zur Erzeugung transgener *Brassica napus* Pflanzen, die die Tyrosin-Aminotransferase-3 aus *Arabidopsis thaliana* unter Kontrolle eines samenspezifischen Promotors exprimieren, und die 2-Methyl-6-Phytylhydrochinon-Methyltransferase aus *Synechocystis* sp. PCC6803 samenspezifischen expremieren, werden die Vektoren pSUN2-LeB4-IPP-SynMT1-nosT und pSUN2-USPP-AtTATase3-nosT miteinander kombiniert.

Das DNA Fragment kodierend für die Expressionskassette bestehend aus: USP-Promotor, Tyrosin-Aminotransferase-3-Gen aus *Arabidopsis thaliana* und nos-Terminator wird aus dem Plasmid pSUN2-USPP-AtTA-Tase3-nosT als Sma1/EcoR1 Fragment isoliert, das EcoR1 Ende wird mit dem Klenow Enzym aufgefüllt und in den mit Srf1 verdauten Vektor pSUN2-LeB4-IPP-SynMT1-nosT kloniert.

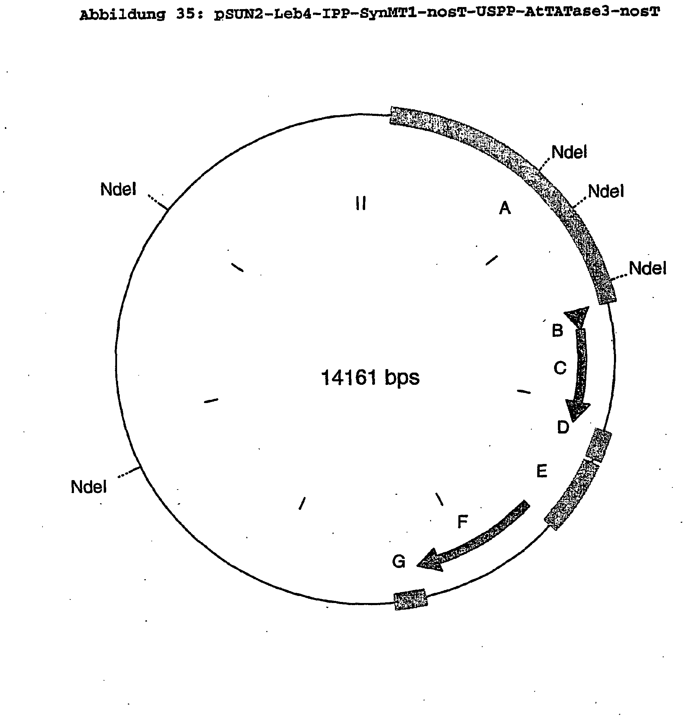

Dieses Plasmid (pSUN2-LeB4-IPP-SynMT1-nosT /USPP-AtTATase3-nosT Abbildung 35) wird zur Erzeugung transgener *Brassica napus* Pflanzen verwendet.

Fragment A (2764Bp) in Abbildung 35 beinhaltet den Promotor des LeguminB4-Gens aus *Vicia faba*, Fragment B (235Bp) kodiert für das Transitpeptid der *A.thaliana* Isopentenyl-pyrophosphat-Isomerase-2. Fragment C (957Bp) kodiert für das 2-Methyl-6-Phytylhydrochinol Methyltransferase Gen aus *Synechocystis* sp. PCC6803. Fragment D (272Bp) kodiert für das Terminationssignal des Nopalin-Synthase-Gens. Fragment E( 678Bp) beinhaltet den Promotor des "Unknow-Seed-protein-Gens" (USPP) aus *Vicia faba*, Fragment F (1334Bp) kodiert für das Tyrosin-Aminotransferase-Gen 3 aus *Arabidopsis thaliana*. Fragment G (272Bp) kodiert für das Terminationssignal des Nopalin-Synthase Gens.

### Beispiel 48

Erzeugung von DNA Konstrukten zur Expression der Tyrosin Aminotransferase 5 aus *Arabidopsis thaliana* unter Kontrolle eines samenspezifischen Promotors und der 2-Methyl-6-Phytylhydrochinon-Methyltransferase aus *Synechocystis* sp. PCC6803 unter Kontrolle eines samenspezifischen Promotors

Zur Herstellung von chimären DNA Konstrukten zur Erzeugung transgener *Brassica napus* Pflanzen, die die Tyrosin-Aminotransferase-5 aus *Arabidopsis thaliana* unter Kontrolle eines samenspezifischen Promotors exprimieren, und die 2-Methyl-6-Phytylhydrochinon-Methyltransferase aus *Synechocystis* sp. PCC6803 samenspezifischen expremieren, werden die Vektoren pSUN2-LeB4-IPP-SynMT1-nosT und pSUN2-USPP-AtTATase5-nosT miteinander kombiniert.

Das DNA Fragment kodierend für die Expressionskassette bestehend aus: USP-Promotor, Tyrosin-Aminotransferase-5-Gen aus *Arabidopsis thaliana* und nos-Terminator wird aus dem Plasmid pSUN2-USPP-AtTA-Tase5-nosT als Sma1/EcoR1 Fragment isoliert, das EcoR1 Ende mit dem Klenow Enzym aufgefüllt und in den mit Srf1 verdauten Vektor pSUN2- LeB4-IPP-SynMT1-nosT kloniert.

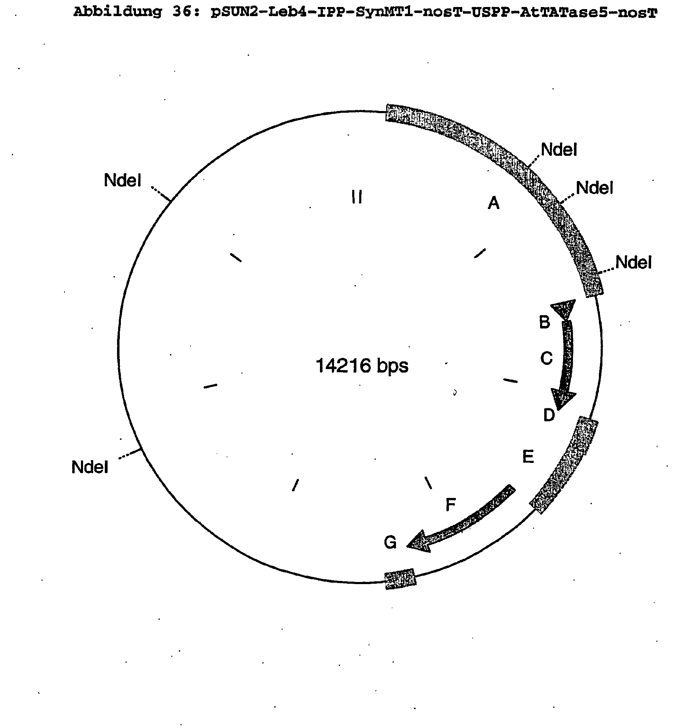

Dieses Plasmid (pSUN2-LeB4-IPP-SynMT1-nosT/USPP-AtTATase5-nosT Abbildung 36) wird zur Erzeugung transgener *Brassica napus* Pflanzen verwendet:

Fragment A (2764Bp) in Abbildung 36 beinhaltet den Promotor des LeguminB4-Gens aus *Vicia faba*, Fragment B (235Bp) kodiert für das Transitpeptid der *A.thaliana* Isopentenyl-pyrophosphat-Isomerase-2. Fragment C (957Bp) kodiert für das 2-Methyl-6-Phytylhydrochinol Methyltransferase Gen aus *Synechocystis* sp. PCC6803. Fragment D (272Bp) kodiert für das Terminationssignal des Nopalin-Synthase-Gens. Fragment E ( 678Bp) beinhaltet den Promotor des "Unknow-Seed-Protein-Gens" aus *Vicia faba*, Fragment F (1389Bp) kodiert für das Tyrosin-Aminotransferase-Gen 5 aus *Arabidopsis thaliana*. Fragment G (272Bp) kodiert für das Terminationssignal des Nopalin-Synthase Gens

### Beispiel 49

Erzeugung von DNA Konstrukten zur Expression der Tyrosin Aminotransferase 6 aus *Arabidopsis thaliana* unter Kontrolle eines samenspezifischen Promotors und der 2-Methyl-6-Phytylhydrochinon-Methyltransferase aus *Synechocystis* sp. PCC6803 unter Kontrolle eines samenspezifischen Promotors

Zur Herstellung von chimären DNA Konstrukten zur Erzeugung transgener *Brassica napus* Pflanzen, die die Tyrosin-Aminotransferase-6 aus *Arabidopsis thaliana* unter Kontrolle eines samenspezifischen Promotors exprimieren, und die 2-Methyl-6-Phytylhydrochinon-Methyltransferase aus *Synechocystis* sp. PCC6803 samenspezifischen expremieren, werden die Vektoren pSUN2-LeB4-IPP-SynMT1-nosT und pSUN2-USPP-AtTATase6-nosT miteinander kombiniert.

Das DNA Fragment kodierend für die Expressionskassette bestehend aus: USP-Promotor, Tyrosin-Aminotransferase-6-Gen aus *Arabidopsis thaliana* und nos-Terminator wird aus dem Plasmid pSUN2-USPP-AtTA-Tase6-nosT als Sma1/EcoR1 Fragment isoliert, das EcoR1 Ende wird mit dem Klenow Enzym aufgefüllt und in den mit Srf1 verdauten Vektor pSUN2-LeB4-IPP-SynMT1-nosT kloniert.

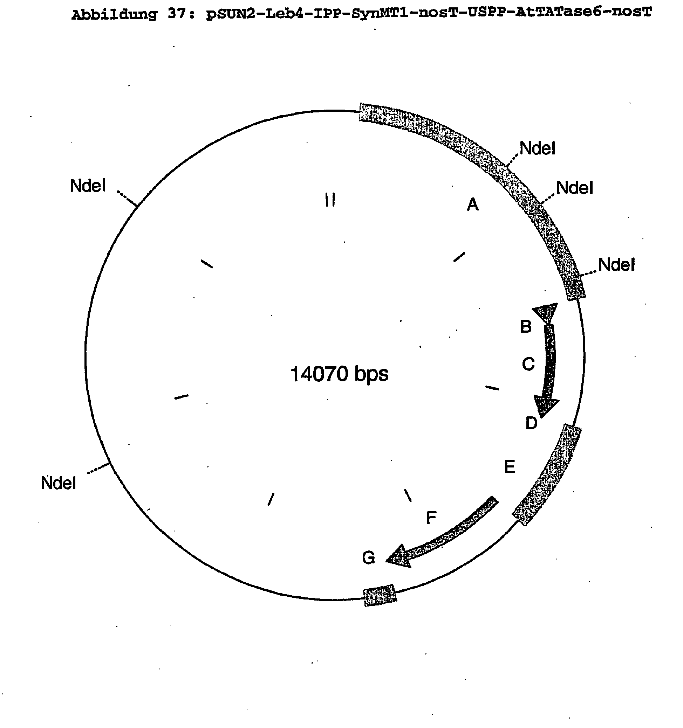

Dieses Plasmid (pSUN2-LeB4-IPP-SynMT1-nosT/USPP-AtTATase6-nosT Abbildung 37) wird zur Erzeugung transgener *Brassica napus* Pflanzen verwendet:

Fragment A (2764Bp) in Abbildung 37 beinhaltet den Promotor des LeguminB4-Gens aus *Vicia faba*, Fragment B (235Bp) kodiert für das Transitpeptid der *A.thaliana* Isopentenyl-pyrophosphat-Isomerase-2. Fragment C (957Bp) kodiert für das 2-Methyl-6-Phytylhydrochinol Methyltransferase Gen aus *Synechocystis* sp. PCC6803. Fragment D (272Bp) kodiert für das Terminationssignal des Nopalin-Synthase-Gens. Fragment E (678Bp) beinhaltet den Promotor des "Unknow-Seed-Protein-Gens" aus *Vicia faba*, Fragment F (1243 Bp) kodiert für das Tyrosin-Aminotransferase-Gen 6 aus *Arabidopsis thaliana*. Fragment G (272Bp) kodiert für das Terminationssignal des Nopalin-Synthase-Gens.

### Beispiel 50

Erzeugung von DNA Konstrukten zur Expression der Tyrosin Aminotransferase aus *Rattus norvegicus* unter Kontrolle eines samenspezifischen Promotors und 2,3-Dimethyl-5-Phytylplastochinol-Zyklase aus *Synechocystis* sp. PCC6803 unter Kontrolle eines samenspezifischen Promotors

Zur Herstellung von chimären DNA Konstrukten zur Erzeugung transgener *Brassica napus* Pflanzen, die die Tyrosin-Aminotransferase aus *Rattus norvegicus* unter Kontrolle eines samenspezifischen Promotors exprimieren, und die 2,3-Dimethyl-5-Phytylplastochinol-Zyklase aus *Synechocystis* sp. PCC6803 samenspezifischen expremieren, werden die Vektoren pSUN2-LeB4-IPP-SynCyc-nosT und pCR4topoblunt-USPP-rbcS-RnTATase1-nosT miteinander kombiniert.

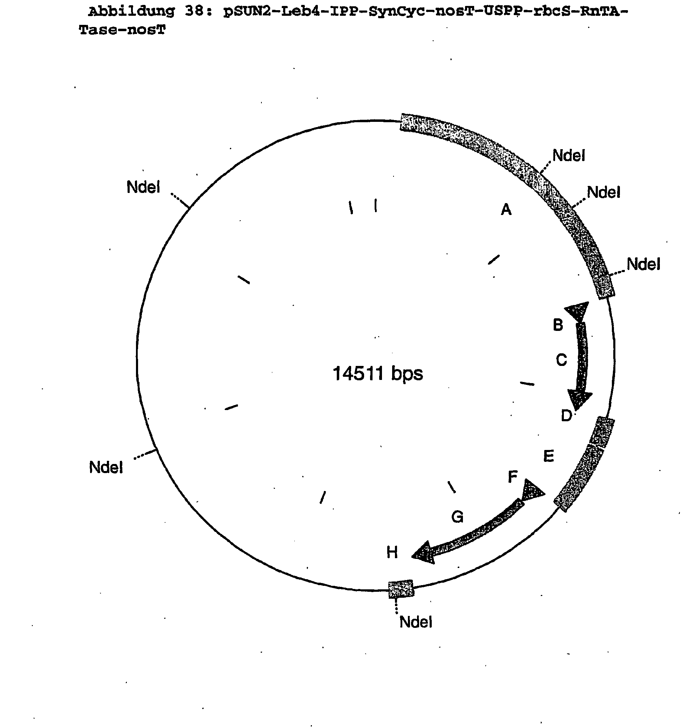

Das DNA Fragment bestehend aus USP-Promotor, Tyrosin-Aminotransferase aus *Rattus norvegicus* und nos-Terminationssequenz wird aus dem Plasmid pCR4TOPOblunt/USPP-rbcS-RnTATase1-nosT als Srf1 Fragment isoliert und in den mit EcoR1 verdauten Vektor pSUN2-LeB4-IPP-SynCyc-nosT kloniert, dessen EcoR1 Enden ebenfalls aufgefüllt werden.
Dieses Plasmid (pSUN2-LeB4-IPP-SynCyc-nosT/USPP-rbcS-RnTA-Tase1-nosT Abbildung 38) wird zur Erzeugung transgener *Brassica napus* Pflanzen verwendet.

Fragment A (2764Bp) in Abbildung 38 beinhaltet den Promotor des LeguminB4-Gens aus *Vicia faba*, Fragment B (235Bp) kodiert für das Transitpeptid der *A.thaliana* Isopentenyl-pyrophosphat-Isomerase-2. Fragment C (1100Bp) kodiert für das 2,3-Dimethyl-5-Phytylplastoquinol Zyklase Gen aus *Synechocystis* sp. PCC6803. Fragment D (272Bp) kodiert für das Terminationssignal des Nopalin-Synthase-Gens. Fragment E ( 678Bp) beinhaltet den Promotor des Unknow-Seed-Protein-Gens (USPP) aus *Vicia faba*, Fragment F (235Bp) kodiert für das Transitpeptid der Ribulose-Bisphosphat-Carboxylase *(rbcS) aus Vicia faba*. Fragment G (1365Bp) kodiert für das Tyrosin-Aminotransferase-Gen aus *Rattus norvegicus.* Fragment H (272Bp) kodiert für das Terminationssignal des Nopalin-Synthase Gens aus *A.tumefaciens*.

### Beispiel 51

Erzeugung von DNA Konstrukten zur Expression der Tyrosin Aminotransferase 1 aus *Arabidopsis thaliana* unter Kontrolle eines samenspezifischen Promotors und der 2,3-Dimethyl-5-Phytylplastochinol-Zyklase aus *Synechocystis* sp. PCC6803 unter Kontrolle eines samenspezifischen Promotors

Zur Herstellung von chimären DNA Konstrukten zur Erzeugung transgener *Brassica napus* Pflanzen, die die Tyrosin-Aminotransferase-1 aus *Arabidopsis thaliana* unter Kontrolle eines samenspezifischen Promotors exprimieren, und 2,3-Dimethyl-5-Phytylplastochinol-Zyklase aus *Synechocystis* sp. PCC6803 samenspezifischen expremieren, werden die Vektoren pSUN2-LeB4-IPP-SynCyc-nosT und pSUN2-USPP-AtTATase1-nosT miteinander kombiniert.

Das DNA Fragment kodierend für die Expressionskassette bestehend aus: USP-Promotor, Tyrosin-Aminotransferase-1-Gen aus *Arabidopsis thaliana* und nos-Terminator wird aus dem Plasmid-pSUN2-USPP-AtTA-Tase1-nosT als Sma1/EcoR1 Fragment isoliert, das EcoR1 Ende mit dem Klenow Enzym aufgefüllt und in den mit EcoR1 verdauten Vektor pSUN2-LeB4-IPP-SynCyc-nosT kloniert, dessen EcoR1 Enden mit dem Klenow Enzym aufgefüllt werden.

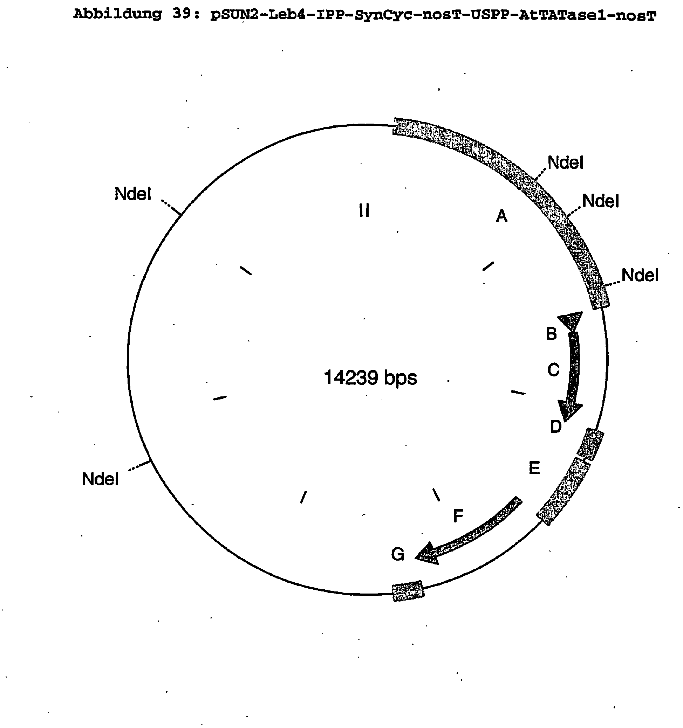

Dieses Plasmid (pSUN2-LeB4-IPP-SynCyc-nosT/USPP-AtTATase1-nosT Abbildung 39) wird zur Erzeugung transgener *Brassica napus* Pflanzen verwendet

Fragment A (2764Bp) in Abbildung 39 beinhaltet den Promotor des LeguminB4-Gens aus *Vicia faba*, Fragment B (235Bp) kodiert für das Transitpeptid der *A.thaliana* Isopentenyl-pyrophosphat-Isomerase-2. Fragment C (1100Bp) kodiert für das 2,3-Dimethyl-5-Phytylplastoquinol Zyklase Gen aus *Synechocystis* sp. PCC6803. Fragment D (272Bp) kodiert für das Terminationssignal des Nopalin-Synthase- Gens. Fragment E (678Bp) beinhaltet den Promotor des Unknow-Seed-Protein-Gens (USPP)aus *Vicia faba*, Fragment F (1269Bp) kodiert für das Tyrosin-Aminotransferase-Gen 1 aus *Arabidopsis thaliana*. Fragment G (272Bp) kodiert für das Terminationssignal des Nopalin-Synthase Gens.

### Beispiel 52

Erzeugung von DNA Konstrukten zur Expression der Tyrosin Aminotransferase 3 aus *Arabidopsis thaliana* unter Kontrolle eines samenspezifischen Promotors und der 2,3-Dimethyl-5-Phytylplastochinol-Zyklase aus *Synechocystis* sp. PCC6803 unter Kontrolle eines samenspezifischen Promotors

Zur Herstellung von chimären DNA Konstrukten zur Erzeugung transgener *Brassica napus* Pflanzen, die die Tyrosin-Aminotransferase-3 aus *Arabidopsis thaliana* unter Kontrolle eines samenspezifischen Promotors exprimieren, und die 2,3-Dimethyl-5-Phytylplastochinol-Zyklase aus *Synechocystis* sp. PCC6803 samenspezifischen expremieren, werden die Vektoren pSUN2-LeB4-IPP-SynCyc-nosT und pSUN2-USPP-AtTATase3-nosT miteinander kombiniert.

Das DNA Fragment kodierend für die Expressionskassette bestehend aus: USP-Promotor, Tyrosin-Aminotransferase-3-Gen aus *Arabidopsis thaliana* und nos-Terminator wird aus dem Plasmid pSUN2-USPP-AtTA-Tase3-nosT als Sma1/EcoR1 Fragment isoliert, das EcoR1 wird mit dem Klenow Enzym aufgefüllt und in den mit EcoR1 verdauten Vektor pSUN2-LeB4-IPP-SynCyc-nosT kloniert, dessen EcoR1 Enden ebenfalls aufgefüllt werden.

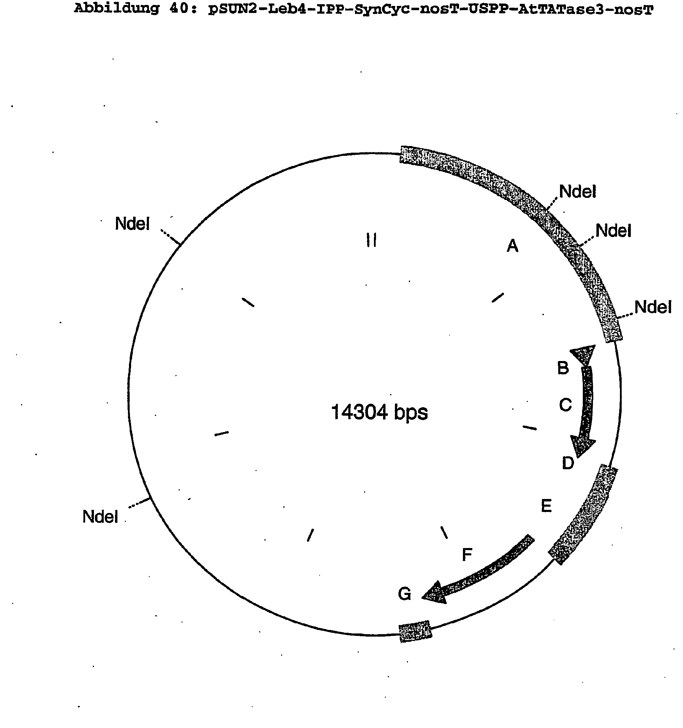

Dieses Plasmid (pSUN2-LeB4-IPP-SynCyc-nosT/USPP-AtTATase3-nosT Abbildung 40) wird zur Erzeugung transgener *Brassica napus* Pflanzen verwendet

Fragment A (2764Bp) in Abbildung 40 beinhaltet den Promotor des LeguminB4-Gens aus *Vicia faba*, Fragment B (235Bp) kodiert für das Transitpeptid der *A.thaliana* Isopentenyl-pyrophosphat-Isomerase-2. Fragment C (1100Bp) kodiert für das 2,3-Dimethyl-5-Phytylplastoquinol Zyklase Gen aus *Synechocystis* sp. PCC6803. Fragment D (272Bp) kodiert für das Terminationssignal des Nopalin-Synthase- Gens. Fragment E(678Bp) beinhaltet den Promotor des "Unknow-Seed-Protein-Gens" (USPP) aus *Vicia faba*, Fragment F (1334Bp) kodiert für das Tyrosin-Aminotransferase-Gen 3 aus *Arabidopsis thaliana*. Fragment G (272Bp) kodiert für das Terminationssignal des Nopalin-Synthase Gens.

### Beispiel 53

Erzeugung von DNA Konstrukten zur Expression der Tyrosin Aminotransferase 5 aus *Arabidopsis thaliana* unter Kontrolle eines samenspezifischen Promotors und der 2,3-Dimethyl-5-Phytylplastochinol-Zyklase aus *Synechocystis* sp. PCC6803 unter Kontrolle eines samenspezifischen Promotors

Zur Herstellung von chimären DNA Konstrukten zur Erzeugung transgener *Brassica napus* Pflanzen, die die Tyrosin-Aminotransferase-5 aus *Arabidopsis thaliana* unter Kontrolle eines samenspezifischen Promotors exprimieren, und die 2,3-Dimethyl-5-Phytylplastochinol-Zyklase aus *Synechocystis* sp. PCC6803 samenspezifischen expremieren, werden die Vektoren pSUN2-LeB4-IPP-SynCyc-nosT und pSUN2-USPP-AtTATase5-nosT miteinander kombiniert.

Das DNA Fragment kodierend für die Expressionskassette bestehend aus: USP-Promotor, Tyrosin-Aminotransferase-5-Gen aus *Arabidopsis thaliana* und nos-Terminator wird aus dem Plasmid pSUN2-USPP-AtTA-Tase5-nosT als Sma1/EcoR1 Fragment isoliert, das EcoR1 Ende mit dem Klenow Enzym aufgefüllt und in den mit EcoR1 verdauten Vektor pSUN2-LeB4-IPP-SynCyc-nosT kloniert, dessen EcoR1 Enden ebenfalls aufgefüllt werden.

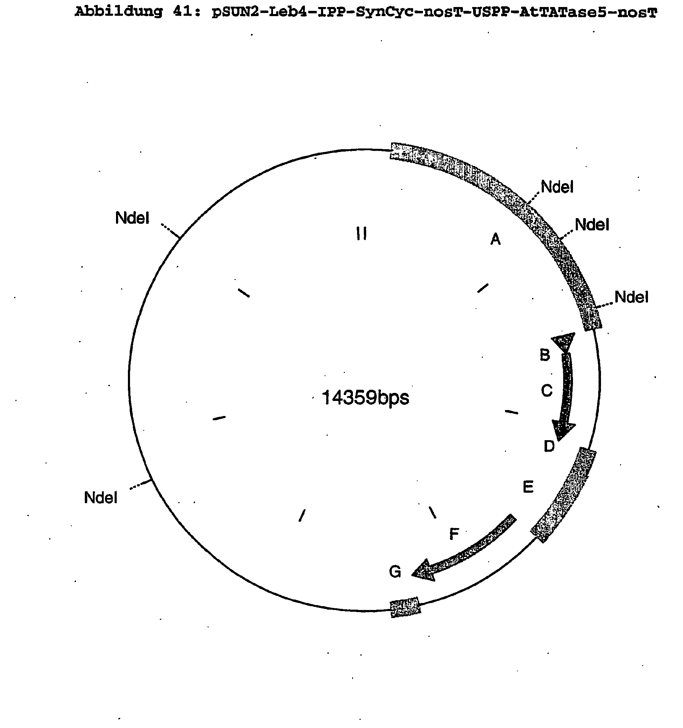

Dieses Plasmid (pSUN2-LeB4-IPP-SynCyc-nosT /USPP-AtTATase5-nosT Abbildung 41) wird zur Erzeugung transgener *Brassica napus* Pflanzen verwendet

Fragment A (2764Bp) in Abbildung 41 beinhaltet den Promotor des LeguminB4-Gens aus *Vicia faba*, Fragment B (235Bp) kodiert für das Transitpeptid der *A.thaliana* Isopentenyl-pyrophosphat-Isomerase-2. Fragment C (1100Bp) kodiert für das 2,3-Dimethyl-5-Phytylplastoquinol Zyklase Gen aus *Synechocystis* sp. PCC6803. Fragment D (272Bp) kodiert für das Terminationssignal des Nopalin-Synthase- Gens. Fragment E (678Bp) beinhaltet den Promotor des "Unknow-Seed-Protein-Gens" aus *Vicia faba*, Fragment F (1389Bp) kodiert für das Tyrosin-Aminotransferase-Gen 5 aus *Arabidopsis thaliana*. Fragment G (272Bp) kodiert für das Terminationssignal des Nopalin-Synthase Gens.

### Beispiel 54

Erzeugung von DNA Konstrukten zur Expression der Tyrosin Aminotransferase 6 aus *Arabidopsis thaliana* unter Kontrolle eines samenspezifischen Promotors und der 2,3-Dimethyl-5-Phytylplastochinol-Zyklase aus *Synechocystis* sp. PCC6803 unter Kontrolle eines samenspezifischen Promotors

Zur Herstellung von chimären DNA Konstrukten zur Erzeugung transgener *Brassica napus* Pflanzen, die die Tyrosin-Aminotransferase-6 aus *Arabidopsis thaliana* unter Kontrolle eines samenspezifischen Promotors exprimieren, und die 2,3-Dimethyl-5-Phytylplastochinol-Zyklase aus *Synechocystis* sp. PCC6803 samenspezifischen expremieren, werden die Vektoren pSUN2-LeB4-IPP-SynCyc-nosT und pSUN2-USPP-AtTATase6-nosT miteinander kombiniert.

Das DNA Fragment kodierend für die Expressionskassette bestehend aus: USP-Promotor, Tyrosin-Aminotransferase-6-Gen aus *Arabidopsis thaliana* und nos-Terminator wird aus dem Plasmid pSUN2-USPP-AtTA-Tase6-nosT als Sma1/EcoR1 Fragment isoliert, das EcoR1 Ende wird mit dem Klenow Enzym aufgefüllt und in den mit EcoR1 verdauten Vektor pSUN2-LeB4-IPP-SynCyc-nosT kloniert, dessen EcoR1 Enden ebenfalls aufgefüllt werden.

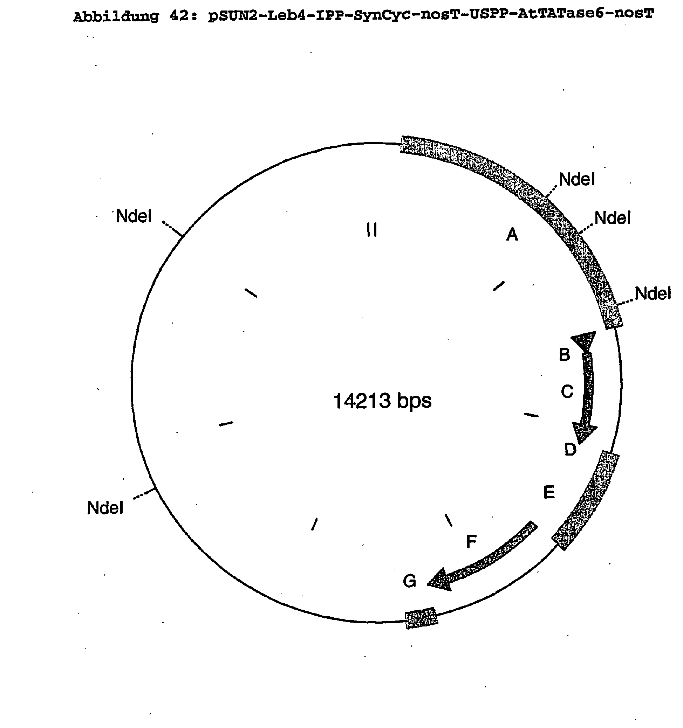

Dieses Plasmid (pSUN2-LeB4-IPP-SynCyc-nosT/USPP-AtTATase6-nosT Abbildung 42) wird zur Erzeugung transgener *Brassica napus* Pflanzen verwendet:

Fragment A (2764Bp) in Abbildung 42 beinhaltet den Promotor des LeguminB4-Gens aus *Vicia faba*, Fragment B (235Bp) kodiert für das Transitpeptid der *A.thaliana* Isopentenyl-pyrophosphat-Isomerase-2. Fragment C (1100Bp) kodiert für das 2,3-Dimethyl-5-Phytylplastoquinol Zyklase Gen aus *Synechocystis* sp. PCC6803. Fragment D (272Bp) kodiert für das Terminationssignal des Nopalin-Synthase- Gens. Fragment E (678Bp) beinhaltet den Promotor des "Unknow-Seed-Protein-Gens" aus *Vicia faba*, Fragment F (1243 Bp) kodiert für das Tyrosin-Aminotransferase-Gen 6 aus *Arabidopsis thaliana*. Fragment G (272Bp) kodiert für das Terminationssignal des Nopalin-Synthase-Gens.

### Beispiel 55

Erzeugung von DNA Konstrukten zur Expression der Tyrosin Aminotransferase aus *Rattus norvegicus* unter Kontrolle eines samenspezifischen Promotors und γ-Tocopherol-Methyltransferase aus *Arabidopsis thaliana* unter Kontrolle eines samenspezifischen Promotors Zur Herstellung von chimären DNA Konstrukten zur Erzeugung transgener *Brassica napus* Pflanzen, die die Tyrosin-Aminotransferase aus *Rattus norvegicus* unter Kontrolle eines samenspezifischen Promotors exprimieren, und die γ-Tocopherol- Methyltransferase aus *Arabidopsis thaliana* samenspezifischen expremieren, werden die Vektoren pSUN2-SBPP-AtγTMT-35sT und pCR4topoblunt-USPP-rbcS-RnTA-Tase1-nosT miteinander kombiniert.

Das DNA Fragment bestehend aus USP-Promotor, Tyrosin-Aminotransferase aus *Rattus norvegicus* und nos-Terminationssequenz wird aus dem Plasmid pCR4TOPOblunt/USPP-rbcS-RnTATase1-nosT als Srf1 Fragment isoliert und in den mit Srf1 verdauten Vektor pSUN2-SBPP-AtγTMT-35sT kloniert.

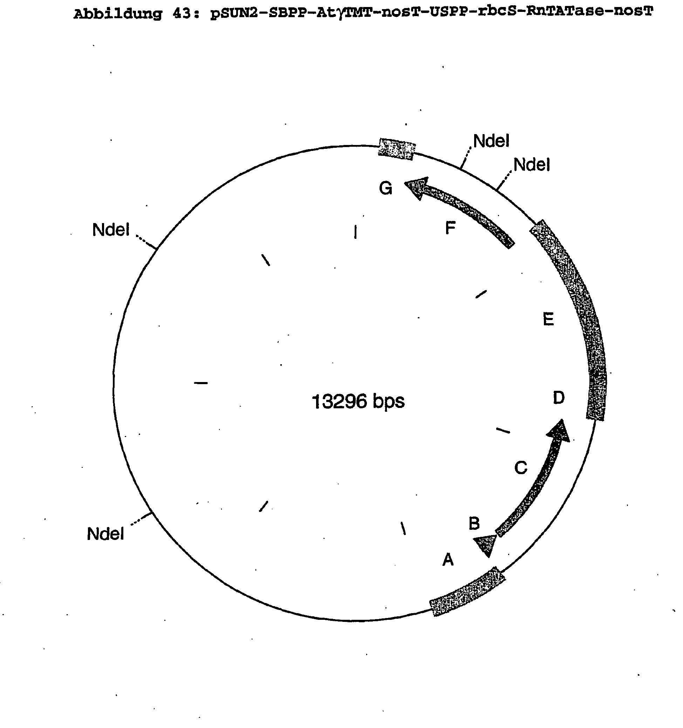

Dieses Plasmid (pSUN2-SBPP-AtγTMT-35sT/USPP-rbcS-RnTATase1-nosT Abbildung 43) wird zur Erzeugung transgener *Brassica napus* Pflanzen verwendet:

Fragment A (678Bp) in Abbildung 43 beinhaltet den Promotor des Unknow-Seed-Protein-Gens (USPP) aus *Vicia faba*, Fragment B (235Bp) kodiert für das Transitpeptid der Ribulose-Bisphosphat-Carboxylase *(rbcS) aus Vicia faba*. Fragment C (1365Bp) kodiert für das Tyrosin-Aminotransferase-Gen aus *Rattus norvegicus.* Fragment D (272Bp) kodiert für das Terminationssignal des Nopalin-Synthase Gens aus *A.tumefaciens*.
Fragment E (1788Bp) beinhaltet den Promotor des SBP-Gens aus *Vicia faba*, Fragment F (1047Bp) kodiert für das γ-Tocopherol-Methyltransferase-Gen aus *Arabidopsis thaliana*, Fragment G (291Bp) kodiert für den 35S-Terminator des Blumenkohlmosaikvirus.

### Beispiel 56

Erzeugung von DNA Konstrukten zur Expression der Tyrosin Aminotransferase 1 aus *Arabidopsis thaliana* unter Kontrolle eines samenspezifischen Promotors und der γ-Tocopherol Methyltransferase aus *Arabidopsis thaliana* unter Kontrolle eines samenspezifischen Promotors

Zur Herstellung von chimären DNA Konstrukten zur Erzeugung transgener *Brassica napus* Pflanzen, die die Tyrosin-Aminotransferase-1 aus *Arabidopsis thaliana* unter Kontrolle eines samenspezifischen Promotors exprimieren, und die γ-Tocopherol Methyltransferase aus *Arabidopsis thaliana* samenspezifischen expremieren, werden die Vektoren pSUN2-SBPP-AtγTMT-35sT und pSUN2-USPP-AtTATasel-nosT miteinander kombiniert.

Das DNA Fragment kodierend für die Expressionskassette bestehend aus: USP-Promotor, Tyrosin-Aminotransferase-1-Gen aus *Arabidopsis thaliana* und nos-Terminator wird aus dem Plasmid pSUN2-USPP-AtTA-Tase1-nosT als Sma1/EcoR1 Fragment isoliert, das EcoR1 Ende mit dem Klenow Enzym aufgefüllt und in den mit Srf1 verdauten Vektor pSUN2-SBPP-AtγTMT-35sT kloniert.

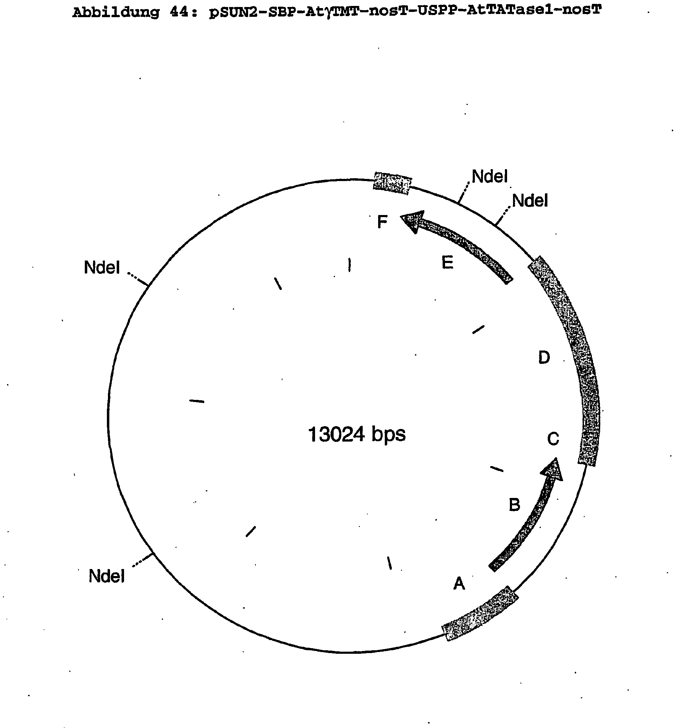

Dieses Plasmid (pSUN2LeB4-SBPP-AtγTMT-35sT/USPP-AtTATasel-nosT Abbildung 44) wird zur Erzeugung transgener *Brassica napus* Pflanzen verwendet:

Fragment A (678Bp) in Abbildung 44 beinhaltet den Promotor des Unknow-Seed-Protein-Gens (USPP)aus *Vicia faba*, Fragment B (1269Bp) kodiert für das Tyrosin-Aminotransferase-Gen 1 aus *Arabidopsis thaliana* und Fragment C (272Bp) kodiert für das Terminationssignal des Nopalin-Synthase Gens. Fragment D (1788Bp) beinhaltet den Promotor des SBP-Gens aus *Vicia faba*, Fragment E (1047Bp) kodiert für das γ-Tocopherol-Methyltransferase-Gen aus *Arabidopsis thaliana*, Fragment F (291Bp) kodiert für den 35S-Terminator des Blumenkoh1-mosaikvirus.

### Beispiel 57

Erzeugung von DNA Konstrukten zur Expression der Tyrosin Aminotransferase 3 aus *Arabidopsis thaliana* unter Kontrolle eines samenspezifischen Promotors und der γ-Tocopherol-Methyltransferase aus *Arabidopsis thaliana* unter Kontrolle eines samenspezifischen Promotors

Zur Herstellung von chimären DNA Konstrukten zur Erzeugung transgener *Brassica napus* Pflanzen, die die Tyrosin-Aminotransferase-3 aus *Arabidopsis thaliana* unter Kontrolle eines samenspezifischen Promotors exprimieren, und die γ-Tocopherol- Methyltransferase aus *Arabidopsis thaliana* samenspezifischen expremieren, werden die Vektoren pSUN2-SBPP-AtγTMT-35sT und pSUN2-USPP-AtTATase3-nosT miteinander kombiniert.

Das DNA Fragment kodierend für die Expressionskassette bestehend aus: USP-Promotor, Tyrosin-Aminotransferase-3-Gen aus *Arabidopsis thaliana* und nos-Terminator wird aus dem Plasmid pSUN2-USPP-AtTA-Tase3-nosT als Sma1/EcoR1 Fragment isoliert, das EcoR1 Ende mit dem Klenow Enzym aufgefüllt und in den mit Srf1 verdauten Vektor pSUN2-SBPP-AtγTMT-35sT kloniert.

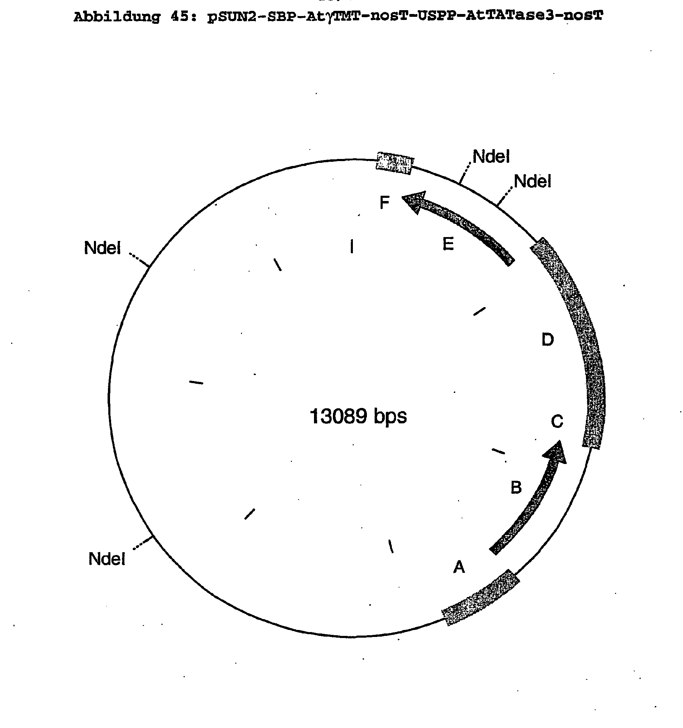

Dieses Plasmid (pSUN2-SBPP-AtγTMT-35sT/USPP-AtTATase3-nosT Abbildung 45) wird zur Erzeugung transgener *Brassica napus* Pflanzen verwendet

Fragment A(678Bp) in Abbildung 45 beinhaltet den Promotor des "Unknow-Seed-Protein-Gens" (USPP) aus *Vicia faba*, Fragment B (1334Bp) kodiert für das Tyrosin-Aminotransferase-Gen 3 aus *Arabidopsis thaliana* und Fragment C (272Bp) kodiert für das Terminationssignal des Nopalin-Synthase Gens. Fragment D (1788Bp) beinhaltet den Promotor des SBP-Gens aus *Vicia faba*, Fragment E (1047Bp) kodiert für das γ-Tocopherol-Methyltransferase-Gen aus *Arabidopsis thaliana*, Fragment F (291Bp) kodiert für den 35S-Terminator des Blumenkohl-mosaikvirus.

### Beispiel 58

Erzeugung von DNA Konstrukten zur Expression der Tyrosin Aminotransferase 5 aus *Arabidopsis thaliana* unter Kontrolle eines samenspezifischen Promotors und der γ-Tocopherol-Methyltransferase aus *Arabidopsis thaliana* unter Kontrolle eines samenspezifischen Promotors

Zur Herstellung von chimären DNA Konstrukten zur Erzeugung transgener *Brassica napus* Pflanzen, die die Tyrosin-Aminotransferase-5 aus *Arabidopsis thaliana* unter Kontrolle eines samenspezifischen Promotors exprimieren, und die γ-Tocopherol- Methyltransferase aus *Arabidopsis thaliana* samenspezifischen expremieren, werden die Vektoren pSUN2-SBPP-AtγTMT-35sT und pSUN2-USPP-AtTATase5-nosT miteinander kombiniert.

Das DNA Fragment kodierend für die Expressionskassette bestehend aus: USP-Promotor, Tyrosin-Aminotransferase-5-Gen aus *Arabidopsis thaliana* und nos-Terminator wird aus dem Plasmid pSUN2-USPP-AtTA-Tase5-nosT als Sma1/EcoR1 Fragment isoliert, das EcoR1 Ende mit dem Klenow Enzym aufgefüllt und in den mit Srf1 verdauten Vektor pSUN2-SBPP-AtγTMT-35sT kloniert.

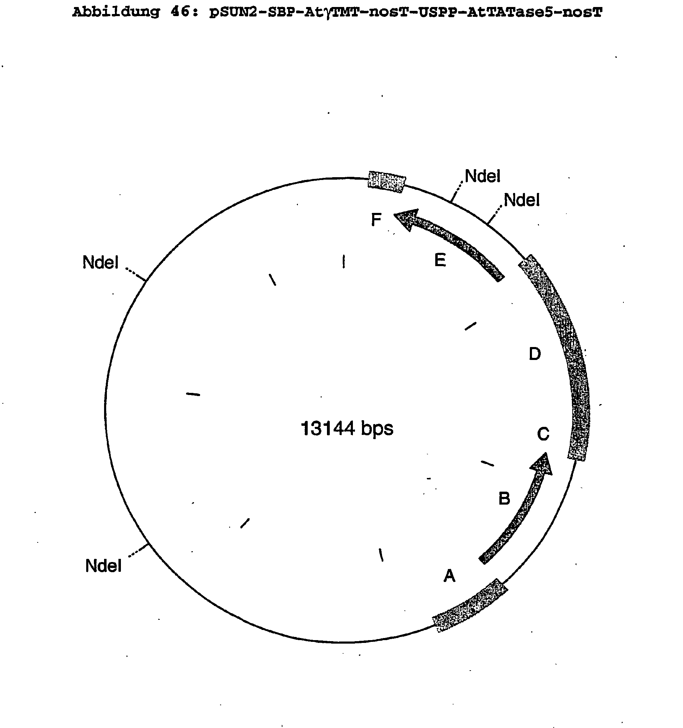

Dieses Plasmid (pSUN2-SBPP-AtγTMT-35sT /USPP-AtTATase5-nosT Abbildung 46) wird zur Erzeugung transgener *Brassica napus* Pflanzen verwendet

Fragment A (678Bp) in Abbildung 46 beinhaltet den Promotor des "Unknow-Seed-Protein-Gens" aus *Vicia faba*, Fragment B (1389Bp) kodiert für das Tyrosin-Aminotransferase-Gen 5 aus *Arabidopsis thaliana* und Fragment C (272Bp) kodiert für das Terminationssignal des Nopalin-Synthase Gens. Fragment D (1788Bp) beinhaltet den Promotor des SBP-Gens aus *Vicia faba*, Fragment E (1047Bp) kodiert für das γ-Tocopherol-Methyltransferase-Gen aus *Arabidopsis thaliana*, Fragment F (291Bp) kodiert für den 35S-Terminator des Blumenkohl-mosaikvirus.

### Beispiel 59

Erzeugung von DNA Konstrukten zur Expression der Tyrosin Aminotransferase 6 aus *Arabidopsis thaliana* unter Kontrolle eines samenspezifischen Promotors und der γ-Tocopherol-Methyltransferase aus *Arabidopsis thaliana* unter Kontrolle eines samenspezifischen Promotors
Zur Herstellung von chimären DNA Konstrukten zur Erzeugung transgener *Brassica napus* Pflanzen, die die Tyrosin-Aminotransferase-6 aus *Arabidopsis thaliana* unter Kontrolle eines samenspezifischen Promotors exprimieren, und die γ-Tocopherol- Methyltransferase aus *Arabidopsis thaliana* samenspezifischen expremieren, werden die Vektoren pSUN2-SBPP-AtγTMT-35sT und pSUN2-USPP-AtTATase6-nosT miteinander kombiniert.

Das DNA Fragment kodierend für die Expressionskassette bestehend aus: USP-Promotor, Tyrosin-Aminotransferase-6-Gen aus *Arabidopsis thaliana* und nos-Terminator wird aus dem Plasmid pSUN2-USPP-AtTA-Tase6-nosT als Sma1/EcoR1 Fragment isoliert, das EcoR1 Ende mit dem Klenow Enzym aufgefüllt und in den mit Srf1 verdauten Vektor pSUN2-SBPP-AtγTMT-35sT kloniert.

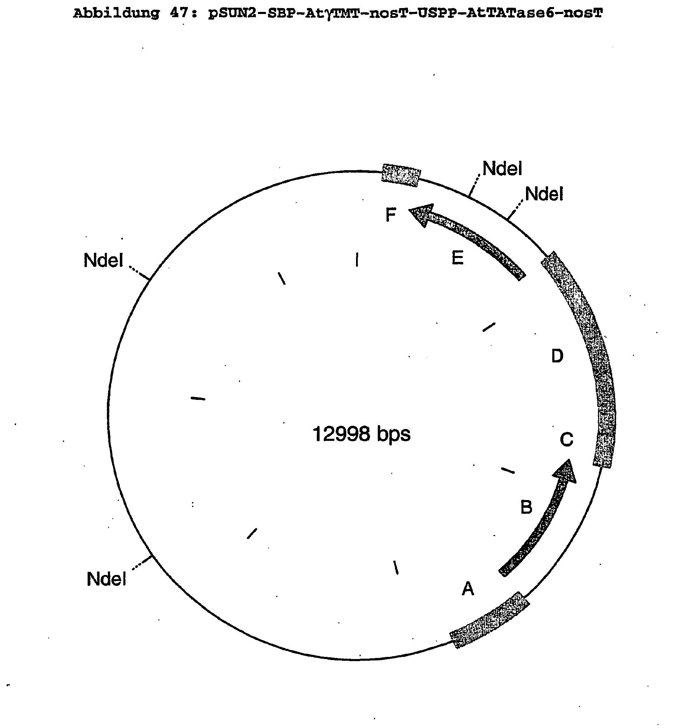

Dieses Plasmid (pSUN2-SBPP-AtγTMT-35sT/USPP-AtTATase6-nosT Abbildung 47) wird zur Erzeugung transgener *Brassica napus* Pflanzen verwendet:

Fragment A (678Bp) in Abbildung 47 beinhaltet den Promotor des "Unknow-Seed-Protein-Gens" aus *Vicia faba*, Fragment B (1243 Bp) kodiert für das Tyrosin-Aminotransferase-Gen 6 aus *Arabidopsis thaliana* und Fragment C (272Bp) kodiert für das Terminationssignal des Nopalin-Synthase-Gens. Fragment D (1788Bp) beinhaltet den Promotor des SBP-Gens aus *Vicia faba*, Fragment E (1047Bp) kodiert für das γ-Tocopherol-Methyltransferase-Gen aus *Arabidopsis thaliana*, Fragment F (291Bp) kodiert für den 35S-Terminator des Blumenkohl-mosaikvirus.

### Beispiel 60

Erzeugung von DNA Konstrukten zur Expression der Geranylgeranylpyrophosphate-Oxidoreduktase aus *Nicotiana tabacum* unter Kontrolle eines samenspezifischen Promotors und zur samenspezifischen Unterdrückung der Expression des Homogentisinsäure-Dioxygenase-Gens aus *Brassica napus*

Zur Herstellung von chimären DNA Konstrukten zur Erzeugung transgener *Brassica napus* Pflanzen, die die Geranylgeranylpyrophosphate-Oxidoreduktase aus *Nicotiana tabacum* unter Kontrolle eines samenspezifischen Promotors exprimieren, und die Expression der endogenen Homogentisinsäure-Dioxygenase-Gens aus *Brassica napus* samenspezifisch unterdrücken, werden die Vektoren pCR4topoblunt-LeB4-NtGGPPOR-nosT (s.u.) und pSUN2-Pvic-*BnHGD-STLS1-α*BnHGD-ocsT miteinander kombiniert.

Mittels PCR wird die Expressionkassette bestehend aus: LeB4-Promotor, Geranylgeranylpyrophosphate-Oxidoreduktase-Gen aus *Nicotiana tabacum* und nos-Terminationssequenz, unter Verwendung eines sense spezifischen Primers (LeB4-SRF1 5': SEQ. ID. No. 54) und eines antisense spezifischen Primers (nosT-SRF1 3' SEQ. ID. No. 53), amplifiziert und in den Vektor pCR4topoblunt (Invitrogene) kloniert.

Das resultierende Plasmid ist pCR4topoblunt-LeB4-NtGGPPOR-nosT
Die PCR Bedingungen waren die folgenden:
Die PCR erfolgte in einem 50µl Reaktionsansatz in dem enthalten war:
- 1µl einer puc19-LeB4-NtGGPPOR-nosT Plasmid-DNA
- 0,2 mM dATP, dTTP, dGTP, dCTP
- 1,5 mM Mg(OAc)₂
- 5µg Rinderserum-Albumin
- 40pmol LeB4-Srf1 5'Primer
- 40pmol nosT-Srf1 3'Primer
- 5µl 10x Pfu1 DNA Polymerase Puffer (Stratagene)
- 5U Pfu1 DNA Polymerase (Stratagene)
Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 55°C (Annealing)
Schritt 4: 10 Minuten 68°C (Elongation)
   30 Wiederholungen der Schritte 2-4
Schritt 5: 10 Minuten 72°C (Post-Elongation)

Das DNA Fragment kodierend für die Expressionskassette bestehend aus: LeB4-Promotor, Geranylgeranylpyrophosphate-Oxidoreduktase-Gen aus *Nicotiana tabacum* und nos-Terminator wird aus dem Plasmid pCR4topoblunt-LeB4-NtGGPPOR-nosT als Srf1 Fragment isoliert und in den mit EcoR5 verdauten pSUN2-Pvic-*BnHGD-STLS1-α*BnHGD-ocsT kloniert.

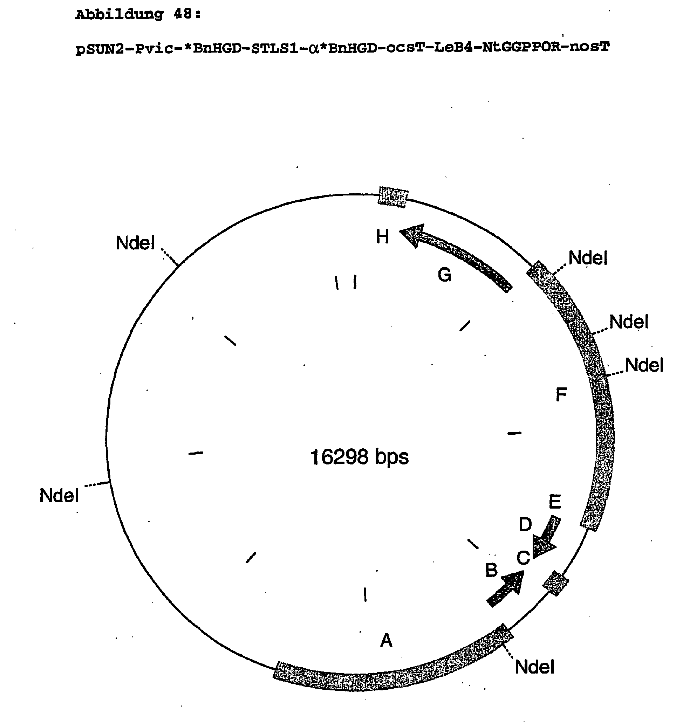

Dieses Plasmid (pSUN2-Pvic-*BnHGD-STLS1-α*BnHGD-ocsT/LeB4-NtGGPPOR-nosT Abbildung 48) wird zur Erzeugung transgener *Brassica napus* Pflanzen verwendet:

Fragment A (2559 Bp) in Abbildung 48 beinhaltet den Promotor des Vicilin-Gens aus Vicia faba, Fragment B (580 Bp) kodiert für einen Fragment des Homogentisinsäure-Dioxygenase-Gen aus *Brassica napus*. Fragment C (190Bp) für das 2 Intron (IV2) des ST-LS1 Gens aus *Solanum tuberosum.* Fragment D ist identisch mit Fragment B jedoch im Vektor gegenläufig zu B orientiert. Fragment E (208Bp) kodiert für das Terminationssignal-2 des Octopin Gens.
Fragment F (2764Bp) beinhaltet den Promotor des LeguminB4-Gens aus *Vicia faba*, Fragment G (1509Bp) kodiert für das Geranylgeranylpyrophosphate-Oxidoreduktase-Gen aus *Nicotiana tabacum* Fragment H (272Bp) kodiert für das Terminationssignal des Nopalin-Synthase-Gens

### Beispiel 61

Erzeugung von DNA Konstrukten zur Expression der Hydroxyphenylpyruvat-Dioxygenase aus *Arabidopsis thaliana* unter Kontrolle eines samenspezifischen Promotors und zur samenspezifischen Unterdrükkung der Expression des Homogentisinsäure-Dioxygenase-Gens aus *Brassica napus.*

Zur Herstellung von chimären DNA Konstrukten zur Erzeugung transgener *Brassica napus* Pflanzen, die die Hydroxyphenylpyruvat-Dioxygenase aus *Arabidopsis thaliana* unter Kontrolle eines samenspezifischen Promotors exprimieren, und die Expression der endogenen Homogentisinsäure-Dioxygenase-Gens aus *Brassica napus* samenspezifisch unterdrücken, werden die Vektoren pCR4topoblunt-USPP-AtHPPD-ocsT (s.u.) und pSUN2-Pvic-*BnHGD-STLS1-α*BnHGD-ocsT miteinander kombiniert.

Mittels PCR wird die Expressionkassette bestehend aus: USP-Promotor, Hydroxyphenylpyruvat-Dioxygenase aus *Arabidopsis thaliana* und ocs-Terminationssequenz-1, unter Verwendung eines sense spezifischen Primers (USPP-SRF1-5': SEQ. ID. No. 52) und eines antisense spezifischen Primers (ocsT-SRF1-3': SEQ. ID. No. 55), amplifiziert und in den Vektor pCR4topoblunt (Invitrogene) kloniert. Das resultierende Plasmid ist pCR4topoblunt-USPP-AtHPPD-ocsT.

Die PCR Bedingungen waren die folgenden:
Die PCR erfolgte in einem 50µl Reaktionsansatz in dem enthalten war:
- 1µl einer pSUN2-USPP-AtHPPD-ocsT P1asmid-DNA
- 0,2 mM dATP, dTTP, dGTP, dCTP
- 1,5 mM Mg(OAc)₂
- 5µg Rinderserum-Albumin
- 40pmol USPP-SRF1 5'Primer
- 40pmol ocsT-SRF1 3'Primer
- 5µl 10x Pfu1 DNA Polymerase Puffer (Stratagene)
- 5U Pfu1 DNA Polymerase (Stratagene)

Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 55°C (Annealing)
Schritt 4: 10 Minuten 68°C (Elongation)
   30 Wiederholungen der Schritte 2-4
Schritt 5: 10 Minuten 72°C (Post-Elongation)
   Das DNA Fragment bestehend aus USP-Promotor, Hydroxyphenylpyruvat- Dioxygenase aus *Arabidopsis thaliana* und ocs-Terminationssequenz wird aus dem Plasmid pCR4TOPOblunt/USPP-AtHPPD-ocsT als Srf1 Fragment isoliert und in den mit EcoR5 verdauten Vektor pSUN2-Pvic-*BnHGD-STLS1-α*BnHGD-ocsT kloniert.

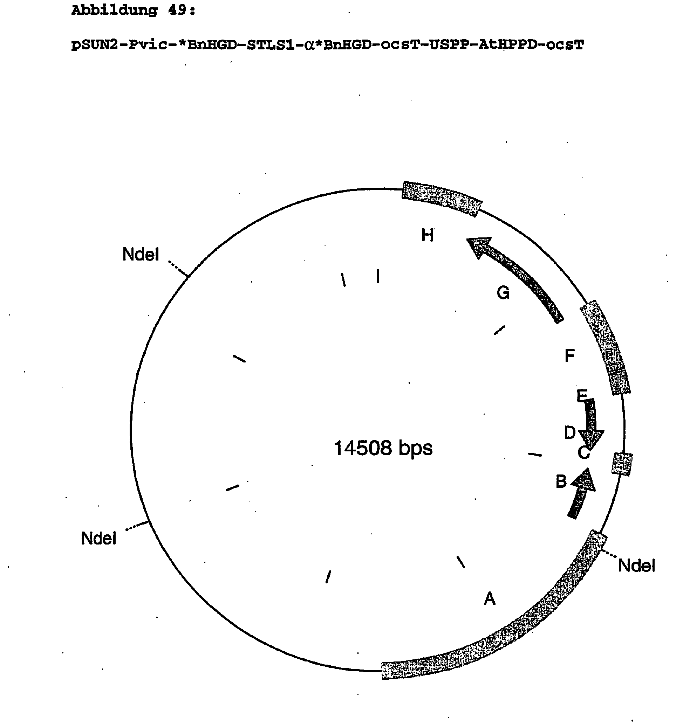

Dieses Plasmid (pSUN2-Pvic-*BnHGD-STLS1-α*BnHGD-ocsT/USPP-AtHPPD-ocsT Abbildung 49) wird zur Erzeugung transgener *Brassica napus* Pflanzen verwendet.

Fragment A (2559 Bp) in Abbildung 49 beinhaltet den Promotor des Vicilin-Gens aus Vicia faba, Fragment B (580 Bp) kodiert für einen Fragment des Homogentisinsäure-Dioxygenase-Gen aus *Brassica* napus. Fragment C (190Bp) für das 2 Intron (IV2) des ST-LS1 Gens aus *Solanum tuberosum*. Fragment D ist identisch mit Fragment B jedoch im Vektor gegenläufig zu B orientiert. Fragment E (208Bp) kodiert für das Terminationssignal-2 des Octopin Gens.
Fragment F (678Bp) kodiert beinhaltet den Promotor des "Unknow-Seed-Protein-Gens" aus *Vicia faba*, Fragment G (1338Bp) kodiert für das Hydroxyphenylpyruvate-Dioxygenase-Gen aus *Arabidopsis thaliana*. Fragment H (713Bp) kodiert für das Terminati-onssignal-1 des Octopin-Synthase.

### Beispiel 62

Erzeugung von DNA Konstrukten zur Expression der Homogentisinsäure Phytyltransferase aus *Arabidopsis thaliana* unter Kontrolle eines samenspezifischen Promotors und zur samenspezifischen Unterdrückung der Expression des Homogentisinsäure Dioxygenase Gens aus *Brassica napus*

Zur Herstellung von chimären DNA Konstrukten zur Erzeugung transgener *Brassica napus* Pflanzen, die die Homogentisinsäure- Phytyltransferase aus *Arabidopsis thaliana* unter Kontrolle eines samenspezifischen Promotors exprimieren, und die Expression der endogenen Homogentisinsäure Dioxygenase Gens aus *Brassica napus* samenspezifisch unterdrücken, werden die Vektoren pCR4topoblunt-USPP-AtHPT-ocsT (siehe nachstehend) und pSUN2-Pvic-*BnHGD-STLS1-α*BnHGD-ocsT miteinander kombiniert.

Mittels PCR wird die Expressionkassette bestehend aus: USP-Promotor, der Homogentisinsäure-Phytyltransferase aus *Arabidopsis thaliana* und ocs-Terminationssequenz, unter Verwendung eines sense spezifischen Primers (USPP-SRF1-5' SEQ. ID. No. 52) und eines antisense spezifischen Primers (ocsT-SRF1-3' SEQ. ID. No. 55), amplifiziert und in den Vektor pCR4topoblunt (Invitrogene) kloniert.

Das resultierende Plasmid ist pCR4topoblunt-USPP-AtHPT-ocsT. Die PCR Bedingungen waren die folgenden:
Die PCR erfolgte in einem 50µl Reaktionsansatz in dem enthalten war:
- 1µl einer pSUN2-USPP-AtHPT-ocsT Plasmid-DNA
- 0,2 mM dATP, dTTP, dGTP, dCTP
- 1,5 mM Mg(OAc)₂
- 5µg Rinderserum-Albumin
- 40pmol USPP-SRF1 5'Primer
- 40pmol ocsT-SRF1 3'Primer
- 5µl 10x Pfu1 DNA Polymerase Puffer (Stratagene)
- 5U Pfu1 DNA Polymerase (Stratagene)

Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 55°C (Annealing)
Schritt 4: 10 Minuten 68°C (Elongation)
   30 Wiederholungen der Schritte 2-4
Schritt 5: 10 Minuten 72°C (Post-Elongation)

Das DNA Fragment bestehend aus USP-Promotor, der Homogentisinsäure- Phytyltransferase aus *Arabidopsis thaliana* und ocs- Terminationssequenz-1 wird aus dem Plasmid pCR4TOPOblunt/USPP-AtHPT-ocsT als Srf1 Fragment isoliert und in den mit EcoR5 verdauten Vektor pSUN2-Pvic-*BnHGD-STLS1-α*BnHGD-ocsT kloniert.

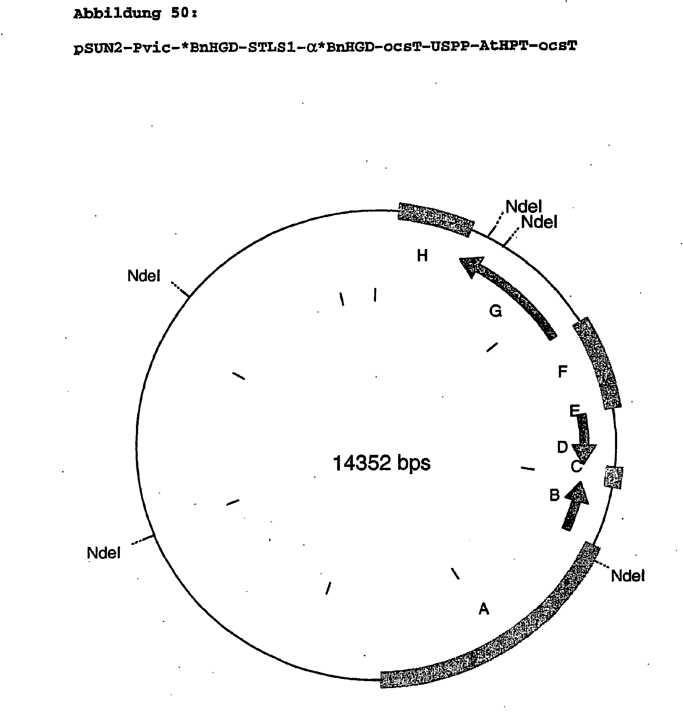

Dieses Plasmid (pSUN2-Pvic-*BnHGD-STLS1-α*BnHGD-ocsT/USPP-AtHPT-ocsT Abbildung 50) wird zur Erzeugung transgener *Brassica napus* Pflanzen verwendet:

Fragment A (2559 Bp) in Abbildung 50 beinhaltet den Promotor des Vicilin-Gens aus Vicia faba, Fragment B (580 Bp) kodiert für einen Fragment des Homogentisinsäure-Dioxygenase-Gen aus *Brassica napus*. Fragment C (190Bp) kodiert für das 2 Intron (IV2) des ST-LS1 Gens aus *Solanum tuberosum*. Fragment D ist identisch mit Fragment B jedoch im Vektor gegenläufig zu B orientiert. Fragment E (208Bp) kodiert für das Terminationssignal-2 des Octopin Gens. Fragment F (678Bp) beinhaltet den Promotor des "Unknow-Seed-Protein-Gens" aus *Vicia faba*, Fragment G (1182Bp) kodiert für das Homogentisinsäure-Phytyltransferase-Gen aus *Arabidopsis thaliana.* Fragment H (713Bp) kodiert für das Terminationssignal-1 des Octopin-Synthase Gens.

### Beispiel 63

Erzeugung von DNA Konstrukten zur Expression der 2-Methyl-6-Phytylhydrochinon-Methyltransferase aus *Synechocystis spec* PC6808 unter Kontrolle eines samenspezifischen Promotors und zur samenspezifischen Unterdrückung der Expression des Homogentisinsäure-Dioxygenase-Gens aus *Brassica napus*

Zur Herstellung von chimären DNA Konstrukten zur Erzeugung transgener *Brassica napus* Pflanzen, die die 2-Methyl-6-Phytylhydrochinon-Methyltransferase aus *Synechocystis spec* PC6808 unter Kontrolle eines samenspezifischen Promotors exprimieren, und die Expression der endogenen Homogentisinsäure-Dioxygenase-Gens aus *Brassica napus* samenspezifisch unterdrücken, werden die Vektoren pCR4topoblunt-LeB4-IPP-SynMt1-nosT (s.u.) und pSUN2-Pvic-*BnHGD-STLS1-α*BnHGD-ocsT miteinander kombiniert.

Mittels PCR wird die Expressionkassette bestehend aus: LeB4-Promotor, die Sequenz kodierend für das Transitpeptid der *A.thaliana* plastiden-spezifischen Isopentenyl-pyrophosphat Isomerase-2 (IPP-2), der 2-Methyl-6-Phytylhydrochinol-Methyltranserase aus *Synechocystis spec* PC6808 und nos-Terminationssequenz, unter Verwendung eines sense spezifischen Primers (LeB4-SRF1-5': SEQ. ID. No. 54) und eines antisense spezifischen Primers (nosT-SRF1-3': SEQ. ID. No. 53), amplifiziert und in den Vektor pCR4topoblunt (Invitrogene) kloniert. Das resultierende Plasmid ist pCR4topoblunt-LeB4-IPP-SynMt1-nosT.

Die PCR Bedingungen waren die folgenden:
Die PCR erfolgte in einem 50µl Reaktionsansatz in dem enthalten war:
- 1µl einer pSUN2-Leb4-IPP-SynMT1-nosT Plasmid-DNA
- 0,2 mM dATP, dTTP, dGTP, dCTP
- 1,5 mM Mg(OAc)₂
- 5µg Rinderserum-Albumin
- 40pmol LeB4-SRF1 5'Primer
- 40pmol nosT-SRF1 3'Primer
- 5µl 10x Pfu1 DNA Polymerase Puffer (Stratagene)
- 5U Pfu1 DNA Polymerase (Stratagene)

Die PCR wurde unter folgenden Zyklus Bedingungen durchgeführt:
Schritt 1: 5 Minuten 94° (Denaturierung)
Schritt 2: 3 Sekunden 94°
Schritt 3: 1 Minute 55° (Annealing)
Schritt 4: 10 Minuten 68° (Elongation)
   30 Wiederholungen der Schritte 2-4
Schritt 5: 10 Minuten 72° (Post-Elongation)

Das DNA Fragment bestehend aus LeB4-Promotor, die Sequenz kodierend für das Transitpeptid der *A.thaliana* plastiden-spezifischen Isopentenyl-pyrophosphat Isomerase-2 (IPP-2) , der 2-Methyl-6-Phytylhydrochinol-Methyltranserase aus *Synechocystis spec* PC6808 und nos-erminationssequenz wird aus dem Plasmid pCR4TOPO-blunt/LeB4-IPP-SynMT1-nosT als Srf1 Fragment isoliert und in den mit EcoR5 verdauten Vektor pSUN2-ic-*nHGD-STLS1-α*nHGD-ocsT kloniert.

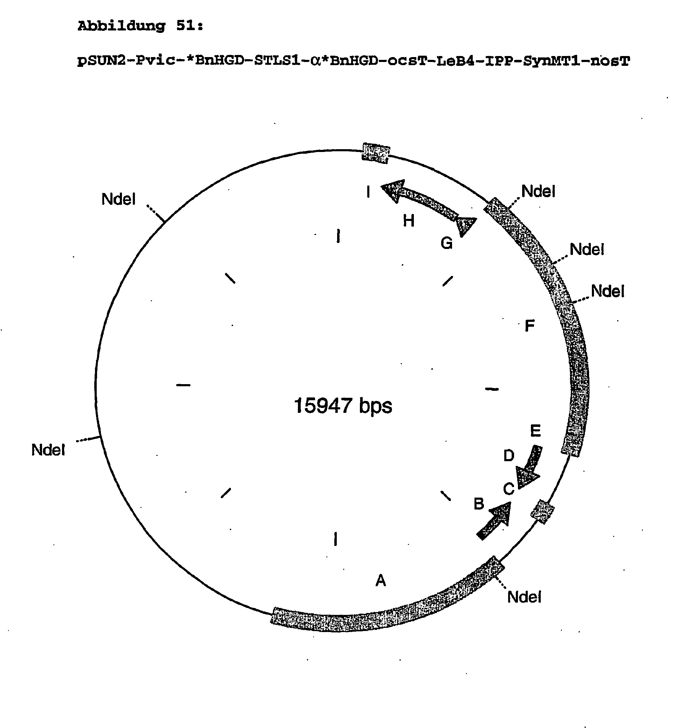

Dieses Plasmid (pSUN2-Pvic-*nHGD-STLS1-α*nHGD-ocsT/USPP-LeB4-IPP-SynMT1-nosT Abbildung 51) wird zur Erzeugung transgener *Brassica napus* Pflanzen verwendet:

Fragment A (2559 Bp) in Abbildung 51 beinhaltet den Promotor des Vicilin-Gens aus Vicia faba, Fragment B (580 Bp) kodiert für einen Fragment des Homogentisinsäure-Dioxygenase-Gen aus *Brassica napus*. Fragment C (190Bp) kodiert für das 2 Intron (IV2) des ST-LS1 Gens aus *Solanum tuberosum*. Fragment D ist identisch mit Fragment B jedoch im Vektor gegenläufig zu B orientiert. Fragment E (208Bp) kodiert für das Terminationssignal-2 des Octopin Gens. Fragment F (2764Bp) beinhaltet den Promotor des LeguminB4-Gens aus *Vicia faba*, Fragment G (235Bp) kodiert für das Transitpeptid der *A.thaliana* Isopentenyl-pyrophosphat-Isomerase-2. Fragment H (957Bp) kodiert für das 2-Methyl-6-Phytylhydrochinol Methyltransferase Gen aus *Synechocystis* sp. PCC6803. Fragment I (272Bp) kodiert für das Terminationssignal des Nopalin-Synthase-Gens.

### Beispiel 64

Erzeugung von DNA Konstrukten zur Expression der 2,3-Dimethyl-5-Phytylplastochinol-Zyklase aus *Synechocystis spec* PC6808 unter Kontrolle eines samenspezifischen Promotors und zur samenspezifischen Unterdrückung der Expression des Homogentisinsäure Dioxygenase Gens aus *Brassica napus*

Zur Herstellung von chimären DNA Konstrukten zur Erzeugung transgener *Brassica napus* Pflanzen, die die 2,3-Dimethyl-5-Phytylplastochinol-Zyklase aus *Synechocystis spec* PC6808 unter Kontrolle eines samenspezifischen Promotors exprimieren, und die Expression der endogenen Homogentisinsäure-Dioxygenase-Gens aus *Brassica napus* samenspezifisch unterdrücken, werden die Vektoren pCR4topoblunt-LeB4-IPP-SynCyc-nosT (s.u.) und pSUN2-Pvic-*BnHGD-STLS1-α*BnHGD-ocsT miteinander kombiniert.

Mittels PCR wird die Expressionkassette bestehend aus: LeB4-Promotor, die Sequenz kodierend für das Transitpeptid der *Arabidopsis thaliana* plastiden-spezifischen Isopentenyl-pyrophosphat Isomerase-2 (IPP-2), der 2,3-Dimethyl-5-Phytylplastochinol-Zyklase aus *Synechocystis spec* PC6808 und nos-Terminationssequenz, unter Verwendung eines sense spezifischen Primers (LeB4-EcoR5-5': SEQ. ID. No. 56) und eines antisense spezifischen Primers (nosT-EcoR5-3': SEQ. ID. No. 57), amplifiziert und in den Vektor pCR4topoblunt (Invitrogene) kloniert. Das resultierende Plasmid ist pCR4topoblunt-LeB4-IPP-SynMt1-nosT

Die PCR Bedingungen waren die folgenden:
Die PCR erfolgte in einem 50µl Reaktionsansatz in dem enthalten war:
- 1µl einer pSUN2-Leb4-IPP-SynCyc-nosT Plasmid-DNA
- 0,2 mM dATP, dTTP, dGTP, dCTP
- 1,5 mM Mg(OAc)₂
- 5µg Rinderserum-Albumin
- 40pmol LeB4-EcoR5 5'Primer
- 40pmol nosT-EcoR5 3'Primer
- 5µl 10x Pfu1 DNA Polymerase Puffer (Stratagene)
- 5U Pfu1 DNA Polymerase (Stratagene)
Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 55°C (Annealing)
Schritt 4: 10 Minuten 68°C (Elongation)
   30 Wiederholungen der Schritte 2-4
Schritt 5: 10 Minuten 72°C (Post-Elongation)

Das DNA Fragment bestehend aus LeB4-Promotor, die Sequenz kodierend für das Transitpeptid der *Arabidopsis thaliana* plastiden-spezifischen Isopentenyl-pyrophosphat Isomerase-2 (IPP-2) , der 2,3-Dimethyl-5-Phytylplastochinol-Zyklase aus *Synechocystis* sp. PCC6803 und nos-Terminationssequenz wird aus dem Plasmid pCR4TO-POblunt/LeB-IPP-SynCyc-nosT als EcoR5 Fragment isoliert und in den mit EcoR5 verdauten Vektor pSUN2-Pvic-*BnHGD-STLS1-α*BnHGD-ocsT kloniert.

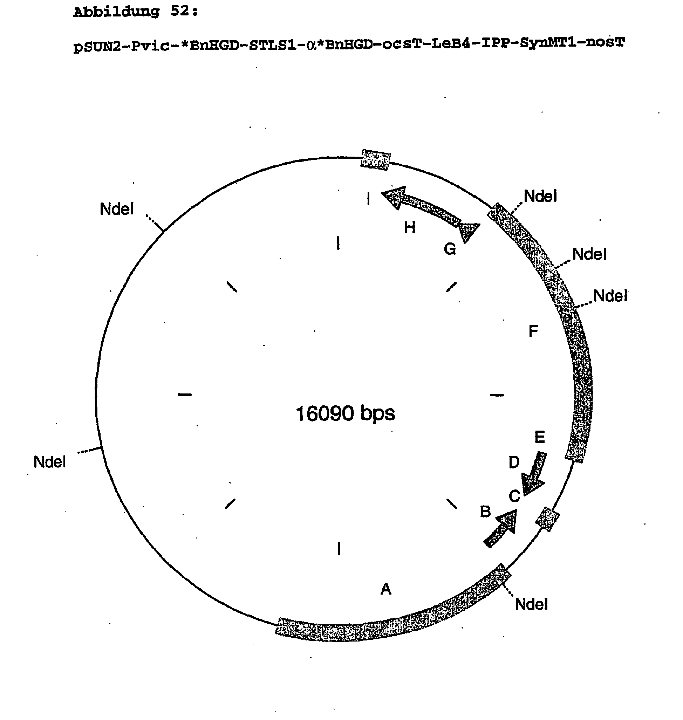

Dieses Plasmid (pSUN2-Pvic-*BnHGD-STLS1-α*BnHGD-ocsT/LeB-IPP-Syn-Cyc-nosT Abbildung 52) wird zur Erzeugung transgener *Brassica napus* Pflanzen verwendet:

Fragment A (2559 Bp) in Abbildung 52 beinhaltet den Promotor des Vicilin-Gens aus Vicia faba, Fragment B (580 Bp) kodiert für einen Fragment des Homogentisinsäure-Dioxygenase-Gen aus *Brassica napus*. Fragment C (190Bp) kodiert für das 2 Intron (IV2) des ST-LS1 Gens aus *Solanum tuberosum*. Fragment D ist identisch mit Fragment B jedoch im Vektor gegenläufig zu B orientiert. Fragment E (208Bp) kodiert für das Terminationssignal des Octopin Gens. Fragment F (2764Bp) beinhaltet den Promotor des LeguminB4-Gens aus *Vicia faba*, Fragment G (235Bp) kodiert für das Transitpeptid der *A.thaliana* Isopentenyl-pyrophosphat-Isomerase-2. Fragment H (1100Bp) kodiert für das 2,3-Dimethyl-5-Phytylplastoquinol Zyklase Gen aus *Synechocystis* sp. PCC6803. Fragment I (272Bp) kodiert für das Terminationssignal des Nopalin-Synthase-Gens.

### Beispiel 65

Erzeugung von DNA Konstrukten zur Expression der γ-Tocopherol Methyltransferase aus *Arabidopsis thaliana* unter Kontrolle eines samenspezifischen Promotors und zur samenspezifischen Unterdrükkung der Expression des Homogentisinsäure-Dioxygenase-Gens aus *Brassica napus.*

Zur Herstellung von chimären DNA Konstrukten zur Erzeugung transgener *Brassica napus* Pflanzen, die die γ-Tocopherol Methyltransferase aus *Arabidopsis thaliana* unter Kontrolle eines samenspezifischen Promotors exprimieren, und die Expression der endogenen Homogentisinsäure-Dioxygenase-Gens aus *Brassica napus* samenspezifisch unterdrücken, werden die Vektoren pCR4topoblunt-SBPP-γTMT-35sT (s.u.) und pSUN2-Pvic-*BnHGD-STLS1-α*BnHGD-ocsT miteinander kombiniert.

Mittels PCR wird die Expressionkassette bestehend aus: LeB4-Promotor, die Sequenz kodierend für das Transitpeptid der *A.thaliana* plastiden-spezifischen Isopentenyl-pyrophosphat Isomerase-2 (IPP-2), der γ-Tocopherol-Methyltransferase aus *Arabidopsis thaliana* und nos-Terminationssequenz, unter Verwendung eines sense spezifischen Primers (SBPP-SRF1-5': SEQ. ID. No. 58) und eines antisense spezifischen Primers (nosT-SRF1-3' SEQ. ID. No. 53), amplifiziert und in den Vektor pCR4topoblunt (Invitrogene) kloniert. Das resultierende Plasmid ist pCR4topoblunt-SBPP-γTMT-35sT. Die PCR Bedingungen waren die folgenden:
Die PCR erfolgte in einem 50µl Reaktionsansatz in dem enthalten war:
- 1µl einer pSUN2-SBPP-γTMT-35sT Plasmid-DNA
- 0,2 mM dATP, dTTP, dGTP, dCTP
- 1,5 mM Mg(OAc)₂
- 5µg Rinderserum-Albumin
- 40pmol SBPP-SRF1 5'Primer
- 40pmol 35sT-SRP1 3'Primer
- 5µl 10x Pfu1 DNA Polymerase Puffer (Stratagene)
- 5U Pfu1 DNA Polymerase (Stratagene)
Die PCR wird unter folgenden Zyklus Bedingungen durchgeführt:
Schritt 1: 5 Minuten 94°C (Denaturierung)
Schritt 2: 3 Sekunden 94°C
Schritt 3: 1 Minute 55°C (Annealing)
Schritt 4: 10 Minuten 68°C (Elongation)
   30 Wiederholungen der Schritte 2-4
Schritt 5: 10 Minuten 72°C (Post-Elongation)

Das DNA Fragment bestehend aus LeB4-Promotor, die Sequenz kodierend für das Transitpeptid der *A.thaliana* plastiden-spezifischen Isopentenyl-pyrophosphat Isomerase-2 (IPP-2), der γ-Tocopherol Methyltransferase aus *Arabidopsis thaliana* und nos- Terminationssequenz wird aus dem Plasmid pCR4TOPOblunt/SBPP-γTMT-35sT als Srf1 Fragment isoliert und in den mit EcoR5 verdauten Vektor pSUN2-Pvic-*BnHGD-STLS1-α*BnHGD-ocsT kloniert.

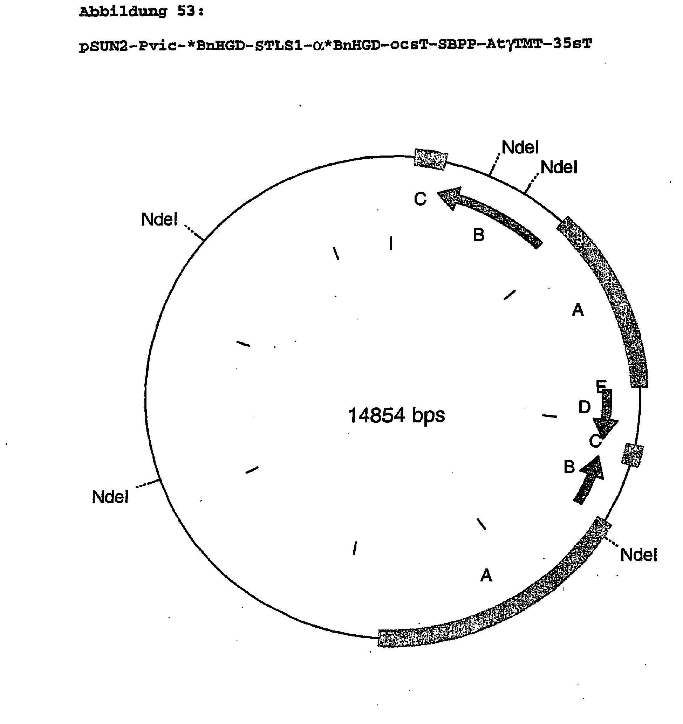

Dieses Plasmid (pSUN2-Pvic-*BnHGD-STLS1-α*BnHGD-ocsT/SBPP-γTMT-35sT Abbildung 53) wird zur Erzeugung transgener *Brassica napus* Pflanzen verwendet:

Fragment A (2559 Bp) in Abbildung 53 beinhaltet den Promotor des Vicilin-Gens aus *Vicia faba*, Fragment B (580 Bp) kodiert für einen Fragment des Homogentisinsäure-Dioxygenase-Gen aus *Brassica napus*. Fragment C (190Bp) kodiert für das 2 Intron (IV2) des ST-LS1 Gens aus *Solanum tuberosum.* Fragment D ist identisch mit Fragment B jedoch im Vektor gegenläufig zu B orientiert. Fragment E (208Bp) kodiert für das Terminationssignal-2 des Octopin Gens. Fragment F (1788Bp) beinhaltet den Promotor des SBP-Gens aus *Vicia faba*, Fragment G (1047Bp) kodiert für das γ-Tocopherol-Methyltransferase-Gen aus *Arabidopsis thaliana*, Fragment H (291Bp) kodiert für den 35S-Terminator des Blumenkohlmosaikvirus.

### Beispiel 66

Erzeugung von DNA Konstrukten zur Expression der Hydroxyphenylpyruvate-Dioxygenase aus *Arabidopsis thaliana* unter Kontrolle eines samenspezifischen Promotors und der Geranylgeranylpyrophosphate-Oxidoreductase aus *Nicotiana tabacum* unter Kontrolle eines samenspezifischen Promotors

Zur Herstellung von chimären DNA Konstrukten zur Erzeugung transgener *Brassica napus* Pflanzen, die die Hydroxyphenylpyruvate-Dioxygenase aus *Arabidopsis thaliana* unter Kontrolle eines samenspezifischen Promotors exprimieren, und die Geranylgeranylpyrophosphate-Oxidoreductase aus *Nicotiana tabacum* samenspezifischen expremieren, werden die Vektoren pSUN2-NtGGPPOR-nosT und pCR4topoblunt-USPP-AtHPPD-ocsT miteinander kombiniert.

Das DNA Fragment bestehend aus USP-Promotor, Hydroxyphenylpyruvat-Dioxygenase-Gen aus *Arabidopsis thaliana* und ocs-Terminationssequenz wird aus dem Plasmid pCR4TOPOblunt/USPP-AtHPPD-ocsT als Srf1 Fragment isoliert und in den mit Sma1 verdauten Vektor pSUN2-LeB4-NtGGPPOR-nosT kloniert.

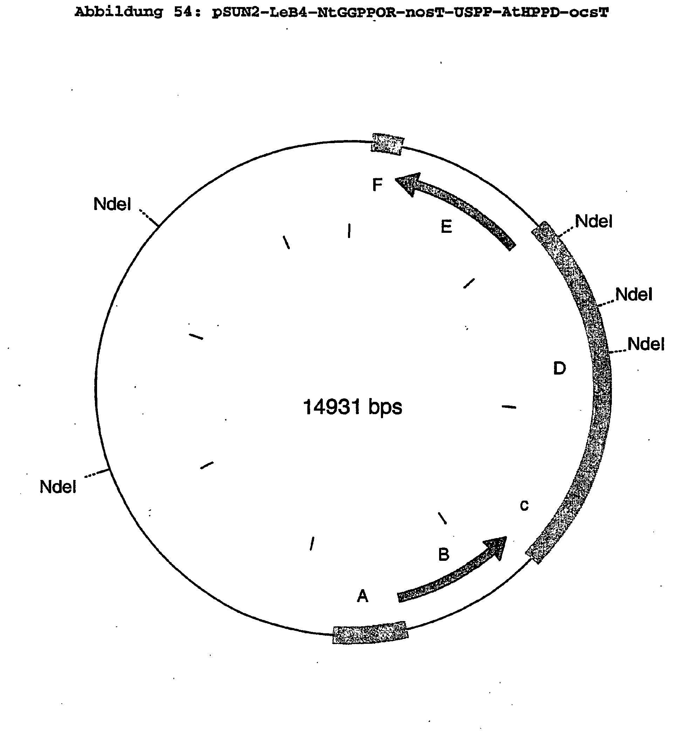

Dieses Plasmid (pSUN2LeB4-NtGGPPORnosT/USPP-AtHPPD-ocsT Abbildung 54) wird zur Erzeugung transgener *Brassica napus* Pflanzen verwendet.

Fragment A (678Bp) in Abbildung 54 beinhaltet den Promotor des "Unknow-Seed-Protein-Gens" aus *Vicia faba*, Fragment B (1338Bp) kodiert für das Hydroxyphenylpyruvate-Dioxygenase-Gen aus Arabi*dopsis thaliana*. Fragment C (713Bp) kodiert für das Terminationssignal-1 des Octopin-Synthase Gens. Fragment D (2764Bp) beinhaltet den Promotor des LeguminB4-Gens aus *Vicia faba*, Fragment E (1509Bp) kodiert für das Geranylgeranylpyrophosphate-Oxidoreduktase-Gen aus *Nicotiana tabacum* Fragment F (272Bp) kodiert für das Terminationssignal des Nopalin-Synthase-Gens.

### Beispiel 67

Erzeugung von DNA Konstrukten zur Expression der Homogentisinsäure-Phytyltransferase aus *Arabidopsis thaliana* unter Kontrolle eines samenspezifischen Promotors und der Geranylgeranylpyrophosphate-Oxidoreductase aus *Nicotiana tabacum* unter Kontrolle eines samenspezifischen Promotors

Zur Herstellung von chimären DNA Konstrukten zur Erzeugung transgener *Brassica napus* Pflanzen, die die Homogentisinsäure- Phytyltransferase aus *Arabidopsis thaliana* unter Kontrolle eines samenspezifischen Promotors exprimieren, und die Geranylgeranylpyrophosphate-Oxidoreductase aus *Nicotiana tabacum* samenspezifischen expremieren, werden die Vektoren pSUN2-LeB4-NtGGPPOR-nosT und pCR4topoblunt-USPP-AtHPT-ocsT miteinander kombiniert.

Das DNA Fragment bestehend aus USP-Promotor, Homogentisinsäure-Phytyltransferase aus *Arabidopsis thaliana* und ocs Terminationssequenz wird aus dem Plasmid pCR4TOPOblunt/USPP-AtHPT-ocsT als Srf1 Fragment isoliert und in den mit Sma1 verdauten Vektor pSUN2-LeB-NtGGPPOR-nosT kloniert.

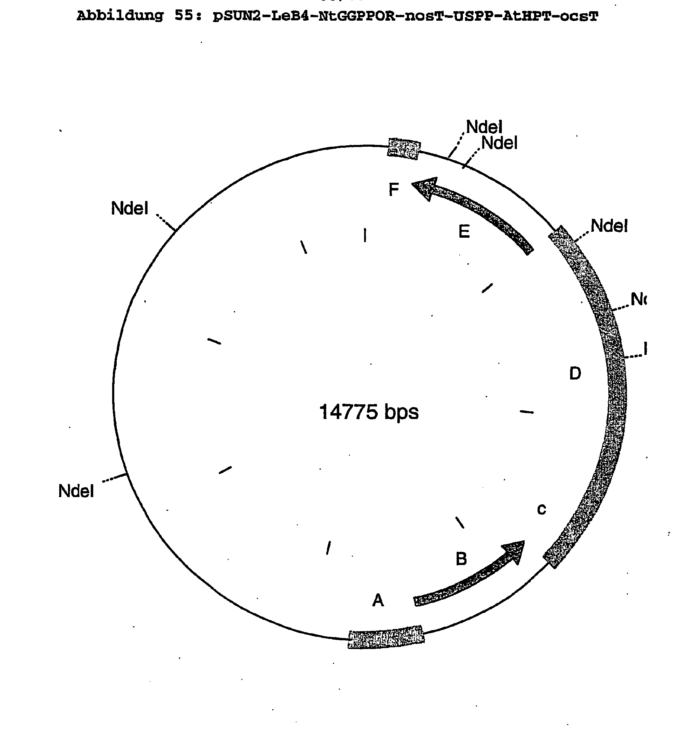

Dieses Plasmid (pSUN2-LeB4-NtGGPPOR-nosT/USPP-AtHPT-ocsT Abbildung 55) wird zur Erzeugung transgener *Brassica napus* Pflanzen verwendet:

Fragment A (678Bp) in Abbildung 55 beinhaltet den Promotor des "Unknow-Seed-Protein-Gens" aus *Vicia faba,* Fragment B (1182Bp) kodiert für das Homogentisinsäure-Phytyltransferase-Gen aus Ara*bidopsis thaliana*. Fragment C (713Bp) kodiert für das Terminationssignal-1 des Octopin-Synthase Gens. Fragment D (2764Bp) beinhaltet den Promotor des LeguminB4-Gens aus *Vicia faba,* Fragment E (1509Bp) kodiert für das Geranylgeranylpyrophosphate-Oxidoreduktase-Gen aus *Nicotiana tabacum* Fragment F (272Bp) kodiert für das Terminationssignal des Nopalin-Synthase-Gens.

### Beispiel 68

Erzeugung von DNA Konstrukten zur Expression der Hydroxyphenylpyruvate-Dioxygenase aus *Arabidopsis thaliana* unter Kontrolle eines samenspezifischen Promotors, der 2-Methyl-6-Phytylhydrochinon-Methyltransferase aus *Synechocystis spec* PC6808 unter Kontrolle eines samenspezifischen Promotors und γ-Tocopherol Methyltransferase aus *Arabidopsis thaliana* unter Kontrolle eines samenspezifischen Promotors

Zur Herstellung von chimären DNA Konstrukten zur Erzeugung transgener *Brassica napus* Pflanzen, die die Hydroxyphenylpyruvate-Dioxygenase aus *Arabidopsis thaliana* unter Kontrolle eines samenspezifischen Promotors exprimieren, 2-Methyl-6-Phytylhydrochinon-Methyltransferase aus *Synechocystis spec* PC6808 unter Kontrolle eines samenspezifischen Promotors exprimieren und γ-Tocopherol-Methyltransferase aus *Arabidopsis thaliana* unter Kontrolle eines samenspezifischen Promotors exprimieren, werden die Vektoren pSUN2-SBPP-AtγTMT35ST und pCR4topoblunt-LeB4-IPP-SynMT1-nosT und pCR4topoblunt-USPP-AtHPPDocsT miteinander kombiniert.

Das DNA Fragment bestehend aus USP-Promotor, Hydroxyphenylpyruvat- Dioxygenase-Gen aus *Arabidopsis thaliana* und ocs-Terminationssequenz-1 wird aus dem Plasmid pCR4TOPOblunt/USPP-AtHPPD-ocsT als Srf1 Fragment isoliert und in den mit Srf1 verdauten Vektor pSUN2-SBPP-AtγTMT 35ST kloniert, der zuvor ebenfalls mit dem Restriktionenzym Srf1 verdaut wird. In das enstande Plasmid pSUN2-SBPP-AtγTMT-35sT/ USPP-AtHPPD-ocsT, wird das DNA Fragment bestehend aus LeB-Promotor, 2-Methyl-6-Phytylhydrochinon-Methyltransferase aus *Synechocystis spec* PC6808 und nos-Terminationssequenz aus dem Plasmid pCR4TOPOblunt/LeB-SynMT1-nosT als Srf1 Fragment isoliert und in den mit Xho1 verdauten Vektor pSUN2-SBPP-AtγTMT35sT/USPP-AtHPPD-ocsT kloniert, nachdem die Xho1 Enden aufgefüllt wurden.

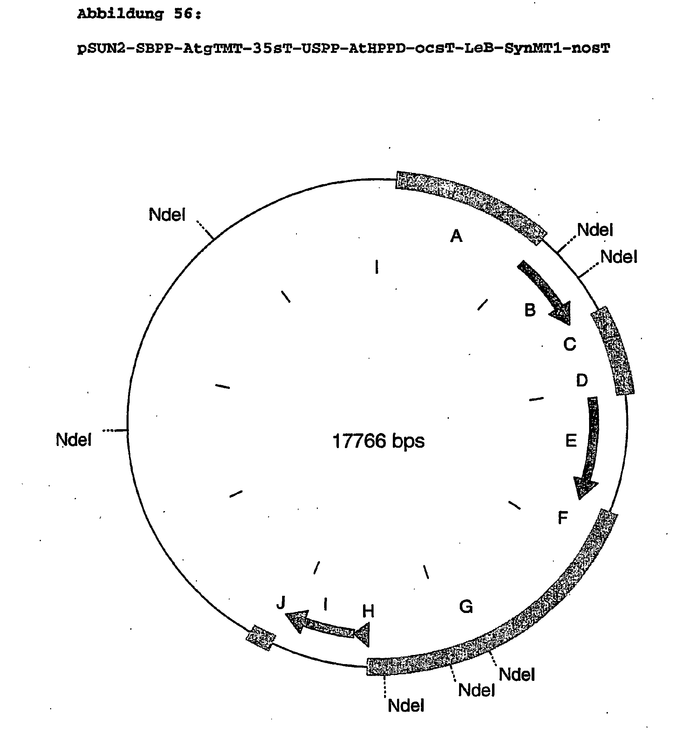

Dieses Plasmid pSUN2-SBPP-AfγTMT35sT/USPP-AtHPPD-ocsT/LeB-SynMT1-nosT Abbildung 56) wird zur Erzeugung transgener *brassica napus* Pflanzen verwendet:

Fragment A (1788Bp) in Abbildung 56 beinhaltet den Promotor des SBP-Gens aus *Vicia faba,* Fragment B (1047Bp) kodiert für das γ-Tocopherol-Methyltransferase-Gen aus *Arabidopsis thaliana,* Fragment C (291Bp) kodiert für den 35*S*-Terminator des Blumenkohlmosaikvirus. Fragment D (678Bp) beinhaltet den Promotor des "Unknow-Seed-Protein-Gens" aus *Vicia faba,* Fragment E (1338Bp) kodiert für das Hydroxyphenylpyruvate-Dioxygenase-Gen aus *Arabidopsis thaliana.* Fragment F (713Bp) kodiert für das Terminationssignal-1 des Octopin-Synthase Gens. Fragment G (2764Bp) beinhaltet den Promotor des LeguminB4-Gens aus *Vicia faba,* Fragment H (235Bp) kodiert für das Transitpeptid der *A*. *thaliana* Isopentenyl-pyrophosphat-Isomerase-2. Fragment I (957Bp) kodiert für das 2-Methyl-6-Phytylhydrochinol Methyltransferase Gen aus *Synechocystis* sp. PCC6803. Fragment J (272Bp) kodiert für das Terminationssignal des Nopalin-Synthase-Gens.

### Beispiel 69

Erzeugung von DNA Konstrukten zur Expression der Hydroxyphenylpyruvate-Dioxygenase aus *Arabidopsis thaliana* unter Kontrolle eines samenspezifischen Promotors, der 2-Methyl-6-Phytylhydrochinon-Methyltransferase aus *Synechocystis spec* PC6808 unter Kontrolle eines samenspezifischen Promotors

Zur Herstellung von chimären DNA Konstrukten zur Erzeugung transgener *Brassica napus* Pflanzen, die die Hydroxyphenylpyruvate Dioxygenase aus *Arabidopsis thaliana* unter Kontrolle eines samenspezifischen Promotors exprimieren, 2-Methyl-6-Phytylhydroquinon-Methyltransferase aus *Synechocystis spec* PC6808 unter Kontrolle eines samenspezifischen Promotors werden und pSUN2-USPP-AtHPPDocsT und der Vektor pCR4topoblunt-LeB4-IPP-SynMT1-nosT miteinander kombiniert.

Das DNA Fragment bestehend aus LeB-Promotor, 2-Methyl-6-Phytylhydrochinon-Methyltransferase aus *Synechocystis spec* PC6808 und nos-Terminationssequenz wird aus dem Plasmid pCR4TOPO-blunt/LeB-SynMT1-nosT als Srf1 Fragment isoliert und in den mit Xho1 verdauten Vektor pSUN2-USPP-AtHPPD-ocsT kloniert, dessen Xho1 Enden aufgefüllt werden.

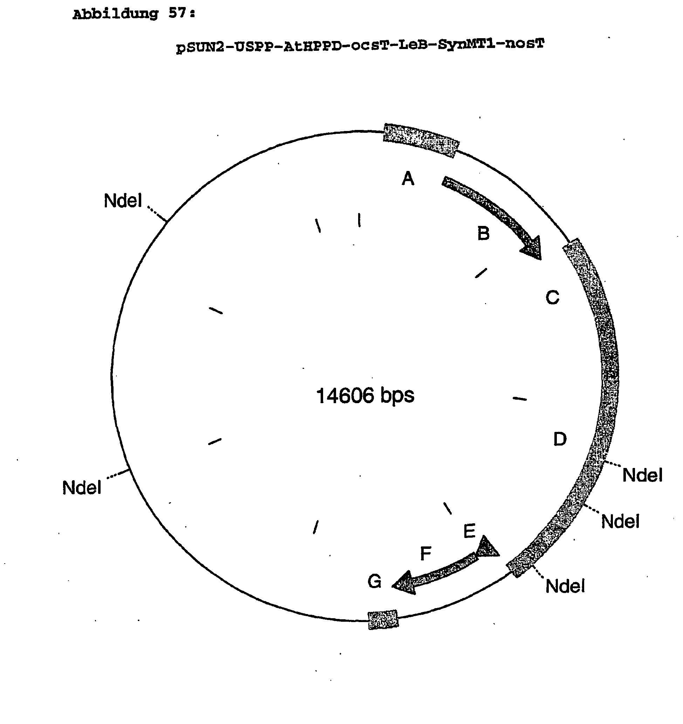

Dieses Plasmid pSUN2-USPP-AtHPPD-ocsT/LeB-SynMT1-nosT Abbildung 57) wird zur Erzeugung transgener *Brassica napus* Pflanzen verwendet:

Fragment A (678Bp) in Abbildung 57 beinhaltet den Promotor des "Unknow-Seed-Protein-Gens" aus *Vicia faba,* Fragment B (1338Bp) kodiert für das Hydroxyphenylpyruvate-Dioxygenase-Gen aus *Arabidopsis thaliana* und Fragment C (713Bp) kodiert für das Terminationssignal-1 des Octopin-Synthase Gens. Fragment D (2764Bp) beinhaltet den Promotor des LeguminB4-Gens aus *Vicia faba*, Fragment E (235Bp) kodiert für das Transitpeptid der *A.thaliana* Isopentenylpyrophosphat-Isomerase-2. Fragment F (957Bp) kodiert für das 2-Methyl-6-Phytylhydrochinol Methyltransferase Gen aus *Synechocystis* sp. PCC6803. Fragment G (272Bp) kodiert für das Terminationssignal des Nopalin-Synthase-Gens.

### Beispiel 70

Erzeugung von DNA Konstrukten zur Expression der Hydroxyphenylpyruvate-Dioxygenase aus *Arabidopsis thaliana* unter Kontrolle eines samenspezifischen Promotors und der Geranylgeranyl-pyrophosphate-Oxidoreductase aus *Nicotiana tabacum* unter Kontrolle eines samenspezifischen Promotors und der Homogentisinsäure-Phytyltransferase aus *Arabidopsis thaliana* unter Kontrolle eines samenspezifischen Promotors.

Zur Herstellung von chimären DNA Konstrukten zur Erzeugung transgener *Brassica napus* Pflanzen, die die Hydroxyphenylpyruvate Dioxygenase aus *Arabidopsis thaliana* unter Kontrolle eines samenspezifischen Promotors exprimieren, die Geranylgeranyl-pyrophosphate-Oxidoreductase aus *Nicotiana tabacum* samenspezifischen expremieren, und Homogentisinsäure-Phytyltransferase aus Arabidopsis thaliana samenspezifischen expremieren, werden die Vektoren pSUN2-LeB4-NtGGPPOR-nosT/USPP-AtHPT-ocsT und pCR4topoblunt-USPP-AtHPPD-ocsT miteinander kombiniert.

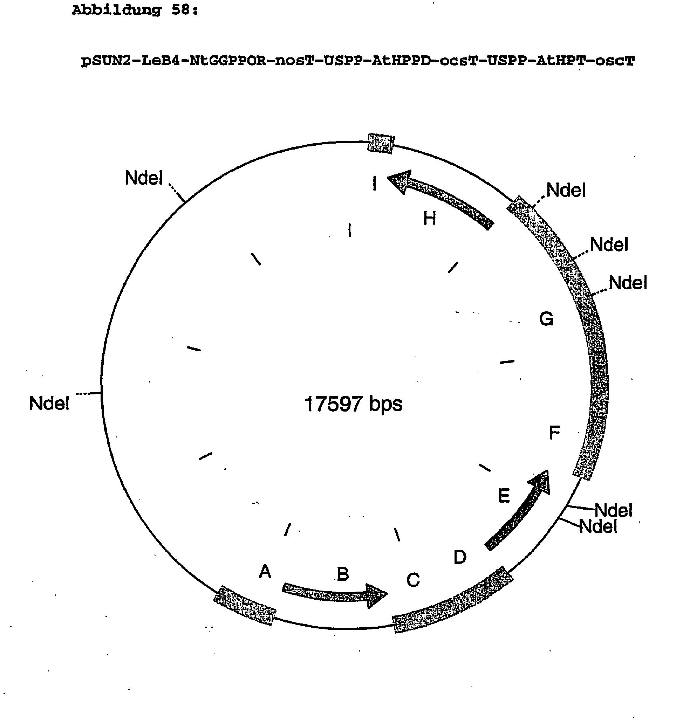

Das DNA Fragment bestehend aus USP-Promotor, Hydroxyphenylpyruvat- Dioxygenase aus *Arabidopsis thaliana* und ocs-Terminationssequenz-1 wird aus dem Plasmid pCR4TOPOblunt/USPP-AtHPPD-ocsT als Srf1 Fragment isoliert und in den mit Xho1 verdauten Vektor pSUN2-LeB4-NtGGPPOR-nosT/USPP-AtHPT-ocsT kloniert, nachdem die Xho1 Enden zuvor mit der Klenow Polymerase geglättet werden. Dieses Plasmid (pSUN2LeB4-NtGGPPORnosT/USPP-AtHPPD-ocsT/USPP-AtHPT-ocsT Abbildung 58) wird zur Erzeugung transgener *Brassica napus* Pflanzen verwendet.

Fragment A (678Bp) in Abbildung 58 beinhaltet den Promotor des "Unknow-Seed-Protein-Gens" aus *Vicia faba,* Fragment B (1338Bp) kodiert für das Hydroxyphenylpyruvate-Dioxygenase-Gen aus *Arabidopsis thaliana*. Fragment C (713Bp) kodiert für das Terminationssignal-1 des Octopin-Synthase Gens. Fragment D (678Bp) beinhaltet den Promotor des "Unknow-Seed-Protein-Gens" aus *Vicia faba,* Fragment E (1182Bp) kodiert für das Homogentisinsäure-Phytyltransferase-Gen aus *Arabidopsis thaliana.* Fragment F (713Bp) kodiert für das Terminationssignal-1 des Octopin-Synthase Gens. Fragment G (2764Bp) beinhaltet den Promotor des LeguminB4-Gens aus *Vicia faba,* Fragment H (1509Bp) kodiert für das Geranylgeranylpyrophosphate-Oxidoreduktase-Gen aus *Nicotiana tabacum* Fragment I (272Bp) kodiert für das Terminationssignal des Nopalin-Synthase-Gens:

### Beispiel 71

Erzeugung von DNA Konstrukten zur Expression der 2,3-Dimethyl-5-Phytylplastochinol-Zyklase aus *Synechocystis spec* PC6808 unter Kontrolle eines samenspezifischen Promotors, der 2-Methyl-6-Phytylhydroquinon-Methyltransferase aus *Synechocystis spec* PC6808 unter Kontrolle eines samenspezifischen Promotors und γ-Tocopherol- Methyltransferase aus *Arabidopsis thaliana* unter Kontrolle eines samenspezifischen Promotors.

Zur Herstellung von chimären DNA Konstrukten zur Erzeugung transgener *Brassica napus* Pflanzen, die die 2,3-Dimethyl-5-Phytylplastochinol-Zyklase aus *Synechocystis spec* PC6808 unter Kontrolle eines samenspezifischen Promotors exprimieren, 2-Methyl-6-Phytylhydrochinon-Methyltransferase aus *Synechocystis spec* PC6808 unter Kontrolle eines samenspezifischen Promotors exprimieren und γ-Tocopherol-Methyltransferase aus *Arabidopsis thaliana* unter Kontrolle eines samenspezifischen Promotors exprimieren, werden die Konstrukte pSUN2-SBPP-AtγTMT35ST/USPP-AtHPPD-ocsT/LeB-SynMT1-nosT und pCR4topoblunt/LeB-IPP-SynCyc-nosT verwendet.

Das DNA Fragment bestehend aus LeB-Promotor, 2,3-Dimethyl-5-Phytylplastochinol-Zyklase aus *Synechocystis spec* PC6808 und nos-Terminationssequenz wird aus dem Plasmid pCR4topoblunt/LeB-IPP-SynCyc-nosT als EcoR5 Fragment isoliert und in den mit Srf1 verdauten Vektor pSUN2-SBPP-AtγTMT-35ST/USPP-AtHPPD-ocsT/LeB-SynMT1-nosT kloniert, der zuvor mit dem Restriktionenzym Srf1 verdaut wird. Dadurch wird die Expressionkassette bestehend aus USP-Promotor, Hydroxyphenylpyruvat-Dehydrogenase-Gen aus *Arabidopsis thaliana* und ocs-Terminationsequenz gegen die Expressionkassette bestehend aus LeB-Promotor, die 2,3-Dimethyl-5-Phytylplastochinol- Zyklase-Genaus *Synechocystis spec* PC6808 und nos-Terminationssequenz, ausgetauscht.

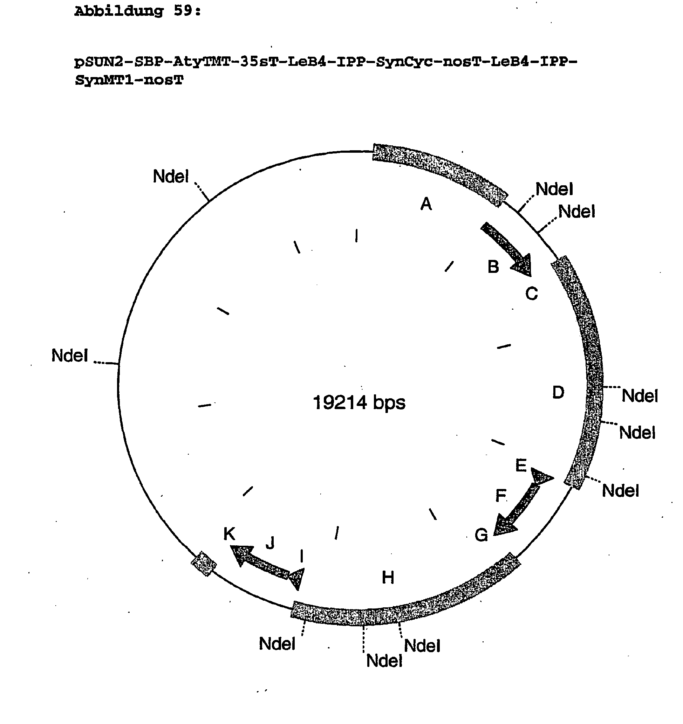

Dieses Plasmid pSUN2-SBPP-AtγTMT35sT/LeB-IPP-SynCyc-nosT/LeB-IPP-SynMT1-nosT Abbildung59) wird zur Erzeugung transgener *Brassica napus* Pflanzen verwendet.

Fragment A (1788Bp) in Abbildung 59 beinhaltet den Promotor des SBP-Gens aus *Vicia faba*, Fragment B (1047Bp) kodiert für das γ-Tocopherol-Methyltransferase-Gen aus *Arabidopsis thaliana,* Fragment C (291Bp) kodiert für den 35*S*-Terminator des Blumenkohlmosaikvirus. Fragment D (2764Bp) beinhaltet den Promotor des LeguminB4-Gens aus *Vicia faba,* Fragment E (235Bp) kodiert für das Transitpeptid der *A.thaliana* Isopentenyl-pyrophosphat-Isomerase-2. Fragment F (1100Bp) kodiert für das 2,3-Dimethyl-5-Phytylplastoquinol Zyklase Gen aus *Synechocystis* sp. PCC6803. Fragment G (272Bp) kodiert für das Terminationssignal des Nopalin-Synthase- Gens. Fragment H (2764Bp) beinhaltet den Promotor des LeguminB4-Gens aus *Vicia faba,* Fragment I (235Bp) kodiert für das Transitpeptid der *A.thaliana* Isopentenyl-pyrophosphat-Isomerase-2. Fragment J (957Bp) kodiert für das 2-Methyl-6-Phytylhydrochinol Methyltransferase Gen aus *Synechocystis* sp. PCC6803. Fragment K (272Bp) kodiert für das Terminationssignal des Nopalin-Synthase-Gens.

### Beispiel 72

Erzeugung von DNA Konstrukten zur Expression der Tyrosin-Aminotransferase aus *Rattus norvegicus* unter Kontrolle eines samenspezifischen Promotors.

Zur Herstellung von chimären DNA Konstrukten zur Erzeugung transgener *A.thaliana, Nicotiana tabacum bzw*. *B*.*napus* Pflanzen, die die Tyrosin-Aminotransferase aus *Rattus norvegicus* (Seq. ID. No. 1) unter Kontrolle eines samenspezifischen Promotors exprimieren, wurde ein Derivat des Vektors pGPTVkan (D.Becker, E. Kemper, J. Schell, R. Masterson. Plant Molecular Biology 20: 1195-1197, 1992) verwendet.

Dieser Vektor wurde so verändert, dass er den samenspezifichen Promotor des Legumin B4 Gens (Kafatos et al., Nuc. Acid. Res.,14(6):2707-2720, 1986), die Sequenz kodierend für das Transitpeptid der *A.thaliana* plastiden-spezifischen Isopentenyl-pyrophosphat Isomerase-2 (IPP-2) (Badur, unveröffentlicht) und das Terminationssignal der Nopalinsynthase aus *A.tumefaciens* (Depikker et al., J. Mol. Appl. Genet. 1, 561-73, 1982) enthält.

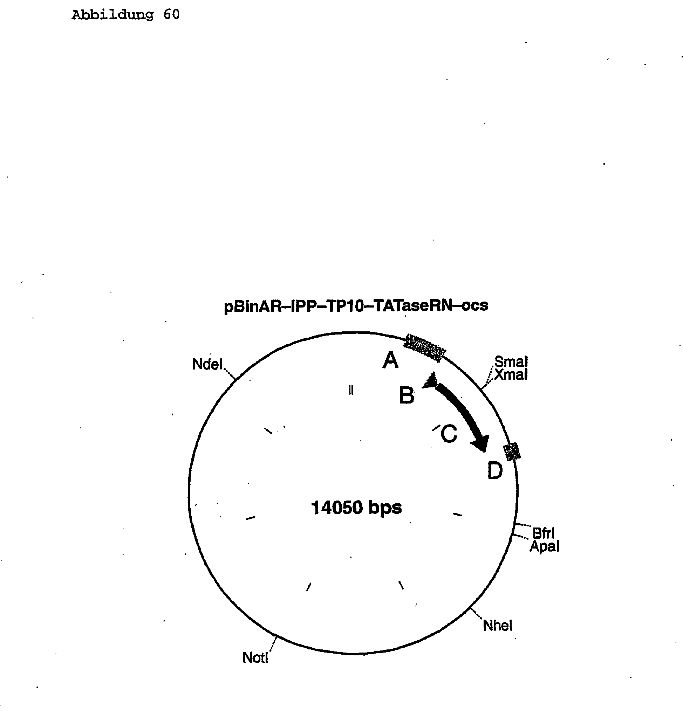

Das DNA Fragment kodierend für das Tyrosin-Aminotransferase-Gen aus Rattus norvegicus wurde als EcoR5 Fragment in den pPTVKan-LeP-IPPTP11 kloniert, nachdem dieser mit dem Restriktionsenzym SalI verdaut und die Enden des linearisierten Plasmides mit dem Klenow Enzym in glatte Enden überführt wurden. Dadurch wurde eine Translationsfusion mit dem Transitpeptid der IPP-2 erzeugt und somit ein Import der Tyrosin-Aminotransferase in die Plastiden gewährleistet. Dieses Plasmid pPTVkan-IPPTP11-TATaseRNnos (oder auch pPTVkan-LeB4-IPP-RnTATase-nosT bezeichnet, Abbildung 60) wurde zur Erzeugung transgener *Brassica Napus* bzw. *A*. *thaliana* Pflanzen verwendet.

Fragment A (2764 bp) in Abbildung 60 beinhaltet den Promotor des LeguminB4-Gens aus Vicia faba, Fragment B (207bp) kodiert für das Transitpeptid der *A.thaliana* Isopentenyl-pyrophosphat-Isomerase-2. Fragment C (1377 Bp) kodiert für das Tyrosin-Aminotransferase-Gen aus *Rattus norvegicus.* Fragment D (272Bp) kodiert für das Terminationssignal des Nopalin-Synthase Gens.

### Beispiel 73

Herstellung von Expressionskassetten enthaltend das Tyrosin-Aminotransferase-Gen aus *Rattus norvegicus*

Transgene *Nicotiana tabacum* und *Arabidopsis thaliana* Pflanzen wurden erzeugt, die die Tyrosin-Aminotransferase aus *Rattus norvegicus* (Seq. ID. No. 1) unter Kontrolle des konstitutiven 35*S*-Promotor des CaMV (Blumenkohlmosaikvirus) (Franck et al., Cell 21: 285-294, 1980) exprimieren.

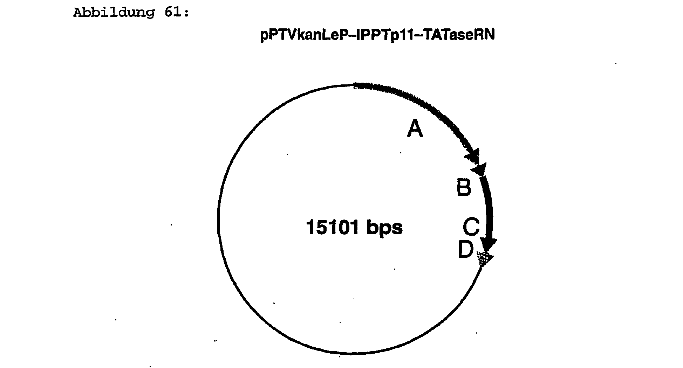

Die Grundlage des zur konstitutiven Expression der Tyrosin-Aminotransferase-1 aus *Rattus norvegicus* erzeugten Plasmides war der pBinAR-IPP-Tp-10 (Ralf Badur, Dissertration Universität Göttingen, 1998). Dieser Vektor ist ein Derivat des pBinAR (Höfgen und Willmitzer, Plant Sei. 66: 221-230, 1990) und enthält den 35*S-*Promotor des CaMV (Blumenkohlmosaikvirus) (Franck et al., 1980) das Terminations-signal des Octopin-Synthase Gens (Gielen et al., EMBO J. 3: 835-846, 1984) und die Sequenz kodierend für das Transitpeptid der *A*.*thaliana* plastiden-spezifischen Isopentenylpyrophosphat Isomerase-2 (IPP-2) (Badur, unveröffentlicht). Die unter Berücksichtigung des korrekten Leserasters erfolgte Klonierung der Tyrosin-Aminotransferase aus Rattus norvegicus in diesen Vektor, erzeugt eine Translationsfusion der Tyrosin-Aminotransferase mit dem plastidären Transitpeptid. Dadurch erfolgt ein Transport des Transgens in die Plastiden.
Zur Erstellung dieses Plasmides wurde das *Tyrosin-Aminotransfe*rase-Gen unter Verwendung der flankierenden EcoRV Restriktionsschnittstellen aus dem Plasmid pGEM-T/Tyrosin-Aminotransferase isoliert. Dieses Fragment wurde unter Anwendung von Standardmethoden in einen SmaI geschnittenen pBinAR-IPP-Tp-10 ligiert (siehe Abbildung 61) Dieses Plasmid pBinAR-IPP-Tp-10/Tyrosin-Aminotransferase (oder auch pBinAr-35sP-IPP-RnTATase-nosT bezeichnet) wurde zur Erzeugung transgener *Nicotiana tabacum* und *A.thaliana* Pflanzen verwendet.

Fragment A (529 bp) in Abbildung 61 beinhaltet den 35*S*-Promotor des Blumenkohlmosaikvirus (Nukleotide 6909 bis 7437 des Blumenkohlmosaikvirus), Fragment B (207bp) kodiert für das Transitpeptid der Isopentenyl-pyrophosphat Isomerase-2, Fragment C (1377 Bp) kodiert für das *Tyrosin-Aminotransferase*-Gen-1 aus *Rattus norvegicus,* Fragment D (208Bp) kodiert für das Terminationssignal des Octopin-Synthase Gens.

### Beispiel 74

### Herstellung transgener Arabidopis thaliana Pflanzen

Wildtyp *Arabidopsis thaliana* Pflanzen.(Columbia) werden mit dem *Agrabacterium tumefaciens* Stamm (GV3101 [pMP90]) auf Grundlage einer modifizierten Vacuuminfiltrationsmethode transformiert (Steve Clough und Andrew Bent. Floral dip: a simplified method for Agrobacterium mediated transformation of A.thaliana. Plant J 16(6):735-43, 1998; der Bechtold, N. Ellis, J. und Pelltier, G., in: Planta Agrobacterium-mediated gene transfer by infiltration of adult Arabidopsis thaliana plants. CRAcad Sci Paris, 1993, 1144(2):204-212). Die verwendeten *Agrobacterium tumefaciens* Zellen werden im Vorfeld mit den vorstehend beschriebenen DNA Konstrukten transformiert.

Samen der Primärtransformanden werden auf Grundlage der Antibiotikaresistenz selektioniert. Antibiotika resistente Keimlinge wurden in Erde gepflanzt und als vollentwickelte Pflanzen zur biochemischen Analyse verwendet.

### Beispiel 75

### Herstellung transgener Nicotiana tabacum Pflanzen.

Zehn ml YEB-Medium mit Antibiotikum (5 g/l Rinder-Extrakt, 1 g/l Hefe-Extrakt, 5 g/l Pepton, 5 g/l Saccharose und 2 mM MgSO₄.) werden mit einer Kolonie von *Agrobacterium tumefaciens* beimpft und über Nacht bei 28°C kultiviert. Die Zellen werden 20 min bei 4°C, 3500 U/min in einer Tischzentrifuge pelletiert und danach in frischem YEB-Medium ohne Antibiotika unter sterilen Bedingungen resuspendiert. Die Zellsuspension wird für die Transformation eingesetzt.

Die Wildtyp-Pflanzen aus Sterilkultur werden durch vegetative Replikation erhalten. Dazu wird nur die Spitze der Pflanze abgeschnitten und auf frisches 2MS-Medium in ein steriles Einweckglas überführt. Vom Rest der Pflanze werden die Haare auf der Blattoberseite und die Mittelrippen der Blätter entfernt. Die Blätter werden mit einer Rasierklinge in etwa 1cm² große Stücke geschnitten. Die Agrobakterienkultur wird in eine kleine Petrischale überführt (Durchmesser 2 cm). Die Blattstücke werden kurz durch diese Lösung gezogen und mit der Blattunterseite auf 2MS-Medium in Petrischalen (Durchmesser 9 cm) gelegt, so daß sie das Medium berühren. Nach zwei Tagen im Dunkeln bei 25°C werden die Explantate auf Platten mit Kallusinduktionsmedium überführt und in der Klimakammer auf 28 °C temperiert. Das Medium muß alle 7-10 Tage gewechselt werden. Sobald sich Kalli bilden, wurden die Explantate in sterile Einweckgläser auf Sproßinduktionsmedium mit Claforan (0,6 % BiTec-Agar (g/v), 2,0 mg/l Zeatinribose, 0,02 mg/l Naphtylessigsäure, 0,02 mg/l Gibberelinsäure, 0,25 g/ml Claforan, 1,6 % Glukose (g/v) und 50 mg/l Kanamycin) überführt. Nach etwa einem Monat tritt Organogenese ein und die gebildeten Sprosse können abgeschnitten werden. Die Kultivierung der Sprosse wird auf 2MS-Medium mit Claforan und Selektionsmarker durchgeführt.
Sobald sich ein kräftiger Wurzelballen bildet, können die Pflanzen in Pikiererde getopft werden.

### Beispiel 76

### Herstellung transgener Brassica napus Pflanzen.

Die Herstellung transgener Raps Pflanzen orientiert sich an einem Protokoll von Bade, J.B. und Damm,B. (in Gene Transfer to Plants, Potrykus, I. und Spangenberg, G., eds, Springer Lab Manual, Springer Verlag, 1995, 30-38), in welchem auch die Zusammensetzung der verwendeten Medien und Puffer angegeben ist.

Die Transformationen erfolgt mit dem *Agrobacterium tumefaciens* Stamm GV3101 [pMP90]. Zur Transformation wird das DNA Konstrukt welches eine spezifische Expression in Samen vermittelt verwendet (Abbildung 60). Darüberhinaus werden Konstrukt welche eine spezifische Expression in Samen vermittelt verwendet, die in den Abbildungen 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 beschrieben sind. Samen von *Brassica napus* var. Westar werden mit 70% Ethanol (v/v) oberflächensteril gemacht, 10 Minuten bei 55°C in Wasser gewaschen, in 1%iger Hypochlorit-Lösung (25% v/v Teepol,0,1% v/v Tween 20) für 20 Minuten inkubiert und sechsmal mit sterilem Wasser für jeweils 20 Minuten gewaschen. Die Samen werden drei Tage auf Filterpapier getrocknet und 10-15 Samen in einem Glaskolben mit 15 ml Keimungsmedium zur Keimung gebracht. Von mehreren Keimlingen (ca. 10 cm groß) werden die Wurzeln und Apices entfernt und die verbleibenden Hypokotyle in ca. 6 mm lange Stücke geschnitten. Die so gewonnenen ca.. 600 Explantate werden 30 Minuten mit 50 ml Basalmedium gewaschen und in einem 300 ml Kolben überführt. Nach Zugabe von 100 ml Kallusinduktionsmedium werden die Kulturen für 24 Stunden bei 100 U/min inkubiert.

Vom Agrobacterium Stamm wird eine Übernachtkultur bei 29°C in Luria Broth-Medium mit Kanamycin (20mg/l) angesetzt, davon 2ml in 50 ml Luria Broth-Medium ohne Kanamycin für 4 Stunden bei 29°C bis zu einer OD₆₀₀ von 0,4-0,5 inkubiert. Nach der Pelletierung der Kultur bei 2000 U/min für 25 min wird das Zellpellet in 25 ml Basalmedium resuspendiert. Die Konzentration der Bakterien in der Lösung wird durch Zugabe von weiterem Basalmedium auf eine OD₆₀₀ von 0,3 eingestellt.

Aus den Raps-Explanten wird das Kallus-Induktionsmedium mit sterilen Pipetten entfernt, 50 ml Agrobakterium-Lösung hinzugefügt, vorsichtig gemischt und für 20 min inkubiert. Die Agrobacterien-Suspension wird entfernt, die Raps-Explante für 1 min mit 50 ml Kallus-Induktionsmedium gewaschen und anschließend 100 ml Kallus-Induktionsmedium hinzugefügt. Die Co-Kultivierung wird für 24 h auf einem Rotiationsschüttler bei 100 U/min durchgeführt. Die Co-Kultivierung wird durch Wegnahme des Kallus-Induktionsmediums gestoppt und die Explante zweimal für jeweils 1 min mit 25 ml und zweimal für 60 min mit jeweils 100 ml Waschmedium bei 100 U/min gewaschen. Das Waschmedium mit den Explanten wird in 15 cm Petrischalen überführt und das Medium mit sterilen Pipetten entfernt.

Zur Regeneration werden jeweils 20-30 Explante in 90 mm Petrischalen überführt, welche 25 ml Sproß-Induktionsmedium mit Kanamycin enthalten. Die Petrischalen werden mit 2 Lagen Leukopor verschlossen und bei 25 °C und 2000 lux bei Photoperioden von 16 Stunden Licht/ 8 Stunden Dunkelheit inkubiert. Alle 12 Tage werden die sich entwickelnden Kalli auf frische Petrischalen mit Sproß-Induktionsmedium umgesetzt. Alle weiteren Schritte zur Regeneration ganzer Pflanzen werden wie von Bade, J.B und Damm, B. (in: Gene Transfer to Plants, Potrykus, I. und Spangenberg, G., eds, Springer Lab Manual, Springer Verlag, 1995, 30-38) beschrieben durchgeführt.

### Beispiel 77

### a) Charakterisierung der transgenen Arabidopsis thaliana und Nicotiana tabacum Pflanzen.

Die Tocopherol- und Tocotrienol-Gehalte in Blätter und Samen der mit den beschriebenen Konstrukten transformierten Pflanzen *(Arabidopsis thaliana* und *Nicotiana tabacum*) werden analysiert. Dazu werden die transgenen Pflanzen im Gewächshaus kultiviert und Pflanzen die das Gen kodierend für die Tyrosin-Aminotransferase-1 aus *Rattus norvegicus* exprimieren auf Northern-Ebene analysiert. In Blättern und Samen dieser Pflanzen wird der Tocopherolgehalt und der Tocotrienolgehalt ermittelt.

Dazu wird das Blattmaterial von Pflanzen direkt nach der Probennahme in flüssigem Stickstoff tiefgefroren. Der daran anschließende Aufschluß der Zellen erfolgt mittels einer Rührapparatur durch dreimalige Inkubation im Eppendorfschüttler bei 30°C, 1000 rpm in 100 % Methanol für 15 Minuten, wobei die jeweils erhaltenen Überstände vereinigt wurden.

Weitere Inkubationsschritte ergaben keine weitere Freisetzung von Tocopherolen oder Tocotrienolen.

Um Oxidation zu vermeiden, wurden die erhaltenen Extrakte direkt nach der Extraktion mit Hilfe einer HPLC-Anlage (Waters Allience 2690) analysiert. Tocopherole und Tocotrienole wurden über eine reverse Phase Säule(ProntoSil 200-3-C30^{(R)}, Fa. Bischoff) mit einer mobilen Phase von 100 % Methanol getrennt und anhand von Standards (Fa. Merck) identifiziert. Als Detektionssystem diente die Fluoreszens der Substanzen (Anregung 295 nm, Emission 320 nm) die mit Hilfe eines Jasco Fluoreszensdetektors FP 920 nachgewiesen wurde.

In allen Fällen war die Tocopherol- und oder Tocotrienol-Konzentration in transgenen Pflanzen, im Vergleich zu nicht transformierten Pflanzen erhöht.

### b) Charakterisierung der transgenen Brassica napus Pflanzen.

Um zu veranschaulichen, daß durch die Expression des Tyrosin-Aminotransferase-Gens aus *Rattus norvegicus,* Tyrosin-Aminotransferase-Gens 1 aus *Arabidposis thaliana,* Tyrosin-Aminotransferase-Gens 3 aus *Arabidposis thaliana*, Tyrosin-Aminotransferase-Gens 5 aus *Arabidposis thaliana* oder Tyrosin-Aminotransferase-Gens 6 aus *Arabidposis thaliana* alleine oder in Kombination mit zumindest einem weiteren Gen ausgewählt aus der Gruppe Hydroxyphenyl-Pyruvat-Dioxygenase-Gen aus *Arabidopsis thaliana*, Homogentisinsäure-Phytyltransferase-Gen aus *Arabidopsis thaliana,* Geranylgeranylpyrophosphat-Oxidoredktase-Gen aus *Nicotiana tabacum,* 2-Methyl-6-Phytylhydrochinon-Methyltransferase-Gen aus *Synechocystis* sp. PCC6803, 2,3-Dimethyl-5-Phytylplastochinol-Zyklase-Gen *Synechocystis* sp. PCC6803, γ-Tocopherol-methyltransferase-Gen aus *Arabidopsis thaliana* und der Unterdrückung der Expression des Homogentisinsäure-Dioxygenase-Gens, der Vitamin E-gehalt in Pflanzen erhöht wird, werden die Tocopherol- und Tocotrienol-Gehalte in den Samen der mit den beschriebenen Konstrukten transformierten Pflanzen *(Brassica napus)* analysiert.

Dazu werden die transgenen Pflanzen im .Gewächshaus kultiviert und auf Northern-Ebene analysiert. In Samen dieser Pflanzen wird der Tocopherolgehalt und der Tocotrienolgehalt analog Beispiel 77 a) ermittelt.

### Beispiel 78

### Herstellung transgener Arabidopsis thaliana Pflanzen, die die Tyrosinaminotransferase überexprimieren

Wildtyp *Arabidopsis thaliana* Pflanzen (Columbia) wurden mit dem *Agrobacterium tumefaciens* Stamm (GV3101 [pMP90]) auf Grundlage einer modifizierten Vakuuminfiltrationsmethode transformiert (Steve Clough und Andrew Bent. Floral dip: a simplified method for Agrobacterium mediated transformation of A.thaliana. Plant J 16(6):735-43, 1998; der Bechtold, N. Ellis, J. und Pelltier, G., in: Planta Agrobacterium-mediated gene transfer by infiltration of adult Arabidopsis thaliana plants. CRAcad Sci Paris, 1993, 1144(2):204-212).

Die verwendeten *Agrobacterium tumefaciens* Zellen waren im Vorfeld mit den Plasmiden pBinAR-35s-IPP-RnTatase-nosT (Beispiel 73, Abbildung 61) und pPTVkan-LeB4-IPP-RnTATase-nosT (Beispiel 72, Abbildung 60) gemäß der von R. HÖFGEN und L. WILLMITZER (Plant Sci. 1990, 66, 221-230 und Nucleic Acids Res. 1988, Oct 25, 16(20), 9877) beschriebenen Methode transformiert worden.

Samen der Primärtransformanden wurden auf Grundlage der Antibiotikaresistenz selektioniert. Antibiotika resistente Keimlinge wurden in Erde gepflanzt und als vollentwickelte Pflanzen zur biochemischen Analyse verwendet.

### Beispiel 79

### Herstellung transgener Brassica napus Pflanzen, die die Tyrosinaminotransferase überexprimieren

Die Herstellung transgener Raps Pflanzen orientierte sich an einem Protokoll von Bade, J.B. und Damm,B. (in Gene Transfer to Plants, Potrykus, I. und Spangenberg, G., eds, Springer Lab Manual, Springer Verlag, 1995, 30-38), in welchem auch die Zusammensetzung der verwendeten Medien und Puffer angegeben ist.

Die Transformationen erfolgten mit dem *Agrobacterium tumefaciens* Stamm GV3101 [pMP90]. Die verwendeten *Agrobacterium tumefaciens* Zellen waren im Vorfeld mit dem Plasmid pPTVkan-LeB4-IPP-RnTA-Tase-nosT (Beispiel 72, Abbildung 60) gemäß der von R. HÖFGEN und L. WILLMITZER (Plant Sci. 1990, 66, 221-230 und Nucleic Acids Res. 1988, Oct 25, 16(20), 9877) beschriebenen Methode transformiert worden. '

Samen von Brassica napus var. Westar wurden mit 70% Ethanol (v/v) oberflächensteril gemacht, 10 Minuten bei 55°C in Wasser gewaschen, in 1%iger Hypochlorit-Lösung (25 % v/v Teepol,0,1 % v/v Tween 20) für 20 Minuten inkubiert und sechsmal mit .sterilem Wasser für jeweils 20 Minuten gewaschen. Die Samen wurden drei Tage auf Filterpapier getrocknet und 10-15 Samen in einem Glaskolben mit 15 ml Keimungsmedium zur Keimung gebracht. Von mehreren Keimlingen (ca. 10 cm groß) wurden die Wurzeln und Apices entfernt und die verbleibenden Hypokotyle in ca. 6 mm lange Stücke geschnitten. Die so gewonnenen ca. 600 Explantate wurden 30 Minuten mit 50 ml Basalmedium gewaschen und in einem 300 ml Kolben überführt. Nach Zugabe von 100 ml Kallusinduktionsmedium wurden die Kulturen für 24 Stunden bei 100 U/min inkubiert.

Vom Agrobacterium Stamm wurde eine Übernachtkultur bei 29°C in Luria Broth-Medium mit Kanamycin (20mg/l) angesetzt, davon 2ml in 50 ml Luria Broth-Medium ohne Kanamycin für 4 Stunden bei 29°C bis zu einer OD₆₀₀ von 0,4-0,5 inkubiert. Nach der Pelletierung der Kultur bei 2000 U/min für 25 min wurde das Zellpellet in 25 ml Basalmedium resuspendiert. Die Konzentration der Bakterien in der Lösung wurde durch Zugabe von weiterem Basalmedium auf eine OD₆₀₀ von 0,3 eingestellt.

Aus den Raps-Explanten wurde das Kallus-Induktionsmedium mit sterilen Pipetten entfernt, 50 ml Agrobakterium-Lösung hinzugefügt, vorsichtig gemischt und für 20 min inkubiert. Die Agrobacterien-Suspension wurde entfernt, die Raps-Explante für 1 min mit 50 ml Kallus-Induktionsmedium gewaschen und anschließend 100 ml Kallus-Induktionsmedium hinzugefügt. Die Co-Kultivierung wurde für 24 h auf einem Rotationsschüttler bei 100 U/min durchgeführt. Die Co-Kultivierung wurde durch Wegnahme des Kallus-Induktionsmediums gestoppt und die Explante zweimal für jeweils 1 min mit 25 ml und zweimal für 60 min mit jeweils 100 ml Waschmedium bei 100 U/min gewaschen. Das Waschmedium mit den Explanten wurde in 15 cm Petrischalen überführt und das Medium mit sterilen Pipetten entfernt.

Zur Regeneration wurden jeweils 20 bis 30 Explante in 90 mm Petrischalen überführt, welche 25 ml Sproß-Induktionsmedium mit Kanamycin enthielten. Die Petrischalen wurden mit 2 Lagen Leukopor verschlossen und bei 25°C und 2000 lux bei Photoperioden von 16 Stunden Licht/8 Stunden Dunkelheit inkubiert. Alle 12 Tage wurden die sich entwickelnden Kalli auf frische Petrischalen mit Sproß-Induktionsmedium umgesetzt. Alle weiteren Schritte zur Regeneration ganzer Pflanzen wurden wie von Bade, J.B und Damm, B. (in: Gene Transfer to Plants, Potrykus, I. und Spangenberg, G., eds, Springer Lab Manual, Springer Verlag, 1995, 30-38) beschrieben durchgeführt.

### Beispiel 80

### Herstellung transgener Nicotiana tabacum Pflanzen, die die Tyrosinaminotransferase überexprimieren

Zehn ml YEB-Medium mit Antibiotikum (5 g/l Rinder-Extrakt, 1 g/l Hefe-Extrakt, 5 g/l Pepton, 5 g/l Saccharose und 2 mM MgSO₄.) wurden mit einer Kolonie von *Agrobacterium tumefaciens* beimpft und über Nacht bei 28°C kultiviert. Die Zellen wurden 20 min bei 4°C, 3500 U/min in einer Tischzentrifuge pelletiert und danach in frischem YEB-Medium ohne Antibiotika unter sterilen Bedingungen resuspendiert. Die Zellsuspension wurde für die Transformation eingesetzt.

Die verwendeten *Agrobacterium tumefaciens* Zellen waren im Vorfeld mit dem Plasmid pBinAR-35s-IPP-RnTatase-nosT (Beispiel 73, Abbildung 61) gemäß der von R. HÖFGEN und L. WILLMITZER (Plant Sci. 1990, 66, 221-230 und Nucleic Acids Res. 1988, Oct 25, 16(20), 9877) beschriebenen Methode transformiert worden.

Die Wildtyp-Pflanzen aus Sterilkultur wurden durch vegetative Replikation erhalten. Dazu wurde nur die Spitze der Pflanze abgeschnitten und auf frisches 2MS-Medium in ein steriles Einweckglas überführt. Vom Rest der Pflanze wurden die Haare auf der Blattoberseite und die Mittelrippen der Blätter entfernt. Die Blätter wurden mit einer Rasierklinge in etwa 1cm² große Stücke geschnitten. Die Agrobakterienkultur wurde in eine kleine Petrischale überführt (Durchmesser 2 cm). Die Blattstücke wurden kurz durch diese Lösung gezogen und mit der Blattunterseite auf 2MS-Medium in Petrischalen (Durchmesser 9 cm) gelegt, so daß sie das Medium berührten.

Nach zwei Tagen im Dunkeln bei 25°C wurden die Explantate auf Platten mit Kallusinduktionsmedium überführt und in der Klimakammer auf 28°C temperiert. Das Medium mußte alle 7 bis 10 Tage gewechselt werden. Sobald sich Kalli bildeten, wurden die Explantate in sterile Einweckgläser auf Sproßinduktionsmedium mit Claforan (0,6 % BiTec-Agar (g/v), 2,0 mg/l Zeatinribose, 0,02 mg/l Naphthylessigsäure, 0,02 mg/l Gibberelinsäure, 0,25 g/ml Claforan, 1,6 % Glukose (g/v) und 50 mg/l Kanamycin) überführt. Nach etwa einem Monat trat Organogenese ein und die gebildeten Sprosse konnten abgeschnitten werden.

Die Kultivierung der Sprosse wurde auf 2MS-Medium mit Claforan und Selektionsmarker durchgeführt. Sobald sich ein kräftiger Wurzelballen gebildet hatte, konnten die Pflanzen in Pikiererde getopft werden.

### Beispiel 81

### Charakterisierung der transgenen Pflanzen aus Beispiel 78, 79 und 80

Die Tocopherol- und Tocotrienol-Gehalte in Blätter und Samen der mit den beschriebenen Konstrukten transformierten Pflanzen aus Beispiel 78, 79 und 80 (*Arabidopsis thaliana*, *Brassica napus* und *Nicotiana tabacum*) werden analysiert. Dazu wurden die transgenen Pflanzen im Gewächshaus kultiviert und Pflanzen die das Gen kodierend für die Tyrosin-Aminotransferase aus Rattus *norvegicus* exprimieren auf Northern-Ebene analysiert. In Blättern und Samen dieser Pflanzen wurde der Tocopherolgehalt und der Tocotrienolgehalt ermittelt.

Dazu wird das Blattmaterial von Pflanzen direkt nach der Probennahme in flüssigem Stickstoff tiefgefroren. Der daran anschließende Aufschluß der Zellen erfolgt mittels einer Rührapparatur durch dreimalige Inkubation im Eppendorfschüttler bei 30°C, 1000 rpm in 100 % Methanol für 15 Minuten, wobei die jeweils erhaltenen Überstände vereinigt wurden.

Weitere Inkubationsschritte ergaben keine weitere Freisetzung von Tocopherolen oder Tocotrienolen.

Um Oxidation zu vermeiden, wurden die erhaltenen Extrakte direkt nach der Extraktion mit Hilfe einer HPLC-Anlage (Waters Allience 269.0) analysiert. Tocopherole und Tocotrienole wurden über eine reverse Phase Säule(ProntoSil 200-3-C30^{(R)}, Fa. Bischoff) mit einer mobilen Phase von 100 % Methanol getrennt und anhand von Standards (Fa. Merck) identifiziert. Als Detektionssystem diente die Fluoreszens der Substanzen (Anregung 295 nm, Emission 320 nm) die mit Hilfe eines Jasco Fluoreszensdetektors FP 920 nachgewiesen wurde.

Tabelle 1 zeigt das Ergebnis der Überexpression der Tyrosinaminotransferase aus Rattus norvegicus in 16 Linien (Linie 1 bis 24) der transgenen *Nicotiana tabacum,* hergestellt nach Beispiel 80 im Vergleich zum Wildtyp (WT, 4 Replikanten). Dargestellt in der zweiten Spalte ist der Gehalt an Vitamin E (Gesamtgehalt = Summe aller 8 Isomere) in jungem Blattmaterial in [µg/gFW]. In der dritten Spalte ist der Tocotrienol-Anteil der jeweiligen Linie am Gesamtgehalt Vitamin E in [Gew.-%] angegeben.

**Tabelle 1**

| Linie transgener *Nicotiana tabacum-* Pflanzen aus Beispiel 80 | Gesamtgehalt Vitamin Ein [µg/gFW] | Anteil Tocotrienole in [Gew.-%] bezogen auf den Gesamtgehalt |
|---|---|---|
| 1 | 9,13 | 48,5 |
| 2 | 2,95 | 4,6 |
| 3 | 5, 94 | 49,5 |
| 4 | 7,24 | 5,8 |
| 6 | 5,97 | 7,6 |
| 7 | 8,02 | 6,4 |
| 9 | 16,26 | 53,1 |
| 10 | 8,95 | 41,3 |
| 11 | 13,28 | 51,6 |
| 16 | 8,96 | 42,9 |
| 17 | 3,99 | 3,2 |
| 18 | 10,58 | 51,7 |
| 19 | 7,57 | 41,3 |
| 24 | 14,76 | 56,7 |
| WT n=4 | 5,4 +/-0,5 | 4,75 +/- 2,4 |

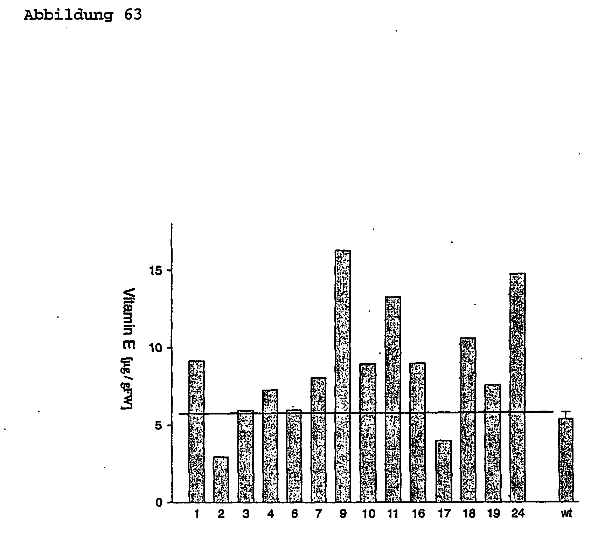

Abbildung 63 zeigt grafisch das Ergebnis der Überexpression der Tyrosinaminotransferase aus Rattus norvegicus in Nicotiana tabacum (Beispiel 80) im Vergleich zum Wildtyp. Dargestellt sind die Gehalte an Vitamin E (Summe aller 8 Isomere) in jungem Blattmaterial. Die Achsenbeschriftung kennzeichnet die einzelnen transgenen Linien. Die dargestellten Werte bei den Wildtyp-pflanzen (wt) entsprechen dem Mittelwert +/- SD von 4 Replikaten.

## Patentansprüche

1. Verfahren zur Herstellung von Vitamin E durch Kultivierung von Pflanzen, die gegenüber dem Wildtyp eine erhöhte Tyrosinaminotransferase-Aktivität aufweisen, durch die Erhöhung der Genexpression einer für eine Tyrosinaminotransferase kodierende Nukleinsäure gegenüber dem Wildtyp, **dadurch gekennzeichnet, daß** man zur Erhöhung der Genexpression, Nukleinsäuren, codierend eine Tyrosinaminotransferase, in die Pflanze einbringt.

2. Verfahren nach Anspruch 1, **dadurch gekennzeichnet, daß** man Nukleinsäuren einbringt, die Proteine kodieren, enthaltend die Aminosäuresequenz SEQ. ID. NO. 2 oder eine von dieser Sequenz durch Substitution, Insertion oder Deletion von Aminosäuren abgeleitete Sequenz, die eine Identität von mindestens 20 % auf Aminosäureebene mit der Sequenz SEQ. ID. NO. 2, und die die enzymatische Eigenschaft einer Tyrosinaminotransferase aufweisen.

3. Verfahren nach Anspruch 2, **dadurch gekennzeichnet, daß** man eine Nukleinsäure, enthaltend die Sequenz SEQ. ID. NO. 1 einbringt.

4. Verfahren nach einem der Ansprüche 1 bis 3, **dadurch gekennzeichnet, daß** die Pflanzen zusätzlich gegenüber dem Wildtyp eine erhöhte Aktivität mindestens einer der Aktivitäten, ausgewählt aus der Gruppe Hydroxyphenylpyruvat-Dioxygenase-Aktivität, Homogentisat-Phytyltransferase-Aktivität, Geranyl-Geranyl-Pyrophosphat-Oxidoreduktase-Aktivität, 2-Methyl-6-Phytylhydrochinon-Methyltransferase-Aktivität, Tocopherolcyclase-Aktivität und γ-Tocopherol-Methyltransferase-Aktivität aufweisen.

5. Verfahren nach Anspruch 4, **dadurch gekennzeichnet, daß** man zur zusätzlichen Erhöhung mindestens einer der Aktivitäten, die Genexpression mindestens einer Nukleinsäure ausgewählt aus der Gruppe, Nukleinsäuren kodierend eine Hydroxyphenylpyruvat-Dioxygenase, Nukleinsäuren kodierend eine Homogentisat-Phytyltransferase, Nukleinsäuren kodierend eine Geranyl-Geranyl-Pyrophosphat-Oxidoreduktase, Nukleinsäuren kodierend eine 2-Methyl-6-Phytylhydrochinon-Methyltransferase, Nukleinsäuren kodierend eine Tocopherolcyclase und Nukleinsäuren kodierend eine γ-Tocopherol-Methyltransferase gegenüber dem Wildtyp erhöht.

6. Verfahren nach Anspruch 5, **dadurch gekennzeichnet, daß** man zur Erhöhung der Genexpression mindestens einer der Nukleinsäuren, mindestens eine Nukleinsäure ausgewählt aus der Gruppe, Nukleinsäuren kodierend eine Hydroxyphenylpyruvat-Dioxygenase, Nukleinsäuren kodierend eine Homogentisat-Phytyltransferase, Nukleinsäuren kodierend eine Geranyl-Geranyl-Pyrophosphat-Oxidoreduktase, Nukleinsäuren kodierend eine 2-Methyl-6-Phytylhydrochinon-Methyltransferase, Nukleinsäuren kodierend eine Tocopherolcyclase und Nukleinsäuren kodierend eine γ-Tocopherol-Methyltransferase in die Pflanze einbringt.

7. Verfahren nach einem der Ansprüche 1 bis 6, **dadurch gekennzeichnet, daß** die Pflanzen gegenüber dem Wildtyp zusätzlich eine reduzierte Aktivität mindestens einer der Aktivitäten, ausgewählt aus der Gruppe Homogentisat-Dioxygenase-Aktivität, Maleylacetoacetat-Isomerase-Aktivität und Fumarylacetoacetat-Hydrolase-Aktivität aufweisen.

8. Verfahren nach Anspruch 7, **dadurch gekennzeichnet, daß** man zur zusätzlichen Reduzierung mindestens einer der Aktivitäten, die Genexpression mindestens einer Nukleinsäure, ausgewählt aus der Gruppe Nukleinsäuren kodierend eine Homogentisat-Dioxygenase, Nukleinsäuren kodierend eine Maleylacetoacetat-Isomerase und Nukleinsäuren kodierend eine Fumarylacetoacetat-Hydrolase gegenüber dem Wildtyp reduziert.

9. Verfahren gemäß Anspruch 7 oder 8, **dadurch gekennzeichnet, daß** die Pflanzen eine reduzierte Homogentisat-Dioxygenase-Aktivität aufweisen.

10. Verfahren nach Anspruch 9, **dadurch gekennzeichnet, daß** man in die Pflanze eine RNA einbringt, die einen Bereich mit Doppel-Strang-Struktur aufweist und in diesem Bereich eine Nukleinsäuresequenz aufweist, die mit einem Teil der Pflanze eigenen Nukleinsäure, codierend eine Homogentisat-Dioxygenase identisch ist.

11. Verfahren nach einem der Ansprüche 1 bis 10, **dadurch gekennzeichnet, daß** man nach dem Kultivieren die Pflanze erntet und die Vitamin-E-Verbindungen anschließend aus der Pflanze isoliert.

12. Nukleinsäurekonstrukt, enthaltend eine Nukleinsäure codierend eine Tyrosinaminotransferase, die mit einem oder mehreren Regulationssignalen funktionell verknüpft sind, **dadurch gekennzeichnet, dass** man Regulationssignale verwendet, die die Transkription und Translation in Pflanzen gewährleisten.

13. Nukleinsäurekonstrukt nach Anspruch 12, enthaltend zusätzlich eine Nukleinsäure codierend ein plastidäres Transitpeptid.

14. Nukleinsäurekonstrukt nach einem der Ansprüche 12 oder 13, enthaltend zusätzlich eine, zwei oder drei Nukleinsäuren, ausgewählt aus der Gruppe Nukleinsäuren kodierend eine Hydroxyphenylpyruvat-Dioxygenase, Nukleinsäuren kodierend eine Homogentisat-Phytyltransferase, Nukleinsäuren kodierend eine Geranyl-Geranyl-Pyrophosphat-Oxidoreduktase, Nukleinsäure kodierend eine 2-Methyl-6-Phytylhydrochinon-Methyltransferase, Nukleinsäuren kodierend eine Tocopherolcyclase und Nukleinsäuren kodierend eine γ-Tocopherol-Methyltransferase, die mit einem oder mehreren Regulationssignalen funktionell verknüpft sind, die die Transkription und Translation in Pflanzen gewährleisten.

15. Nukleinsäurekonstrukt nach einem der Ansprüche 12 bis 14, enthaltend zusätzlich funktionell verknüpft eine RNA, die einen Bereich mit Doppel-Strang-Struktur aufweist und in diesem Bereich eine Nukleinsäuresequenz aufweist, die mit einem Teil einer Nukleinsäure, codierend eine Homogentisat-Dioxygenase identisch ist.

16. Kombination aus Nukleinsäurekonstrukten, wobei die Kombination ein Nukleinsäurekonstrukt gemäß einem der Ansprüche 12 bis 15 und
a) mindestens ein weiteres Nukleinsäurekonstrukt, ausgewählt aus der Gruppe A bis F
A Nukleinsäurekonstrukt, enthaltend Nukleinsäuren codierend eine Hydroxyphenylpyruvat-Dioxygenase, die mit einem oder mehreren Regulationssignalen funktionell verknüpft sind, die die Transkription und Translation in Pflanzen gewährleisten,
B Nukleinsäurekonstrukt, enthaltend Nukleinsäuren codierend eine Homogentisat-Phytyltransferase, die mit einem oder mehreren Regulationssignalen funktionell verknüpft sind, die die Transkription und Translation in Pflanzen gewährleisten und
C Nukleinsäurekonstrukt, enthaltend Nukleinsäuren codierend eine Geranyl-Geranyl-Pyrophosphat-Oxidoreduktase, die mit einem oder mehreren Regulationssignalen funktionell verknüpft sind, die die Transkription und Translation in Pflanzen gewährleisten,
D Nukleinsäurekonstrukt, enthaltend Nukleinsäuren codierend eine 2-Methyl-6-Phytylhydrochinon-Methyltransferase, die mit einem oder mehreren Regulationssignalen funktionell verknüpft sind, die die Transkription und Translation in Pflanzen gewährleisten,
E Nukleinsäurekonstrukt, enthaltend Nukleinsäuren codierend eine Tocopherolcyclase, die mit einem oder mehreren Regulationssignalen funktionell verknüpft sind, die die Transkription und Translation in Pflanzen gewährleisten und
F Nukleinsäurekonstrukt, enthaltend Nukleinsäuren codierend eine γ-Tocopherol-Methyltransferase, die mit einem oder mehreren Regulationssignalen funktionell verknüpft sind, die die Transkription und Translation in Pflanzen gewährleisten,
oder
b) mindestens ein weiteres Nukleinsäurekonstrukt, enthaltend zwei, drei oder vier Nukleinsäurekonstrukte, ausgewählt aus der Gruppe der Nukleinsäurekonstrukte A bis F,
umfasst.

17. Kombination von Nukleinsäurekonstrukten gemäß Anspruch 16, **dadurch gekennzeichnet, daß** die Regulationssignale einen oder mehrere Promotoren und einen oder mehrere Terminatoren enthalten, die die Transkription und Translation in Pflanzen gewährleisten.

18. Genetisch veränderte Pflanze mit einer gegenüber dem Wildtyp erhöhten Aktivität einer Tyrosinaminotransferase **dadurch gekennzeichnet, daß** die Pflanze ein Nukleinsäure Konstrukt nach Anspruch 12 enthält und daß die genetische Veränderung zusätzlich mindestens eine der Aktivitäten, ausgewählt aus der Gruppe Hydroxyphenylpyruvat-Dioxygenase-Aktivität, Homogentisat-Phytyltransferase-Aktivität, Geranyl-Geranyl-Pyrophosphat-Oxidoreduktase-Aktivität, 2-Methyl-6-Phytylhydrochinon-Methyltransferase-Aktivität, Tocopherolcyclase-Aktivität und γ-Tocopherol-Methyltransferase-Aktivität gegenüber einem Wildtyp erhöht, wobei die Erhöhung mindestens einer der Aktivitäten durch eine Erhöhung der Genexpression mindestens einer Nukleinsäure ausgewählt aus der Gruppe Nukleinsäuren kodierend eine Hydroxyphenylpyruvat-Dioxygenase, Nukleinsäuren kodierend eine Homogentisat-Phytyltransferase, Nukleinsäuren kodierend eine Geranyl-Geranyl-Pyrophosphat-Oxidoreduktase, Nukleinsäuren kodierend eine 2-Metyhl-6-Phytylhydrochinon-Methyltransferase, Nukleinsäuren kodierend eine Tocopherolcyclase und Nukleinsäuren kodierend eine γ-Tocopherol-Methyltransferase gegenüber dem Wildtyp bewirkt wird.

19. Genetisch veränderte Pflanze nach Anspruch 18, **dadurch gekennzeichnet, daß** die Pflanze mindestens eine exogene Nukleinsäure kodierend eine Hydroxyphenylpyruvat-Dioxygenase oder zwei oder mehr endogene Nukleinsäuren kodierend eine Hydroxyphenylpyruvat-Dioxygenase und/oder mindestens eine exogene Nukleinsäure kodierend eine Homogentisat-Phytyltransferase oder zwei oder mehr endogene Nukleinsäuren kodieren eine Homogentisat-Phytyltransferase und/oder mindestens eine exogene Nukleinsäure kodierend eine Geranyl-Geranyl-Pyrophosphat-Oxidoreduktase oder zwei oder mehr endogene Nukleinsäuren kodierend eine Geranyl-Geranyl-Pyrophosphat-Oxidoreduktase und/oder mindestens eine exogene Nukleinsäure kodierend eine 2-Methyl-6-Phytylhydrochinon - Methyltransferase oder zwei oder mehr endogene Nukleinsäure kodierend eine 2-Methyl-6-Phytylhydrochinon - Methyltransferase und/oder mindestens eine exogene Nukleinsäure kodierend eine Tocopherolcyclase oder zwei oder mehr endogene Nukleinsäuren kodierend eine Tocopherolcyclase und/oder mindestens eine exogene Nukleinsäure kodierend eine γ-Tocopherol-Methyltransferase oder zwei oder mehr endogene Nukleinsäuren kodierend eine γ-Tocopherol-Methyltransferase, enthält.

20. Genetisch veränderte Pflanze nach einem der Ansprüche 18 oder 19, **dadurch gekennzeichnet, daß** die genetische Veränderung zusätzlich mindestens eine der Aktivitäten, ausgewählt aus der Gruppe, Homogentisat-Dioxygenase-Aktivität, Maleylacetoacetat-Isomerase-Aktivität und Fumarylacetoacetat-Hydrolase-Aktivität gegenüber einem Wildtyp reduziert.

21. Genetisch veränderte Pflanze nach Anspruch 20, **dadurch gekennzeichnet, daß** die Reduzierung mindestens einer der Aktivitäten durch eine Reduzierung der Genexpression mindestens einer Nukleinsäure, ausgewählt aus der Gruppe Nukleinsäuren kodierend eine Homogentisat-Dioxygenase, Nukleinsäuren kodierend eine Maleylacetoacetat-Isomerase und Nukleinsäuren kodierend eine Fumarylacetoacetat-Hydrolase, gegenüber dem Wildtyp bewirkt wird.

22. Verwendung einer genetisch veränderten Pflanze nach einem der Ansprüche 18 bis 21 zur Herstellung von Vitamin E.

23. Verwendung der genetisch veränderten Pflanze nach einem der Ansprüche 18 bis 21 als Futter- und Nahrungsmittel, zur Herstellung von prozessierten Lebensmittel, zur Herstellung von Vitamin E-haltigen Extrakten der Pflanzen oder zur Herstellung von Futter- und Nahrungsergänzungsmittel.

24. Verfahren zur Herstellung von genetisch veränderten Pflanzen gemäß einem der Ansprüche 18 bis 21, **dadurch gekennzeichnet, daß** man Nukleinsäuren wie in einem der Ansprüche 1 bis 10 definiert oder Nukleinsäurekonstrukte gemäß einem der Ansprüche 12 bis 15 oder Kombinationen von Nukleinsäurekonstrukten gemäß einem der Ansprüche 16 oder 17 in das Genom der Ausgangspflanze einführt.

25. Verwendung der Nukleinsäuren definiert gemäß einem der Ansprüche 1 bis 10 oder der Nukleinsäurekonstrukte gemäß einem der Ansprüche 12 bis 15 oder der Kombinationen von Nukleinsäurekonstrukten gemäß einem der Ansprüche 16 oder 17 zur Erhöhung des Gehalts an Vitamin E in Pflanzen, die als Wildtyp in der Lage sind, Vitamin E zu produzieren.

## Claims

1. A process for the production of vitamin E by growing plants with an increased tyrosine aminotransferase activity in comparison with the wild type, by increasing the gene expression of a nucleic acid encoding a tyrosine aminotransferase in comparison with the wild type, wherein, to increase the gene expression, nucleic acids encoding a tyrosine aminotransferase are introduced into the plant.

2. The process according to claim 1, wherein nucleic acids are introduced which encode proteins comprising the amino acid sequence SEQ. ID. NO. 2 or a sequence derived from this sequence by amino acid substitution, insertion or deletion, which proteins have at least 20% identity at the amino acid level with the sequence SEQ. ID. NO. 2 and which have the enzymatic characteristic of a tyrosine aminotransferase.

3. The process according to claim 2, wherein a nucleic acid comprising the sequence SEQ. ID. NO. 1 is introduced.

4. The process according to any of claims 1 to 3, wherein the plants additionally have at least one of the activities selected from the group consisting of hydroxyphenylpyruvate dioxygenase activity, homogentisate phytyltransferase activity, geranylgeranyl-pyrophosphate oxidoreductase activity, 2-methyl-6-phytylhydroquinone methyltransferase activity, tocopherol cyclase activity and γ-tocopherol methyltransferase activity which is increased in comparison with the wild type.

5. The process according to claim 4, wherein, for the additional increase in at least one of the activities, the gene expression of at least one nucleic acid selected from the group consisting of nucleic acids encoding a hydroxyphenylpyruvate dioxygenase, nucleic acids encoding a homogentisate phytyltransferase, nucleic acids encoding a geranylgeranyl-pyrophosphate oxidoreductase, nucleic acids encoding a 2-methyl-6-phytylhydroquinone methyltransferase, nucleic acids encoding a tocopherol cyclase and nucleic acids encoding a γ-tocopherol methyltransferase is increased in comparison with the wild type.

6. The process according to claim 5, wherein, to increase the gene expression of at least one of the nucleic acids, at least one nucleic acid selected from the group consisting of nucleic acids encoding a hydroxyphenylpyruvate dioxygenase, nucleic acids encoding a homogentisate phytyltransferase, nucleic acids encoding a geranylgeranyl-pyrophosphate oxidoreductase, nucleic acids encoding a 2-methyl-6-phytylhydroquinone methyltransferase, nucleic acids encoding a tocopherol cyclase and nucleic acids encoding a γ-tocopherol methyltransferase is introduced into the plant.

7. The process according to any of claims 1 to 6, wherein the plants additionally have a reduced activity of at least one of the activities selected from the group consisting of homogentisate dioxygenase activity, maleylacetoacetate isomerase activity and fumarylacetoacetate hydrolase activity in comparison with the wild type.

8. The process according to claim 7, wherein, to additionally reduce at least one of the activities, the gene expression of at least one nucleic acid selected from the group consisting of nucleic acids encoding a homogentisate dioxygenase, nucleic acids encoding a maleylacetoacetate isomerase and nucleic acids encoding a fumarylacetoacetate hydrolase is reduced in comparison with the wild type.

9. The process according to claim 7 or 8, wherein the plants have a reduced homogentisate dioxygenase activity.

10. The process according to claim 9, wherein an RNA which has a region with duplex structure and within this region has a nucleic acid sequence which is identical to part of the homologous nucleic acid encoding a homogentisate dioxygenase is introduced into the plant.

11. The process according to any of claims 1 to 10, wherein, after growing the plant, the latter is harvested and the vitamin E compounds are subsequently isolated from the plant.

12. A nucleic acid construct comprising a nucleic acid encoding a tyrosine aminotransferase which is linked operably to one or more regulatory signals, wherein regulatory signals are used which ensure the transcription and translation in plants.

13. The nucleic acid construct according to claim 12, additionally comprising a nucleic acid encoding a plastid transit peptide.

14. The nucleic acid construct according to either of claims 12 or 13, additionally comprising one, two or three nucleic acids, selected from the group consisting of nucleic acids encoding a hydroxyphenylpyruvate dioxygenase, nucleic acids encoding a homogentisate phytyltransferase, nucleic acids encoding a geranylgeranyl-pyrophosphate oxidoreductase, nucleic acids encoding a 2-methyl-6-phytylhydroquinone methyltransferase, nucleic acids encoding a tocopherol cyclase and nucleic acids encoding a γ-tocopherol methyltransferase, which nucleic acids are linked operably to one or more regulatory signals and which ensure the transcription and translation in plants.

15. The nucleic acid construct according to any of claims 12 to 14, additionally comprising, in operable linkage, an RNA which has a region with duplex structure and within this region has a nucleic acid sequence which is identical to part of a nucleic acid encoding a homogentisate dioxygenase.

16. A combination of nucleic acid constructs, where the combination comprises a nucleic acid construct according to any of claims 12 to 15 and
a) at least one further nucleic acid construct selected from among groups A to F
A nucleic acid construct comprising nucleic acids encoding a hydroxyphenylpyruvate dioxygenase, which nucleic acids are linked operably with one or more regulatory signals which ensure the transcription and translation in plants,
B nucleic acid construct comprising nucleic acids encoding a homogentisate phytyltransferase, which nucleic acids are linked operably with one or more regulatory signals which ensure the transcription and translation in plants,
C nucleic acid construct comprising nucleic acids encoding a geranylgeranyl-pyrophosphate oxidoreductase, which nucleic acids are linked operably with one or more regulatory signals which ensure the transcription and translation in plants,
D nucleic acid construct comprising nucleic acids encoding a 2-methyl-6-phytylhydroquinone methyltransferase, which nucleic acids are linked operably with one or more regulatory signals which ensure the transcription and translation in plants,
E nucleic acid construct comprising nucleic acids encoding a tocopherol cyclase, which nucleic acids are linked operably with one or more regulatory signals which ensure the transcription and translation in plants, and
F nucleic acid construct comprising nucleic acids encoding a γ-tocopherol methyltransferase, which nucleic acids are linked operably with one or more regulatory signals which ensure the transcription and translation in plants,
or
b) at least one further nucleic acid construct comprising two, three or four nucleic acid constructs selected from the group of the nucleic acid constructs A to F.

17. The combination of nucleic acid constructs according to claim 16, wherein the regulatory signals comprise one or more promoters and one or more terminators which ensure the transcription and translation in plants.

18. A genetically modified plant with an increased activity of a tyrosine aminotransferase in comparison with the wild type, wherein the plant comprises a nucleic acid construct according to claim 12 and wherein the genetic modification additionally increases at least one of the activities selected from the group consisting of hydroxyphenylpyruvate dioxygenase activity, homogentisate phytyltransferase activity, geranylgeranyl-pyrophosphate oxidoreductase activity, 2-methyl-6-phytylhydroquinone methyltransferase activity, tocopherol cyclase activity and γ-tocopherol methyltransferase activity in comparison with a wild type, the increase in at least one of the activities being brought about by an increase in the gene expression of at least one nucleic acid selected from the group consisting of nucleic acids encoding a hydroxyphenylpyruvate dioxygenase, nucleic acids encoding a homogentisate phytyltransferase, nucleic acids encoding a geranylgeranyl-pyrophosphate oxidoreductase, nucleic acids encoding a 2-methyl-6-phytylhydroquinone methyltransferase, nucleic acids encoding a tocopherol cyclase and nucleic acids encoding a γ-tocopherol methyltransferase in comparison with the wild type.

19. The genetically modified plant according to claim 18, wherein the plant comprises at least one exogenous nucleic acid encoding a hydroxyphenylpyruvate dioxygenase or two or more endogenous nucleic acids encoding a hydroxyphenylpyruvate dioxygenase and/or at least one exogenous nucleic acid encoding a homogentisate phytyltransferase or two or more endogenous nucleic acids encoding a homogentisate phytyltransferase and/or at least one exogenous nucleic acid encoding a geranylgeranyl-pyrophosphate oxidoreductase or two or more endogenous nucleic acids encoding a geranylgeranyl-pyrophosphate oxidoreductase and/or at least one exogenous nucleic acid encoding a 2-methyl-6-phytylhydroquinone methyltransferase or two or more endogenous nucleic acids encoding a 2-methyl-6-phytylhydroquinone methyltransferase and/or at least one exogenous nucleic acid encoding a tocopherol cyclase or two or more endogenous nucleic acids encoding a tocopherol cyclase and/or at least one exogenous nucleic acid encoding a γ-tocopherol methyltransferase or two or more endogenous nucleic acids encoding a γ-tocopherol methyltransferase.

20. The genetically modified plant according to either of claims 18 or 19, wherein the genetic modification additionally reduces at least one of the activities selected from the group consisting of homogentisate dioxygenase activity, maleylacetoacetate isomerase activity and fumarylacetoacetate hydrolase activity in comparison with a wild type.

21. The genetically modified plant according to claim 20, wherein the reduction in at least one of the activities is brought about by reducing the gene expression of at least one nucleic acid selected from the group consisting of nucleic acids encoding a homogentisate dioxygenase, nucleic acids encoding a maleylacetoacetate isomerase and nucleic acids encoding a fumarylacetoacetate hydrolase in comparison with the wild type.

22. The use of a genetically modified plant according to any of claims 18 to 21 for the production of vitamin E.

23. The use of the genetically modified plant according to any of claims 18 to 21 as feed and food, for the production of processed foodstuffs, for the production of vitamin E-containing extracts of the plants, or for the production of feed and food supplements.

24. A process for the production of genetically modified plants according to any of claims 18 to 21, wherein nucleic acids as defined in any of claims 1 to 10 or nucleic acid constructs according to any of claims 12 to 15 or combinations of nucleic acid constructs according to either of claims 16 or 17 are introduced into the genome of the starting plant.

25. The use of the nucleic acids defined according to any of claims 1 to 10 or nucleic acid constructs according to any of claims 12 to 15 or combinations of nucleic acid constructs according to either of claims 16 to 17 for increasing the vitamin E content in plants who as wild types are capable of producing vitamin E.

## Revendications

1. Procédé pour la production de vitamine E par cultures de plantes qui présentent une activité tyrosine aminotransférase accrue par rapport au type sauvage, par l'augmentation de l'expression de gène d'un acide nucléique codant la tyrosine aminotransférase par rapport au type sauvage, **caractérisé en ce que,** pour l'augmentation de l'expression de gène, on introduit dans la plante des acides nucléiques codant une tyrosine aminotransférase.

2. Procédé selon la revendication 1, **caractérisé en ce qu'**on introduit des acides nucléiques qui codent des protéines contenant la séquence d'aminoacides SEQ ID n° 2 ou une séquence dérivée de cette séquence par remplacement, insertion ou délétion d'aminoacides, qui présentent une identité d'au moins 20 % sur le plan aminoacide avec la SEQ ID n° 2 et qui ont la propriété enzymatique d'une tyrosine aminotransférase.

3. Procédé selon la revendication 2, **caractérisé en ce qu'**on introduit un acide nucléique contenant la SEQ ID n° 1.

4. Procédé selon l'une quelconque des revendications 1 à 3, **caractérisé en ce que** les plantes présentent en outre par rapport au type sauvage une activité accrue d'au moins l'une des activités, choisie dans le groupe constitué par l'activité hydroxyphénylpyruvate dioxygénase, l'activité homogentisate phytyltransférase, l'activité géranyl-géranyl-pyrophosphate oxydoréductase, l'activité 2-méthyl-6-phytylhydroquinone méthyltransférase, l'activité tocophérol cyclase et l'activité γ-tocophérol méthyltransférase.

5. Procédé selon la revendication 4, **caractérisé en ce que**, pour l'accroissement supplémentaire d'au moins l'une des activités, on augmente par rapport au type sauvage l'expression de gène d'au moins un acide nucléique choisi dans le groupe constitué par les acides nucléiques codant une hydroxyphénylpyruvate dioxygénase, les acides nucléiques codant une homogentisate phytyltransférase, les acides nucléiques codant une géranyl-géranyl-pyrophosphate oxydoréductase, les acides nucléiques codant une 2-méthyl-6-phytylhydroquinone méthyltransférase, les acides nucléiques codant une tocophérol cyclase et les acides nucléiques codant une γ-tocophérol méthyltransférase.

6. Procédé selon la revendication 5, **caractérisé en ce que** pour l'accroissement de l'expression de gène d'au moins l'un des acides nucléiques, on introduit dans la plante au moins un acide nucléique choisi dans le groupe constitué par les acides nucléiques codant une hydroxyphénylpyruvate dioxygénase, les acides nucléiques codant une homogentisate phytyltransférase, les acides nucléiques codant une géranyl-géranyl-pyrophosphate oxydoréductase, les acides nucléiques codant une 2-méthyl-6-phytylhydroquinone méthyltransférase, les acides nucléiques codant une tocophérol cyclase et les acides nucléiques codant une γ-tocophérol méthyltransférase.

7. Procédé selon l'une quelconque des revendications 1 à 6, **caractérisé en ce que** les plantes présentent par rapport au type sauvage en outre une activité réduite d'au moins l'une des activités, choisie dans le groupe constitué par l'activité homogentisate dioxygénase, l'activité maléylacétoacétate isomérase et l'activité fumarylacétoacétate hydrolase.

8. Procédé selon la revendication 7, **caractérisé en ce que** pour la réduction supplémentaire d'au moins l'une des activités on réduit par rapport au type sauvage l'expression de gène d'au moins un acide nucléique choisi dans le groupe constitué par les acides nucléiques codant une homogentisate dioxygénase, les acides nucléiques codant une maléylacétoacétate isomérase et les acides nucléiques codant une fumarylacétoacétate hydrolase.

9. Procédé selon la revendication 7 ou 8, **caractérisé en ce que** les plantes présentent une activité homogentisate dioxygénase réduite.

10. Procédé selon la revendication 9, **caractérisé en ce qu'**on introduit dans la plante un ARN qui comporte une région à structure double brin et dans cette région comporte une séquence d'acide nucléique qui est identique à une partie de l'acide nucléique endogène de la plante, codant une homogentisate dioxygénase.

11. Procédé selon l'une quelconque des revendications 1 à 10, **caractérisé en ce qu'**on récolte la plante après la culture et ensuite on isole à partir de la plante les composés de vitamine E.

12. Produit de construction d'acide nucléique, contenant un acide nucléique codant une tyrosine aminotransférase qui est fonctionnellement lié à une ou plusieurs signaux de régulation, **caractérisé en ce qu'**on utilise des signaux de régulation qui assurent la transcription et la traduction chez les plantes.

13. Produit de construction selon la revendication 12, contenant en outre un acide nucléique codant un peptide de transit de plaste.

14. Produit de construction d'acide nucléique selon la revendication 12 ou 13, contenant en outre un, deux ou trois acides nucléiques choisis dans le groupe constitué par les acides nucléiques codant une hydroxyphénylpyruvate dioxygénase, les acides nucléiques codant une homogentisate phytyltransférase, les acides nucléiques codant une géranyl-géranyl-pyrophosphate oxydoréductase, les acides nucléiques codant une 2-méthyl-6-phytylhydroquinone méthyltransférase, les acides nucléiques codant une tocophérol cyclase et les acides nucléiques codant une γ-tocophérol méthyltransférase, qui sont fonctionnellement liés à un ou plusieurs signaux de régulation qui assurent la transcription et la traduction chez des plantes.

15. Produit de construction d'acide nucléique selon l'une quelconque des revendications 12 à 14, contenant en outre, fonctionnellement lié, un ARN qui comporte une région à structure double brin et dans cette région comporte une séquence d'acide nucléique qui est identique à une partie d'un acide nucléique codant une homogentisate dioxygénase.

16. Association de produits de construction d'acide nucléique, l'association comprenant un produit de construction d'acide nucléique selon l'une quelconque des revendications 12 à 15 et
a) au moins un autre produit de construction d'acide nucléique, choisi dans l'ensemble A à F
A un produit de construction d'acide nucléique, contenant des acides nucléiques codant une hydroxyphénylpyruvate dioxygénase, qui sont fonctionnellement liés à une ou plusieurs signaux de régulation qui assurent la transcription et la traduction chez les plantes,
B un produit de construction d'acide nucléique, contenant des acides nucléiques codant une homogentisate phytyltransférase, qui sont fonctionnellement liés à une ou plusieurs signaux de régulation qui assurent la transcription et la traduction chez les plantes, et
C un produit de construction d'acide nucléique, contenant des acides nucléiques codant une géranyl-géranyl-pyrophosphate oxydoréductase, qui sont fonctionnellement liés à une ou plusieurs signaux de régulation qui assurent la transcription et la traduction chez les plantes,
D un produit de construction d'acide nucléique, contenant des acides nucléiques codant une 2-méthyl-6-phytylhydroquinone méthyltransférase, qui sont fonctionnellement liés à une ou plusieurs signaux de régulation qui assurent la transcription et la traduction chez les plantes,
E un produit de construction d'acide nucléique, contenant des acides nucléiques codant une tocophérol cyclase, qui sont fonctionnellement liés à une ou plusieurs signaux de régulation qui assurent la transcription et la traduction chez les plantes,
F un produit de construction d'acide nucléique, contenant des acides nucléiques codant une γ-tocophérol méthyltransférase, qui sont fonctionnellement liés à une ou plusieurs signaux de régulation qui assurent la transcription et la traduction chez des plantes,
ou
b) au moins un autre produit de construction, contenant deux, trois ou quatre produits de construction d'acide nucléique, choisis dans le groupe des produits de construction d'acide nucléique A à F.

17. Association de produits de construction d'acide nucléique selon la revendication 16, **caractérisée en ce que** les signaux de régulation contiennent un ou plusieurs promoteurs et un ou plusieurs terminateurs, qui assurent la transcription et la traduction chez les plantes.

18. Plante génétiquement modifiée ayant une activité, accrue par rapport au type sauvage, d'une tyrosine aminotransférase, **caractérisée en ce que** la plante contient un produit de construction d'acide nucléique selon la revendication 12 et **en ce que** la modification génétique en outre accroît, par rapport au type sauvage, au moins l'une des activités, choisie dans le groupe constitué par l'activité hydroxyphénylpyruvate dioxygénase, l'activité homogentisate phytyltransférase, l'activité géranyl-géranyl-pyrophosphate oxydoréductase, l'activité 2-méthyl-6-phytylhydroquinone méthyltransférase, l'activité tocophérol cyclase et l'activité γ-tocophérol méthyltransférase, l'accroissement d'au moins l'une des activités étant provoqué par une augmentation de l'expression de gène, par rapport au type sauvage, d'au moins un acide nucléique choisi dans le groupe constitué par les acides nucléiques codant une hydroxyphénylpyruvate dioxygénase, les acides nucléiques codant une homogentisate phytyltransférase, les acides nucléiques codant une géranyl-géranyl-pyrophosphate oxydoréductase, les acides nucléiques codant une 2-méthyl-6-phytylhydroquinone méthyltransférase, les acides nucléiques codant une tocophérol cyclase et les acides nucléiques codant une γ-tocophérol méthyltransférase.

19. Plante génétiquement modifiée selon la revendication 18, **caractérisée en ce que** la plante contient au moins un acide nucléique exogène codant une hydroxyphénylpyruvate dioxygénase ou deux ou plus de deux acides nucléiques endogènes codant une hydroxyphénylpyruvate dioxygénase et/ou au moins un acide nucléique exogène codant une homogentisate phytyltransférase ou deux ou plus de deux acides nucléiques endogènes codant une homogentisate phytyltransférase et/ou au moins un acide nucléique exogène codant une géranyl-géranyl-pyrophosphate oxydoréductase ou deux ou plus de deux acides nucléiques endogènes codant une géranyl-géranyl-pyrophosphate oxydoréductase et/ou au moins un acide nucléique exogène codant une 2-méthyl-6-phytylhydroquinone méthyltransférase ou deux ou plus de deux acides nucléiques endogènes codant une 2-méthyl-6-phytylhydroquinone méthyltransférase et/ou au moins un acide nucléique exogène codant une tocophérol cyclase ou deux ou plus de deux acides nucléiques endogènes codant une tocophérol cyclase et/ou au moins un acide nucléique exogène codant une γ-tocophérol méthyltransférase ou deux ou plus de deux acides nucléiques endogènes codant une γ-tocophérol méthyltransférase.

20. Plante génétiquement modifiée selon la revendication 18 ou 19, **caractérisée en ce que** la modification génétique en outre réduit, par rapport au type sauvage, au moins l'une des activités, choisie dans le groupe constitué par l'activité homogentisate dioxygénase, l'activité maléylacétoacétate isomérase et l'activité fumarylacétoacétate hydrolase.

21. Plante génétiquement modifiée selon la revendication 20, **caractérisée en ce que** la réduction d'au moins l'une des activités est provoquée par une réduction de l'expression de gène d'au moins un acide nucléique choisi dans le groupe constitué par les acides nucléiques codant une homogentisate dioxygénase, les acides nucléiques codant une maléylacétoacétate isomérase et les acides nucléiques codant une fumarylacétoacétate hydrolase.

22. Utilisation d'une plante génétiquement modifiée selon l'une quelconque des revendications 18 à 21, pour la production de vitamine E.

23. Utilisation de la plante génétiquement modifiée selon l'une quelconque des revendications 18 à 21, en tant produit alimentaire ou aliment pour animaux, pour la fabrication de produits alimentaires transformés, pour la fabrication d'extraits, contenant de la vitamine E, des plantes ou pour la fabrication de compléments nutritionnels et de compléments pour aliments pour animaux.

24. Procédé pour la production de plantes génétiquement modifiées selon l'une quelconque des revendications 18 à 21, **caractérisé en ce qu'**on introduit dans le génome de la plante de départ des acides nucléiques tels que définis dans l'une quelconque des revendications 1 à 10 ou des produits de construction d'acide nucléique selon l'une quelconque des revendications 12 à 15 ou des associations de produits de construction selon la revendication 16 ou 17.

25. Utilisation des acides nucléiques définis selon l'une quelconque des revendications 1 à 10 ou des produits de construction d'acide nucléique selon l'une quelconque des revendications 12 à 15 ou des associations de produits de construction selon la revendication 16 ou 17, pour l'accroissement de la teneur en vitamine E de plantes qui sont capables, en tant que type sauvage, de produire de la vitamine E.


### SEQUENZPROTOKOLL

<110> SunGene GmbH & Co. KGaA
<120> Erhöhung des Vitamin-E-Gehalts in Organismen durch Erhöhung der Tyrosinaminotransferase-Aktivität
<130> 0817/00021
<140>
   <141>
<160> 58
<170> PatentIn Vers. 2.0
<210> 1
   <211> 1377
   <212> DNA
   <213> Rattus norvegicus
<220>
   <221> CDS
   <222> (1)..(1371)
<400> 1
   gat atc atg gac tcc tac gtg att cag acg gat gtc gac gac agc ttg    48
   Asp Ile Met Asp Ser Tyr Val Ile Gln Thr Asp Val Asp Asp Ser Leu
    1               5                  10                  15
   tcc tca gtt ctg gat gtg cat gtc aat att ggt ggg aga aac tcg gta    96
   Ser Ser Val Leu Asp Val His Val Asn Ile Gly Gly Arg Asn Ser Val
               20                  25                  30
   caa gga aga aag aaa ggc agg aag gcc aga tgg gac gtg aga ccc tct   144
   Gln Gly Arg Lys Lys Gly Arg Lys Ala Arg Trp Asp Val Arg Pro Ser
           35                  40                  45
   gac atg tcc aat aag acc ttc aat ccc atc cga gcc atc gtg gac aac   192
   Asp Met Ser Asn Lys Thr Phe Asn Pro Ile Arg Ala Ile Val Asp Asn
       50                  55                  60
   atg aag gtg cag ccc aat ccg aac aag acc gtg att tct ctg tca att   240
   Met Lys Val Gln Pro Asn Pro Asn Lys Thr Val Ile Ser Leu Ser Ile
   65                  70                  75                  80
   ggg gac cct act gtg ttt ggg aac ctg cct aca gac cct gaa gtt acc   288
   Gly Asp Pro Thr Val Phe Gly Asn Leu Pro Thr Asp Pro Glu Val Thr
                   85                  90                  95
   caa gcc atg aaa gat gcc ctg gac tcg ggg aag tac aat ggc tat gcc     336
   Gln Ala Met Lys Asp Ala Leu Asp Ser Gly Lys Tyr Asn Gly Tyr Ala
               100                 105                 110
   ccg tcc atc ggc tac cta tcc agt cgg gag gag gtc gct tct tac tac     384
   Pro Ser Ile Gly Tyr Leu Ser Ser Arg Glu Glu Val Ala Ser Tyr Tyr
           115                 120                 125
   cac tgt cat gag gct cct ctg gaa gct aag gat gtc att ctg aca agc     432
   His Cys His Glu Ala Pro Leu Glu Ala Lys Asp Val Ile Leu Thr Ser
       130                 135                 140
   ggc tgc agt cag gcc att gag cta tgt cta gct gtg ttg gcc aat cct     480
   Gly Cys Ser Gln Ala Ile Glu Leu Cys Leu Ala Val Leu Ala Asn Pro
   145                 150                 155                 160
   gga caa aac atc ctc att cca agg ccc ggg ttt tcc ctc tat agg act     528
   Gly Gln Asn Ile Leu Ile Pro Arg Pro Gly Phe Ser Leu Tyr Arg Thr
                   165                 170                 175
   ttg gct gag tct atg gga att gag gtc aag ctc tac aat ctc ctg cct     576
   Leu Ala Glu Ser Met Gly Ile Glu Val Lys Leu Tyr Asn Leu Leu Pro
               180                 185                 190
   gag aag tct tgg gaa att gac cta aaa caa ctg gaa tct ctg atc gat     624
   Glu Lys Ser Trp Glu Ile Asp Leu Lys Gln Leu Glu Ser Leu Ile Asp
           195                 200                 205
   gaa aaa aca gcg tgt ctt gtt gtc aac aac cca tcc aat ccc tgt ggc     672
   Glu Lys Thr Ala Cys Leu Val Val Asn Asn Pro Ser Asn Pro Cys Gly
       210                 215                 220
   tcc gtg ttc agt aag cga cac ctt cag aag att ttg gca gtg gct gaa     720
   Ser Val Phe Ser Lys Arg His Leu Gln Lys Ile Leu Ala Val Ala Glu
   225                 230                 235                 240
   agg cag tgt gtc ccc atc tta gct gac gag atc tat ggt gac atg gtg     768
   Arg Gln Cys Val Pro Ile Leu Ala Asp Glu Ile Tyr Gly Asp Met Val
                   245                 250                 255
   ttt tca gat tgc aaa tac gaa cca ctg gcc aac ctc agc acc aat gtt     816
   Phe Ser Asp Cys Lys Tyr Glu Pro Leu Ala Asn Leu Ser Thr Asn Val
               260                 265                 270
   ccc atc ctg tcc tgt ggt ggg ctg gcc aag cgc tgg ctg gtt cct ggc     864
   Pro Ile Leu Ser Cys Gly Gly Leu Ala Lys Arg Trp Leu Val Pro Gly
           275                 280                 285
   tgg agg ttg ggc tgg atc ctc att cat gat cga aga gac att ttt ggc     912
   Trp Arg Leu Gly Trp Ile Leu Ile His Asp Arg Arg Asp Ile Phe Gly
       290                 295                 300
   aat gag att cga gac ggg ctg gtg aaa ctg agt cag cgg atc ctg gga     960
   Asn Glu Ile Arg Asp Gly Leu Val Lys Leu Ser Gln Arg Ile Leu Gly
   305                 310                 315                 320
   cca tgc acc ata gtc cag ggt gct ctg aag agc atc ctt cag cga acc    1008
   Pro Cys Thr Ile Val Gln Gly Ala Leu Lys Ser Ile Leu Gln Arg Thr
                   325                 330                 335
   cct cag gag ttc tat cac gac acg tta agc ttc ctc aag tcc aat gcg    1056
   Pro Gln Glu Phe Tyr His Asp Thr Leu Ser Phe Leu Lys Ser Asn Ala
               340                 345                 350
   gac ctc tgc tat ggg gca ctg gct gcc atc cct gga ctc cag ccg gtc    1104
   Asp Leu Cys Tyr Gly Ala Leu Ala Ala Ile Pro Gly Leu Gln Pro Val
           355                 360                 365
   cgc cct tct gga gcc atg tac ctt atg gtg gga att gag atg gag cat    1152
   Arg Pro Ser Gly Ala Met Tyr Leu Met Val Gly Ile Glu Met Glu His
       370                 375                 380
   ttc ccg gaa ttc gag aac gac gtg gag ttc aca gag cgg ttg att gcg    1200
   Phe Pro Glu Phe Glu Asn Asp Val Glu Phe Thr Glu Arg Leu Ile Ala
   385                 390                 395                 400
   gag cag gct gtc cac tgt ctc cca gca acg tgc ttc gag tac cca aat    1248
   Glu Gln Ala Val His Cys Leu Pro Ala Thr Cys Phe Glu Tyr Pro Asn
                   405                 410                 415
   ttc ttc cga gtg gtc atc aca gtc ccc gag gtg atg atg ctg gag gct    1296
   Phe Phe Arg Val Val Ile Thr Val Pro Glu Val Met Met Leu Glu Ala
               420                 425                 430
   tgt agc cgg atc cag gag ttc tgt gaa cag cac tac cac tgt gct gaa    1344
   Cys Ser Arg Ile Gln Glu Phe Cys Glu Gln His Tyr His Cys Ala Glu
           435                 440                 445
   ggc agc cag gag gag tgt gac aaa taa gatatc                         1377
   Gly Ser Gln Glu Glu Cys Asp Lys
       450                 455
<210> 2
   <211> 456
   <212> PRT
   <213> Rattus norvegicus
<400> 2
   Asp Ile Met Asp Ser Tyr Val Ile Gln Thr Asp Val Asp Asp Ser Leu
   1 5 10 15
   Ser Ser Val Leu Asp Val His Val Asn Ile Gly Gly Arg Asn Ser Val
   20 25 30
   Gln Gly Arg Lys Lys Gly Arg Lys Ala Arg Trp Asp Val Arg Pro Ser
   35 40 45
   Asp Met Ser Asn Lys Thr Phe Asn Pro Ile Arg Ala Ile Val Asp Asn
   50 55 60
   Met Lys Val Gln Pro Asn Pro Asn Lys Thr Val Ile Ser Leu Ser Ile
   65 70 75 80
   Gly Asp Pro Thr Val Phe Gly Asn Leu Pro Thr Asp Pro Glu Val Thr
   85 90 95
   Gln Ala Met Lys Asp Ala Leu Asp Ser Gly Lys Tyr Asn Gly Tyr Ala
   100 105 110
   Pro Ser Ile Gly Tyr Leu Ser Ser Arg Glu Glu Val Ala Ser Tyr Tyr
   115 120 125
   His Cys His Glu Ala Pro Leu Glu Ala Lys Asp Val Ile Leu Thr Ser
   130 135 140
   Gly Cys Ser Gln Ala Ile Glu Leu Cys Leu Ala Val Leu Ala Asn Pro
   145 150 155 160
   Gly Gln Asn Ile Leu Ile Pro Arg Pro Gly Phe Ser Leu Tyr Arg Thr
   165 170 175
   Leu Ala Glu Ser Met Gly Ile Glu Val Lys Leu Tyr Asn Leu Leu Pro
   180 185 190
   Glu Lys Ser Trp Glu Ile Asp Leu Lys Gln Leu Glu Ser Leu Ile Asp
   195 200 205
   Glu Lys Thr Ala Cys Leu Val Val Asn Asn Pro Ser Asn Pro Cys Gly
   210 215 220
   Ser Val Phe Ser Lys Arg His Leu Gln Lys Ile Leu Ala Val Ala Glu
   225 230 235 240
   Arg Gln Cys Val Pro Ile Leu Ala Asp Glu Ile Tyr Gly Asp Met Val
   245 250 255
   Phe Ser Asp Cys Lys Tyr Glu Pro Leu Ala Asn Leu Ser Thr Asn Val
   260 265 270
   Pro Ile Leu Ser Cys Gly Gly Leu Ala Lys Arg Trp Leu Val Pro Gly
   275 280 285
   Trp Arg Leu Gly Trp Ile Leu Ile His Asp Arg Arg Asp Ile Phe Gly
   290 295 300
   Asn Glu Ile Arg Asp Gly Leu Val Lys Leu Ser Gln Arg Ile Leu Gly
   305 310 315 320
   Pro Cys Thr Ile Val Gln Gly Ala Leu Lys Ser Ile Leu Gln Arg Thr
   325 330 335
   Pro Gln Glu Phe Tyr His Asp Thr Leu Ser Phe Leu Lys Ser Asn Ala
   340 345 350
   Asp Leu Cys Tyr Gly Ala Leu Ala Ala Ile Pro Gly Leu Gln Pro Val
   355 360 365
   Arg Pro Ser Gly Ala Met Tyr Leu Met Val Gly Ile Glu Met Glu His
   370 375 380
   Phe Pro Glu Phe Glu Asn Asp Val Glu Phe Thr Glu Arg Leu Ile Ala
   385 390 395 400
   Glu Gln Ala Val His Cys Leu Pro Ala Thr Cys Phe Glu Tyr Pro Asn
   405 410 415
   Phe Phe Arg Val Val Ile Thr Val Pro Glu Val Met Met Leu Glu Ala
   420 425 430
   Cys Ser Arg Ile Gln Glu Phe Cys Glu Gln His Tyr His Cys Ala Glu
   435 440 445
   Gly Ser Gln Glu Glu Cys Asp Lys
   450 455
<210> 3
   <211> 1365
   <212> DNA
   <213> Rattus norvegicus
<220>
   <221> CDS
   <222> (1)..(1365)
<400> 3
   atg gac tcc tac gtg att cag acg gat gtc gac gac agc ttg tcc tca 48
   Met Asp Ser Tyr Val Ile Gln Thr Asp Val Asp Asp Ser Leu Ser Ser
   1 5 10 15
   gtt ctg gat gtg cgt gtc aat gtt ggt ggg aga aac tcg gta caa gga     96
   Val Leu Asp Val Arg Val Asn Val Gly Gly Arg Asn Ser Val Gln Gly
                   20                  25                  30
   aga aag aaa ggc agg aag gcc aga tgg gac gtg aga ccc tct gac atg    144
   Arg Lys Lys Gly Arg Lys Ala Arg Trp Asp Val Arg Pro Ser Asp Met
               35                  40                  45
   tcc aat aag acc ttc aat ccc atc cga gcc atc gtg gac aac atg aag    192
   Ser Asn Lys Thr Phe Asn Pro Ile Arg Ala Ile Val Asp Asn Met Lys
           50                  55                  60
   gtg cag ccc aat ccg aac aag acc gtg att tct ctg tca att ggg gac    240
   Val Gln Pro Asn Pro Asn Lys Thr Val Ile Ser Leu Ser Ile Gly Asp
    65                  70                  75                  80
   cct act gtg ttt ggg aac ctg cct aca gac cct gaa gtt acc caa gcc    288
   Pro Thr Val Phe Gly Asn Leu Pro Thr Asp Pro Glu Val Thr Gln Ala
                    85                  90                  95
   atg aaa gat gcc ctg gac tcg ggg aag tac aat ggc tat gcc ccg tcc    336
   Met Lys Asp Ala Leu Asp Ser Gly Lys Tyr Asn Gly Tyr Ala Pro Ser
               100                 105                 110
   atc ggc tac cta tcc agt cgg gag gag gtc gct tct tac tac cac tgt    384
   Ile Gly Tyr Leu Ser Ser Arg Glu Glu Val Ala Ser Tyr Tyr His Cys
           115                 120                 125
   cat gag gct cct ctg gaa gct aag gat gtc att ctg aca agc ggc tgc    432
   His Glu Ala Pro Leu Glu Ala Lys Asp Val Ile Leu Thr Ser Gly Cys
       130                 135                 140
   agt cag gcc att gag cta tgt cta gct gtg ttg gcc aat cct gga caa    480
   Ser Gln Ala Ile Glu Leu Cys Leu Ala Val Leu Ala Asn Pro Gly Gln
   145                 150                 155                 160
   aac atc ctc att cca agg ccc ggg ttt tcc ctc tat agg act ttg gct    528
   Asn Ile Leu Ile Pro Arg Pro Gly Phe Ser Leu Tyr Arg Thr Leu Ala
                   165                 170                 175
   gag tct atg gga att gag gtc aag ctc tac aat ctc ctg ccc gag aag    576
   Glu Ser Met Gly Ile Glu Val Lys Leu Tyr Asn Leu Leu Pro Glu Lys
               180                 185                 190
   tct tgg gaa att gac cta aaa caa ctg gaa tct ctg atc gat gaa aaa    624
   Ser Trp Glu Ile Asp Leu Lys Gln Leu Glu Ser Leu Ile Asp Glu Lys
           195                 200                 205
   aca gcg tgt ctt gtt gtc aac aac cca tcc aat ccc tgt ggc tcc gtg    672
   Thr Ala Cys Leu Val Val Asn Asn Pro Ser Asn Pro Cys Gly Ser Val
       210                 215                 220
   ttc agt aag cgg cac ctt cag aag att ttg gca gtg gct gaa agg cag    720
   Phe Ser Lys Arg His Leu Gln Lys Ile Leu Ala Val Ala Glu Arg Gln
   225                 230                 235                 240
   tgt gtc ccc atc tta gct gac gag atc tat ggt gac atg gtg ttt tca    768
   Cys Val Pro Ile Leu Ala Asp Glu Ile Tyr Gly Asp Met Val Phe Ser
                   245                 250                 255
   gac tgc aaa tac gaa cca ctg gcc aac ctc agc acc aat gtt ccc atc    816
   Asp Cys Lys Tyr Glu Pro Leu Ala Asn Leu Ser Thr Asn Val Pro Ile
               260                 265                 270
   ctg tcc tgt ggt ggg ctg gcc aag cgc tgg ctg gtt cct ggc tgg agg    864
   Leu Ser Cys Gly Gly Leu Ala Lys Arg Trp Leu Val Pro Gly Trp Arg
           275                 280                 285
   ttg ggc tgg atc ctc att cat gat cga aga gac att ttt ggc aat gag    912
   Leu Gly Trp Ile Leu Ile His Asp Arg Arg Asp Ile Phe Gly Asn Glu
       290                 295                 300
   att cga gac ggg ctg gtg aaa ctg agt cag cgg atc ctg gga cca tgc    960
   Ile Arg Asp Gly Leu Val Lys Leu Ser Gln Arg Ile Leu Gly Pro Cys
   305                 310                 315                 320
   acc ata gtc cag ggt gct ctg aag agc atc ctt cag cga acc cct cag    1008
   Thr Ile Val Gln Gly Ala Leu Lys Ser Ile Leu Gln Arg Thr Pro Gln
                   325                 330                 335
   gag ttc tat cac gac acg tta agc ttc ctc aag tcc aat gcg gac ctc    1056
   Glu Phe Tyr His Asp Thr Leu Ser Phe Leu Lys Ser Asn Ala Asp Leu
               340                 345                 350
   tgc tat ggg gca ctg gct gcc atc cct gga ctc cag ccg gtc cgc cct    1104
   Cys Tyr Gly Ala Leu Ala Ala Ile Pro Gly Leu Gln Pro Val Arg Pro
           355                 360                 365
   tct gga gcc atg tac ctt atg gtg gga att gag atg gag cat ctc ccg    1152
   Ser Gly Ala Met Tyr Leu Met Val Gly Ile Glu Met Glu His Leu Pro
       370                 375                 380
   gaa ttc gag aac gac gtg gag ttc aca gag cgg ttg att gcg gag cag    1200
   Glu Phe Glu Asn Asp Val Glu Phe Thr Glu Arg Leu Ile Ala Glu Gln
   385                 390                 395                 400
   gct gtc cac tgt ctc cca gca acg tgc ttc gag tac cca aat ttc ttc    1248
   Ala Val His Cys Leu Pro Ala Thr Cys Phe Glu Tyr Pro Asn Phe Phe
                   405                 410                 415
   cga gtg gtc atc aca gtc ccc gag gtg atg atg ctg gag gct tgt agc    1296
   Arg Val Val Ile Thr Val Pro Glu Val Met Met Leu Glu Ala Cys Ser
               420                 425                 430
   cgg atc cag gag ttc tgt gaa cag cac tac cac tgt gct gaa ggc agc    1344
   Arg Ile Gln Glu Phe Cys Glu Gln His Tyr His Cys Ala Glu Gly Ser
           435                 440                 445
   cag gag gag tgt gac aaa taa                                        1365
   Gln Glu Glu Cys Asp Lys
       450                 455
<210> 4
   <211> 454
   <212> PRT
   <213> Rattus norvegicus
<400> 4
   Met Asp Ser Tyr Val Ile Gln Thr Asp Val Asp Asp Ser Leu Ser Ser
     1               5                  10                  15
   Val Leu Asp Val Arg Val Asn Val Gly Gly Arg Asn Ser Val Gln Gly
               20                  25                  30
   Arg Lys Lys Gly Arg Lys Ala Arg Trp Asp Val Arg Pro Ser Asp Met
           35                  40                  45
   Ser Asn Lys Thr Phe Asn Pro Ile Arg Ala Ile Val Asp Asn Met Lys
       50                  55                  60
   Val Gln Pro Asn Pro Asn Lys Thr Val Ile Ser Leu Ser Ile Gly Asp
    65                  70                  75                  80
   Pro Thr Val Phe Gly Asn Leu Pro Thr Asp Pro Glu Val Thr Gln Ala
                    85                  90                  95
   Met Lys Asp Ala Leu Asp Ser Gly Lys Tyr Asn Gly Tyr Ala Pro Ser
               100                 105                 110
   Ile Gly Tyr Leu Ser Ser Arg Glu Glu Val Ala Ser Tyr Tyr His Cys
           115                 120                 125
   His Glu Ala Pro Leu Glu Ala Lys Asp Val Ile Leu Thr Ser Gly Cys
       130                 135                 140
   Ser Gln Ala Ile Glu Leu Cys Leu Ala Val Leu Ala Asn Pro Gly Gln
   145                 150                 155                 160
   Asn Ile Leu Ile Pro Arg Pro Gly Phe Ser Leu Tyr Arg Thr Leu Ala
   165 170 175
   Glu Ser Met Gly Ile Glu Val Lys Leu Tyr Asn Leu Leu Pro Glu Lys
   180 185 190
   Ser Trp Glu Ile Asp Leu Lys Gln Leu Glu Ser Leu Ile Asp Glu Lys
   195 200 205
   Thr Ala Cys Leu Val Val Asn Asn Pro Ser Asn Pro Cys Gly Ser Val
   210 215 220
   Phe Ser Lys Arg His Leu Gln Lys Ile Leu Ala Val Ala Glu Arg Gln
   225 230 235 240
   Cys Val Pro Ile Leu Ala Asp Glu Ile Tyr Gly Asp Met Val Phe Ser
   245 250 255
   Asp Cys Lys Tyr Glu Pro Leu Ala Asn Leu Ser Thr Asn Val Pro Ile
   260 265 270
   Leu Ser Cys Gly Gly Leu Ala Lys Arg Trp Leu Val Pro Gly Trp Arg
   275 280 285
   Leu Gly Trp Ile Leu Ile His Asp Arg Arg Asp Ile Phe Gly Asn Glu
   290 295 300
   Ile Arg Asp Gly Leu Val Lys Leu Ser Gln Arg Ile Leu Gly Pro Cys
   305 310 315 320
   Thr Ile Val Gln Gly Ala Leu Lys Ser Ile Leu Gln Arg Thr Pro Gln
   325 330 335
   Glu Phe Tyr His Asp Thr Leu Ser Phe Leu Lys Ser Asn Ala Asp Leu
   340 345 350
   Cys Tyr Gly Ala Leu Ala Ala Ile Pro Gly Leu Gln Pro Val Arg Pro
   355 360 365
   Ser Gly Ala Met Tyr Leu Met Val Gly Ile Glu Met Glu His Leu Pro
   370 375 380
   Glu Phe Glu Asn Asp Val Glu Phe Thr Glu Arg Leu Ile Ala Glu Gln
   385 390 395 400
   Ala Val His Cys Leu Pro Ala Thr Cys Phe Glu Tyr Pro Asn Phe Phe
   405 410 415
   Arg Val Val Ile Thr Val Pro Glu Val Met Met Leu Glu Ala Cys Ser
   420 425 430
   Arg Ile Gln Glu Phe Cys Glu Gln His Tyr His Cys Ala Glu Gly Ser
   435 440 445
   Gln Glu Glu Cys Asp Lys
   450
<210> 5
   <211> 1269
   <212> DNA
   <213> Arabidopsis thaliana
<220>
   <221> CDS
   <222> (1)..(1269)
<400> 5
   atg gca acc ctt aag tgc att gat tgg caa ttc agc gga agc gag gcg   48
   Met Ala Thr Leu Lys Cys Ile Asp Trp Gln Phe Ser Gly Ser Glu Ala
    1               5                  10                  15
   gcc aaa gat gct gct gcg gcc tcc tta ggc tca tat acc tct gca ctc   96
   Ala Lys Asp Ala Ala Ala Ala Ser Leu Gly Ser Tyr Thr Ser Ala Leu
               20                  25                  30
   tat gcc ctg tgc gat cct cat ggc aaa ccc att ttg ccc cca cga aat   144
   Tyr Ala Leu Cys Asp Pro His Gly Lys Pro Ile Leu Pro Pro Arg Asn
           35                  40                  45
   gag atc ctg gag acc agc aat aca gcc gaa aaa gca gtt gtt aaa gct   192
   Glu Ile Leu Glu Thr Ser Asn Thr Ala Glu Lys Ala Val Val Lys Ala
       50                  55                  60
   gtt ctt tat ggc tcg gga aac gcc tat gct cct agc tta ggc ctc gcg   240
   Val Leu Tyr Gly Ser Gly Asn Ala Tyr Ala Pro Ser Leu Gly Leu Ala
   65                  70                  75                  80
   gcc gcc aaa agt gcc gta gca gag tat cta aac caa ggt ctt cca aag   288
   Ala Ala Lys Ser Ala Val Ala Glu Tyr Leu Asn Gln Gly Leu Pro Lys
                   85                  90                  95
   aag ctt acc gca gat gac gtg ttt atg act ctg gga tgc aaa caa gct   336
   Lys Leu Thr Ala Asp Asp Val Phe Met Thr Leu Gly Cys Lys Gln Ala
               100                 105                 110
   att gag ctc gcg gta gac att ctc gct aaa ccg aaa gcc aac gtt ttg   384
   Ile Glu Leu Ala Val Asp Ile Leu Ala Lys Pro Lys Ala Asn Val Leu
           115                 120                 125
   ctt ccg agt ccc ggc ttc cca tgg gac cta gtc cgc tcc atc tac aag      432
   Leu Pro Ser Pro Gly Phe Pro Trp Asp Leu Val Arg Ser Ile Tyr Lys
       130                 135                 140
   aac ctt gag gtc cgc cac tat aat ttc ctt cca gaa aag aac ttt gaa      480
   Asn Leu Glu Val Arg His Tyr Asn Phe Leu Pro Glu Lys Asn Phe Glu
   145                 150                 155                 160
   atc gac ttt gat agc gtc cga gcg ctc gtg gac gag aac aca ttt gcc      528
   Ile Asp Phe Asp Ser Val Arg Ala Leu Val Asp Glu Asn Thr Phe Ala
                   165                 170                 175
   ata ttt ata atc aac ccc cac aac ccc aat ggt aac acc tac tcc gag      576
   Ile Phe Ile Ile Asn Pro His Asn Pro Asn Gly Asn Thr Tyr Ser Glu
               180                 185                 190
   gct cat ctc aaa cag ctg gct gaa ctg gct aag gaa ctc aag att atg      624
   Ala His Leu Lys Gln Leu Ala Glu Leu Ala Lys Glu Leu Lys Ile Met
           195                 200                 205
   gtg gtt tct gac gag gtt ttt aga tgg aca ctc ttt ggt agt aac cct      672
   Val Val Ser Asp Glu Val Phe Arg Trp Thr Leu Phe Gly Ser Asn Pro
       210                 215                 220
   ttt gtt cct atg gga aaa ttc tcg tcg atc gta cca gtg gtt aca ctc      720
   Phe Val Pro Met Gly Lys Phe Ser Ser Ile Val Pro Val Val Thr Leu
   225                 230                 235                 240
   gga tcc ata tca aag gga tgg aaa gtc cca gga tgg cga act ggt tgg      768
   Gly Ser Ile Ser Lys Gly Trp Lys Val Pro Gly Trp Arg Thr Gly Trp
                   245                 250                 255
   ctc acg cta cat gat cta gac ggt gtc ttc aga aac acc aag gtc tta      816
   Leu Thr Leu His Asp Leu Asp Gly Val Phe Arg Asn Thr Lys Val Leu
               260                 265                 270
   caa gct gct caa gat ttt ctc cag ata aac aat aac cct ccg aca gtt      864
   Gln Ala Ala Gln Asp Phe Leu Gln Ile Asn Asn Asn Pro Pro Thr Val
           275                 280                 285
   atc cag gcg gct att cct gac atc ttg gag aaa act cct caa gag ttt      912
   Ile Gln Ala Ala Ile Pro Asp Ile Leu Glu Lys Thr Pro Gln Glu Phe
       290                 295                 300
   ttt gat aag agg cag agt ttt ctg aaa gat aaa gta gaa ttt ggt tat      960
   Phe Asp Lys Arg Gln Ser Phe Leu Lys Asp Lys Val Glu Phe Gly Tyr
   305                 310                 315                 320
   tct aag ctc aag tac att cct agc ctc act tgc tac atg aaa ccc gaa     1008
   Ser Lys Leu Lys Tyr Ile Pro Ser Leu Thr Cys Tyr Met Lys Pro Glu
                   325                 330                 335
   gcc tgc acc ttc tta tgg acc gag ctt gat tta tcg agc ttt gtg gac    1056
   Ala Cys Thr Phe Leu Trp Thr Glu Leu Asp Leu Ser Ser Phe Val Asp
               340                 345                 350
   atc gaa gac gat caa gac ttt tgc aat aag ctt gct aaa gaa gaa aac    1104
   Ile Glu Asp Asp Gln Asp Phe Cys Asn Lys Leu Ala Lys Glu Glu Asn
           355                 360                 365
   ctc gtc gtt tta cca ggg att gca ttc agt cag aag aac tgg ttg agg    1152
   Leu Val Val Leu Pro Gly Ile Ala Phe Ser Gln Lys Asn Trp Leu Arg
       370                 375                 380
   cat tct atc gat atg gag act ccg gta ttg gag gat gca ttg gaa aga    1200
   His Ser Ile Asp Met Glu Thr Pro Val Leu Glu Asp Ala Leu Glu Arg
   385                 390                 395                 400
   ttg aag agc ttc tgc gat cgc cat tcc aac aaa aaa gct ccc ctc aaa    1248
   Leu Lys Ser Phe Cys Asp Arg His Ser Asn Lys Lys Ala Pro Leu Lys
                   405                 410                 415
   gac gtc aat ggt gtt aag taa                                        1269
   Asp Val Asn Gly Val Lys
               420
<210> 6
   <211> 422
   <212> PRT
   <213> Arabidopsis thaliana
<400> 6
   Met Ala Thr Leu Lys Cys Ile Asp Trp Gln Phe Ser Gly Ser Glu Ala
   1 5 10 15
   Ala Lys Asp Ala Ala Ala Ala Ser Leu Gly Ser Tyr Thr Ser Ala Leu
   20 25 30
   Tyr Ala Leu Cys Asp Pro His Gly Lys Pro Ile Leu Pro Pro Arg Asn
   35 40 45
   Glu Ile Leu Glu Thr Ser Asn Thr Ala Glu Lys Ala Val Val Lys Ala
   50 55 60
   Val Leu Tyr Gly Ser Gly Asn Ala Tyr Ala Pro Ser Leu Gly Leu Ala
   65 70 75 80
   Ala Ala Lys Ser Ala Val Ala Glu Tyr Leu Asn Gln Gly Leu Pro Lys
   85 90 95
   Lys Leu Thr Ala Asp Asp Val Phe Met Thr Leu Gly Cys Lys Gln Ala
   100 105 110
   Ile Glu Leu Ala Val Asp Ile Leu Ala Lys Pro Lys Ala Asn Val Leu
   115 120 125
   Leu Pro Ser Pro Gly Phe Pro Trp Asp Leu Val Arg Ser Ile Tyr Lys
   130 135 140
   Asn Leu Glu Val Arg His Tyr Asn Phe Leu Pro Glu Lys Asn Phe Glu
   145 150 155 160
   Ile Asp Phe Asp Ser Val Arg Ala Leu Val Asp Glu Asn Thr Phe Ala
   165 170 175
   Ile Phe Ile Ile Asn Pro His Asn Pro Asn Gly Asn Thr Tyr Ser Glu
   180 185 190
   Ala His Leu Lys Gln Leu Ala Glu Leu Ala Lys Glu Leu Lys Ile Met
   195 200 205
   Val Val Ser Asp Glu Val Phe Arg Trp Thr Leu Phe Gly Ser Asn Pro
   210 215 220
   Phe Val Pro Met Gly Lys Phe Ser Ser Ile Val Pro Val Val Thr Leu
   225 230 235 240
   Gly Ser Ile Ser Lys Gly Trp Lys Val Pro Gly Trp Arg Thr Gly Trp
   245 250 255
   Leu Thr Leu His Asp Leu Asp Gly Val Phe Arg Asn Thr Lys Val Leu
   260 265 270
   Gln Ala Ala Gln Asp Phe Leu Gln Ile Asn Asn Asn Pro Pro Thr Val
   275 280 285
   Ile Gln Ala Ala Ile Pro Asp Ile Leu Glu Lys Thr Pro Gln Glu Phe
   290 295 300
   Phe Asp Lys Arg Gln Ser Phe Leu Lys Asp Lys Val Glu Phe Gly Tyr
   305 310 315 320
   Ser Lys Leu Lys Tyr Ile Pro Ser Leu Thr Cys Tyr Met Lys Pro Glu
   325 330 335
   Ala Cys Thr Phe Leu Trp Thr Glu Leu Asp Leu Ser Ser Phe Val Asp
   340 345 350
   Ile Glu Asp Asp Gln Asp Phe Cys Asn Lys Leu Ala Lys Glu Glu Asn
   355 360 365
   Leu Val Val Leu Pro Gly Ile Ala Phe Ser Gln Lys Asn Trp Leu Arg
   370 375 380
   His Ser Ile Asp Met Glu Thr Pro Val Leu Glu Asp Ala Leu Glu Arg
   385 390 395 400
   Leu Lys Ser Phe Cys Asp Arg His Ser Asn Lys Lys Ala Pro Leu Lys
   405 410 415
   Asp Val Asn Gly Val Lys
   420
<210> 7
   <211> 1334
   <212> DNA
   <213> Arabidopsis thaliana
<220>
   <221> CDS
   <222> (1)..(1332)
<400> 7
<210> 8
   <211> 444
   <212> PRT
   <213> Arabidopsis thaliana
<400> 8
   Met Ala Ser Asn Gly Val Thr Asn Cys Asn Ala Asn Ala Asn Val Trp
   1               5                   10                  15
   Arg Phe Lys Gly Asn Gly Ala Thr Ser Asp Ala Thr Ala Val Thr Leu
               20                  25                  30
   Arg Lys Leu Ala Phe Gly Met Phe Lys Asn Cys Thr Met Asn Ser Gly
   35 40 45
   Lys Thr Ile Leu Phe Pro Thr Pro Gly Glu Pro Ser Ala His Ser Asn
   50 55 60
   Phe Arg Thr Cys Pro Glu Ala Glu Glu Ala Val Ala Asp Ala Ala Arg
   65 70 75 80
   Ser Gly Met Ala Asn Ser Tyr Ala Pro Ser Pro Gly Val Phe Lys Ala
   85 90 95
   Arg Arg Ala Val Ala Glu Tyr Leu Asn Gly Glu Leu Pro Thr Lys Leu
   100 105 110
   Lys Ala Glu Asp Val Tyr Ile Thr Gly Gly Cys Asn Gln Ala Ile Glu
   115 120 125
   Ile Val Ile Asp Ser Leu Ala Gly Asn Pro Ser Thr Asn Ile Leu Leu
   130 135 140
   Pro Arg Pro Gly Tyr Pro His Tyr Asp Ala Arg Ala Val Tyr Ser Gly
   145 150 155 160
   Leu Glu Ile Arg Glu Tyr Asp Leu Leu Pro Glu Ser Asp Trp Glu Ile
   165 170 175
   Asn Leu Asp Gly Leu Glu Ala Ala Ala Asp Glu Asn Thr Val Ala Met
   180 185 190
   Val Ile Ile Asn Pro Asn Asn Pro Cys Gly Asn Val Tyr Thr Tyr Asp
   195 200 205
   His Leu Asn Lys Val Ala Glu Met Ala Arg Lys Leu Gly Ile Met Ile
   210 215 220
   Ile Ser Asp Glu Val Tyr Asp His Val Val Tyr Gly Asp Lys Pro Phe
   225 230 235 240
   Ile Pro Met Gly Lys Phe Ala Ser Ile Ala Pro Val Ile Thr Leu Gly
   245 250 255
   Ser Ile Ser Lys Gly Trp Val Asn Pro Gly Trp Arg Val Gly Trp Ile
   260 265 270
   Ala Met Asn Asp Pro Asn Gly Ile Phe Val Ser Thr Gly Val Val Gln
   275 280 285
   Ala Ile Glu Asp Phe Leu Asp Leu Thr Pro Gln Pro Ser Phe Ile Leu
   290 295 300
   Gln Glu Ala Leu Pro Asp Ile Leu Glu Lys Thr Pro Lys Glu Phe Phe
   305                 310                 315                 320
   Glu Lys Lys Ile Lys Ala Met Arg Arg Asn Val Glu Leu Ser Cys Glu
                   325                 330                 335
   Arg Leu Lys Asp Ile Pro Cys Leu Phe Cys Pro Lys Lys Pro Glu Ser
               340                 345                 350
   Cys Ser Tyr Leu Trp Leu Lys Leu Asp Thr Ser Met Leu Asn Asn Ile
           355                 360                 365
   Lys Asn Asp Phe Asp Phe Cys Thr Lys Leu Val Ser Glu Glu Ser Leu
       370                 375                 380
   Ile Leu Ile Pro Gly Val Ala Leu Gly Ala Glu Asn Trp Val Arg Ile
   385                 390                 395                 400
   Ser Ile Gly Thr Asp Glu Ser Val Val Gln Glu Ile Phe Asp Arg Leu
                   405                 410                 415
   Lys Gly Phe Tyr Asp Arg His Ala Ile Ser Lys Glu Ala Ile Lys Leu
               420                 425                 430
   Ser Gly His Ala Ile Asn Gln Ile Val Val Ser Val
           435                 440
<210> 9
   <211> 1389
   <212> DNA
   <213> Arabidopsis thaliana
<220>
   <221> CDS
   <222> (1)..(1389)
<400> 9
   atg agc gaa gaa caa caa cac gcc aat cta gcg gtt ccc gcg ttt aaa    48
   Met Ser Glu Glu Gln Gln His Ala Asn Leu Ala Val Pro Ala Phe Lys
    1               5                  10                  15
   act gag aaa gat ccc gta acg caa acg caa aat ggt caa agt agc gtt    96
   Thr Glu Lys Asp Pro Val Thr Gln Thr Gln Asn Gly Gln Ser Ser Val
               20                  25                  30
   tgg cgt ttc ggt gga agt gat aag gca gcg aaa gca tcc acc gta acg    144
   Trp Arg Phe Gly Gly Ser Asp Lys Ala Ala Lys Ala Ser Thr Val Thr
           35                  40                  45
   ctt aga ggt gtc atc tac atg ctc ttc gac aac tgc agc aaa gac gtc    192
   Leu Arg Gly Val Ile Tyr Met Leu Phe Asp Asn Cys Ser Lys Asp Val
        50                  55                  60
   aat aag acc att tta ccc ctc ggc cac ggt gac cct tcc gtc tac cct    240
   Asn Lys Thr Ile Leu Pro Leu Gly His Gly Asp Pro Ser Val Tyr Pro
   65                  70                  75                  80
   tgc ttc cgt acc tgt atc gaa gct gaa gac gcc gtc gtc gac gtc ctt    288
   Cys Phe Arg Thr Cys Ile Glu Ala Glu Asp Ala Val Val Asp Val Leu
                   85                  90                  95
   cgc tcc ggc aaa ggc aat tct tac ggt ccc gga gct ggg att ctc ccc    336
   Arg Ser Gly Lys Gly Asn Ser Tyr Gly Pro Gly Ala Gly Ile Leu Pro
               100                 105                 110
   gca aga cga gcc gtt gct gat tat atg aac cga gat ctt ccg cac aag    384
   Ala Arg Arg Ala Val Ala Asp Tyr Met Asn Arg Asp Leu Pro His Lys
           115                 120                 125
   tta acg ccc gaa gat att ttt ctg acc gct gga tgc aac caa ggg ata    432
   Leu Thr Pro Glu Asp Ile Phe Leu Thr Ala Gly Cys Asn Gln Gly Ile
       130                 135                 140
   gag atc gtg ttc gaa tcg ttg gct cga cca aac gca aac atc ttg ctc    480
   Glu Ile Val Phe Glu Ser Leu Ala Arg Pro Asn Ala Asn Ile Leu Leu
   145                 150                 155                 160
   cca cgt cct ggc ttc cct cat tac gac gct cgt gct gct tac agt ggt    528
   Pro Arg Pro Gly Phe Pro His Tyr Asp Ala Arg Ala Ala Tyr Ser Gly
                   165                 170                 175
   ctc gag gtt cgc aag ttt gat ctt ctt ccc gag aaa gaa tgg gag att    576
   Leu Glu Val Arg Lys Phe Asp Leu Leu Pro Glu Lys Glu Trp Glu Ile
               180                 185                 190
   gat ctt gaa ggt atc gaa gcc att gca gac gag aaa act gtg gct atg    624
   Asp Leu Glu Gly Ile Glu Ala Ile Ala Asp Glu Lys Thr Val Ala Met
           195                 200                 205
   gtt gta att aac ccc aac aat ccc tgt gga aat gtc tac tct cac gac    672
   Val Val Ile Asn Pro Asn Asn Pro Cys Gly Asn Val Tyr Ser His Asp
       210                 215                 220
   cat ctc aaa aag gtt gca gag acg gct agg aag ctc ggg ata atg gtg    720
   His Leu Lys Lys Val Ala Glu Thr Ala Arg Lys Leu Gly Ile Met Val
   225                 230                 235                 240
   atc tca gac gaa gta tat gac cga act ata ttc gga gac aat cca ttt    768
   Ile Ser Asp Glu Val Tyr Asp Arg Thr Ile Phe Gly Asp Asn Pro Phe
                   245                     250                 255
   gtt cca atg ggg aag ttt gct tcg ata gtc cct gta ttg aca cta gca      816
   Val Pro Met Gly Lys Phe Ala Ser Ile Val Pro Val Leu Thr Leu Ala
               260                 265                 270
   ggc ata tct aag gga tgg gtt gtt cct gga tgg aaa att ggc tgg att      864
   Gly Ile Ser Lys Gly Trp Val Val Pro Gly Trp Lys Ile Gly Trp Ile
           275                 280                 285
   gcc ttg aat gat ccc gag ggc gtt ttc gag acc acc aag gtg tta caa      912
   Ala Leu Asn Asp Pro Glu Gly Val Phe Glu Thr Thr Lys Val Leu Gln
       290                 295                 300
   tcc atc aaa cag aat ctt gac gta act cct gac cct gcc aca ata att      960
   Ser Ile Lys Gln Asn Leu Asp Val Thr Pro Asp Pro Ala Thr Ile Ile
   305                 310                 315                 320
   cag gct gca ctt cca gcg atc ctg gag aaa gcg gac aaa aac ttc ttt     1008
   Gln Ala Ala Leu Pro Ala Ile Leu Glu Lys Ala Asp Lys Asn Phe Phe
                   325                 330                 335
   gca aag aag aac aag ata ctc aaa cat aat gtt gat ttg gtg tgt gat     1056
   Ala Lys Lys Asn Lys Ile Leu Lys His Asn Val Asp Leu Val Cys Asp
               340                 345                 350
   agg ctc aag gac atc ccc tgt gtc gtc tgt ccc aag aaa cct gag tct     1104
   Arg Leu Lys Asp Ile Pro Cys Val Val Cys Pro Lys Lys Pro Glu Ser
           355                 360                 365
   tgc act tac tta ttg aca aag ttg gag ctg tca ctg atg gat aat atc     1152
   Cys Thr Tyr Leu Leu Thr Lys Leu Glu Leu Ser Leu Met Asp Asn Ile
       370                 375                 380
   aag gac gat ata gat ttt tgc gta aaa ctg gcc aga gag gag aat ctc     1200
   Lys Asp Asp Ile Asp Phe Cys Val Lys Leu Ala Arg Glu Glu Asn Leu
   385                 390                 395                 400
   gtg ttt cta cca ggg gat gct ctg ggt ttg aag aac tgg acg agg ata     1248
   Val Phe Leu Pro Gly Asp Ala Leu Gly Leu Lys Asn Trp Thr Arg Ile
                   405                 410                 415
   acc atc gga gtc gaa gct cat atg ctt gag gat gca ctt gag aga ctg     1296
   Thr Ile Gly Val Glu Ala His Met Leu Glu Asp Ala Leu Glu Arg Leu
               420                 425                 430
   aag ggt ttc tgt aca cgt cat gcc aag aag aca gag aca gaa act gag     1344
   Lys Gly Phe Cys Thr Arg His Ala Lys Lys Thr Glu Thr Glu Thr Glu
           435                 440                 445
   tca ctt caa gct ttg aaa ctg agt gat aat aat ctc gaa atg taa        1389
   Ser Leu Gln Ala Leu Lys Leu Ser Asp Asn Asn Leu Glu Met
       450                 455                 460
<210> 10
   <211> 462
   <212> PRT
   <213> Arabidopsis thaliana
<400> 10
   Met Ser Glu Glu Gln Gln His Ala Asn Leu Ala Val Pro Ala Phe Lys
    1               5                  10                  15
   Thr Glu Lys Asp Pro Val Thr Gln Thr Gln Asn Gly Gln Ser Ser Val
               20                  25                  30
   Trp Arg Phe Gly Gly Ser Asp Lys Ala Ala Lys Ala Ser Thr Val Thr
           35                  40                  45
   Leu Arg Gly Val Ile Tyr Met Leu Phe Asp Asn Cys Ser Lys Asp Val
       50                  55                  60
   Asn Lys Thr Ile Leu Pro Leu Gly His Gly Asp Pro Ser Val Tyr Pro
   65                  70                  75                  80
   Cys Phe Arg Thr Cys Ile Glu Ala Glu Asp Ala Val Val Asp Val Leu
                   85                  90                  95
   Arg Ser Gly Lys Gly Asn Ser Tyr Gly Pro Gly Ala Gly Ile Leu Pro
               100                 105                 110
   Ala Arg Arg Ala Val Ala Asp Tyr Met Asn Arg Asp Leu Pro His Lys
           115                 120                 125
   Leu Thr Pro Glu Asp Ile Phe Leu Thr Ala Gly Cys Asn Gln Gly Ile
       130                 135                 140
   Glu Ile Val Phe Glu Ser Leu Ala Arg Pro Asn Ala Asn Ile Leu Leu
   145                 150                 155                 160
   Pro Arg Pro Gly Phe Pro His Tyr Asp Ala Arg Ala Ala Tyr Ser Gly
                   165                 170                 175
   Leu Glu Val Arg Lys Phe Asp Leu Leu Pro Glu Lys Glu Trp Glu Ile
               180                 185                 190
   Asp Leu Glu Gly Ile Glu Ala Ile Ala Asp Glu Lys Thr Val Ala Met
           195                 200                 205
   Val Val Ile Asn Pro Asn Asn Pro Cys Gly Asn Val Tyr Ser His Asp
       210                 215                 220
   His Leu Lys Lys Val Ala Glu Thr Ala Arg Lys Leu Gly Ile Met Val
   225                 230                 235                 240
   Ile Ser Asp Glu Val Tyr Asp Arg Thr Ile Phe Gly Asp Asn Pro Phe
                   245                 250                 255
   Val Pro Met Gly Lys Phe Ala Ser Ile Val Pro Val Leu Thr Leu Ala
               260                 265                 270
   Gly Ile Ser Lys Gly Trp Val Val Pro Gly Trp Lys Ile Gly Trp Ile
           275                 280                 285
   Ala Leu Asn Asp Pro Glu Gly Val Phe Glu Thr Thr Lys Val Leu Gln
       290                 295                 300
   Ser Ile Lys Gln Asn Leu Asp Val Thr Pro Asp Pro Ala Thr Ile Ile
   305                 310                 315                 320
   Gln Ala Ala Leu Pro Ala Ile Leu Glu Lys Ala Asp Lys Asn Phe Phe
                   325                 330                 335
   Ala Lys Lys Asn Lys Ile Leu Lys His Asn Val Asp Leu Val Cys Asp
               340                 345                 350
   Arg Leu Lys Asp Ile Pro Cys Val Val Cys Pro Lys Lys Pro Glu Ser
           355                 360                 365
   Cys Thr Tyr Leu Leu Thr Lys Leu Glu Leu Ser Leu Met Asp Asn Ile
       370                 375                 380
   Lys Asp Asp Ile Asp Phe Cys Val Lys Leu Ala Arg Glu Glu Asn Leu
   385                 390                 395                 400
   Val Phe Leu Pro Gly Asp Ala Leu Gly Leu Lys Asn Trp Thr Arg Ile
                   405                 410                 415
   Thr Ile Gly Val Glu Ala His Met Leu Glu Asp Ala Leu Glu Arg Leu
               420                 425                 430
   Lys Gly Phe Cys Thr Arg His Ala Lys Lys Thr Glu Thr Glu Thr Glu
           435                 440                 445
   Ser Leu Gln Ala Leu Lys Leu Ser Asp Asn Asn Leu Glu Met
       450                 455                 460
<210> 11
   <211> 1243
   <212> DNA
   <213> Arabidopsis thaliana
<220>
   <221> CDS
   <222> (1)..(1242)
<400> 11
   atg gag aat gga gca acg acg acg agc aca att acc atc aaa ggg att    48
   Met Glu Asn Gly Ala Thr Thr Thr Ser Thr Ile Thr Ile Lys Gly Ile
   1               5                   10                  15
   ctg agt ttg cta atg gaa agc atc aca aca gag gaa gat gaa gga gga    96
   Leu Ser Leu Leu Met Glu Ser Ile Thr Thr Glu Glu Asp Glu Gly Gly
               20                  25                  30
   aag aga gta ata tct ctg gga atg gga gac cca aca ctc tac tcg tgt    144
   Lys Arg Val Ile Ser Leu Gly Met Gly Asp Pro Thr Leu Tyr Ser Cys
           35                  40                  45
   ttt cgt aca aca caa gtc tct ctt caa gct gtt tct gat tct ctt ctc    192
   Phe Arg Thr Thr Gln Val Ser Leu Gln Ala Val Ser Asp Ser Leu Leu
       50                  55                  60
   tcc aac aag ttc cat ggt tac tct cct acc gtc ggt ctt ccc caa gct    240
   Ser Asn Lys Phe His Gly Tyr Ser Pro Thr Val Gly Leu Pro Gln Ala
   65                  70                  75                  80
   cga agg gca ata gca gag tat cta tcg cgt gat ctt cca tac aaa ctt    288
   Arg Arg Ala Ile Ala Glu Tyr Leu Ser Arg Asp Leu Pro Tyr Lys Leu
                   85                  90                  95
   tca cag gat gat gtg ttt atc aca tcg ggt tgc acg caa gcg atc gat    336
   Ser Gln Asp Asp Val Phe Ile Thr Ser Gly Cys Thr Gln Ala Ile Asp
               100                 105                 110
   gta gca ttg tcg atg tta gct cgt ccc agg gct aat ata ctt ctt cca    384
   Val Ala Leu Ser Met Leu Ala Arg Pro Arg Ala Asn Ile Leu Leu Pro
           115                 120                 125
   agg cct ggt ttc cca atc tat gaa ctc tgt gct aag ttt aga cac ctt    432
   Arg Pro Gly Phe Pro Ile Tyr Glu Leu Cys Ala Lys Phe Arg His Leu
       130                 135                 140
   gaa gtt cgc tac gtc gat ctt ctt ccg gaa aat gga tgg gag atc gat    480
   Glu Val Arg Tyr Val Asp Leu Leu Pro Glu Asn Gly Trp Glu Ile Asp
   145                 150                 155                 160
   ctt gat gct gtc gag gct ctt gca gac gaa aac acg gtt gct ttg gtt     528
   Leu Asp Ala Val Glu Ala Leu Ala Asp Glu Asn Thr Val Ala Leu Val
                   165                 170                 175
   gtt ata aac cct ggt aat cct tgc ggg aat gtc tat agc tac cag cat     576
   Val Ile Asn Pro Gly Asn Pro Cys Gly Asn Val Tyr Ser Tyr Gln His
               180                 185                 190
   ttg atg aag att gcg gaa tcg gcg aaa aaa cta ggg ttt ctt gtg att     624
   Leu Met Lys Ile Ala Glu Ser Ala Lys Lys Leu Gly Phe Leu Val Ile
           195                 200                 205
   gct gat gag gtt tac ggt cat ctt gct ttt ggt agc aaa ccg ttt gtg     672
   Ala Asp Glu Val Tyr Gly His Leu Ala Phe Gly Ser Lys Pro Phe Val
       210                 215                 220
   cca atg ggt gtg ttt gga tct att gtt cct gtg ctt act ctt ggc tct     720
   Pro Met Gly Val Phe Gly Ser Ile Val Pro Val Leu Thr Leu Gly Ser
   225                 230                 235                 240
   tta tca aag aga tgg ata gtt cca ggt tgg cga ctc ggg tgg ttt gtc     768
   Leu Ser Lys Arg Trp Ile Val Pro Gly Trp Arg Leu Gly Trp Phe Val
                   245                 250                 255
   acc act gat cct tct ggt tcc ttt aag gac cct aag atc att gag agg     816
   Thr Thr Asp Pro Ser Gly Ser Phe Lys Asp Pro Lys Ile Ile Glu Arg
               260                 265                 270
   ttt aag aaa tac ttt gat att ctt ggt gga cca gct aca ttt att cag     864
   Phe Lys Lys Tyr Phe Asp Ile Leu Gly Gly Pro Ala Thr Phe Ile Gln
           275                 280                 285
   gct gca gtt ccc act att ttg gaa cag acg gat gag tct ttc ttc aag     912
   Ala Ala Val Pro Thr Ile Leu Glu Gln Thr Asp Glu Ser Phe Phe Lys
       290                 295                 300
   aaa acc ttg aac tcg ttg aag aac tct tcg gat att tgt tgt gac tgg     960
   Lys Thr Leu Asn Ser Leu Lys Asn Ser Ser Asp Ile Cys Cys Asp Trp
   305                 310                 315                 320
   atc aag gag att cct tgc att gat tcc tcg cat cga cca gaa gga tcc    1008
   Ile Lys Glu Ile Pro Cys Ile Asp Ser Ser His Arg Pro Glu Gly Ser
                   325                 330                 335
   atg gca atg atg gtc aag ctg aat ctc tca tta ctt gaa gat gta agt    1056
   Met Ala Met Met Val Lys Leu Asn Leu Ser Leu Leu Glu Asp Val Ser
               340                 345                 350
   gac gat atc gac ttc tgt ttc aag tta gct agg gaa gaa tca gtc atc    1104
   Asp Asp Ile Asp Phe Cys Phe Lys Leu Ala Arg Glu Glu Ser Val Ile
           355                 360                 365
   ctt ctt cct ggt acc gcg gtg ggg ctg aag aac tgg ctg agg ata acg      1152
   Leu Leu Pro Gly Thr Ala Val Gly Leu Lys Asn Trp Leu Arg Ile Thr
       370                 375                 380
   ttt gca gca gat gca act tcg att gaa gaa gct ttt aaa agg atc aaa      1200
   Phe Ala Ala Asp Ala Thr Ser Ile Glu Glu Ala Phe Lys Arg Ile Lys
   385                 390                 395                 400
   tgt ttc tat ctt aga cat gcc aag act caa tat cca acc ata t            1243
   Cys Phe Tyr Leu Arg His Ala Lys Thr Gln Tyr Pro Thr Ile
                   405                 410
<210> 12
   <211> 414
   <212> PRT
   <213> Arabidopsis thaliana
<400> 12
   Met Glu Asn Gly Ala Thr Thr Thr Ser Thr Ile Thr Ile Lys Gly Ile
    1               5                  10                  15
   Leu Ser Leu Leu Met Glu Ser Ile Thr Thr Glu Glu Asp Glu Gly Gly
               20                  25                  30
   Lys Arg Val Ile Ser Leu Gly Met Gly Asp Pro Thr Leu Tyr Ser Cys
           35                  40                  45
   Phe Arg Thr Thr Gln Val Ser Leu Gln Ala Val Ser Asp Ser Leu Leu
       50                  55                  60
   Ser Asn Lys Phe His Gly Tyr Ser Pro Thr Val Gly Leu Pro Gln Ala
   65                  70                  75                  80
   Arg Arg Ala Ile Ala Glu Tyr Leu Ser Arg Asp Leu Pro Tyr Lys Leu
                   85                  90                  95
   Ser Gln Asp Asp Val Phe Ile Thr Ser Gly Cys Thr Gln Ala Ile Asp
               100                 105                 110
   Val Ala Leu Ser Met Leu Ala Arg Pro Arg Ala Asn Ile Leu Leu Pro
           115                 120                 125
   Arg Pro Gly Phe Pro Ile Tyr Glu Leu Cys Ala Lys Phe Arg His Leu
       130                 135                 140
   Glu Val Arg Tyr Val Asp Leu Leu Pro Glu Asn Gly Trp Glu Ile Asp
   145                 150                 155                 160
   Leu Asp Ala Val Glu Ala Leu Ala Asp Glu Asn Thr Val Ala Leu Val
   165 170 175
   Val Ile Asn Pro Gly Asn Pro Cys Gly Asn Val Tyr Ser Tyr Gln His
   180 185 190
   Leu Met Lys Ile Ala Glu Ser Ala Lys Lys Leu Gly Phe Leu Val Ile
   195 200 205
   Ala Asp Glu Val Tyr Gly His Leu Ala Phe Gly Ser Lys Pro Phe Val
   210 215 220
   Pro Met Gly Val Phe Gly Ser Ile Val Pro Val Leu Thr Leu Gly Ser
   225 230 235 240
   Leu Ser Lys Arg Trp Ile Val Pro Gly Trp Arg Leu Gly Trp Phe Val
   245 250 255
   Thr Thr Asp Pro Ser Gly Ser Phe Lys Asp Pro Lys Ile Ile Glu Arg
   260 265 270
   Phe Lys Lys Tyr Phe Asp Ile Leu Gly Gly Pro Ala Thr Phe Ile Gln
   275 280 285
   Ala Ala Val Pro Thr Ile Leu Glu Gln Thr Asp Glu Ser Phe Phe Lys
   290 295 300
   Lys Thr Leu Asn Ser Leu Lys Asn Ser Ser Asp Ile Cys Cys Asp Trp
   305 310 315 320
   Ile Lys Glu Ile Pro Cys Ile Asp Ser Ser His Arg Pro Glu Gly Ser
   325 330 335
   Met Ala Met Met Val Lys Leu Asn Leu Ser Leu Leu Glu Asp Val Ser
   340 345 350
   Asp Asp Ile Asp Phe Cys Phe Lys Leu Ala Arg Glu Glu Ser Val Ile
   355 360 365
   Leu Leu Pro Gly Thr Ala Val Gly Leu Lys Asn Trp Leu Arg Ile Thr
   370 375 380
   Phe Ala Ala Asp Ala Thr Ser Ile Glu Glu Ala Phe Lys Arg Ile Lys
   385 390 395 400
   Cys Phe Tyr Leu Arg His Ala Lys Thr Gln Tyr Pro Thr Ile
   405 410
<210> 13
   <211> 1338
   <212> DNA
   <213> Arabidopsis thaliana
<220>
   <221> CDS
   <222> (1)..(1338)
<400> 13
   atg ggc cac caa aac gcc gcc gtt tca gag aat caa aac cat gat gac      48
   Met Gly His Gln Asn Ala Ala Val Ser Glu Asn Gln Asn His Asp Asp
     1               5                  10                  15
   ggc gct gcg tcg tcg ccg gga ttc aag ctc gtc gga ttt tcc aag ttc      96
   Gly Ala Ala Ser Ser Pro Gly Phe Lys Leu Val Gly Phe Ser Lys Phe
                20                  25                  30
   gta aga aag aat cca aag tct gat aaa ttc aag gtt aag cgc ttc cat     144
   Val Arg Lys Asn Pro Lys Ser Asp Lys Phe Lys Val Lys Arg Phe His
            35                  40                  45
   cac atc gag ttc tgg tgc ggc gac gca acc aac gtc gct cgt cgc ttc     192
   His Ile Glu Phe Trp Cys Gly Asp Ala Thr Asn Val Ala Arg Arg Phe
        50                  55                  60
   tcc tgg ggt ctg ggg atg aga ttc tcc gcc aaa tcc gat ctt tcc acc     240
   Ser Trp Gly Leu Gly Met Arg Phe Ser Ala Lys Ser Asp Leu Ser Thr
    65                  70                  75                  80
   gga aac atg gtt cac gcc tct tac cta ctc acc tcc ggt gac ctc cga     288
   Gly Asn Met Val His Ala Ser Tyr Leu Leu Thr Ser Gly Asp Leu Arg
                    85                  90                  95
   ttc ctt ttc act gct cct tac tct ccg tct ctc tcc gcc gga gag att     336
   Phe Leu Phe Thr Ala Pro Tyr Ser Pro Ser Leu Ser Ala Gly Glu Ile
               100                 105                 110
   aaa ccg aca acc aca gct tct atc cca agt ttc gat cac ggc tct tgt     384
   Lys Pro Thr Thr Thr Ala Ser Ile Pro Ser Phe Asp His Gly Ser Cys
           115                 120                 125
   cgt tcc ttc ttc tct tca cat ggt ctc ggt gtt aga gcc gtt gcg att     432
   Arg Ser Phe Phe Ser Ser His Gly Leu Gly Val Arg Ala Val Ala Ile
       130                 135                 140
   gaa gta gaa gac gca gag tca gct ttc tcc atc agt gta gct aat ggc     480
   Glu Val Glu Asp Ala Glu Ser Ala Phe Ser Ile Ser Val Ala Asn Gly
   145                 150                 155                 160
   gct att cct tcg tcg cct cct atc gtc ctc aat gaa gca gtt acg atc    528
   Ala Ile Pro Ser Ser Pro Pro Ile Val Leu Asn Glu Ala Val Thr Ile
                   165                 170                 175
   gct gag gtt aaa cta tac ggc gat gtt gtt ctc cga tat gtt agt tac    576
   Ala Glu Val Lys Leu Tyr Gly Asp Val Val Leu Arg Tyr Val Ser Tyr
               180                 185                 190
   aaa gca gaa gat acc gaa aaa tcc gaa ttc ttg cca ggg ttc gag cgt    624
   Lys Ala Glu Asp Thr Glu Lys Ser Glu Phe Leu Pro Gly Phe Glu Arg
           195                 200                 205
   gta gag gat gcg tcg tcg ttc cca ttg gat tat ggt atc cgg cgg ctt    672
   Val Glu Asp Ala Ser Ser Phe Pro Leu Asp Tyr Gly Ile Arg Arg Leu
       210                 215                 220
   gac cac gcc gtg gga aac gtt cct gag ctt ggt ccg gct tta act tat    720
   Asp His Ala Val Gly Asn Val Pro Glu Leu Gly Pro Ala Leu Thr Tyr
   225                 230                 235                 240
   gta gcg ggg ttc act ggt ttt cac caa ttc gca gag ttc aca gca gac    768
   Val Ala Gly Phe Thr Gly Phe His Gln Phe Ala Glu Phe Thr Ala Asp
                   245                 250                 255
   gac gtt gga acc gcc gag agc ggt tta aat tca gcg gtc ctg gct agc    816
   Asp Val Gly Thr Ala Glu Ser Gly Leu Asn Ser Ala Val Leu Ala Ser
               260                 265                 270
   aat gat gaa atg gtt ctt cta ccg att aac gag cca gtg cac gga aca    864
   Asn Asp Glu Met Val Leu Leu Pro Ile Asn Glu Pro Val His Gly Thr
           275                 280                 285
   aag agg aag agt cag att cag acg tat ttg gaa cat aac gaa ggc gca    912
   Lys Arg Lys Ser Gln Ile Gln Thr Tyr Leu Glu His Asn Glu Gly Ala
       290                 295                 300
   ggg cta caa cat ctg gct ctg atg agt gaa gac ata ttc agg acc ctg    960
   Gly Leu Gln His Leu Ala Leu Met Ser Glu Asp Ile Phe Arg Thr Leu
   305                 310                 315                 320
   aga gag atg agg aag agg agc agt att gga gga ttc gac ttc atg cct    1008
   Arg Glu Met Arg Lys Arg Ser Ser Ile Gly Gly Phe Asp Phe Met Pro
                   325                 330                 335
   tct cct ccg cct act tac tac cag aat ctc aag aaa cgg gtc ggc gac    1056
   Ser Pro Pro Pro Thr Tyr Tyr Gln Asn Leu Lys Lys Arg Val Gly Asp
               340                 345                 350
   gtg ctc agc gat gat cag atc aag gag tgt gag gaa tta ggg att ctt    1104
   Val Leu Ser Asp Asp Gln Ile Lys Glu Cys Glu Glu Leu Gly Ile Leu
               355                 360                 365
   gta gac aga gat gat caa ggg acg ttg ctt caa atc ttc aca aaa cca    1152
   Val Asp Arg Asp Asp Gln Gly Thr Leu Leu Gln Ile Phe Thr Lys Pro
           370                 375                 380
   cta ggt gac agg ccg acg ata ttt ata gag ata atc cag aga gta gga    1200
   Leu Gly Asp Arg Pro Thr Ile Phe Ile Glu Ile Ile Gln Arg Val Gly
   385                 390                 395                 400
   tgc atg atg aaa gat gag gaa ggg aag gct tac cag agt gga gga tgt    1248
   Cys Met Met Lys Asp Glu Glu Gly Lys Ala Tyr Gln Ser Gly Gly Cys
                   405                 410                 415
   ggt ggt ttt ggc aaa ggc aat ttc tct gag ctc ttc aag tcc att gaa    1296
   Gly Gly Phe Gly Lys Gly Asn Phe Ser Glu Leu Phe Lys Ser Ile Glu
               420                 425                 430
   gaa tac gaa aag act ctt gaa gcc aaa cag tta gtg gga tga            1338
   Glu Tyr Glu Lys Thr Leu Glu Ala Lys Gln Leu Val Gly
           435                 440                 445
<210> 14
   <211> 445.
   <212> PRT
   <213> Arabidopsis thaliana
<400> 14
   Met Gly His Gln Asn Ala Ala Val Ser Glu Asn Gln Asn His Asp Asp
   1               5                   10                  15
   Gly Ala Ala Ser Ser Pro Gly Phe Lys Leu Val Gly Phe Ser Lys Phe
               20                  25                  30
   Val Arg Lys Asn Pro Lys Ser Asp Lys Phe Lys Val Lys Arg Phe His
           35                  40                  45
   His Ile Glu Phe Trp Cys Gly Asp Ala Thr Asn Val Ala Arg Arg Phe
       50                  55                  60
   Ser Trp Gly Leu Gly Met Arg Phe Ser Ala Lys Ser Asp Leu Ser Thr
   65                  70                  75                  80
   Gly Asn Met Val His Ala Ser Tyr Leu Leu Thr Ser Gly Asp Leu Arg
                   85                  90                  95
   Phe Leu Phe Thr Ala Pro Tyr Ser Pro Ser Leu Ser Ala Gly Glu Ile
               100                 105                 110
   Lys Pro Thr Thr Thr Ala Ser Ile Pro Ser Phe Asp His Gly Ser Cys
   115 120 125
   Arg Ser Phe Phe Ser Ser His Gly Leu Gly Val Arg Ala Val Ala Ile
   130 135 140
   Glu Val Glu Asp Ala Glu Ser Ala Phe Ser Ile Ser Val Ala Asn Gly
   145 150 155 160
   Ala Ile Pro Ser Ser Pro Pro Ile Val Leu Asn Glu Ala Val Thr Ile
   165 170 175
   Ala Glu Val Lys Leu Tyr Gly Asp Val Val Leu Arg Tyr Val Ser Tyr
   180 185 190
   Lys Ala Glu Asp Thr Glu Lys Ser Glu Phe Leu Pro Gly Phe Glu Arg
   195 200 205
   Val Glu Asp Ala Ser Ser Phe Pro Leu Asp Tyr Gly Ile Arg Arg Leu
   210 215 220
   Asp His Ala Val Gly Asn Val Pro Glu Leu Gly Pro Ala Leu Thr Tyr
   225 230 235 240
   Val Ala Gly Phe Thr Gly Phe His Gln Phe Ala Glu Phe Thr Ala Asp
   245 250 255
   Asp Val Gly Thr Ala Glu Ser Gly Leu Asn Ser Ala Val Leu Ala Ser
   260 265 270
   Asn Asp Glu Met Val Leu Leu Pro Ile Asn Glu Pro Val His Gly Thr
   275 280 285
   Lys Arg Lys Ser Gln Ile Gln Thr Tyr Leu Glu His Asn Glu Gly Ala
   290 295 300
   Gly Leu Gln His Leu Ala Leu Met Ser Glu Asp Ile Phe Arg Thr Leu
   305 310 315 320
   Arg Glu Met Arg Lys Arg Ser Ser Ile Gly Gly Phe Asp Phe Met Pro
   325 330 335
   Ser Pro Pro Pro Thr Tyr Tyr Gln Asn Leu Lys Lys Arg Val Gly Asp
   340 345 350
   Val Leu Ser Asp Asp Gln Ile Lys Glu Cys Glu Glu Leu Gly Ile Leu
   355 360 365
   Val Asp Arg Asp Asp Gln Gly Thr Leu Leu Gln Ile Phe Thr Lys Pro
   370 375 380
   Leu Gly Asp Arg Pro Thr Ile Phe Ile Glu Ile Ile Gln Arg Val Gly
   385 390 395 400
   Cys Met Met Lys Asp Glu Glu Gly Lys Ala Tyr Gln Ser Gly Gly Cys
   405 410 415
   Gly Gly Phe Gly Lys Gly Asn Phe Ser Glu Leu Phe Lys Ser Ile Glu
   420 425 430
   Glu Tyr Glu Lys Thr Leu Glu Ala Lys Gln Leu Val Gly
   435 440 445
<210> 15
   <211> 1182
   <212> DNA
   <213> Arabidopsis thaliana
<220>
   <221> CDS
   <222> (1)..(1182)
<400> 15
   atg gag tct ctg ctc tct agt tct tct ctt gtt tcc gct gct ggt ggg 48
   Met Glu Ser Leu Leu Ser Ser Ser Ser Leu Val Ser Ala Ala Gly Gly
   1 5 10 15
   ttt tgt tgg aag aag cag aat cta aag ctc cac tct tta tca gaa atc 96
   Phe Cys Trp Lys Lys Gln Asn Leu Lys Leu His Ser Leu Ser Glu Ile
   20 25 30
   cga gtt ctg cgt tgt gat tcg agt aaa gtt gtc gca aaa ccg aag ttt 144
   Arg Val Leu Arg Cys Asp Ser Ser Lys Val Val Ala Lys Pro Lys Phe
   35 40 45
   agg aac aat ctt gtt agg cct gat ggt caa gga tct tca ttg ttg ttg 192
   Arg Asn Asn Leu Val Arg Pro Asp Gly Gln Gly Ser Ser Leu Leu Leu
   50 55 60
   tat cca aaa cat aag tcg aga ttt cgg gtt aat gcc act gcg ggt cag 240
   Tyr Pro Lys His Lys Ser Arg Phe Arg Val Asn Ala Thr Ala Gly Gln
   65 70 75 80
   ccc gag gct ttc gac tcg aat agc aaa cag aag tct ttt aga gac tcg 288
   Pro Glu Ala Phe Asp Ser Asn Ser Lys Gln Lys Ser Phe Arg Asp Ser
   85 90 95
   tta gat gcg ttt tac agg ttt tct agg cct cat aca gtt att ggc aca 336
   Leu Asp Ala Phe Tyr Arg Phe Ser Arg Pro His Thr Val Ile Gly Thr
                   100                     105                     110
   gtg ctt agc att tta tct gta tct ttc tta gca gta gag aag gtt tct     384
   Val Leu Ser Ile Leu Ser Val Ser Phe Leu Ala Val Glu Lys Val Ser
           115                     120                     125
   gat ata tct cct tta ctt ttc act ggc atc ttg gag gct gtt gtt gca     432
   Asp Ile Ser Pro Leu Leu Phe Thr Gly Ile Leu Glu Ala Val Val Ala
       130                     135                     140
   gct ctc atg atg aac att tac ata gtt ggg cta aat cag ttg tct gat     480
   Ala Leu Met Met Asn Ile Tyr Ile Val Gly Leu Asn Gln Leu Ser Asp
   145                     150                     155                 160
   gtt gaa ata gat aag gtt aac aag ccc tat ctt cca ttg gca tca gga     528
   Val Glu Ile Asp Lys Val Asn Lys Pro Tyr Leu Pro Leu Ala Ser Gly
                   165                     170                     175
   gaa tat tct gtt aac acc ggc att gca ata gta gct tcc ttc tcc atc     576
   Glu Tyr Ser Val Asn Thr Gly Ile Ala Ile Val Ala Ser Phe Ser Ile
               180                     185                     190
   atg agt ttc tgg ctt ggg tgg att gtt ggt tca tgg cca ttg ttc tgg     624
   Met Ser Phe Trp Leu Gly Trp Ile Val Gly Ser Trp Pro Leu Phe Trp
           195                     200                     205
   gct ctt ttt gtg agt ttc atg ctc ggt act gca tac tct atc aat ttg     672
   Ala Leu Phe Val Ser Phe Met Leu Gly Thr Ala Tyr Ser Ile Asn Leu
       210                     215                     220
   cca ctt tta cgg tgg aaa aga ttt gca ttg gtt gca gca atg tgt atc     720
   Pro Leu Leu Arg Trp Lys Arg Phe Ala Leu Val Ala Ala Met Cys Ile
   225                     230                     235                 240
   ctc gct gtc cga gct att att gtt caa atc gcc ttt tat cta cat att     768
   Leu Ala Val Arg Ala Ile Ile Val Gln Ile Ala Phe Tyr Leu His Ile
                   245                     250                     255
   cag aca cat gtg ttt gga aga cca atc ttg ttc act agg cct ctt att     816
   Gln Thr His Val Phe Gly Arg Pro Ile Leu Phe Thr Arg Pro Leu Ile
               260                     265                     270
   ttc gcc act gcg ttt atg agc ttt ttc tct gtc gtt att gca ttg ttt     864
   Phe Ala Thr Ala Phe Met Ser Phe Phe Ser Val Val Ile Ala Leu Phe
           275                     280                     285
   aag gat ata cct gat atc gaa ggg gat aag ata ttc gga atc cga tca     912
   Lys Asp Ile Pro Asp Ile Glu Gly Asp Lys Ile Phe Gly Ile Arg Ser
       290                     295                     300
   ttc tct gta act ctg ggt cag aaa cgg gtg ttt tgg aca tgt gtt aca    960
   Phe Ser Val Thr Leu Gly Gln Lys Arg Val Phe Trp Thr Cys Val Thr
   305                 310                 315                 320
   cta ctt caa atg gct tac gct gtt gca att cta gtt gga gcc aca tct    1008
   Leu Leu Gln Met Ala Tyr Ala Val Ala Ile Leu Val Gly Ala Thr Ser
                   325                 330                 335
   cca ttc ata tgg agc aaa gtc atc tcg gtt gtg ggt cat gtt ata ctc    1056
   Pro Phe Ile Trp Ser Lys Val Ile Ser Val Val Gly His Val Ile Leu
               340                 345                 350
   gca aca act ttg tgg gct cga gct aag tcc gtt gat ctg agt agc aaa    1104
   Ala Thr Thr Leu Trp Ala Arg Ala Lys Ser Val Asp Leu Ser Ser Lys
           355                 360                 365
   acc gaa ata act tca tgt tat atg ttc ata tgg aag ctc ttt tat gca    1152
   Thr Glu Ile Thr Ser Cys Tyr Met Phe Ile Trp Lys Leu Phe Tyr Ala
       370                 375                 380
   gag tac ttg ctg tta cct ttt ttg aag tga                            1182
   Glu Tyr Leu Leu Leu Pro Phe Leu Lys
   385                 390
<210> 16
   <211> 393
   <212> PRT
   <213> Arabidopsis thaliana
<400> 16
   Met Glu Ser Leu Leu Ser Ser Ser Ser Leu Val Ser Ala Ala Gly Gly
     1               5                  10                  15
   Phe Cys Trp Lys Lys Gln Asn Leu Lys Leu His Ser Leu Ser Glu Ile
               20                  25                  30
   Arg Val Leu Arg Cys Asp Ser Ser Lys Val Val Ala Lys Pro Lys Phe
           35                  40                  45
   Arg Asn Asn Leu Val Arg Pro Asp Gly Gln Gly Ser Ser Leu Leu Leu
       50                  55                  60
   Tyr Pro Lys His Lys Ser Arg Phe Arg Val Asn Ala Thr Ala Gly Gln
    65                  70                  75                  80
   Pro Glu Ala Phe Asp Ser Asn Ser Lys Gln Lys Ser Phe Arg Asp Ser
                    85                  90                  95
   Leu Asp Ala Phe Tyr Arg Phe Ser Arg Pro His Thr Val Ile Gly Thr
   100 105 110
   Val Leu Ser Ile Leu Ser Val Ser Phe Leu Ala Val Glu Lys Val Ser
   115 120 125
   Asp Ile Ser Pro Leu Leu Phe Thr Gly Ile Leu Glu Ala Val Val Ala
   130 135 140
   Ala Leu Met Met Asn Ile Tyr Ile Val Gly Leu Asn Gln Leu Ser Asp
   145 150 155 160
   Val Glu Ile Asp Lys Val Asn Lys Pro Tyr Leu Pro Leu Ala Ser Gly
   165 170 175
   Glu Tyr Ser Val Asn Thr Gly Ile Ala Ile Val Ala Ser Phe Ser Ile
   180 185 190
   Met Ser Phe Trp Leu Gly Trp Ile Val Gly Ser Trp Pro Leu Phe Trp
   195 200 205
   Ala Leu Phe Val Ser Phe Met Leu Gly Thr Ala Tyr Ser Ile Asn Leu
   210 215 220
   Pro Leu Leu Arg Trp Lys Arg Phe Ala Leu Val Ala Ala Met Cys Ile
   225 230 235 240
   Leu Ala Val Arg Ala Ile Ile Val Gln Ile Ala Phe Tyr Leu His Ile
   245 250 255
   Gln Thr His Val Phe Gly Arg Pro Ile Leu Phe Thr Arg Pro Leu Ile
   260 265 270
   Phe Ala Thr Ala Phe Met Ser Phe Phe Ser Val Val Ile Ala Leu Phe
   275 280 285
   Lys Asp Ile Pro Asp Ile Glu Gly Asp Lys Ile Phe Gly Ile Arg Ser
   290 295 300
   Phe Ser Val Thr Leu Gly Gln Lys Arg Val Phe Trp Thr Cys Val Thr
   305 310 315 320
   Leu Leu Gln Met Ala Tyr Ala Val Ala Ile Leu Val Gly Ala Thr Ser
   325 330 335
   Pro Phe Ile Trp Ser Lys Val Ile Ser Val Val Gly His Val Ile Leu
   340 345 350
   Ala Thr Thr Leu Trp Ala Arg Ala Lys Ser Val Asp Leu Ser Ser Lys
   355 360 365
   Thr Glu Ile Thr Ser Cys Tyr Met Phe Ile Trp Lys Leu Phe Tyr Ala
   370 375 380
   Glu Tyr Leu Leu Leu Pro Phe Leu Lys
   385 390
<210> 17
   <211> 1509
   <212> DNA
   <213> Nicotiana tabacum
<220>
   <221> CDS
   <222> (1)..(1395)
<400> 17
<210> 18
   <211> 464
   <212> PRT
   <213> Nicotiana tabacum
<400> 18
   Met Ala Ser Ile Ala Leu Lys Thr Phe Thr Gly Leu Arg Gln Ser Ser
   1 5 10 15
   Pro Glu Asn Asn Ser Ile Thr Leu Ser Lys Ser Leu Pro Phe Thr Gln
               20                  25                  30
   Thr His Arg Arg Leu Arg Ile Asn Ala Ser Lys Ser Ser Pro Arg Val
           35                  40                  45
   Asn Gly Arg Asn Leu Arg Val Ala Val Val Gly Gly Gly Pro Ala Gly
       50                  55                  60
   Gly Ala Ala Ala Glu Thr Leu Ala Lys Gly Gly Ile Glu Thr Phe Leu
   65                  70                  75                  80
   Ile Glu Arg Lys Met Asp Asn Cys Lys Pro Cys Gly Gly Ala Ile Pro
                   85                  90                  95
   Leu Cys Met Val Gly Glu Phe Asp Leu Pro Leu Asp Ile Ile Asp Arg
               100                 105                 110
   Lys Val Thr Lys Met Lys Met Ile Ser Pro Ser Asn Val Ala Val Asp
           115                 120                 125
   Ile Gly Gln Thr Leu Lys Pro His Glu Tyr Ile Gly Met Val Arg Arg
       130                 135                 140
   Glu Val Leu Asp Ala Tyr Leu Arg Asp Arg Ala Ala Glu Ala Gly Ala
   145                 150                 155                 160
   Ser Val Leu Asn Gly Leu Phe Leu Lys Met Asp Met Pro Lys Ala Pro
                   165                 170                 175
   Asn Ala Pro Tyr Val Leu His Tyr Thr Ala Tyr Asp Ser Lys Thr Asn
               180                 185                 190
   Gly Ala Gly Glu Lys Arg Thr Leu Glu Val Asp Ala Val Ile Gly Ala
           195                 200                 205
   Asp Gly Ala Asn Ser Arg Val Ala Lys Ser Ile Asn Ala Gly Asp Tyr
       210                 215                 220
   Glu Tyr Ala Ile Ala Phe Gln Glu Arg Ile Lys Ile Ser Asp Asp Lys
   225                 230                 235                 240
   Met Lys Tyr Tyr Glu Asn Leu Ala Glu Met Tyr Val Gly Asp Asp Val
                   245                 250                 255
   Ser Pro Asp Phe Tyr Gly Trp Val Phe Pro Lys Cys Asp His Val Ala
               260                 265                 270
   Val Gly Thr Gly Thr Val Thr His Lys Ala Asp Ile Lys Lys Phe Gln
   275 280 285
   Leu Ala Thr Arg Leu Arg Ala Asp Ser Lys Ile Thr Gly Gly Lys Ile
   290 295 300
   Ile Arg Val Glu Ala His Pro Ile Pro Glu His Pro Arg Pro Arg Arg
   305 310 315 320
   Leu Gln Asp Arg Val Ala Leu Val Gly Asp Ala Ala Gly Tyr Val Thr
   325 330 335
   Lys Cys Ser Gly Glu Gly Ile Tyr Phe Ala Ala Lys Ser Gly Arg Met
   340 345 350
   Cys Ala Glu Ala Ile Val Glu Gly Ser Glu Met Gly Lys Arg Met Val
   355 360 365
   Asp Glu Ser Asp Leu Arg Lys Tyr Leu Glu Lys Trp Asp Lys Thr Tyr
   370 375 380
   Trp Pro Thr Tyr Lys Val Leu Asp Ile Leu Gln Lys Val Phe Tyr Arg
   385 390 395 400
   Ser Asn Pro Ala Arg Glu Ala Phe Val Glu Met Cys Ala Asp Glu Tyr
   405 410 415
   Val Gln Lys Met Thr Phe Asp Ser Tyr Leu Tyr Lys Lys Val Ala Pro
   420 425 430
   Gly Asn Pro Ile Glu Asp Leu Lys Leu Ala Val Asn Thr Ile Gly Ser
   435 440 445
   Leu Val Arg Ala Asn Ala Leu Arg Arg Glu Met Asp Lys Leu Ser Val
   450 455 460
<210> 19
   <211> 957
   <212> DNA
   <213> Synechocystis PCC6803
<220>
   <221> CDS
   <222> (1)..(957)
<400> 19
   atg ccc gag tat ttg ctt ctg ccc gct ggc cta att tcc ctc tcc ctg 48
   Met Pro Glu Tyr Leu Leu Leu Pro Ala Gly Leu Ile Ser Leu Ser Leu
   1 5 10 15
   gcg atc gcc gct gga ctg tat ctc cta act gcc cgg ggc tat cag tca     96
   Ala Ile Ala Ala Gly Leu Tyr Leu Leu Thr Ala Arg Gly Tyr Gln Ser
               20                  25                  30
   tcg gat tcc gtg gcc aac gcc tac gac caa tgg aca gag gac ggc att    144
   Ser Asp Ser Val Ala Asn Ala Tyr Asp Gln Trp Thr Glu Asp Gly Ile
           35                  40                  45
   ttg gaa tat tac tgg ggc gac cat atc cac ctc ggc cat tat ggc gat    192
   Leu Glu Tyr Tyr Trp Gly Asp His Ile His Leu Gly His Tyr Gly Asp
       50                  55                  60
   ccg cca gtg gcc aag gat ttc atc caa tcg aaa att gat ttt gtc cat    240
   Pro Pro Val Ala Lys Asp Phe Ile Gln Ser Lys Ile Asp Phe Val His
   65                  70                  75                  80
   gcc atg gcc cag tgg ggc gga tta gat aca ctt ccc ccc ggc aca acg    288
   Ala Met Ala Gln Trp Gly Gly Leu Asp Thr Leu Pro Pro Gly Thr Thr
                   85                  90                  95
   gta ttg gat gtg ggt tgc ggc att ggc ggt agc agt cgc att ctc gcc    336
   Val Leu Asp Val Gly Cys Gly Ile Gly Gly Ser Ser Arg Ile Leu Ala
               100                 105                 110
   aaa gat tat ggt ttt aac gtt acc ggc atc acc att agt ccc caa cag    384
   Lys Asp Tyr Gly Phe Asn Val Thr Gly Ile Thr Ile Ser Pro Gln Gln
           115                 120                 125
   gtg aaa cgg gcg acg gaa tta act cct ccc gat gtg acg gcc aag ttt    432
   Val Lys Arg Ala Thr Glu Leu Thr Pro Pro Asp Val Thr Ala Lys Phe
       130                 135                 140
   gcg gtg gac gat gct atg gct ttg tct ttt cct gac ggt agt ttc gac    480
   Ala Val Asp Asp Ala Met Ala Leu Ser Phe Pro Asp Gly Ser Phe Asp
   145                 150                 155                 160
   gta gtt tgg tcg gtg gaa gca ggg ccc cac atg cct gac aaa gct gtg    528
   Val Val Trp Ser Val Glu Ala Gly Pro His Met Pro Asp Lys Ala Val
                   165                 170                 175
   ttt gcc aag gaa tta ctg cgg gtc gtg aaa cca ggg ggc att ctg gtg    576
   Phe Ala Lys Glu Leu Leu Arg Val Val Lys Pro Gly Gly Ile Leu Val
               180                 185                 190
   gtg gcg gat tgg aat caa cgg gac gat cgc caa gtg ccc ctc aac ttc    624
   Val Ala Asp Trp Asn Gln Arg Asp Asp Arg Gln Val Pro Leu Asn Phe
           195                 200                 205
   tgg gaa aaa cca gtg atg cga caa ctg ttg gat caa tgg tcc cac cct    672
   Trp Glu Lys Pro Val Met Arg Gln Leu Leu Asp Gln Trp Ser His Pro
       210                 215                 220
   gcc ttt gcc agc att gaa ggt ttt gcg gaa aat ttg gaa gcc acg ggt     720
   Ala Phe Ala Ser Ile Glu Gly Phe Ala Glu Asn Leu Glu Ala Thr Gly
   225                 230                 235                 240
   ttg gtg gag ggc cag gtg act act gct gat tgg act gta ccg acc ctc     768
   Leu Val Glu Gly Gln Val Thr Thr Ala Asp Trp Thr Val Pro Thr Leu
                   245                 250                 255
   ccc gct tgg ttg gat acc att tgg cag ggc att atc cgg ccc cag ggc     816
   Pro Ala Trp Leu Asp Thr Ile Trp Gln Gly Ile Ile Arg Pro Gln Gly
               260                 265                 270
   tgg tta caa tac ggc att cgt ggg ttt atc aaa tcc gtg cgg gaa gta     864
   Trp Leu Gln Tyr Gly Ile Arg Gly Phe Ile Lys Ser Val Arg Glu Val
           275                 280                 285
   ccg act att tta ttg atg cgc ctt gcc ttt ggg gta gga ctt tgt cgc     912
   Pro Thr Ile Leu Leu Met Arg Leu Ala Phe Gly Val Gly Leu Cys Arg
       290                 295                 300
   ttc ggt atg ttc aaa gca gtg cga aaa aac gcc act caa gct taa         957
   Phe Gly Met Phe Lys Ala Val Arg Lys Asn Ala Thr Gln Ala
   305                 310                 315
<210> 20
   <211> 318
   <212> PRT
   <213> Synechocystis PCC6803
<400> 20
   Met Pro Glu Tyr Leu Leu Leu Pro Ala Gly Leu Ile Ser Leu Ser Leu
    1               5                  10                  15
   Ala Ile Ala Ala Gly Leu Tyr Leu Leu Thr Ala Arg Gly Tyr Gln Ser
               20                  25                  30
   Ser Asp Ser Val Ala Asn Ala Tyr Asp Gln Trp Thr Glu Asp Gly Ile
           35                  40                  45
   Leu Glu Tyr Tyr Trp Gly Asp His Ile His Leu Gly His Tyr Gly Asp
       50                  55                  60
   Pro Pro Val Ala Lys Asp Phe Ile Gln Ser Lys Ile Asp Phe Val His
   65                  70                  75                  80
   Ala Met Ala Gln Trp Gly Gly Leu Asp Thr Leu Pro Pro Gly Thr Thr
                   85                  90                  95
   Val Leu Asp Val Gly Cys Gly Ile Gly Gly Ser Ser Arg Ile Leu Ala
               100                 105                 110
   Lys Asp Tyr Gly Phe Asn Val Thr Gly Ile Thr Ile Ser Pro Gln Gln
           115                 120                 125
   Val Lys Arg Ala Thr Glu Leu Thr Pro Pro Asp Val Thr Ala Lys Phe
       130                 135                 140
   Ala Val Asp Asp Ala Met Ala Leu Ser Phe Pro Asp Gly Ser Phe Asp
   145                 150                 155                 160
   Val Val Trp Ser Val Glu Ala Gly Pro His Met Pro Asp Lys Ala Val
                   165                 170                 175
   Phe Ala Lys Glu Leu Leu Arg Val Val Lys Pro Gly Gly Ile Leu Val
               180                 185                 190
   Val Ala Asp Trp Asn Gln Arg Asp Asp Arg Gln Val Pro Leu Asn Phe
           195                 200                 205
   Trp Glu Lys Pro Val Met Arg Gln Leu Leu Asp Gln Trp Ser His Pro
       210                 215                 220
   Ala Phe Ala Ser Ile Glu Gly Phe Ala Glu Asn Leu Glu Ala Thr Gly
   225                 230                 235                 240
   Leu Val Glu Gly Gln Val Thr Thr Ala Asp Trp Thr Val Pro Thr Leu
                   245                 250                 255
   Pro Ala Trp Leu Asp Thr Ile Trp Gln Gly Ile Ile Arg Pro Gln Gly
               260                 265                 270
   Trp Leu Gln Tyr Gly Ile Arg Gly Phe Ile Lys Ser Val Arg Glu Val
           275                 280                 285
   Pro Thr Ile Leu Leu Met Arg Leu Ala Phe Gly Val Gly Leu Cys Arg
       290                 295                 300
   Phe Gly Met Phe Lys Ala Val Arg Lys Asn Ala Thr Gln Ala
   305                 310                 315
<210> 21
   <211> 1100
   <212> DNA
   <213> Synechocystis PCC6803
<220>
   <221> CDS
   <222> (1)..(1092)
<400> 21
   atg aaa ttt ccg ccc cac agt ggt tac cat tgg caa ggt caa tca cct    48
   Met Lys Phe Pro Pro His Ser Gly Tyr His Trp Gln Gly Gln Ser Pro
    1               5                  10                  15
   ttc ttt gaa ggt tgg tac gtg cgc ctg ctt ttg ccc caa tcc ggg gaa    96
   Phe Phe Glu Gly Trp Tyr Val Arg Leu Leu Leu Pro Gln Ser Gly Glu
               20                  25                  30
   agt ttt gct ttt atg tac tcc atc gaa aat cct gct agc gat cat cat   144
   Ser Phe Ala Phe Met Tyr Ser Ile Glu Asn Pro Ala Ser Asp His His
           35                  40                  45
   tac ggc ggc ggt gct gtg caa att tta ggg ccg gct acg aaa aaa caa   192
   Tyr Gly Gly Gly Ala Val Gln Ile Leu Gly Pro Ala Thr Lys Lys Gln
       50                  55                  60
   gaa aat cag gaa gac caa ctt gtt tgg cgg aca ttt ccc tcg gta aaa   240
   Glu Asn Gln Glu Asp Gln Leu Val Trp Arg Thr Phe Pro Ser Val Lys
    65                  70                  75                  80
   aaa ttt tgg gcc agt cct cgc cag ttt gcc cta ggg cat tgg gga aaa   288
   Lys Phe Trp Ala Ser Pro Arg Gln Phe Ala Leu Gly His Trp Gly Lys
                   85                  90                  95
   tgt agg gat aac agg cag gcg aaa ccc cta ctc tcc gaa gaa ttt ttt   336
   Cys Arg Asp Asn Arg Gln Ala Lys Pro Leu Leu Ser Glu Glu Phe Phe
               100                 105                 110
   gcc acg gtc aag gaa ggt tat caa atc cat caa aat cag cac caa gga   384
   Ala Thr Val Lys Glu Gly Tyr Gln Ile His Gln Asn Gln His Gln Gly
           115                 120                 125
   caa atc att cat ggc gat cgc cat tgt cgt tgg cag ttc acc gta gaa   432
   Gln Ile Ile His Gly Asp Arg His Cys Arg Trp Gln Phe Thr Val Glu
       130                 135                 140
   ccg gaa gta act tgg ggg agt cct aac cga ttt cct cgg gct aca gcg   480
   Pro Glu Val Thr Trp Gly Ser Pro Asn Arg Phe Pro Arg Ala Thr Ala
   145                 150                 155                 160
   ggt tgg ctt tcc ttt tta ccc ttg ttt gat ccc ggt tgg caa att ctt   528
   Gly Trp Leu Ser Phe Leu Pro Leu Phe Asp Pro Gly Trp Gln Ile Leu
                   165                 170                 175
   tta gcc caa ggt aga gcg cac ggc tgg ctg aaa tgg cag agg gaa cag   576
   Leu Ala Gln Gly Arg Ala His Gly Trp Leu Lys Trp Gln Arg Glu Gln
                   180                 185                 190
   tat gaa ttt gac cac gcc cta gtt tat gcc gaa aaa aat tgg ggt cac      624
   Tyr Glu Phe Asp His Ala Leu Val Tyr Ala Glu Lys Asn Trp Gly His
           195                 200                 205
   tcc ttt ccc tcc cgc tgg ttt tgg ctc caa gca aat tat ttt cct gac      672
   Ser Phe Pro Ser Arg Trp Phe Trp Leu Gln Ala Asn Tyr Phe Pro Asp
       210                 215                 220
   cat cca gga ctg agc gtc act gcc gct ggc ggg gaa cgg att gtt ctt      720
   His Pro Gly Leu Ser Val Thr Ala Ala Gly Gly Glu Arg Ile Val Leu
   225                 230                 235                 240
   ggt cgc ccc gaa gag gta gct tta att ggc tta cat cac caa ggt aat      768
   Gly Arg Pro Glu Glu Val Ala Leu Ile Gly Leu His His Gln Gly Asn
                   245                 250                 255
   ttt tac gaa ttt ggc ccg ggc cat ggc aca gtc act tgg caa gta gct      816
   Phe Tyr Glu Phe Gly Pro Gly His Gly Thr Val Thr Trp Gln Val Ala
               260                 265                 270
   ccc tgg ggc cgt tgg caa tta aaa gcc agc aat gat agg tat tgg gtc      864
   Pro Trp Gly Arg Trp Gln Leu Lys Ala Ser Asn Asp Arg Tyr Trp Val
           275                 280                 285
   aag ttg tcc gga aaa aca gat aaa aaa ggc agt tta gtc cac act ccc      912
   Lys Leu Ser Gly Lys Thr Asp Lys Lys Gly Ser Leu Val His Thr Pro
       290                 295                 300
   acc gcc cag ggc tta caa ctc aac tgc cga gat acc act agg ggc tat      960
   Thr Ala Gln Gly Leu Gln Leu Asn Cys Arg Asp Thr Thr Arg Gly Tyr
   305                 310                 315                 320
   ttg tat ttg caa ttg gga tct gtg ggt cac ggc ctg ata gtg caa ggg     1008
   Leu Tyr Leu Gln Leu Gly Ser Val Gly His Gly Leu Ile Val Gln Gly
                   325                 330                 335
   gaa acg gac acc gcg ggg cta gaa gtt gga ggt gat tgg ggt tta aca     1056
   Glu Thr Asp Thr Ala Gly Leu Glu Val Gly Gly Asp Trp Gly Leu Thr
               340                 345                 350
   gag gaa aat ttg agc aaa aaa aca gtg cca ttc tga gggaataa            1100
   Glu Glu Asn Leu Ser Lys Lys Thr Val Pro Phe
           355                 360
<210> 22
   <211> 363
   <212> PRT
   <213> Synechocystis PCC6803
<400> 22
   Met Lys Phe Pro Pro His Ser Gly Tyr His Trp Gln Gly Gln Ser Pro
   1 5 10 15
   Phe Phe Glu Gly Trp Tyr Val Arg Leu Leu Leu Pro Gln Ser Gly Glu
   20 25 30
   Ser Phe Ala Phe Met Tyr Ser Ile Glu Asn Pro Ala Ser Asp His His
   35 40 45
   Tyr Gly Gly Gly Ala Val Gln Ile Leu Gly Pro Ala Thr Lys Lys Gln
   50 55 60
   Glu Asn Gln Glu Asp Gln Leu Val Trp Arg Thr Phe Pro Ser Val Lys
   65 70 75 80
   Lys Phe Trp Ala Ser Pro Arg Gln Phe Ala Leu Gly His Trp Gly Lys
   85 90 95
   Cys Arg Asp Asn Arg Gln Ala Lys Pro Leu Leu Ser Glu Glu Phe Phe
   100 105 110
   Ala Thr Val Lys Glu Gly Tyr Gln Ile His Gln Asn Gln His Gln Gly
   115 120 125
   Gln Ile Ile His Gly Asp Arg His Cys Arg Trp Gln Phe Thr Val Glu
   130 135 140
   Pro Glu Val Thr Trp Gly Ser Pro Asn Arg Phe Pro Arg Ala Thr Ala
   145 150 155 160
   Gly Trp Leu Ser Phe Leu Pro Leu Phe Asp Pro Gly Trp Gln Ile Leu
   165 170 175
   Leu Ala Gln Gly Arg Ala His Gly Trp Leu Lys Trp Gln Arg Glu Gln
   180 185 190
   Tyr Glu Phe Asp His Ala Leu Val Tyr Ala Glu Lys Asn Trp Gly His
   195 200 205
   Ser Phe Pro Ser Arg Trp Phe Trp Leu Gln Ala Asn Tyr Phe Pro Asp
   210 215 220
   His Pro Gly Leu Ser Val Thr Ala Ala Gly Gly Glu Arg Ile Val Leu
   225 230 235 240
   Gly Arg Pro Glu Glu Val Ala Leu Ile Gly Leu His His Gln Gly Asn
   245 250 255
   Phe Tyr Glu Phe Gly Pro Gly His Gly Thr Val Thr Trp Gln Val Ala
               260                 265                 270
   Pro Trp Gly Arg Trp Gln Leu Lys Ala Ser Asn Asp Arg Tyr Trp Val
           275                 280                 285
   Lys Leu Ser Gly Lys Thr Asp Lys Lys Gly Ser Leu Val His Thr Pro
       290                 295                 300
   Thr Ala Gln Gly Leu Gln Leu Asn Cys Arg Asp Thr Thr Arg Gly Tyr
   305                 310                 315                 320
   Leu Tyr Leu Gln Leu Gly Ser Val Gly His Gly Leu Ile Val Gln Gly
                   325                 330                 335
   Glu Thr Asp Thr Ala Gly Leu Glu Val Gly Gly Asp Trp Gly Leu Thr
               340                 345                 350
   Glu Glu Asn Leu Ser Lys Lys Thr Val Pro Phe
           355                 360
<210> 23
   <211> 1047
   <212> DNA
   <213> Arabidopsis thaliana
<220>
   <221> CDS
   <222> (1)..(1047)
<400> 23
   atg aaa gca act cta gca gca ccc tct tct ctc aca agc ctc cct tat      48
   Met Lys Ala Thr Leu Ala Ala Pro Ser Ser Leu Thr Ser Leu Pro Tyr
   1               5                   10                  15
   cga acc aac tct tct ttc ggc tca aag tca tcg ctt ctc ttt cgg tct      96
   Arg Thr Asn Ser Ser Phe Gly Ser Lys Ser Ser Leu Leu Phe Arg Ser
               20                  25                  30
   cca tcc tcc tcc tcc tca gtc tct atg acg aca acg cgt gga aac gtg      144
   Pro Ser Ser Ser Ser Ser Val Ser Met Thr Thr Thr Arg Gly Asn Val
           35                  40                  45
   gct gtg gcg gct gct gct aca tcc act gag gcg cta aga aaa gga ata      192
   Ala Val Ala Ala Ala Ala Thr Ser Thr Glu Ala Leu Arg Lys Gly Ile
       50                  55                  60
   gcg gag ttc tac aat gaa act tcg ggt ttg tgg gaa gag att tgg gga      240
   Ala Glu Phe Tyr Asn Glu Thr Ser Gly Leu Trp Glu Glu Ile Trp Gly
   65 70 75 80
   gat cat atg cat cat ggc ttt tgt gac cct gat tct tct gtt caa ctt 288
   Asp His Met His His Gly Phe Cys Asp Pro Asp Ser Ser Val Gln Leu
   85 90 95
   tct gat tct ggt cac aag gaa gct cag atc cgt atg att gaa gag tct 336
   Ser Asp Ser Gly His Lys Glu Ala Gln Ile Arg Met Ile Glu Glu Ser
   100 105 110
   ctc cgt ttt gcc ggt gtt act gat gaa gag gag gag aaa aag ata aag 384
   Leu Arg Phe Ala Gly Val Thr Asp Glu Glu Glu Glu Lys Lys Ile Lys
   115 120 125
   aaa gta gtg gat gtt ggg tgt ggg att gga gga agc tca aga tat ctt 432
   Lys Val Val Asp Val Gly Cys Gly Ile Gly Gly Ser Ser Arg Tyr Leu
   130 135 140
   gcc tct aaa ttt gga gct gaa tgc att ggc att act ctc agc cct gtt 480
   Ala Ser Lys Phe Gly Ala Glu Cys Ile Gly Ile Thr Leu Ser Pro Val
   145 150 155 160
   cag gcc aag aga gcc aat gat ctc gcg gct gct caa tca ctc gct cat 528
   Gln Ala Lys Arg Ala Asn Asp Leu Ala Ala Ala Gln Ser Leu Ala His
   165 170 175
   aag gct tcc ttc caa gtt gcg gat gcg ttg gat cag cca ttc gaa gat 576
   Lys Ala Ser Phe Gln Val Ala Asp Ala Leu Asp Gln Pro Phe Glu Asp
   180 185 190
   gga aaa ttc gat ata gtg tgg tcg atg gag agt ggt gag cat atg cct 624
   Gly Lys Phe Asp Ile Val Trp Ser Met Glu Ser Gly Glu His Met Pro
   195 200 205
   gac aag gcc aag ttt gta aaa gag ttg gta cgt gtg gcg gct cca gga 672
   Asp Lys Ala Lys Phe Val Lys Glu Leu Val Arg Val Ala Ala Pro Gly
   210 215 220
   ggt agg ata ata ata gtg aca tgg tgc cat aga aat cta tct gcg ggg 720
   Gly Arg Ile Ile Ile Val Thr Trp Cys His Arg Asn Leu Ser Ala Gly
   225 230 235 240
   gag gaa gct ttg cag ccg tgg gag caa aac atc ttg gac aaa atc cgt 768
   Glu Glu Ala Leu Gln Pro Trp Glu Gln Asn Ile Leu Asp Lys Ile Arg
   245 250 255
   aag acg ttc tat ctc ccg gct tgg tgc tcc acc gat gat tat gtc aac 816
   Lys Thr Phe Tyr Leu Pro Ala Trp Cys Ser Thr Asp Asp Tyr Val Asn
   260 265 270
   ttg ctt caa tcc cat tct ctc cag gat att aag tgt gcg gat tgg tca     864
   Leu Leu Gln Ser His Ser Leu Gln Asp Ile Lys Cys Ala Asp Trp Ser
           275                     280                     285
   gag aac gta gct cct ttc tgg cct gcg gtt ata cgg act gca tta aca     912
   Glu Asn Val Ala Pro Phe Trp Pro Ala Val Ile Arg Thr Ala Leu Thr
       290                     295                     300
   tgg aag ggc ctt gtg tct ctg ctt cgt agt ggt atg aaa agt att aaa     960
   Trp Lys Gly Leu Val Ser Leu Leu Arg Ser Gly Met Lys Ser Ile Lys
   305                     310                     315                 320
   gga gca ttg aca atg cca ttg atg att gaa ggt tac aag aaa ggt gtc     1008
   Gly Ala Leu Thr Met Pro Leu Met Ile Glu Gly Tyr Lys Lys Gly Val
                   325                     330                     335
   att aag ttt ggt atc atc act tgc cag aag cca ctc taa                 1047
   Ile Lys Phe Gly Ile Ile Thr Cys Gln Lys Pro Leu
               340                     345
<210> 24
   <211> 348
   <212> PRT
   <213> Arabidopsis thaliana
<400> 24
   Met Lys Ala Thr Leu Ala Ala Pro Ser Ser Leu Thr Ser Leu Pro Tyr
   1 5 10 15
   Arg Thr Asn Ser Ser Phe Gly Ser Lys Ser Ser Leu Leu Phe Arg Ser
   20 25 30
   Pro Ser Ser Ser Ser Ser Val Ser Met Thr Thr Thr Arg Gly Asn Val
   35 40 45
   Ala Val Ala Ala Ala Ala Thr Ser Thr Glu Ala Leu Arg Lys Gly Ile
   50 55 60
   Ala Glu Phe Tyr Asn Glu Thr Ser Gly Leu Trp Glu Glu Ile Trp Gly
   65 70 75 80
   Asp His Met His His Gly Phe Cys Asp Pro Asp Ser Ser Val Gln Leu
   85 90 95
   Ser Asp Ser Gly His Lys Glu Ala Gln Ile Arg Met Ile Glu Glu Ser
   100 105 110
   Leu Arg Phe Ala Gly Val Thr Asp Glu Glu Glu Glu Lys Lys Ile Lys
   115 120 125
   Lys Val Val Asp Val Gly Cys Gly Ile Gly Gly Ser Ser Arg Tyr Leu
   130 135 140
   Ala Ser Lys Phe Gly Ala Glu Cys Ile Gly Ile Thr Leu Ser Pro Val
   145 150 155 160
   Gln Ala Lys Arg Ala Asn Asp Leu Ala Ala Ala Gln Ser Leu Ala His
   165 170 175
   Lys Ala Ser Phe Gln Val Ala Asp Ala Leu Asp Gln Pro Phe Glu Asp
   180 185 190
   Gly Lys Phe Asp Ile Val Trp Ser Met Glu Ser Gly Glu His Met Pro
   195 200 205
   Asp Lys Ala Lys Phe Val Lys Glu Leu Val Arg Val Ala Ala Pro Gly
   210 215 220
   Gly Arg Ile Ile Ile Val Thr Trp Cys His Arg Asn Leu Ser Ala Gly
   225 230 235 240
   Glu Glu Ala Leu Gln Pro Trp Glu Gln Asn Ile Leu Asp Lys Ile Arg
   245 250 255
   Lys Thr Phe Tyr Leu Pro Ala Trp Cys Ser Thr Asp Asp Tyr Val Asn
   260 265 270
   Leu Leu Gln Ser His Ser Leu Gln Asp Ile Lys Cys Ala Asp Trp Ser
   275 280 285
   Glu Asn Val Ala Pro Phe Trp Pro Ala Val Ile Arg Thr Ala Leu Thr
   290 295 300
   Trp Lys Gly Leu Val Ser Leu Leu Arg Ser Gly Met Lys Ser Ile Lys
   305 310 315 320
   Gly Ala Leu Thr Met Pro Leu Met Ile Glu Gly Tyr Lys Lys Gly Val
   325 330 335
   Ile Lys Phe Gly Ile Ile Thr Cys Gln Lys Pro Leu
   340 345
<210> 25
   <211> 580
   <212> DNA
   <213> Brassica napus
<220>
   <221> misc_structure
   <222> (1)..(580)
<400> 25
   gtcgacgagc tcatggggcg aagggtcttg ctgcaccaag agattttctt gcaccaacgg 60
   catggtttga ggaagggcta cggcctgact acactattgt tcagaagttt ggcggtgaac 120
   tctttactgc taaacaagat ttctctccgt tcaatgtggt tgcctggcat ggcaattacg 180
   tgccttataa gtatgacctg cacaagttct gtccatacaa cactgtcctt gtagaccatg 240
   gagatccatc tgtaaataca gttctgacag caccaacgga taaacctggt gtggccttgc 300
   ttgattttgt catattccct cctcgttggt tggttgctga gcataccttt cgacctcctt 360
   actaccatcg taactgcatg agtgaattta tgggcctaat ctatggtgct tacgaggcca 420
   aagctgatgg atttctacct ggtggcgcaa gtcttcacag ttgtatgaca cctcatggtc 480
   cagatacaac cacatacgag gcgacgattg ctcgtgtaaa tgcaatggct ccttataagc 540
   tcacaggcac catggccttc atgtttgagg taccagtact 580
<210> 26
   <211> 30
   <212> DNA
   <213> Artificial Sequence
<220>
   <223> Die Beschreibung von Knstliche Sequenz:Primer
<220>
   <221> primer_bind
   <222> (1)..(30)
<400> 26
   gatatcatgg actcctacgt gattcagacg 30
<210> 27
   <211> 29
   <212> DNA
   <213> Artificial Sequence
<220>
   <223> Die Beschreibung von Knstliche Sequenz: Primer
<220>
   <221> primer_bind
   <222> (1)..(29)
<400> 27
   gatatcttat ttgtcacact cctcctggc 29
<210> 28
   <211> 24
   <212> DNA
   <213> Artificial Sequence
<220>
   <223> Die Beschreibung von Knstliche Sequenz:Primer
<220>
   <221> primer_bind
   <222> (1)..(24)
<400> 28
   gtcgacatgg caacccttaa gtgc 24
<210> 29
   <211> 25
   <212> DNA
   <213> Artificial Sequence
<220>
   <223> Die Beschreibung von Knstliche Sequenz: Primer
<220>
   <221> primer_bind
   <222> (1)..(25)
<400> 29
   gtcgacttac ttaacaccat tgacg 25
<210> 30
   <211> 24
   <212> DNA
   <213> Artificial Sequence
<220>
   <223> Die Beschreibung von Knstliche Sequenz: Primer
<220>
   <221> primer_bind
   <222> (1)..(24)
<400> 30
   gtcgacatgg cgagcaacgg agtt 24
<210> 31
   <211> 25
   <212> DNA
   <213> Artificial Sequence
<220>
   <223> Die Beschreibung von Knstliche Sequenz: Primer
<220>
   <221> primer_bind
   <222> (1)..(25)
<400> 31
   gtcgactcag ttgacagaga cgacg 25
<210> 32
   <211> 30
   <212> DNA
   <213> Artificial Sequence
<220>
   <223> Die Beschreibung von Knstliche Sequenz: Primer
<220>
   <221> primer_bind
   <222> (1).. (30)
<400> 32
   ggatccgatc catgagcgaa gaacaaccac 30
<210> 33
   <211> 27
   <212> DNA
   <213> Artificial Sequence
<220>
   <223> Die Beschreibung von Knstliche Sequenz: Primer
<220>
   <221> primer_bind
   <222> (1)..(27)
<400> 33
   ggatccttac atttcgagat tattatc 27
<210> 34
   <211> 27
   <212> DNA
   <213> Artificial Sequence
<220>
   <223> Die Beschreibung von Knstliche Sequenz: Primer
<220>
   <221> primer_bind
   <222> (1)..(27)
<400> 34
   agatctatgg agaatggagc aacgacg 27
<210> 35
   <211> 31
   <212> DNA
   <213> Artificial Sequence
<220>
   <223> Die Beschreibung von Knstliche Sequenz: Primer
<220>
   <221> primer_bind
   <222> (1)..(31)
<400> 35
   agatctatat ggttggatat tgagtcttgg c 31
<210> 36
   <211> 26
   <212> DNA
   <213> Artificial Sequence
<220>
   <223> Die Beschreibung von Knstliche Sequenz: Primer
<220>
   <221> primer_bind
   <222> (1)..(26)
<400> 36
   gcccgggcat ggcttccatt gctctc 26
<210> 37
   <211> 26
   <212> DNA
   <213> Artificial Sequence
<220>
   <223> Die Beschreibung von Knstliche Sequenz: Primer
<220>
   <221> primer_bind
   <222> (1)..(26)
<400> 37
   gcccgggcgc tcaaattatg aagtta 26
<210> 38
   <211> 24
   <212> DNA
   <213> Artificial Sequence
<220>
   <223> Die Beschreibung von Knstliche Sequenz: Primer
<220>
   <221> primer_bind
   <222> (1)..(24)
<400> 38
   ggatccatgg gccaccaaaa cgcc 24
<210> 39
   <211> 26
   <212> DNA
   <213> Artificial Sequence
<220>
   <223> Die Beschreibung von Knstliche Sequenz: Primer
<220>
   <221> primer_bind
   <222> (1)..(26)
<400> 39
   gtcgactcat cccactaact gtttgg 26
<210> 40
   <211> 26
   <212> DNA
   <213> Artificial Sequence
<220>
   <223> Die Beschreibung von Knstliche Sequenz: Primer
<220>
   <221> primer_bind
   <222> (1)..(26)
<400> 40
   ggatccatgg agtctctgct ctctag 26
<210> 41
   <211> 32
   <212> DNA
   <213> Artificial Sequence
<220>
   <223> Die Beschreibung von Knstliche Sequenz: Primer
<220>
   <221> primer_bind
   <222> (1)..(32)
<400> 41
   ccatggatcc tcacttcaaa aaaggtaaca gc 32
<210> 42
   <211> 31
   <212> DNA
   <213> Artificial Sequence
<220>
   <223> Die Beschreibung von Knstliche Sequenz: Primer
<220>
   <221> primer_bind
   <222> (1)..(31)
<400> 42
   gatatcacca tggccgctgg actgtatctc c 31
<210> 43
   <211> 30
   <212> DNA
   <213> Artificial Sequence
<220>
   <223> Die Beschreibung von Knstliche Sequenz: Primer
<220>
   <221> primer_bind
   <222> (1)..(30)
<400> 43
   gtcgacctta agaatttaag cttgagtggc 30
<210> 44
   <211> 27
   <212> DNA
   <213> Artificial Sequence
<220>
   <223> Die Beschreibung von Knstliche Sequenz: Primer
<220>
   <221> primer_bind
   <222> (1)..(27)
<400> 44
   gatatcatgg aaatttccgc cccacag 27
<210> 45
   <211> 28
   <212> DNA
   <213> Artificial Sequence
<220>
   <223> Die Beschreibung von Knstliche Sequenz: Primer
<220>
   <221> primer_bind
   <222> (1)..(28)
<400> 45
   gatatccagt gttattccct cagaatgg 28
<210> 46
   <211> 26
   <212> DNA
   <213> Artificial Sequence
<220>
   <223> Die Beschreibung von Knstliche Sequenz: Primer
<220>
   <221> primer_bind
   <222> (1)..(26)
<400> 46
   ggatccatga aagcaactct agcagc 26
<210> 47
   <211> 26
   <212> DNA
   <213> Artificial Sequence
<220>
   <223> Die Beschreibung von Knstliche Sequenz: Primer
<220>
   <221> primer_bind
   <222> (1)..(26)
<400> 47
   gtcgacttag agtggcttct ggcaag 26
<210> 48
   <211> 24
   <212> DNA
   <213> Artificial Sequence
<220>
   <223> Die Beschreibung von Knstliche Sequenz: Primer
<220>
   <221> primer_bind
   <222> (1)..(24)
<400> 48
   gtcgacgagc tcatgggggc gaag 24
<210> 49
   <211> 21
   <212> DNA
   <213> Artificial Sequence
<220>
   <223> Die Beschreibung von Knstliche Sequenz: Primer
<220>
   <221> primer_bind
   <222> (1)..(21)
<400> 49
   agtactggta cctcaaacat g 21
<210> 50
   <211> 23
   <212> DNA
   <213> Artificial Sequence
<220>
   <223> Die Beschreibung von Knstliche Sequenz: Primer
<220>
   <221> primer_bind
   <222> (1)..(23)
<400> 50
   tctagactag aatccaactt ctg 23
<210> 51
   <211> 24
   <212> DNA
   <213> Artificial Sequence
<220>
   <223> Die Beschreibung von Knstliche Sequenz: Primer
<220>
   <221> primer_bind
   <222> (1)..(24)
<400> 51
   tctagagctc gatcgagcgg ccgc 24
<210> 52
   <211> 26
   <212> DNA
   <213> Artificial Sequence
<220>
   <223> Die Beschreibung von Knstliche Sequenz: Primer
<220>
   <221> primer_bind
   <222> (1)..(26)
<400> 52
   gcccgggcca aatttacaat tgccac 26
<210> 53
   <211> 23
   <212> DNA
   <213> Artificial Sequence
<220>
   <223> Die Beschreibung von Knstliche Sequenz: Primer
<220>
   <221> primer_bind
   <222> (1)..(23)
<400> 53
   gcccgggcta attcccgatc tag 23
<210> 54
   <211> 26
   <212> DNA
   <213> Artificial Sequence
<220>
   <223> Die Beschreibung von Knstliche Sequenz: Primer
<220>
   <221> primer_bind
   <222> (1)..(26)
<400> 54
   gcccgggcat ctgtcgtctc aaactc 26
<210> 55
   <211> 28
   <212> DNA
   <213> Artificial Sequence
<220>
   <223> Die Beschreibung von Knstliche Sequenz: Primer
<220>
   <221> primer_bind
   <222> (1)..(28)
<400> 55
   gcccgggctg ttgtcgcaaa attcgccc 28
<210> 56
   <211> 26
   <212> DNA
   <213> Artificial Sequence
<220>
   <223> Die Beschreibung von Knstliche Sequenz: Primer
<220>
   <221> primer_bind
   <222> (1)..(26)
<400> 56
   gcccgggcat ctgtcgtctc aaactc 26
<210> 57
   <211> 23
   <212> DNA
   <213> Artificial Sequence
<220>
   <223> Die Beschreibung von Knstliche Sequenz: Primer
<220>
   <221> primer_bind
   <222> (1)..(23)
<400> 57
   gcccgggcta attcccgatc tag 23
<210> 58
   <211> 25
   <212> DNA
   <213> Artificial Sequence
<220>
   <223> Die Beschreibung von Knstliche Sequenz: Primer
<220>
   <221> primer_bind
   <222> (1)..(25)
<400> 58
   gcccgggcct agaatccaac ttctg 25